(19) Europäisches Patentamt
European Patent Office
Office européen des brevets

(11) Veröffentlichungsnummer: **0 045 081**
**B1**

(12) # EUROPÄISCHE PATENTSCHRIFT

(45) Veröffentlichungstag der Patentschrift: **08.01.86**

(21) Anmeldenummer: **81105914.6**

(22) Anmeldetag: **27.07.81**

(51) Int. Cl.⁴: **C 07 D 401/04**, C 07 D 233/64, C 07 D 263/32, C 07 D 403/04, C 07 D 405/04, C 07 D 409/04, C 07 D 413/04, A 61 K 7/42, A 61 K 31/415, A 61 K 31/42

(54) **Neue trisubstituierte Imidazolderivate, Verfahren zu ihrer Herstellung, diese enthaltende pharmazeutische Präparate und ihre Verwendung.**

(30) Priorität: **25.07.80 CH 5715/80**

(43) Veröffentlichungstag der Anmeldung: **03.02.82 Patentblatt 82/5**

(45) Bekanntmachung des Hinweises auf die Patenterteilung: **08.01.86 Patentblatt 86/2**

(84) Benannte Vertragsstaaten: **AT BE CH DE FR IT LI LU NL SE**

(56) Entgegenhaltungen:
**DE - A - 2 221 546**
**US - A - 3 558 645**

**CHEMICAL ABSTRACTS, Band 89 Nr. 25, 18. Dezember 1978, Seite 593, Zusammenfassung 215367y Columbus, Ohio, US J. NYITRAI et al. "Some propionic acids and derivatives, substituted in position 2 with heterocyclic groups"**

(73) Patentinhaber: **CIBA-GEIGY AG, Klybeckstrasse 141, CH-4002 Basel (CH)**

(72) Erfinder: **Sallmann, Alfred, Dr., Joachimsackerstrasse 12, CH-4103 Bottmingen (CH)**

(74) Vertreter: **Zumstein, Fritz, Dr. et al, Bräuhausstrasse 4, D-8000 München 2 (DE)**

**EP 0 045 081 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Die Erfindung betrifft neue trisubstituierte Imidazolderivate, insbesondere Verbindungen der allgemeinen Formel I

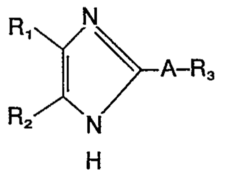

(I) ,

worin einerseits $R_1$ und $R_2$ unabhängig voneinander Phenyl und/oder durch Halogen, Niederalkyl, Hydroxy, Niederalkoxy und/oder Niederalkanoyl substituiertes Phenyl bedeuten oder worin andererseits einer der Reste $R_1$ und $R_2$ Pyrrolyl, Furyl, Thienyl, Pyridyl, 1-Oxidopyridyl oder Pyrimidyl bedeutet, welche jeweils unsubstituiert oder durch Halogen, Niederalkyl, Hydroxy, Niederalkoxy und/oder Niederalkanoyloxy substituiert sein können, und der andere Phenyl, Pyrrolyl, Furyl, Thienyl, Pyridyl, 1-Oxidopyridyl oder Pyrimidyl bedeutet, welche jeweils unsubstituiert oder durch Halogen, Niederalkyl, Hydroxy, Niederalkoxy und/oder Niederalkanoyloxy substituiert sein können, und jeweils A Niederalkylen, Niederalkyliden, Niederalkenylen, Niederalkenyliden, Cycloalkylen, Cycloalkyliden oder Cycloalkyl-niederalkyliden darstellen, und $R_3$ Carboxy, mit einem Niederalkanol, 3- bis 8-gliedrigem Cycloalkanol, Phenol, einem Hydroxypyridin, einem substituierten Phenol bzw. Hydroxypyridin verestertes Carboxy, Carbamoyl oder durch Hydroxy, Amino, Phenyl oder substituiertes Phenyl mono-substituiertes, durch Niederalkyl mono- oder disubstituiertes oder durch 4- bis 7-gliedriges Alkylen bzw. 3-Aza-, 3-Niederalkylaza-, 3-Oxa- oder 3-Thiaalkylen disubstituiertes Carbamoyl bedeutet, wobei ein Niederalkanol bzw. Cycloalkanol unsubstituiert oder durch Hydroxy, Mercapto, gegebenenfalls substituiertes Phenyl, Niederalkoxy, gegebenenfalls im Phenylteil substituiertes Phenylniederalkoxy, Niederalkylthio, gegebenenfalls im Phenylteil substituiertes Phenylniederalkylthio, Hydroxyniederalkoxy, Niederalkoxyniederalkoxy, gegebenenfalls im Phenylteil substituiertes Phenylniederalkoxyniederalkoxy, Carboxyniederalkoxy, Niederalkoxycarbonyl-niederalkoxy, gegebenenfalls substituiertes Phenyl aufweisendes Niederalkoxycarbonyl-niederalkoxy oder Niederalkanoyloxy und substituiertes Phenyl, Phenol bzw. Hydroxypyridin jeweils durch Niederalkyl, Niederalkoxy, Halogen und/oder Trifluormethyl substituiert sein kann, ihre Isomeren sowie ihre Salze, insbesondere pharmazeutisch verwendbare Salze, mit der Massgabe, dass, wenn A für Methylen bzw. Ethyliden und $R_3$ für Ethoxycarbonyl stehen, nicht beide Reste $R_1$ und $R_2$ zugleich Phenyl bedeuten, und der weiteren Massgabe, dass, wenn A für Ethyliden und $R_3$ für Carboxy bzw. Carbamoyl stehen, nicht beide Reste $R_1$ und $R_2$ zugleich Phenyl, p-Methoxyphenyl oder p-Chlorphenyl bedeuten, wobei unter mit «nieder» bezeichneten Resten solche zu verstehen sind, die bis und mit 7 C-Atome enthalten, Verfahren zu ihrer Herstellung, pharmazeutische Präparate, die solche Verbindungen enthalten, und ihre Verwendung, z.B. als Arzneimittelwirkstoffe.

Pyrrolyl ist z.B. 2-Pyrrolyl, Furyl z.B. 2-Furyl, Thienyl z.B. 2- oder 3-Thienyl, Pyridyl z.B. 2-, 3- oder 4-Pyridyl, 1-Oxidopyridyl z.B. 1-Oxido-3-pyridyl oder 1-Oxido-4-pyridyl, und Pyrimidyl z.B. 2-Pyrimidyl.

Ein Niederalkanol ist beispielsweise Methanol, Ethanol, Propanol, Isopropanol, n-Butanol, sec- oder tert-Butanol. Ein 3- bis 8-gliedriges Cycloalkanol ist z.B. Cyclopentanol, -hexanol oder -heptanol. Als Substituenten solcher Niederalkanole bzw. Cycloalkanole kommen beispielsweise Hydroxy, Mercapto, gegebenenfalls substituiertes Phenyl, Niederalkoxy, gegebenenfalls im Phenylteil substituiertes Phenylniederalkoxy, Niederalkylthio, gegebenenfalls im Phenylteil substituiertes Phenylniederalkylthio, Hydroxyniederalkoxy, Niederalkoxyniederalkoxy, gegebenenfalls im Phenylteil substituiertes Phenylniederalkoxyniederalkoxy, Carboxyniederalkoxy, Niederalkoxycarbonyl-niederalkoxy, gegebenenfalls substituiertes Phenyl aufweisendes Niederalkoxycarbonylniederalkoxy oder Niederalkanoyloxy in Betracht. Ein Phenol oder Hydroxypyridin, z.B. 2-, 3- oder 4-Hydroxypyridin kann gegebenenfalls durch Niederalkyl, Niederalkoxy, Halogen und/oder Trifluormethyl substituiert sein.

N-Mono- oder N,N-Diniederalkylcarbamoyl ist z.B. N-Methyl-, N-Ethyl-, N,N-Dimethyl-, N,N-Diethyl- oder N,N-Dipropylcarbamoyl. Durch 4- bis 7-gliedriges Alkylen bzw. 3-Aza-, 3-Niederalkylaza-, 3-Oxa- oder 3-Thiaalkylen disubstituiertes Carbamoyl ist z.B. Pyrrolidino- oder Piperidinocarbonyl, Morpholino-, Piperazino- bzw. 4-Methylpiperazino- sowie Thiomorpholinocarbonyl.

In der vorliegenden Beschreibung sind unter «niederen» organischen Resten und Verbindungen solche zu verstehen, die bis und mit 7, vor allem bis und mit 4 Kohlenstoffatome (C-Atome) enthalten.

Die vor- und nachstehend verwendeten Allgemeindefinitionen haben im Rahmen der vorliegenden Anmeldung in erster Linie die folgenden Bedeutungen:

Halogen ist z.B. Halogen bis und mit Atomnummer 35, wie Fluor, Chlor oder Brom, ferner Iod.

Niederalkyl ist z.B. Methyl, Ethyl, n-Propyl, Isopropyl, n-Butyl, Isobutyl, sec.-Butyl, tert.-Butyl, ferner ein Pentyl-, Hexyl oder Heptylrest.

Niederalkoxy ist z.B. Methoxy, Ethoxy, n-Propyloxy, Isopropyloxy, n-Butyloxy, Isobutyloxy, sec.-Butyloxy oder tert.-Butyloxy.

Niederalkylthio ist z.B. Methyl-, Ethyl-, n-Propyl-, Isopropyl-, n-Butyl-, Isobutyl-, sec.-Butyl- oder tert.-Butylthio.

Phenylniederalkoxy ist z.B. Phenylmethoxy, Phenylethoxy oder Phenylpropyloxy.

Phenylniederalkylthio ist z.B. Benzyl-, Phenylethyl- oder Phenylpropylthio.

Hydroxyniederalkoxy ist z.B. Hydroxyethoxy, Hydroxypropyloxy oder 1,2-Dihydroxypropyloxy.

Niederalkoxyniederalkoxy ist z.B. Methoxyethoxy, Ethoxyethoxy, Methoxypropyloxy oder Methoxybutyloxy.

Phenylniederalkoxyniederalkoxy ist z.B. 2-Benzyloxyethoxy oder 2-(2-Phenylethoxy)-ethoxy.

Niederalkanoyloxy ist z.B. Acetyl-, Propionyl-, Butyryl-, Iso-, sec- oder tert.-Butyryloxy.

Niederalkylen ist z.B. geradkettig, wie Methylen, Ethylen, 1,3-Propylen oder 1,4-Butylen, oder verzweigt, wie 1,2-Propylen, 1,2- oder 1,3-(2-Methyl)-propylen oder 1,2-Butylen.

Niederalkyliden weist ein tertiäres oder insbesondere ein quartäres C-Atom auf und ist z.B. Ethyliden oder 1,1- oder 2,2-Propyliden, ferner 1,1-oder 2,2-Butyliden oder 1,1-, 2,2- oder 3,3-Pentyliden.

Niederalkenylen ist z.B. Ethenylen, 1,2- oder 1,3-Propenylen oder 1,2-, 1,3- oder 1,4-Buten-2-ylen.

Niederalkenyliden ist z.B. Ethenyliden, 1,1-Propen-1-yliden, 1,1-Propen-2-yliden, ferner ein Butenyliden, wie 1,1-Buten-3-yliden.

Cycloalkylen ist z.B. Cyclopropylen, 1,2- oder 1,3-Cyclobutylen, 1,2-, 1,3- oder 1,4-Cyclopentylen, ferner ein Cyclohexylen.

Cycloalkyliden ist z.B. Cyclopropyliden, Cyclobutyliden, Cyclopentyliden oder Cyclohexyliden.

Cycloalkyl-niederalkyliden ist z.B. ein Cyclopropyl-, Cyclobutyl-, Cyclopentyl- oder Cyclohexyl-methylen, -ethyliden oder -propyliden, ferner ein Cyclohexyl-butyliden.

Carboxyniederalkoxy ist z.B. Carboxymethoxy, 2-Carboxyethoxy, 2-, 3-Carboxypropyloxy, 1-Carboxy-2-propyloxy, 2-, 3- oder 4-Carboxy-n-butyloxy, 1-Carboxy-2-methyl-propyl-3-oxy oder 1-Carboxy-2-methyl-propyl-2-oxy.

Niederalkoxycarbonyl-niederalkoxy enthält jeweils im Niederalkoxyteil unabhängig voneinander die vorstehend unter Niederalkoxy aufgeführten Bedeutungen.

Salze von erfindungsgemässen Verbindungen der Formel I sind vorzugsweise pharmazeutisch verwendbare Salze, wie pharmazeutisch verwendbare Säureadditionssalze, und/oder, wenn $R_3$ Carboxy bedeutet und/oder $R_1$ und $R_2$ unabhängig voneinander durch Hydroxy substituiertes Phenyl bzw. Heteroaryl darstellen, innere Salze oder Salze mit Basen. Geeignete Säureadditionssalze sind beispielsweise Salze mit anorganischen Säuren, wie Mineralsäure, mit Sulfaminsäuren, wie Cyclohexylsulfaminsäure, mit organischen Carbonsäuren, wie Niederalkancarbonsäuren, gegebenenfalls ungesättigte Dicarbonsäuren, mit durch Hydroxy und/oder Oxo substituierten Carbonsäuren oder mit Sulfonsäuren, beispielsweise Sulfate oder Hydrohalogenide, wie Hydrobromide oder Hydrochloride, Oxalate, Malonate, Fumarate oder Maleinate, Tartrate, Pyruvate oder Citronate, Sulfonate, wie Methan-, Benzol- oder p-Toluolsulfonate.

Geeignete Salze mit Basen sind beispielsweise Alkali- oder Erdalkalimetallsalze, z.B. Natrium-, Kalium- oder Magnesiumsalze, pharmazeutisch verwendbare Übergangsmetallsalze, wie Zink- oder Kupfersalze, oder Salze mit Ammoniak oder von substituierten organischen Aminen, wie cyclische Amine, z.B. Morpholin, Thiomorpholin, Piperidin, Pyrrolidin, wie Mono-, Di- bzw. Triniederalkylamine oder Mono-, Di- bzw. Trihydroxyniederalkylamine, z.B. Mono-, Di- bzw. Triethanolamin. Mononiederalkylamine sind beispielsweise Ethyl- oder tert.-Butylamin. Diniederalkylamine sind z.B. Diethyl- oder Diisopropylamin, und als Triniederalkylamin kommt z.B. Triethylamin in Betracht.

Die neuen Verbindungen der Formel I und ihre pharmazeutisch verwendbaren Salze weisen wertvolle pharmakologische Eigenschaften auf. Insbesondere besitzen sie, z.B. bei lokaler Anwendung, eine ausgeprägte antiinflammatorische Wirkung.

Diese Eigenschaft lässt sich beispielsweise nach der von G. Tonelli, L. Thibault, Endocrinology 77, 625 (1965) entwickelten Methode durch Hemmung des mit Crotonöl induzierten Rattenohrödems bei der Normalratte im Dosisbereich von etwa 1 bis etwa 100 mg/ml nachweisen.

Die ausgezeichnete entzündungshemmende Wirkung lässt sich ebenfalls im Ultraviolettstrahlen-Dermatitis-Hemmtest am Meerschweinchen [Methodik: Weirich, E.G.; Longauer, J.; Kirkwood, A.H.; Dermatologica 152, 87–99 (1976)] und mit Hilfe des Crotonöl-Dermatitis-Hemmtests am Kaninchen [Methodik: Weirich, E.G.; Longauer, J.; Kirkwood, A.H.; Arch. Derm. Res. 259, 141–9 (1977)] jeweils im Dosisbereich von etwa 0,01 bis etwa 1,0% G/G bei topischer Applikation einer entsprechenden Lösung erfassen. Weiterhin zeigen die erfindungsgemässen Verbindungen im Hyperplasie-Hemmtest am Meerschweinchen im Dosisbereich von etwa 0,01 bis 1,0% G/G bei topischer Anwendung eine ausgeprägte Hemmwirkung [Methodik: Weirich, E.G.; Longauer, J.; Kirkwood, A.H.; Dermatologica, 151, 321–332 (1975)]. Ausserdem wurden die Substanzen einer Aktivitätsprüfung am Menschen unterzogen. Im Hautvasokonstriktions-Lösungs-Test konnte im Dosisbereich von etwa $1 \cdot 10^{-5}$ bis $1 \cdot 10^{-1}$ G/G ein beträchtlicher Vasokonstriktionseffekt festgestellt werden [Methodik: Weirich, E.G.; Lutz, U.; Dermatologica 155, 328–334 (1977)].

Weiterhin zeichnen sich die erfindungsgemässen Verbindungen durch deutliche Effekte gegen einige Virusstämme aus. So wird beispielsweise bei Experimenten am Meerschweinchen, die mit HVH 2/Angelotti infiziert sind [Methodik: B. Lukas et al., Arch. Ges. Virusforsch. 44, 153–5 (1974) und 49, 1–11 (1975)], nach 2mal täglicher intravaginaler Applikation über 5 Tage von 0,1 ml eines Gels mit 0,2%iger Konzentration eine rasche Regression bzw. völlige Reduktion der durch Herpes genitalis verursachten Symptome festgestellt.

Die erfindungsgemässen Verbindungen der Formel I sind daher als Arzneimittel, insbesondere externe (topische) Hautphlogistatika für die Behandlung entzündlicher Dermatosen jeglicher Genese, wie bei leichten Hautirritationen, Kontaktdermatitis, Exanthemen, Verbrennungen, sowie als Schleimhautphlogostatika für die Behandlung von Mukosaentzündungen, z.B. der Augen, Nase, Lippen, Mund, Genital-, Analregion, geeignet. Ferner können die Verbindungen als Sonnenschutzmittel sowie als antivirale Mittel, z. B. Anti-Herpes-Mittel, verwendet werden.

Die vorliegende Erfindung betrifft ebenfalls die Verwendung der erfindungsgemässen Verbindungen und deren Salze sowie pharmazeutische Präparate, die diese enthalten, und deren Verwendung zur Behandlung von Entzündungen, z. B. von entzündlichen Erkrankungen unterschiedlichster Genese, sowie zur Herstellung von Arzneimitteln.

Die durch Disclaimer ausgeschlossenen Verbindungen sind in der Publikation von J. Nyitrai et al., Acta Chim. Acad. Sci. Hung. 97, 91–103 (1978) offenbart.

Die Erfindung betrifft beispielsweise Verbindungen der Formel I, worin $R_1$ und $R_2$ unabhängig voneinander Phenyl und/oder durch Halogen, Hydroxy, Niederalkyl, Niederalkoxy und/oder Niederalkanoyloxy substituiertes Phenyl bedeuten, A Niederalkylen mit bis und mit 4 C-Atomen, wie Methylen, Niederalkyliden mit bis und mit 7 C-Atomen, wie 2,2-Propyliden, Niederalkenylen mit bis und mit 4 C-Atomen, wie 1,3-Propen-2-ylen, Niederalkenyliden mit bis und mit 7 C-Atomen, wie 1,1-Buten-3-yliden, 3- bis 8-gliedriges Cycloalkylen, wie Cyclopropylen, 3- bis 8-gliedriges Cycloalkyliden, wie Cyclopentyliden, oder Cycloalkylniederalkyliden mit bis und mit 7 C-Atomen im Alkylidenteil und mit einem 3- bis 8-gliedrigen Cycloalkylteil, wie 2-Cyclohexyl-1,1-ethyliden, bedeutet und $R_3$ Carboxy, mit einem Niederalkanol, einem 3- bis 8-gliedrigen Cycloalkanol, Phenol oder einem substituierten Phenol verestertes Carboxy, Carbamoyl, N-Mono-, N,N-Diniederalkylcarbamoyl, Pyrrolidino-, Piperidino-, Morpholino-, Piperazino-, 4-Niederalkyl-piperazino-, Thiomorpholino-, Anilino- oder durch Niederalkyl, Niederalkoxy, und/oder Halogen substituiertes Anilinocarbonyl darstellt, wobei das Niederalkanol bzw. Cycloalkanol unsubstituiert oder durch Hydroxy, Mercapto, Phenyl, substituiertes Phenyl, Niederalkoxy, Phenylniederalkoxy, im Phenylteil substituiertes Phenylniederalkoxy, Niederalkylthio, Phenylniederalkylthio, im Phenylteil substituiertes Phenylniederalkylthio, Hydroxyniederalkoxy, Niederalkoxyniederalkoxy, Phenylniederalkoxyniederalkoxy, im Phenylteil substituiertes Phenylniederalkoxyniederalkoxy, Carboxyniederalkoxy, Niederalkoxycarbonyl-niederalkoxy, gegebenenfalls substituiertes Phenyl aufweisendes Niederalkoxycarbonyl-niederalkoxy oder Niederalkanoyloxy und substituiertes Phenol bzw. Phenyl jeweils durch Niederalkyl, Niederalkoxy, Halogen und/oder Trifluormethyl substituiert sein kann, ihre Isomeren sowie ihre Salze, insbesondere pharmazeutisch verwendbare Salze, mit der Massgabe, dass, wenn A für Methylen oder Ethyliden und $R_3$ für Ethoxycarbonyl stehen, mindestens einer der Reste $R_1$ und $R_2$ von Phenyl verschieden ist, und der weiteren Massgabe, dass, wenn A für Ethyliden und $R_3$ für Carboxy stehen, mindestens einer der Reste $R_1$ und $R_2$ von Phenyl, p-Methoxyphenyl und p-Chlorphenyl verschieden ist.

Die Erfindung betrifft beispielsweise Verbindungen der Formel I, worin einer der Reste $R_1$ und $R_2$ Pyridyl oder 1-Oxido-pyridyl, die unsubstituiert und/oder jeweils durch Halogen, Hydroxy, Niederalkyl, Niederalkoxy und/oder Niederalkanoyloxy substituiert sein können, und der andere Phenyl, Pyridyl oder 1-Oxidopyridyl, die unsubstituiert und/oder jeweils durch Halogen, Hydroxy, Niederalkyl, Niederalkoxy und/oder Niederalkanoyloxy substituiert sein können, bedeutet, A Niederalkylen mit bis und mit 4 C-Atomen, wie Methylen, Niederalkyliden mit bis und mit 7 C-Atomen, wie 2,2-Propyliden, Niederalkenylen mit bis und mit 4 C-Atomen, wie 1,3-Propen-2-ylen, Niederalkenyliden mit bis und mit 7 C-Atomen, wie 1,1-Buten-3-yliden, 3- bis 8-gliedriges Cycloalkylen, wie Cyclopropylen, 3- bis 8-gliedriges Cycloalkyliden, wie Cyclopentyliden, oder Cycloalkyl-niederalkyliden mit bis und mit 7 C-Atomen im Alkylidenteil und mit einem 3- bis 8-gliedrigen Cycloalkylteil, wie 2-Cyclohexyl-1,1-ethyliden, bedeutet und $R_3$ Carboxy, mit einem Niederalkanol, einem 3- bis 8-gliedrigen Cycloalkanol, Phenol oder einem substituierten Phenol verestertes Carboxy, Carbamoyl, N-Mono-, N,N-Diniederalkylcarbamoyl, Pyrrolidino-, Piperidino-, Morpholino-, Piperazino-, 4-Niederalkyl-piperazino-, Thiomorpholino-, Anilino- oder durch Niederalkyl, Niederalkoxy und/oder Halogen substituiertes Anilinocarbonyl darstellt, wobei das Niederalkanol bzw. Cycloalkanol unsubstituiert oder durch Hydroxy, Mercapto, Phenyl, substituiertes Phenyl, Niederalkoxy, Phenylniederalkoxy, im Phenylteil substituiertes Phenylniederalkoxy, Niederalkylthio, Phenylniederalkylthio, im Phenylteil substituiertes Phenylniederalkylthio, Hydroxyniederalkoxy, Niederalkoxyniederalkoxy, Phenylniederalkoxyniederalkoxy, im Phenylteil substituiertes Phenylniederalkoxyniederalkoxy, Carboxyniederalkoxy, Niederalkoxycarbonyl-niederalkoxy, gegebenenfalls substituiertes Phenyl aufweisendes Niederalkoxycarbonyl-niederalkoxy oder Niederalkanoyloxy und substituiertes Phenol bzw. Phenyl jeweils durch Niederalkyl, Niederalkoxy, Halogen und/oder Trifluormethyl substituiert sein kann, ihre Isomeren sowie ihre Salze, insbesondere pharmazeutisch verwendbare Salze.

Die Erfindung betrifft beispielsweise Verbindungen der Formel I, worin $R_1$ und $R_2$ unabhängig voneinander gegebenenfalls durch Halogen mit Atomnummer bis und mit 35, Hydroxy, Niederalkyl mit bis und mit 4 C-Atomen, Niederalkoxy mit bis und mit 4 C-Atomen und/oder Niederalkanoyloxy mit bis und mit 5 C-Atomen substituiertes Phenyl, Pyridyl, und/oder 1-Oxidopyridyl bedeuten, A Niederalkylen mit bis und mit 4 C-Atomen oder Niederalkyliden mit bis und mit 4 C-Atomen bedeutet, $R_3$ gegebenenfalls mit einem Niederalkanol mit bis und mit 4 C-Atomen verestertes Carboxy darstellt, wobei das Niederalkanol gegebenenfalls durch Hydroxy, Mercapto, gegebenenfalls substituiertes Phenyl, Niederalkoxy mit bis und mit 4 C-Atomen, gegebenenfalls im Phenylteil substituiertes Phenylniederalkoxy mit bis und mit 4 C-Atomen im Alkylteil, Niederalkylthio mit bis und mit 4 C-Atomen, Hydroxyniederalkoxy mit bis und mit 4 C-Atomen im Niederalkylteil, Niederalkoxyniederalkoxy jeweils mit bis und mit 4 C-Atomen im Niederalkylteil oder Niederalkanoyloxy mit bis und mit 5 C-Atomen und Phenyl gegebenenfalls durch Niederalkyl mit bis und mit 4 C-Atomen, Niederalkoxy mit bis und mit 4 C-Atomen, Halogen mit Atomnummer bis und mit 35 und/oder Trifluormethyl substituiert sein kann, ihre Isomeren sowie ihre Salze, mit der Massgabe, dass, wenn A für Methylen bzw. Ethyliden und $R_3$ für Ethoxycarbonyl stehen, nicht beide Reste $R_1$ und $R_2$ zugleich Phenyl bedeuten, und der weiteren Massgabe, dass wenn A für Ethyliden und $R_3$ für Carboxy stehen, nicht beide Reste $R_1$ und $R_2$ zugleich Phenyl, p-Methoxyphenyl oder p-Chlorphenyl bedeuten.

Die Erfindung betrifft beispielsweise Verbindungen der Formel I, worin $R_1$ und $R_2$ unabhängig voneinander Phenyl und/oder durch Halogen mit Atomnummer bis und mit 35, wie Chlor, Hydroxy, Niederalkyl mit bis und mit 4 C-Atomen, wie Methyl, und/oder Niederalkoxy mit bis und mit 4 C-Atomen, wie Methoxy, substituiertes Phenyl bedeuten, A Niederalkylen mit bis und mit 4 C-Atomen, wie Methylen, Niederalkyliden mit bis und mit 7 C-Atomen, wie 2,2-Propyliden, Niederalkenyliden mit bis und mit 7 C-Atomen, wie 1,1-Buten-3-yliden, oder 3- bis 8-gliedriges Cycloniederalkyliden, wie 1,1-Cyclopentyliden, darstellen und $R_3$ Carboxy, Niederalkoxycarbonyl mit bis und mit 5 C-Atomen, wie Ethoxycarbonyl, Carbamoyl, N-Mononiederalkyl-carbamoyl mit bis und mit 4 C-Atomen im Niederalkyl, wie N-Methyl-carbamoyl, oder N,N-Diniederalkylcarbamoyl jeweils mit bis und mit 4 C-Atomen im Niederalkyl, wie N,N-Dimethylcarbamoyl, bedeuten, wobei das Niederalkoxycarbonyl durch Niederalkanoyloxy mit bis und mit 5 C-Atomen, wie Pivaloyloxy, substituiert sein kann, ihre Isomeren sowie ihre Salze, insbesondere pharmazeutisch verwendbare Salze, mit der Massgabe, dass, wenn A für Methylen oder Ethyliden und $R_3$ für Ethoxycarbonyl stehen, mindestens einer der Reste $R_1$ und $R_2$ von Phenyl verschieden ist, und der weiteren Massgabe, dass, wenn A für Ethyliden und $R_3$ für Carboxy stehen, mindestens einer der Reste $R_1$ und $R_2$ von Phenyl, p-Methoxyphenyl und p-Chlorphenyl verschieden ist.

Die Erfindung betrifft beispielsweise Verbindungen der Formel I, worin einer der Reste $R_1$ und $R_2$ Phenyl oder durch Halogen mit Atomnummer bis und mit 35, wie Chlor, Hydroxy, Niederalkyl mit bis und mit 4 C-Atomen, wie Methyl, und/oder Niederalkoxy mit bis und mit 4 C-Atomen, wie Methoxy, substituiertes Phenyl bedeutet und der andere Pyridyl, wie 3-Pyridyl, oder 1-Oxido-pyridyl, wie 1-Oxido-3-pyridyl, bedeutet, die jeweils unsubstituiert oder durch Halogen mit Atomnummer bis und mit 35, wie Chlor, Hydroxy und/oder Niederalkoxy mit bis und mit 4 C-Atomen, wie Methoxy, substituiert sein können, A Niederalkylen mit bis und mit 4 C-Atomen, wie Methylen, Niederalkyliden mit bis und mit 7 C-Atomen, wie 2,2-Propyliden, Niederalkenyliden mit bis und mit 7 C-Atomen, wie 1,1-Buten-3-yliden, oder 3- bis 8-gliedriges Cycloniederalkyliden, wie 1,1-Cyclopentyliden, darstellen und $R_3$ Carboxy, Niederalkoxycarbonyl mit bis und mit 5 C-Atomen, wie Ethoxycarbonyl, Carbamoyl, N-Mononiederalkyl-carbamoyl mit bis und mit 4 C-Atomen im Niederalkyl, wie N-Methyl-carbamoyl, oder N,N-Diniederalkyl-carbamoyl jeweils mit bis und mit 4 C-Atomen im Niederalkyl, wie N,N-Dimethylcarbamoyl, bedeuten, wobei das Niederalkoxycarbonyl durch Niederalkanoyloxy mit bis und mit 5 C-Atomen, wie Pivaloyloxy, substituiert sein kann, ihre Isomereren sowie ihre Salze, insbesondere pharmazeutisch verwendbare Salze.

Die Erfindung betrifft insbesondere Verbindungen der Formel I, worin $R_1$ und $R_2$ unabhängig voneinander Phenyl und/oder durch Niederalkoxy mit bis und mit 4 C-Atomen, wie Methoxy, substituiertes Phenyl bedeuten, A Niederalkylen mit bis und mit 4 C-Atomen, wie Methylen, oder insbesondere Niederalkyliden mit bis und mit 4 C-Atomen, wie 2,2-Propyliden, darstellt und $R_3$ Carboxy oder Niederalkoxycarbonyl mit bis und mit 5 C-Atomen, wie Ethoxycarbonyl, bedeutet, ihre Isomeren so wie ihre Salze, insbesondere pharmazeutisch verwendbare Salze, mit der Massgabe, dass, wenn A für Methylen oder Ethyliden und $R_3$ für Ethoxycarbonyl steht, mindestens einer der Reste $R_1$ und $R_2$ von Phenyl verschieden ist, und der weiteren Massgabe, dass, wenn A für Ethyliden und $R_3$ für Carboxy stehen, mindestens einer der Reste $R_1$ und $R_2$ von Phenyl und p-Methoxyphenyl verschieden ist.

Die Erfindung betrifft in erster Linie Verbindungen der Formel I, worin einer der Reste $R_1$ und $R_2$ Phenyl oder durch Halogen mit Atomnummer bis und mit 35, wie Chlor, Hydroxy oder Niederalkoxy mit bis und mit 4 C-Atomen, wie Methoxy, substituiertes Phenyl bedeutet und der andere Pyridyl, wie 3- oder 4-Pyridyl, oder 1-Oxidopyridyl, wie 1-Oxido-3-pyridyl oder 1-Oxido-4-pyridyl, darstellt, A Niederalkyliden mit bis und mit 4 C-Atomen, wie 2,2-Propyliden, bedeutet, und $R_3$ Niederalkoxycarbonyl mit bis und mit 5 C-Atomen, wie Ethoxycarbonyl, darstellt, ihre Isomeren sowie ihre Salze, insbesondere pharmazeutisch verwendbare Salze.

Die Erfindung betrifft in erster Linie Verbindungen der Formel I, worin einer der Reste $R_1$ und $R_2$ Phenyl oder durch Halogen mit Atomnummer bis und mit 35, wie Chlor, Hydroxy oder Niederalkoxy mit bis und mit 4 C-Atomen, wie Methoxy substituiertes Phenyl bedeutet und der andere Pyridyl, wie 3- oder 4-Pyridyl, oder 1-Oxidopyridyl, wie 1-Oxido-3-pyridyl oder 1-Oxido-4-pyridyl, darstellt, A ein ein quartäres C-Atom aufweisendes Niederalkyliden mit bis und mit 4 C-Atomen, wie 2,2-Propyliden, wobei das quartäre C-Atom direkt an den Imidazolring gebunden ist, bedeutet, und $R_3$ Niederalkoxycarbonyl mit bis und mit 5 C-Atomen, wie Ethoxycarbonyl, darstellt, ihre Isomeren sowie ihre Salze, insbesondere pharmazeutisch verwendbare Salze.

Die Erfindung betrifft in allererster Linie Verbindungen der Formel I, worin einer der Reste $R_1$ und $R_2$ Phenyl und der andere 1-Oxidopyridyl, wie 1-Oxido-3-pyridyl, bedeutet, A 2,2-Propyliden darstellt und $R_3$ Niederalkoxycarbonyl mit bis und mit

5 C-Atomen, wie Ethoxycarbonyl, bedeutet, ihre Isomeren sowie ihre Salze, insbesondere pharmazeutisch verwendbare Salze.

Die Erfindung betrifft namentlich die in den Beispielen genannten Verbindungen und ihre Salze, insbesondere pharmazeutisch verwendbare Salze von solchen Verbindungen mit salzbildenden Gruppen, sowie die in den Beispielen aufgeführten Herstellungsverfahren.

Die Erfindung betrifft namentlich die in den Beispielen genannten Verbindungen der Formel I und ihre Salze, insbesondere pharmazeutisch verwendbare Salze von solchen Verbindungen mit salzbildenden Gruppen.

Die neuen Verbindungen der Formel I oder deren Salze lassen sich in an sich bekannter Weise herstellen.

Eine Verfahrensweise besteht beispielsweise darin, dass man aus einer Verbindung der Formel

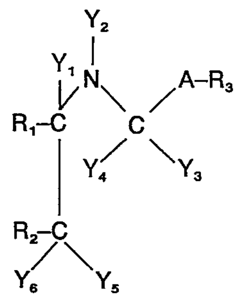

(II),

worin einer der Reste $Y_1$ und $Y_6$ Hydroxy oder Amino und der andere sowie $Y_2$ Wasserstoff darstellt und $Y_3$ gemeinsam mit $Y_4$ und $Y_5$ eine Gruppe der Formel =N– bedeutet oder worin $Y_1$ gemeinsam mit $Y_6$ eine Bindung darstellt, $Y_2$ Wasserstoff ist, $Y_3$ Hydroxy oder Amino bedeutet und $Y_4$ gemeinsam mit $Y_5$ eine Gruppe der Formel –NH– darstellt oder worin $Y_1$ gemeinsam mit $Y_6$ eine Bindung darstellt, $Y_2$ gemeinsam mit $Y_3$ eine zusätzliche Bindung und einer der Reste $Y_4$ und $Y_5$ Amino und der andere Amino, Hydroxy oder reaktionsfähiges verestertes Hydroxy, insbesondere Halogen oder Sulfonyloxy, darstellt oder worin $Y_1$ Hydroxy ist, $Y_2$ sowie $Y_3$ Wasserstoff bedeutet, $Y_4$ Hydroxy oder Amino darstellt und $Y_5$ gemeinsam mit $Y_6$ eine Gruppe der Formel =NH oder, sofern $Y_4$ Amino ist, Oxo bedeutet, oder einem Tautomeren und/oder Salz davon, unter Einführung einer gegebenenfalls zusätzlichen Bindung H–Z abspaltet, und gewünschtenfalls die verfahrensgemäss erhältliche freie Verbindung in ein Salz oder ein verfahrensgemäss erhältliches Salz in die freie Verbindung oder in ein anderes Salz überführt und/oder, wenn erwünscht, ein erfindungsgemäss erhältliches Gemisch von isomeren Verbindungen der Formel I in die einzelnen Isomeren auftrennt.

Dabei bedeutet Z Hydroxy oder Amino $Y_1$ bzw. $Y_6$, $Y_3$ oder $Y_4$ bzw. $Y_5$ oder Halogen bzw. Sulfonyloxy $Y_4$ bzw. $Y_5$.

Reaktionsfähiges verestertes Hydroxy ist beispielsweise mit einer anorganischen Mineralsäure, wie Halogenwasserstoffsäure, oder mit einer organischen Sulfonsäure, wie Niederalkan- oder gegebenenfalls substituierte Benzolsulfonsäure, verestertes Hydroxy und bedeutet in erster Linie Halogen, z.B. Chlor oder Brom, sowie Sulfonyloxy, z.B. Methan- oder p-Toluolsulfonyloxy.

Tautomere von Verbindungen der Formel II sind beispielsweise solche, in denen eine partielle Enol- bzw. Enamin-Gruppierung der Formel

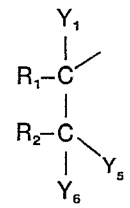

(IIa),

worin $Y_1$ gemeinsam mit $Y_6$ eine zusätzliche Bindung darstellt und $Y_5$ Hydroxy bzw. Amino bedeutet, in Form der entsprechenden tautomeren Keto- bzw. Ketiminform, worin $Y_1$ Wasserstoff ist und $Y_5$ gemeinsam mit $Y_6$ Oxo bzw. Imino bedeutet, vorliegt und/oder solche, in denen eine partielle Enol- bzw. Enamingruppierung der Formel

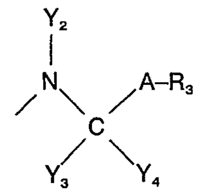

(IIb),

worin $Y_2$ gemeinsam mit $Y_3$ eine Bindung darstellt und $Y_4$ Hydroxy bzw. Amino bedeutet, in Form der entsprechenden tautomeren Form vorliegt, worin $Y_2$ Wasserstoff ist und $Y_3$ gemeinsam mit $Y_4$ Oxo bzw. Imino darstellt, vorliegt, wobei die genannten Tautomeren miteinander im Gleichgewicht stehen.

Die Abspaltung von H–Z aus einer Verbindung der Formel II, einem Tautomeren und/oder Salz davon erfolgt in üblicher, insbesondere in der aus der Literatur für analoge Reaktionen bekannten Weise, erforderlichenfalls unter Erwärmen, wie in einem Temperaturbereich von etwa 20° bis etwa 250 °C, unter Druck und/oder in Gegenwart eines katalytischen Mittels, vorzugsweise einer Säure. Als Säuren eignen sich beispielsweise anorganische Säuren, wie Mineralsäuren, z.B. Schwefelsäure, Polyphosphorsäure oder Halogenwasserstoffsäure, wie Chlorwasserstoffsäure, oder organische Säuren, wie Niederalkancarbonsäuren, z.B. Essigsäure. Dabei arbeitet man erforderlichenfalls in einem inerten Lösungsmittel, beispielsweise einem gegebenenfalls halogenierten Kohlenwasserstoff, wie Chloroform, Chlorbenzol, Hexan oder Toluol, einem Niederalkanol, wie Methanol oder Ethanol, einem Carbonsäureamid, wie Niederalkancarbonsäureamid, z.B. Dimethylformamid oder Formamid, oder einer Niederalkancarbonsäure, wie Ameisen- oder Essigsäure, und/oder unter Inertgas, wie Stickstoff.

Die Ausgangsstoffe der Formel II, ihre Tautomeren und/oder Salze werden nach an sich bekannten Verfahren zum überwiegenden Teil in situ gebildet und unter den Reaktionsbedingungen ohne Isolierung weiter zu der Verbindung der Formel I

umgesetzt. Dabei kann die Abspaltung von H–Z unter direkter Cyclisierung oder im Anschluss an eine vorgelagerte Cyclisierung erfolgen.

So kann man in einer bevorzugten Ausführungsform des vorstehenden Verfahrens beispielsweise das Diketon der Formel

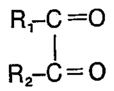

(IIIa)

mit einem Aldehyd der Formel $R_3$–A–C(=O)–H (IIIb) oder einem Salz davon, und einem Überschuss an Ammoniak unter Erwärmen umsetzen. Dabei wird beispielsweise intermediär eine Verbindung der Formel II, z. B. eine solche, worin $Y_1$ Hydroxy, $Y_2$ sowie $Y_6$ Wasserstoff bedeutet und $Y_3$ gemeinsam mit $Y_4$ und $Y_5$ eine Gruppe der Formel =N– darstellt, z. B.

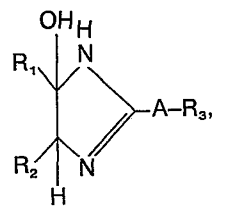

oder eine tautomere Form davon gebildet, die unter den Reaktionsbedingungen erfindungsgemäss weiterreagiert.

Ferner kann man in weiteren bevorzugten Ausführungsformen des vorstehend beschriebenen Verfahrens ein acyliertes α-Aminoketon der Formel

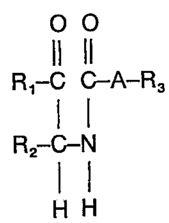

(IIIc)

oder ein Salz davon mit Ammoniak umsetzen. Dabei arbeitet man beispielsweise unter Erwärmen, z. B. in einem Temperaturbereich von etwa 50° bis etwa 250 °C, und unter inerten Bedingungen.

Die Ausgangsstoffe der Formel (IIIc) sind bekannt oder werden nach an sich bekannten Verfahren hergestellt. Beispielsweise geht man von einer Verbindung der Formel

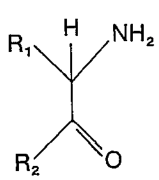

(IIIh)

oder einem Salz davon aus und setzt diese mit einem Säurederivat der Formel $R_3$–A–COOH (IIIi), beispielsweise einem entsprechenden Anhydrid, wie einer Halogencarbonylverbindung, um.

In einer weiteren, besonders bevorzugten Variante des eingangs beschriebenen Verfahrens setzt man eine Verbindung der Formel

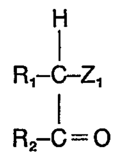

(IIId)

worin $Z_1$ gegebenenfalls reaktionsfähiges verestertes Hydroxy bedeutet, oder ein Salz davon mit einer Verbindung der Formel

$R_3$–A–$Z_2$ (IIIe),

worin $Z_2$ ein Amidinorest oder Ammoniumcarboxylat bedeutet, oder ein Salz davon gegebenenfalls mit Ammoniak um.

Die Umsetzung mit einem Amidin der Formel (IIIe) erfolgt üblicherweise unter Erwärmen, beispielsweise in einem Temperaturbereich von etwa 50° bis etwa 250 °C.

Die Reaktion des Ammoniumcarboxylats der Formel (IIIe) mit einer Verbindung der Formel (IIId) wird mit einem mindestens 3-molaren, oder, falls die Verbindung der Formel (IIId) in Salzform vorliegt, mindestens 4-molaren Überschuss des Ammoniumsalzes der Verbindung der Formel (IIIe), gegebenenfalls unter Erwärmen, z. B. in einem Temperaturbereich von etwa 50° bis etwa 250 °C, vorzugsweise bei 90° bis 120 °C, durchgeführt, wobei die Verbindung der Formel (IIIe) gleichzeitig als Lösungsmittel dienen kann. Diese Variante kann beispielsweise auch dahingehend abgewandelt werden, dass man das Ammoniumsalz der Formel III, in bezug auf den reaktionsfähigen Ester $Z_1$, in etwa äquimolarer Menge einsetzt und zusätzlich Ammoniak, gegebenenfalls in Form eines Salzes einer gegenüber $R_3$–COOH schwächeren Säure, in überschüssiger Menge, vorzugsweise in 3- bis 5-fachem Überschuss, zugibt.

Eine reaktionsfähige veresterte Hydroxygruppe $Z_1$ ist beispielsweise eine vorzugsweise durch starke anorganische oder organische Säuren, wie starke Mineralsäuren, z. B. Halogenwasserstoffsäuren, wie Chlor- oder Bromwasserstoffsäure, oder starke organische Sulfonsäuren, wie entsprechende Niederalkan- oder Arylsulfonsäuren, z. B. Methan- oder eine gegebenenfalls substituierte Benzolsulfonsäure, veresterte Hydroxygruppe und stellt z. B. Halogen, wie Chlor oder Brom, Niederalkylsulfonyloxy, z. B. Methyl- oder Ethylsulfonyloxy, oder Arylsulfonyloxy, z. B. p-Toluol- oder Benzolsulfonyloxy, dar.

Das Ammoniumsalz der Formel (IIIe) kann auch unter den Reaktionsbedingungen in situ gebildet werden, beispielsweise indem man im Reaktionsgemisch die freie Säure der Formel (IIIe) vorlegt und mit flüssigem oder gasförmigem Ammoniak versetzt. Bei dieser Ausführungsform kann das Ammoniak auch in Form eines Salzes mit einer gegenüber $R_3$–A–COOH schwächeren Säure, wie Kohlensäure, zugegeben werden.

Als geeignete Lösungsmittel kommen z.B. gegebenenfalls halogenierte Kohlenwasserstoffe, wie gegebenenfalls halogenierte aliphatische, cycloaliphatische oder aromatische Kohlenwasserstoffe, wie Hexan, Cyclohexan, Toluol, Chloroform, oder Chlorbenzol, Alkanole, wie Propanol, Isopropanol, Butanole, Pentanole oder Octanole, Ether, wie Dimethoxyethan, Ethylenglykolmonoethylether, Dioxan oder Tetrahydrofuran, Niederalkancarbonsäuren, wie Ameisen- oder Essigsäure oder vorzugsweise Säuren der Formel (IIIh), Amide, wie Niederalkancarbonsäureamide, z.B. Formamid oder Dimethylformamid, sowie Lactame, z.B. N-Methylpyrrolidon, Sulfoxide, wie Dimethylsulfoxid, oder Wasser in Frage.

Eine bevorzugte Ausführung dieser Variante zur erfindungsgemässen Darstellung von Verbindungen der Formel I über Verbindungen der Formel II besteht darin, dass man eine Verbindung der Formel IIId, worin $Z_1$ beispielsweise Halogen, wie Brom, bedeutet, mit einem Ammoniumsalz der Formel (IIIe) bei einer Reaktionstemperatur von etwa 100 °C umsetzt. Die Verbindung der Formel (IIIe) wird im Überschuss zugegeben, beispielsweise in einem Verhältnis gegenüber dem Ester der Formel (IIId) von etwa 4:1 bis etwa 6:1, und lässt sich in situ bilden, indem man z.B. die entsprechende Säure unter den Reaktionsbedingungen mit flüssigem Ammoniak umsetzt.

Die Ausgangsstoffe der Formel (IIId) sind bekannt oder können nach an sich bekannten Verfahren hergestellt werden.

So lassen sie sich beispielsweise durch Esterkondensation von veresterten Säuren der Formeln $R_1$–$CH_2$–COOH bzw. $R_2$–$CH_2$–COOH mit veresterten Säuren der Formeln $R_1$–COOH bzw. $R_2$–COOH, vorzugsweise in Gegenwart einer Base erhalten. Das resultierende α-Methylenketon der Formel

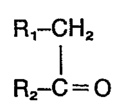

wird beispielsweise bromiert und somit in eine Verbindung der Formel (IIId) bzw. ein Salz, z.B. Hydrohalogenid, davon überführt, worin $Z_1$ Brom bedeutet.

Ausserdem kann man in einer weiteren bevorzugten Ausführungsform ein Oxazol der Formel

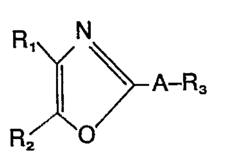

(IIIf)

mit Ammoniak über Verbindungen der Formel II zu Verbindungen der Formel I umsetzen.

Diese Reaktion wird gegebenenfalls unter Druck, beispielsweise bei $1{,}8 \cdot 10^7$ Pa, und/oder Erwärmen, z.B. auf etwa 100° bis etwa 250 °C, durchgeführt.

Die Verbindungen der Formel (IIIf) ihrerseits lassen sich nach an sich bekanntem Verfahren herstellen, beispielsweise durch Umsetzung von Verbindungen der Formel

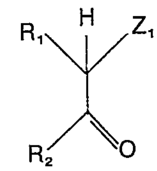

(IIId),

worin $Z_1$ gegebenenfalls reaktionsfähiges verestertes Hydroxy bedeutet, mit einer Carbonsäure der Formel $R_3$–A–COOH (IIIp), einem funktionellen Derivat oder Salz davon, wie einem entsprechenden Anhydrid, z.B. einem Halogencarbonylderivat, gegebenenfalls über eine Verbindung der Formel

$$R_2\text{–C(=O)–CH(}R_1\text{)–O–C(=O)–A–}R_3 \qquad \text{(IIIq)},$$

und mit Ammoniak.

Dabei wird jeweils eine Verbindung der Formel II gebildet, die erfindungsgemäss, insbesondere in situ, zu einer Verbindung der Formel I weiterreagiert.

Einige der vorstehend beschriebenen Verfahrensvarianten lassen sich durch Anwendung milder Bedingungen so durchführen, dass die Verbindungen der Formel II bzw. ihre Tautomeren und/oder Salze isoliert werden können.

Verbindungen der Formel I oder Salze davon kann man weiterhin herstellen, indem man beispielsweise eine Verbindung der Formel

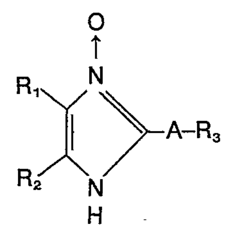

(IV)

oder ein Salz davon zu einer Verbindung der Formel I reduziert und gewünschtenfalls die verfahrensgemäss erhältliche freie Verbindung in ein Salz oder ein verfahrensgemäss erhältliches Salz in die freie Verbindung oder in ein anderes Salz überführt.

Die Reduktion erfolgt nach an sich bekannten Verfahren. So behandelt man Verbindungen der Formel (IV) oder deren Salze mit Wasserstoff in Gegenwart eines Hydrierungskatalysators oder mit einem Dithionit, z.B. Natriumdithionit, oder mit einem Phosphorhalogenid, z.B. Phosphortrichlorid. Als Hydrierungskatalysatoren lassen sich beispielsweise Elemente der VIII. Nebengruppe sowie Derivate davon, wie Platin, Palladium oder Palladiumchlorid, die gegebenenfalls auf einem üblichen Trägermaterial, wie Aktivkohle oder Erdalkalimetallverbindungen, z.B. Bariumcarbonat, aufgezogen sind, oder Raney-Nickel, verwenden.

Die Reduktion lässt sich erforderlichenfalls unter Kühlen oder Erwärmen, beispielsweise in einem Temperaturbereich von etwa 0° bis etwa

150 °C, in einem inerten Lösungsmittel, wie einem halogenierten Kohlenwasserstoff, z.B. Chloroform, Tetrachlorkohlenstoff oder Chlorbenzol, oder einem Ether, wie Dimethoxyethan, Diethylether, Dioxan oder Tetrahydrofuran, und/oder unter Inertgas, z.B. Stickstoff, durchführen.

Die Ausgangsstoffe der Formel IV oder deren Salze sind in an sich bekannter Weise erhältlich, beispielsweise in dem eine Verbindung der Formel

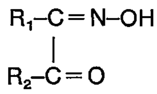
(IVa),

ein Tautomeres oder ein Salz davon in situ mit einem Überschuss an Ammoniak und einem Aldehyd der Formel $R_3$–A–C(=O)–H (IIIb) bei erhöhter Temperatur umsetzt.

Verbindungen der Formel I lassen sich ferner herstellen, indem man in einer Verbindung der Formel

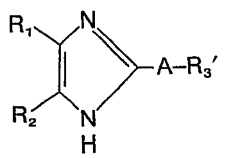
(V),

worin $R_3'$ einen im $R_3$ überführbaren Rest bedeutet, den Rest $R_3'$ in einen Rest $R_3$ überführt und gewünschtenfalls die verfahrensgemäss erhältliche freie Verbindung in ein Salz oder ein verfahrensgemäss erhältliches Salz in die freie Verbindung oder in ein anderes Salz überführt.

Derartige Gruppen $R_3'$ sind beispielsweise von gegebenenfalls verestertem oder amidiertem Carboxy verschiedene funktionell abgewandelte Carboxygruppierungen, wie Cyano oder Orthoestergruppierungen, welche beispielsweise durch Solvolyse, z.B. Hydrolyse oder Alkoholyse, in einen Rest $R_3$ überführbar sind.

Als Orthoestergruppierungen kommen in Frage: mit einem Niederalkanol veretherte oder mit einer Mineralsäure veresterte Orthoestergruppen wie Triniederalkoxy-, Trihalogen- oder Niederalkoxydihalogenmethyl, vor allem Triethoxy- oder Trichlormethyl.

Die Solvolyse wird in üblicher Weise durchgeführt, erforderlichenfalls in Gegenwart einer Base, wie eines Alkalimetallhydroxides, z.B. Natrium- oder Kaliumhydroxid, oder einer Protonsäure, z.B. einer Mineralsäure, wie Schwefel- oder Halogenwasserstoff-, beispielsweise Chlorwasserstoffsäure, oder z.B. einer organischen Carbon- oder Sulfonsäure, wie von Essigsäure oder p-Toluolsulfonsäure. Dabei arbeitet man erforderlichenfalls in einem inerten Lösungsmittel, wie in einem Niederalkanol, z.B. Methanol, Ethanol, einem Keton, wie Aceton, oder einem Ether, wie Dioxan, und erforderlichenfalls unter Kühlen oder Erwärmen, z.B. bei etwa 0° bis etwa 150 °C. So lässt sich Cyano, gegebenenfalls substituiertes Carbamoyl oder eine Orthosäuregruppierung $R_3'$ durch Hydrolyse in freies Carboxy, durch Alkoholyse in verestertes Carboxy oder durch Ammonolyse bzw. Aminolyse in Carbamoyl bzw. N-substituiertes Carbamoyl überführen.

Weitere in Carboxy bzw. verestertes Carboxy $R_3$ überführbare Gruppen sind beispielsweise oxidativ in diesen überführbare Reste, wie gegebenenfalls verestertes oder verethertes Hydroxymethyl oder gegebenenfalls acetalisiertes Formyl.

Verestertes Hydroxymethyl ist beispielsweise mit einer Mineralsäure, wie Halogenwasserstoff-, wie Chlorwasserstoffsäure, oder einer Carbonsäure, wie Niederalkancarbonsäure, z.B. Essigsäure, oder gegebenenfalls substituierten Benzoesäure verestertes Hydroxymethyl. Acetalisiertes Formyl ist beispielsweise mit einem Niederalkanol oder einem Niederalkandiol acetalisiertes Formyl, wie Dimethoxy-, Diethoxy- oder Ethylendioxyformyl.

Die Oxidation derartiger Gruppen $R_3'$ erfolgt in an sich bekannter Weise, beispielsweise durch Umsetzung in einem geeigneten Oxidationsmittel, beispielsweise in einem inerten Lösungsmittel, wie einer Niederalkancarbonsäure z.B. Essigsäure, einem Keton, z.B. Aceton, einem Ether, z.B. Tetrahydrofuran, einem heterocyclischen Aromaten, z.B. Pyridin, oder Wasser oder einem Gemisch davon, erforderlichenfalls unter Kühlen oder Erwärmen, z.B. von etwa 0° bis etwa 150 °C. Als Oxidationsmittel kommen beispielsweise oxidierende Übergangsmetallverbindungen, insbesondere solche mit Elementen der I., VI., VII., oder VIII. Nebengruppe, in Frage. Als Beispiele seien genannt: Silberverbindungen, wie Silbernitrat, -oxid oder -picolinat, Chromverbindungen, wie Chromtrioxid oder Kaliumdichromat, Manganverbindungen, wie Kaliumferrat, Tetrabutylammonium- oder Benzyl(triethyl)ammoniumpermanganat. Weitere Oxidationsmittel sind beispielsweise geeignete Verbindungen mit Elementen der 4. Hauptgruppe, wie Bleidioxid, oder Halogen-Sauerstoff-Verbindungen, wie Natriumiodat oder Kaliumperiodat.

So wird beispielsweise Hydroxymethyl und gegebenenfalls acetalisiertes Formyl zu Carboxy $R_3$ oxidiert, während die Oxidation von verethertem Hydroxymethyl R zu verestertem Carboxy $R_3$ führt.

Die Ausgangsstoffe der Formel V werden nach an sich bekannten Verfahren hergestellt. Beispielsweise geht man von einem Diketon der Formel

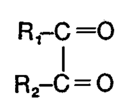
(IIIa)

aus und setzt dieses mit Ammoniak und einem Aldehyd der Formel $R_3'$–A–C(=O)–H in einem inerten Lösungsmittel und unter Erwärmen und die in situ gebildete Verbindung der Formel V ohne Isolierung weiter um.

Eine weitere, an sich bekannte Herstellungsweise für Verbindungen der Formel I oder deren Salze besteht beispielsweise darin, dass in einer Verbindung der Formel

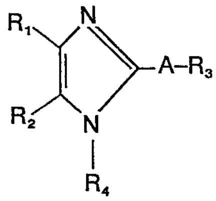

(VI),

worin $R_4$ eine in Wasserstoff überführbare Gruppe darstellt, oder einem Salz davon $R_4$ in Wasserstoff überführt und gewünschtenfalls die verfahrensgemäss erhältliche freie Verbindung in ein Salz oder ein verfahrensgemäss erhältliches Salz in die freie Verbindung oder in ein anderes Salz überführt.

Ein in Wasserstoff überführbarer Rest $R_4$ ist beispielsweise eine der üblichen geeigneten Aminoschutzgruppen. Aus der Fülle der geeigneten in Wasserstoff überführbaren Gruppen sind beispielsweise durch Sauerstoff unterbrochene, gegebenenfalls cyclische Alkyl-, gegebenenfalls substituierte Aralkyl-, Sulfenyl- oder Acylreste zu nennen. Als Alkylreste, die durch Sauerstoff unterbrochen sind, kommen z.B. Niederalkoxyniederalkyl, wie Ethoxyethyl, Phenylniederalkoxyniederalkyl, wie Benzylmethylether, Tetrahydrofuranyl oder Tetrahydropyranyl in Frage. Gegebenenfalls substituierte Aralkylreste sind z.B. solche, die im Arylteil z.B. Phenyl, Biphenyl, Anthryl und/oder Pyridyl und im Alkylteil Niederalkyl, wie Methyl oder Isopropyl, aufweisen, wie im Phenylteil durch Niederalkyl, Niederalkoxy oder Halogen substituiertes Benzyl, wie 3,5-Dimethoxy-, 2,4,6-Trimethoxy- oder 4-Brombenzyl, 2-Biphenyl-2-propyl, Di- oder Triarylmethyl, wie Diphenyl-, Triphenyl- oder α,α-Diphenyl-4-pyridylmethyl. Sulfenylreste sind beispielsweise gegebenenfalls durch Nitro substituierte Phenylsulfenylreste, wie 3-Nitrophenyl-sulfenyl. Unter Acylresten sind beispielsweise solche zu verstehen, die sich von aromatischen Carbonsäuren oder Alkancarbonsäuren sowie von Sulfonsäuren ableiten, wie gegebenenfalls substituiertes Benzoyloxycarbonyl, Niederalkanoyloxycarbonyl, z.B. tert.-Butyloxycarbonyl, Alkanoyl, wie Acetyl, oder Sulfonyl, wie p-Toluolsulfonyl.

Die Abspaltung der Amino-Schutzgruppe $R_4$ erfolgt nach an sich bekannten Methoden. Sie kann beispielsweise reduktiv, z.B. hydrogenolytisch mit gegebenenfalls nascierendem Wasserstoff, oder acidolytisch, z.B. mit Mineralsäuren, wie Halogenwasserstoffsäuren, wie Chlor- oder Bromwasserstoffsäure, oder mit gegebenenfalls substituierten Niederalkancarbonsäuren, wie Essigsäure oder Trifluoressigsäure, erforderlichenfalls unter Kühlen oder Erwärmen, z.B. im Temperaturbereich von etwa 0° bis etwa 150 °C, und in einem inerten Lösungsmittel erfolgen. Inerte Lösungsmittel sind beispielsweise Amide, wie Dimethylformamid, halogenierte Kohlenwasserstoffe, wie Chloroform oder Tetrachlorkohlenstoff, Niederalkanole, wie Methanol oder Ethanol, Ketone, wie Diniederalkylketone, z.B. Aceton, Ether, wie Tetrahydrofuran, oder Nitrile, wie Acetonitril. Insbesondere erfolgt die Überführung von Benzyl $R_4$ in Wasserstoff mittels Wasserstoff in Essigsäure und in Gegenwart eines Hydrierungskatalysators, wie Palladiumkohle, mit Natrium in flüssigem Ammoniak, mit Bromwasserstoffsäure in Essigsäure oder mit Fluorwasserstoff, von Triphenylmethyl $R_4$ mit Chlorwasserstoffsäure in Acetonitril, mit Trifluoressigsäure in Essigsäure oder mit Essigsäure oder von tert.-Butyl $R_4$ mit Trifluoressigsäure.

Die Ausgangsstoffe der Formel (VI) werden nach an sich bekannten Verfahren hergestellt, beispielsweise geht man von Verbindungen der Formel VIII oder V aus und führt die Gruppe $R_4$ ein, z.B. durch übliche Aralkylierung oder Acylierung. Entsprechend N-substituierte Verbindungen der Formel VIII werden anschliessend in der nachstehend beschriebenen Weise mit Hilfe eines geeigneten Dehydrierungsmittels zu Verbindungen der Formel VI dehydriert.

In der so erhältlichen Verbindung der Formel

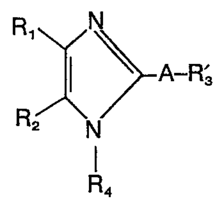

(VIc)

wird $R_3'$ in $R_3$ überführt. Die Überführung erfolgt oxidativ, wobei Hydroxymethyl bzw. Niederalkoxymethyl $R_3'$ zu Carboxy bzw. Niederalkoxycarbonyl $R_3$ oxidiert wird. In einem weiteren Reaktionsschritt kann sich gewünschtenfalls eine Ammonolyse bzw. Aminolyse anschliessen.

In einer weiteren Verfahrensweise gelangt man zu Verbindungen der Formel I, indem man Verbindungen der Formel

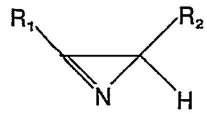

(VIIa)

oder Tautomere davon mit Verbindungen der Formel $R_3$–A–CN (VIIb) umsetzt und gewünschtenfalls die verfahrensgemäss erhältlichen freien Verbindungen in Salze oder verfahrensgemäss erhältlichen Salze in die freien Verbindungen oder andere Salze überführt.

Die Umsetzung erfolgt in an sich bekannter Weise, beispielsweise in Gegenwart einer Lewissäure. Dabei wird erforderlichenfalls in einem inerten Lösungsmittel, unter Inertgas, z.B. Stickstoff, und/oder in einem Temperaturbereich von etwa −50° bis etwa +100 °C, insbesondere zwischen −10° und etwa +30 °C, gearbeitet. Vorteilhaft verwendet man Verbindungen der Formel VIIb zusätzlich als Lösungsmittel.

Als Lewissäuren, d.h. Elektronenakzeptoren, werden z.B. Verbindungen von Elementen der 3. und 5. Hauptgruppe sowie der II. und VIII. Nebengruppe des Periodensystems verwendet. In Betracht kommen vor allem Halogenide des Bors, Aluminium, Zinns, Antimons und Eisens, insbesondere Bortrifluorid-Etherat, ferner Aluminiumchlorid, Zinn-IV-chlorid, Zink- und Eisenchlorid.

Inerte Lösungsmittel sind beispielsweise gegebenenfalls Nitro enthaltende Kohlenwasserstoffe, wie Nitroaromaten, z.B. Nitrobenzol.

Die Bildung von Verbindungen der Formel I ausgehend von Ausgangsstoffen der Formeln VIIa und VIIb erfolgt unter den Reaktionsbedingungen überwiegend in situ und ohne Isolierung von möglichen Zwischenstufen. Allerdings kann die Umsetzung auch ausgehend von stabilen Vinylnitrenium-Verbindungen der Formel

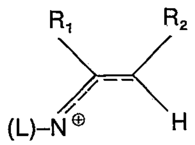

(VIIc)

erfolgen, worin gegebenenfalls vorhandenes L eine komplex gebundene Lewissäure bedeutet.

Die Bildung von Vinylnitrenium-Verbindungen der Formel VIIc wird durch Energiezufuhr, beispielsweise durch Thermo- oder Photolyse, oder durch die vorstehend genannten Lewissäuren induziert.

Die Ausgangsstoffe der Formeln VIIa, VIIb und VIIc sind z.B. bekannt oder werden nach an sich bekannten Methoden hergestellt.

In den vorstehend beschriebenen Verfahren zur Herstellung von Verbindungen der Formeln II, IV, Va und Vb, kann das Ammoniak, welches überwiegend im Überschuss zugegeben wird, auch in Form eines Ammoniak abgebenden Mittels eingesetzt werden, wobei die Freisetzung bei erhöhter Temperatur und gegebenenfalls unter Druck erfolgt. Als Ammoniak abgebende Mittel kommen z.B. Ammoniumsalze von Niederalkancarbonsäuren, vorzugsweise Ammoniumacetat, oder einer Carbonsäure der Formel $R_3$–A–COOH, ferner ein geeignetes Niederalkancarbonsäureamid, insbesondere Formamid, in Frage.

Die Verbindungen der Formel I können weiterhin hergestellt werden, indem man eine Verbindung der Formel

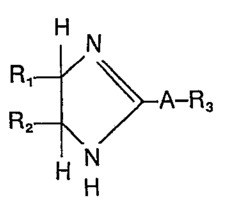

(VIII)

oder ein Isomeres davon zu Verbindungen der Formel I dehydriert und gewünschtenfalls die verfahrensgemäss erhältliche freie Verbindung in ein Salz oder ein verfahrensgemäss erhältliches Salz in die freie Verbindung oder in ein anderes Salz überführt.

Verbindungen der Formel VIII können beispielsweise in Form eines individuellen Stereoisomeren, optischen Isomeren wie Enantiomeren, oder als Gemische derselben, wie Racemate, sowie als geometrische (cis-trans) Isomere vorliegen.

Dehydrierung von Verbindungen der Formel VIII erfolgt in an sich bekannter Weise, insbesondere bei erhöhter Temperatur, beispielsweise in einem Temperaturintervall von etwa 100° bis etwa 300 °C, gegebenenfalls unter Verwendung eines Dehydrierungsmittels. Als solche Mittel kommen beispielsweise Dehydrierungskatalysatoren, z.B. Nebengruppenelemente, vorzugsweise der Nebengruppe VIII, wie Palladium oder Platin, oder deren Salze, wie Ruthenium-triphenyl-phosphidchlorid, in Betracht, wobei die Katalysatoren gegebenenfalls auf geeignetem Trägermaterial, wie Kohle, Aluminiumoxid oder Siliziumdioxid, aufgezogen sind. Weitere Dehydrierungsmittel sind beispielsweise Chinone, wie p-Benzochinone, z.B. Tetrachlor-p-benzochinon oder 2,3-Dichlor-5,6-dicyano-p-benzochinon, oder wie Anthrachinone, z.B. Phenanthren-9,10-chinon, N-Halogensuccinimide, wie N-Chlorsuccinimid, oder Manganate, wie Bariummanganat. Die Umsetzung wird in einem inerten, gegebenenfalls hochsiedenden Lösungsmittel, wie einem Ether, z.B. Diphenylether, erforderlichenfalls unter Druck, in einem geschlossenen Gefäss und/oder unter Inertgas, z.B. Stickstoff, durchgeführt.

Die als Ausgangsstoffe zu verwendenden Verbindungen der Formel VIII können beispielsweise hergestellt werden, indem man eine Verbindung der Formel

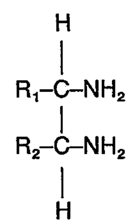

(VIIIa)

oder ein Salz davon mit Verbindungen der Formel $R_3$–A–$Z_3$ (VIIIb), worin $Z_3$ gegebenenfalls funktionell abgewandeltes Carboxy bedeutet, umsetzt. Funktionell abgewandeltes Carboxy ist unter anderem verestertes Carboxy, wie Niederalkoxycarbonyl, amidiertes Carboxy, wie gegebenenfalls substituiertes Carbamoyl, oder anhydridisiertes Carboxy, wie Halogencarbonyl.

Ein weiterer Verfahrensweg, der gegebenenfalls über Verbindungen der Formel II verläuft, besteht in der Umsetzung von Verbindungen der Formel $R_1$–$C(NH_2)=C(NH_2)$–$R_2$ (IIIk) oder deren Salzen, mit gegebenenfalls funktionell abgewandelten Verbindungen der Formel (IIIp) oder, unter oxidierenden Bedingungen, z.B. in Gegenwart eines der vorstehend genannten Oxidationsmittels oder insbesondere Nitrobenzols mit Verbindungen der Formel IIIb, gegebenenfalls unter Kühlen oder Erwärmen, und unter inerten Bedingungen. In einer bevorzugten Variante erhitzt man Verbindungen der Formeln (IIIk) und (IIIb) in Nitrobenzol, wobei ohne Isolierung von Zwischenprodukten direkt eine entsprechende Verbindung der Formel I erhalten wird.

Der Aldehyd der Formel $R_3$–A–C(=O)–H (IIIb) kann in den vorstehend beschriebenen Herstel-

lungsverfahren für Verbindungen der Formeln II, IV und V beispielsweise auch unter den Reaktionsbedingungen aus einem Oxazinderivat der Formel

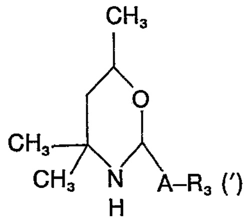

(IX)

freigesetzt werden. Die Verbindungen der Formel IX lassen sich beispielsweise herstellen, indem man 2-Methyl-2,4-pentandiol mit einem Nitril der Formel $R_3(')$–A–CN in Gegenwart von Schwefelsäure umsetzt. Das dabei gebildete, entsprechend substituierte Dihydro-1,3-oxazin wird in einem Gemisch aus Tetrahydrofuran und Ethanol bei −45 °C und einem pH-Wert von etwa 7 unter Einwirkung von Natriumborhydrid zu dem Tetrahydro-1,3-oxazin der Formel IX reduziert.

Eine erfindungsgemäss erhältliche Verbindung kann in üblicher Weise in eine andere Verbindung der Formel I überführt werden.

So kann man in erfindungsgemäss erhältlichen Verbindungen der Formel I freie und veresterte Carboxygruppen $R_3$ ineinander umwandeln.

Eine freie Carboxylgruppe $R_3$ lässt sich beispielsweise in üblicher Weise, z.B. durch Behandeln mit einem Diazoniederalkan, Diniederalkylformamidacetal, Alkylhalogenid oder Triniederalkyloxonium-, Triniederalkylcarboxonium- oder Diniederalkylcarboniumsalze, wie Hexachloroantimonat oder Hexafluorophosphat, oder vor allem durch Umsetzung mit dem entsprechenden Alkohol oder einem reaktionsfähigen Derivat, wie einem Carbon-, Phosphorig-, Schweflig- oder Kohlensäureester, z.B. einem Niederalkancarbonsäureester, Triniederalkylphosphit, Diniederalkylsulfit oder dem Pyrocarbonat, oder einem Mineralsäure- oder Sulfonsäureester, z.B. dem Chlor- oder Bromwasserstoffsäure- oder Schwefelsäure-, Benzolsulfonsäure-, Toluolsulfonsäure- oder Methansulfonsäureester, des entsprechenden Alkohols oder einem davon abgeleiteten Olefin, zu einer veresterten Carboxylgruppe $R_3$ verestern.

Die Umsetzung mit dem entsprechenden Alkohol selbst kann vorteilhaft in Gegenwart eines sauren Katalysators erfolgen, wie einer Protonensäure, z.B. von Chlor- oder Bromwasserstoff-, Schwefel-, Phosphor-, Bor-, Benzolsulfon- und/oder Toluolsulfonsäure, oder eine Lewissäure, z.B. von Bortrifluorid-Etherat, in einem inerten Lösungsmittel, insbesondere einem Überschuss des eingesetzten Alkohols und erforderlichenfalls in Gegenwart eines wasserbindenden Mittels und/oder unter destillativer, z.B. azeotroper, Entfernung des Reaktionswassers und/oder bei erhöhter Temperatur.

Die Umsetzung mit einem reaktionsfähigen Derivat des entsprechenden Alkohols kann in üblicher Weise durchgeführt werden, ausgehend von einem Carbon-, Phosphorig-, Schweflig- oder Kohlensäureester beispielsweise in Gegenwart eines sauren Katalysators, wie eines der vorstehend genannten, in einem inerten Lösungsmittel, wie einem aromatischen Kohlenwasserstoff, z.B. in Benzol oder Toluol, oder einem Überschuss des eingesetzten Alkoholderivates oder des entsprechenden Alkohols. Ausgehend von einem Mineralsäure- oder Sulfonsäureester setzt man die zu veresternde Säure vorteilhaft in Form eines Salzes, z.B. des Natrium- oder Kaliumsalzes, und arbeitet erforderlichenfalls in Gegenwart eines basischen Kondensationsmittels, wie einer anorganischen Base, z.B. von Natrium- oder Kalium- oder Calciumhydroxid oder -carbonat, oder einer tertiären organischen Stickstoffbase, z.B. von Triethylamin oder Pyridin, und/oder in einem inerten Lösungsmittel, wie einer der vorstehend tertiären Stickstoffbasen oder eines polaren Lösungsmittels, z.B. in Dimethylformamid und/oder bei erhöhter Temperatur.

Die Reaktion mit einem Diniederalkyl-formamidacetal, wie Dimethyl-formamidacetal, erfolgt gegebenenfalls unter Erwärmen, während die Umsetzung mit einem Alkylhalogenid in Gegenwart einer Base, wie einem Amin, z.B. Triethylamin, durchgeführt wird.

Die Umsetzung mit einem Olefin kann beispielsweise in Gegenwart eines sauren Katalysators, z.B. einer Lewissäure, wie Bortrifluorid, einer Sulfonsäure, z.B. von p-Toluolsulfonsäure, oder vor allem eines basischen Katalysators, z.B. von Natrium- oder Kaliumhydroxid, vorteilhaft in einem inerten Lösungsmittel, wie einem Ether, z.B. Diethylether oder Tetrahydrofuran, erfolgen.

Die vorstehend beschriebenen Umwandlungen freier in veresterte Carboxylgruppen $R_3$ können aber auch so durchgeführt werden, dass man eine Verbindung der Formel I, worin $R_3$ Carboxyl ist, zunächst in üblicher Weise in ein reaktionsfähiges Derivat, beispielsweise mittels eines Halogenides des Phosphors oder Schwefels, z.B. mittels Phosphortrichlorid oder -bromid, Phosphorpentachlorid oder Thionylchlorid, in ein Säurehalogenid oder durch Umsetzung mit einem entsprechenden Alkohol in einen reaktiven Ester, d.h. Ester mit elektronenanziehenden Strukturen, wie den Ester mit Phenol, Thiophenol, p-Nitrophenol oder Cyanmethylalkohol, überführt und das erhaltene reaktionsfähige Derivat dann in üblicher Weise z.B. wie nachstehend für die Umesterung bzw. gegenseitige Umwandlung veresterter Carboxylgruppen $R_3$ beschrieben, mit einem entsprechenden Alkohol zu der gewünschten Gruppe $R_3$ umsetzt.

Eine veresterte Carboxylgruppe $R_3$ kann in üblicher Weise, z.B. durch Hydrolyse in Gegenwart eines Katalysators, beispielsweise eines basischen oder sauren Mittels, wie einer starken Base, z.B. von Natrium- oder Kaliumhydroxid, oder einer Mineralsäure, z.B. von Salzsäure, Schwefelsäure oder Phosphorsäure, zur freien Carboxylgruppe $R_3$ überführt werden.

Eine veresterte Carboxylgruppe $R_3$ kann ferner in üblicher Weise, z.B. durch Umsetzung mit einem Metallsalz, wie dem Natrium- oder Kaliumsalz, eines entsprechenden Alkohols oder mit diesem selbst in Gegenwart eines Katalysators, beispielsweise einer starken Base, z. B. von Natrium- oder Kaliumhydroxid, oder einer starken Säure, wie einer Mineralsäure, z. B. von Salzsäure, Schwefelsäure oder Phosphorsäure, oder einer organischen Sulfonsäure, z. B. von p-Toluolsulfonsäure, oder einer Lewissäure, z. B. von Bortrifluorid-Etherat, zu einer anderen veresterten Carboxylgruppe $R_3$ umgeestert werden.

Weiterhin kann man freies Carboxy bzw. reaktionsfähig funktionelle Carboxyderivate durch Solvolyse mit Ammoniak oder einem primären bzw. sekundären Amin, wobei auch Hydroxylamine bzw. Hydrazine eingesetzt werden können, in üblicher Weise unter Dehydratisierung, gegebenenfalls in Anwesenheit eines Kondensationsmittels, in eine gewünschte amidierte Form überführen. Als Kondensationsmittel werden vorzugsweise Basen verwendet, beispielsweise anorganische Basen, wie Alkalimetallhydroxide, z. B. Natrium- oder Kaliumhydroxid, organische Stickstoffbasen, wie tert.-Amine, z. B. Pyridin, Tributylamin oder N-Dimethylanilin, oder Tetrahalogensilane, wie Tetrachlorsilan. Ebenso kann man in erfindungsgemäss erhältlichen Verbindungen der Formel I, worin $R_3$ amidiertes Carboxy bedeutet, nach an sich bekannten Methoden die Amidbindung spalten und so das Carbamoyl in freies Carboxy überführen. Hierzu arbeitet man in Gegenwart eines Katalysators, beispielsweise einer Base, wie eines Alkalimetall- oder Erdalkalimetallhydroxides oder -carbonates, z. B. Natrium-, Kalium- oder Calciumhydroxid oder -carbonat, oder einer Säure, wie einer Mineralsäure, z. B. Salzsäure, Schwefelsäure oder Phosphorsäure.

Enthält mindestens einer der Substituenten $R_1$, $R_2$ und $R_3$ als zusätzlichen Substituenten Hydroxy, so lässt sich dieser nach an sich bekannter Weise verethern. Die Umsetzung mit einer Alkoholkomponente, z. B. mit einem Niederalkanol, wie Ethanol, in Gegenwart von Säuren, z. B. Mineralsäure, wie Schwefelsäure, oder von Dehydratisierungsmitteln, wie Dicyclohexylcarbodiimid, führt zu Niederalkoxy. Phenole bzw. deren Salze lassen sich z. B. in Gegenwart von Basen, wie Alkalimetallhydroxiden oder -carbonaten, z. B. Natriumhydroxid oder Kaliumcarbonat, mit Hilfe von Diniederalkylsulfaten, Diazoniederalkanen oder Alkyl- bzw. Arylhalogeniden in entsprechende Niederalkylphenylether bzw. Arylphenylether überführen. Umgekehrt kann man Ether in Alkohole spalten. So entstehen z. B. aus Alkoxyarylverbindungen aromatische Alkohole, indem man die Etherspaltung mittels Säuren, wie Mineralsäuren, z. B. Halogenwasserstoffsäure, wie Bromwasserstoffsäure, oder wie Lewissäuren, z. B. Halogeniden von Elementen der 3. Hauptgruppe, wie Bortribromid, oder mittels Basen, z. B. Niederalkylaminen, wie Methylamin, durchführt.

Weiterhin lässt sich Hydroxy in Niederalkanoyloxy umwandeln, beispielsweise durch Umsetzung mit einer gewünschten Niederalkancarbonsäure, wie Essigsäure oder einem reaktionsfähigen Derivat davon, beispielsweise in Gegenwart einer Säure, wie eine Protonsäure, z. B. Chlor-, Bromwasserstoff-, Schwefel-, Phosphor- oder einer Benzolsulfonsäure, in Gegenwart einer Lewissäure, z. B. von Bortrifluorid-Etherat, oder in Gegenwart eines wasserbindenden Mittels. Umgekehrt kann verestertes Hydroxy, z. B. durch Basen-Katalyse, zu Hydroxy solvolysiert werden.

Erhaltene freie Verbindungen der Formel I können in an sich bekannter Weise in Salze überführt werden. Hydroxy aufweisende Gruppen $R_1$ bzw. $R_2$ sowie Carboxy $R_3'$ werden mit entsprechenden Basen, wie Alkalimetallhydroxiden, in die eingangs aufgeführten Salze mit Basen, oder durch Behandeln mit einer wie vorstehend aufgeführten, Säureadditionssalze bildenden Säure in Säureadditionssalze umgewandelt werden.

Erhaltene Salze können in an sich bekannter Weise in die freien Verbindungen umgewandelt werden, z. B. durch Behandeln mit einem sauren Reagenz, wie einer Mineralsäure, bzw. einer Base, z. B. Alkalihydroxid.

Infolge der engen Beziehung zwischen der neuen Verbindung in freier Form und in Form ihrer Salze sind im vorausgegangenen und nachfolgend unter der freien Verbindung oder ihren Salzen sinn- und zweckgemäss gegebenenfalls auch die entsprechenden Salze bzw. die freie Verbindung zu verstehen.

Die neue Verbindung kann, je nach der Wahl der Ausgangsstoffe und Arbeitsweisen, in Form eines der möglichen Isomeren oder als Gemische derselben vorliegen.

Die neue Verbindung einschliesslich ihrer Salze kann auch in Form ihrer Hydrate erhalten werden oder andere zur Kristallisation verwendete Lösungsmittel einschliessen.

Die neuen Verbindungen können, je nach der Wahl der Ausgangsstoffe und Arbeitsweisen, in Form eines der möglichen Isomeren oder als Gemische derselben, z. B. je nach der Anzahl der asymmetrischen Kohlenstoffatome als reine optische Isomere, wie Antipoden, oder als Isomerengemische, wie Racemate, Diastereoisomerengemische oder Racematgemische, ferner als Tautomere vorliegen.

Erhaltene Diastereomerengemische und Racematgemische können auf Grund der physikalischchemischen Unterschiede der Bestandteile in bekannter Weise in die reinen Isomeren, Diastereomeren oder Racemate aufgetrennt werden, beispielsweise durch Chromatographie und/oder fraktionierte Kristallisation. Erhaltene Racemate lassen sich ferner nach bekannten Methoden in die optischen Antipoden zerlegen, beispielsweise durch Umkristallisation aus einem optisch aktiven Lösungsmittel, mit Hilfe von Mikroorganismen oder durch Umsetzung eines sauren Endstoffes mit einer mit der racemischen Säure Salze bildenden optisch aktiven Base und Trennung der auf diese Weise erhaltenen Salze, z. B. auf Grund ihrer verschiedenen Löslichkeiten, in die Diastereomeren, aus denen die Antipoden durch Einwirkung geeigneter Mittel freigesetzt werden können, zerlegt. Vorteilhaft isoliert man den wirksameren der beiden Antipoden.

Die Erfindung betrifft auch diejenigen Ausführungsformen des Verfahrens, nach denen man von einer auf irgendeiner Stufe des Verfahrens als Zwischenprodukt erhältlichen Verbindungen ausgeht und die fehlenden Schritte durchführt oder einen Ausgangsstoff in Form eines Salzes verwendet oder insbesondere unter den Reaktionsbedingungen bildet.

Die Ausgangsstoffe der Formel IIIf, die speziell für die Herstellung der erfindungsgemässen Verbindungen entwickelt wurden, die Verfahren zu ihrer Herstellung sowie ihre Verwendung bilden ebenfalls einen Gegenstand der Erfindung.

So weisen beispielsweise Verbindungen der Formel IIIf worin einer der Reste $R_1$ und $R_2$ Heteroaryl und der andere carbocyclisches Aryl oder Heteroaryl bedeutet und A einen zweiwertigen Kohlenwasserstoff darstellt und $R_3$ Carboxy, verestertes Carboxy oder amidiertes Carboxy bedeutet, ihre Isomeren und ihre Salze, mit der Massgabe, dass $R_1$ bzw. $R_2$ von Naphthyl, Thienyl oder Furyl verschieden sind, eine ausgeprägte antiinflammatorische Aktivität auf, insbesondere bei topischer Applikation. Diese Wirkung lässt sich beispielsweise aufgrund der Hemmwirkung auf das durch Crotonöl induzierten Ohrödems bei der Normalratte im Dosisbereich von etwa 1 bis etwa 100 mg/ml ermitteln [Methodik: G. Tonelli, L. Thibault, Endocinology 77, 625 (1965)]. Infolgedessen können entsprechende Verbindungen der Formel IIIf als Arzneimittel, insbesondere externe (topische) Hautphlogistatika für die Behandlung entzündlicher Dermatosen, verwendet werden.

Die entsprechenden Verbindungen der Formel IIIf, Verfahren zu ihrer Herstellung, pharmazeutischer Präparate, die solche Verbindungen enthalten, und ihre Verwendung, z.B. als Arzneimittelwirkstoffe bilden ebenfalls Gegenstände der Erfindung.

Bei den erfindungsgemässen pharmazeutischen Präparaten, welche die erfindungsgemässe Verbindung oder pharmazeutisch verwendbaren Salze davon enthalten, handelt es sich vorzugsweise um solche zur topischen Anwendung an Warmblüter(n), wobei der pharmakologische Wirkstoff allein oder zusammen mit einem pharmazeutisch anwendbaren Trägermaterial enthalten ist. Die tägliche Dosierung des Wirkstoffs hängt von dem Alter und dem individuellen Zustand sowie von der Applikationsweise ab. Entsprechende Mittel mit einem Konzentrationsbereich von etwa 1 bis etwa 10% G/G, z.B. in Form von Cremen, Salben oder Lösungen, können beispielsweise 2 bis 3 × täglich appliziert werden.

Als topisch anwendbare pharmazeutische Präparate kommen in erster Linie Cremen, Salben, Pasten, Schäume, Tinkturen und Lösungen in Frage, die von etwa 0,1 bis etwa 10% des Wirkstoffs enthalten.

Cremen sind Öl-in-Wasser-Emulsionen, die mehr als 50% Wasser aufweisen. Als ölige Grundlage verwendet man in erster Linie Fettalkohole, z.B. Lauryl-, Cetyl- oder Stearylalkohol, Fettsäuren, z.B. Palmitin- oder Stearinsäure, flüssige bis feste Wachse, z.B. Isopropylmyristat, Wollwachs oder Bienenwachs, und/oder Kohlenwasserstoffe, z.B. Vaseline (Petrolatum) oder Paraffinöl. Als Emulgatoren kommen oberflächenaktive Substanzen mit vorwiegend hydrophilen Eigenschaften in Frage, wie entsprechende nichtionische Emulgatoren, z.B. Fettsäureester von Polyalkoholen oder Ethylenoxidaddukte davon, wie Polyglycerinfettsäureester oder Polyoxyethylensorbitanfettsäureester (Tweens), ferner Polyoxyethylenfettalkoholether oder -fettsäureester, oder entsprechende ionische Emulgatoren, wie Alkalimetallsalze von Fettalkoholsulfaten, z.B. Natriumlaurylsulfat, Natriumcetylsulfat oder Natriumstearylsulfat, die man üblicherweise in Gegenwart von Fettalkoholen, z.B. Cetylalkohol oder Stearylalkohol, verwendet. Zusätze zur Wasserphase sind u.a. Mittel, welche die Austrocknung der Creme vermindern, z.B. Polyalkohole, wie Glycerin, Sorbit, Propylenglykol und/oder Polyethylenglykole, ferner Konservierungsmittel, Riechstoffe, etc.

Salben sind Wasser-in-Öl-Emulsionen, die bis zu 70%, vorzugsweise jedoch von etwa 20% bis etwa 50% Wasser oder wässrige Phasen enthalten. Als Fettphase kommen in erster Linie Kohlenwasserstoffe, z.B. Vaseline, Paraffinöl und/oder Hartparaffine in Frage, die zur Verbesserung des Wasserbindungsvermögens vorzugsweise geeignete Hydroxyverbindungen, wie Fettalkohole oder Ester davon, z.B. Cetylalkohol oder Wollwachsalkohole bzw. Wollwachs, enthalten. Emulgatoren sind entsprechende lipophile Substanzen, wie Sorbitanfettsäureester (Spans), z.B. Sorbitanoleat und/oder Sorbitanisostearat. Zusätze zur Wasserphase sind u.a. Feuchthaltungsmittel, wie Polyalkohole, z.B. Glycerin, Propylenglykol, Sorbit und/oder Polyethylenglykol, sowie Konservierungsmittel, Riechstoffe, etc.

Fettsalben sind wasserfrei und enthalten als Grundlage insbesondere Kohlenwasserstoff, z.B. Paraffin, Vaseline und/oder flüssige Paraffine, ferner natürliches oder partialsynthetisches Fett, z.B. Kokosfettsäuretriglycerid, oder vorzugsweise gehärtete Öle, z.B. hydriertes Erdnuss- oder Rizinusöl, ferner Fettsäurepartialester des Glycerins, z.B. Glycerinmono- und -distearat, sowie z.B. die im Zusammenhang mit den Salben erwähnten, die Wasseraufnahmefähigkeit steigernden Fettalkohole, Emulgatoren und/oder Zusätze.

Pasten sind Cremen und Salben mit sekretabsorbierenden Puderbestandteilen, wie Metalloxiden, z.B. Titanoxid oder Zinkoxid, ferner Talk und/oder Aluminiumsilikate, welche die Aufgabe haben, vorhandene Feuchtigkeit oder Sekrete zu binden.

Schäume werden z.B. aus Druckbehältern verabreicht und sind in Aerosolform vorliegende flüssige Öl-in-Wasser-Emulsionen, wobei halogenierte Kohlenwasserstoffe, wie Chlorfluorniederalkane, z.B. Dichlordifluormethan und Dichlortetrafluorethan, als Treibmittel verwendet werden. Als Ölphase verwendet man u.a. Kohlenwasserstoffe, z.B. Paraffinöl, Fettalkohole, z.B. Cetylalkohol, Fettsäureester, z.B. Isopropylmyristat, und/oder andere Wachse. Als Emulgatoren verwendet man u.a. Gemische von solchen mit vorwiegend hydrophilen Eigenschaften, wie Polyoxyethylensorbitan-fettsäureester (Tweens), und solchen mit vorwiegend lipophilen Eigenschaften, wie Sorbitanfettsäureester (Spans). Dazu kommen die üblichen Zusätze, wie Konservierungsmittel, etc.

Tinkturen und Lösungen weisen meistens eine wässerig-äthanolische Grundlage auf, der u.a. Polyalkohole, z.B. Glycerin, Glykole und/oder Polyethylenglykol, als Feuchthaltemittel zur Herabsetzung der Verdunstung, und rückfettende Substanzen, wie Fettsäureester mit niedrigen Polyethylenglykolen, d.h. im wässrigen Gemisch lösliche, lipophile Substanzen als Ersatz für die der Haut mit dem Äthanol entzogenen Fettsubstanzen, und, falls notwendig, andere Hilfs- und Zusatzmittel beigegeben sind.

Die Herstellung der topisch verwendbaren pharmazeutischen Präparate erfolgt in an sich bekannter Weise, z.B. durch Lösen oder Suspendieren des Wirkstoffs in der Grundlage oder in einem Teil davon, falls notwendig. Bei Verarbeitung des Wirkstoffs als Lösung wird dieser in der Regel vor der Emulgierung in einer der beiden Phasen gelöst; bei Verarbeitung als Suspension wird er nach der Emulgierung mit einem Teil der Grundlage vermischt und dann dem Rest der Formulierung beigegeben.

Die nachfolgenden Beispiele illustrieren die oben beschriebene Erfindung, sie sollen jedoch diese in ihrem Umfang in keiner Weise einschränken. Temperaturen sind in Celsiusgraden angegeben.

Beispiel 1:

Ein Gemisch aus 13,9 g 1-Phenyl-2-(3-pyridyl)-glyoxal, 9,5 g α-Formyl-α,α-dimethyl-essigsäureethylester [A.J. Meyers et al., J. Org. Chem. 38 (1) 41 (1973)], 35,6 g Ammoniumacetat und 100 ml Eisessig wird eine Stunde unter Rückfluss gekocht und anschliessend unter kräftigem Rühren in ein Gemisch aus 200 g Eis und 145 ml konzentrierter wässriger Ammoniaklösung gegossen. Der Kristallbrei wird zweimal mit je 150 ml Ethylacetat extrahiert und die organische Phase mit Wasser neutral gewaschen, mit Magnesiumsulfat getrocknet und unter 1466,5 Pa bei 40° zur Trockne eingedampft. Der Rückstand wird aus Ether umkristallisiert. Man erhält den 2-[4(5)-Phenyl-5-(4)-(3-pyridyl)-imidazol-2-yl]-2-methyl-propionsäureethylester als weisse Kristalle vom Smp. 134–136°.

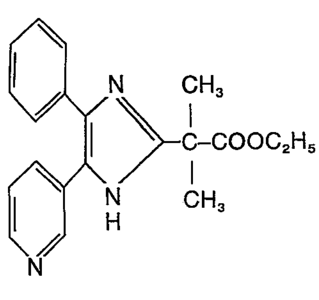

In analoger Weise kann man herstellen:
2-[4(5)-Phenyl-5(4)-(3-pyridyl)-imidazol-2-yl]-2-allyl-essigsäureethylester, Öl, ausgehend von 1-Phenyl-2-(3-pyridyl)-glyoxal und α-Formyl-α-allyl-essigsäureethylester.
1-[4(5)-Phenyl-5(4)-(3-pyridyl)-imidazol-2-yl]-1-carbethoxy-cyclopentan, Smp. 115–117°, ausgehend von 1-Phenyl-2-(3-pyridyl)-glyoxal und 1-Formyl-1-carbethoxy-cyclopentan.
2-[4(5)-(p-Methoxyphenyl)-5(4)-(3-pyridyl)-imidazol-2-yl]-2-methyl-propionsäureethylester, Smp. 126–128°, ausgehend von 1-(p-Methoxyphenyl)-2-(3-pyridyl)-glyoxal und α-Formyl-α,α-dimethyl-essigsäureethylester.

Beispiel 2:

In eine Lösung von 5 g α-[4-Phenyl-5-(3-pyridyl)-3-oxidoimidazol-2-yl]-2-methyl-propionsäureethylester in 50 ml Methylenchlorid wird 0,5 g Palladiumkohle gegeben und anschliessend unter Rühren Wasserstoff eingeleitet. Das Reaktionsgemisch wird filtriert und bei 1466,5 Pa zur Trockene eingedampft. Der Rückstand wird mit Ethylacetat extrahiert und mit gesättigter Natriumchlorid-Lösung gewaschen. Nach Trocknen über Natriumsulfat und Eindampfen unter vermindertem Druck kristallisiert man den Rückstand aus Ether. Man erhält den 2-[4(5)-Phenyl-5(4)-(3-pyridyl)-imidazol-2-yl]-2-methyl-propionsäureethylester vom Smp. 134–136°.

Das Ausgangsmaterial kann wie folgt hergestellt werden:

Eine Mischung aus 6,0 g α-Hydroximinobenzyl-(3-pyridyl)-keton, 6,5 g 2-(2,2-Dimethyl-carbethoxymethyl)-4,4,6-trimethyl-2,3,5,6-tetrahydro-1,3-oxazin, 5,3 g Ammoniumacetat und 26,5 ml Eisessig wird 2 Stunden unter Rückfluss gekocht. Man kühlt ab und giesst auf ein Gemisch von 50 ml konzentriertem wässrigem Ammoniak und 100 g Eis. Die Suspension wird filtriert. Man löst die Kristalle in 100 ml Ethylacetat und extrahiert die organische Phase zweimal mit 20 ml Wasser, trocknet über Magnesiumsulfat und engt unter vermindertem Druck zur Trockne ein. Den Rückstand kristallisiert man aus Ethanol. Der 2-[4-Phenyl-5-(3-pyridyl)-3-oxido-imidazol-2-yl]-2-methyl-propionsäureethylester schmilzt bei 200–204°.

Beispiel 3:

Ein Gemisch aus 7,0 g 1-Phenyl-2-(1-oxido-3-pyridyl)-glyoxal, 4,5 g α-Formyl-α,α-dimethyl-essigsäureethylester, 17,8 g Ammoniumacetat und 50 ml Eisessig wird eine Stunde unter Rückfluss gekocht und anschliessend unter Rühren in ein Gemisch aus 100 g Eis und 70 ml konzentrierter wässriger Ammoniaklösung gegossen. Das ausgeschiedene Öl wird zweimal mit je 70 ml Ethylacetat extrahiert und die organische Phase mit Wasser neutral gewaschen, über Magnesiumsulfat getrocknet und

unter 1466,5 Pa bei 40° zur Trockene eingedampft. Der Rückstand wird aus Methanol-Wasser umkristallisiert. Der
2-[4(5)-Phenyl-5(4)-(1-oxido-3-pyridyl)-imidazol-2-yl]-2-methyl-propionsäureethylester
schmilzt bei 82–85° (als Monohydrat).

Beispiel 4:

Eine Mischung aus 38,9 g 1-Phenyl-2-(3-pyridyl)-glyoxal, 44,6 g 2-(2,2-Dimethyl-carbethoxymethyl)-4,4,6-trimethyl-2,3,5,6-tetrahydro-1,3-oxazin, 99,1 g Ammoniumacetat und 275 ml Eisessig wird drei Stunden unter Einleiten von Stickstoff unter Rückfluss erhitzt, abgekühlt und unter Rühren in eine Mischung aus 900 g Eis und 550 ml konzentrierter wässriger Ammoniaklösung gegossen. Die Suspension wird zweimal mit je 700 ml Ethylacetat extrahiert und die organische Phase mit 500 ml Wasser gewaschen, über Magnesiumsulfat getrocknet und bei 40° unter vermindertem Druck zur Trockene eingedampft. Der Rückstand wird aus Ether kristallisiert. Der
2-[4(5)-Phenyl-5(4)-(3-pyridyl)-imidazol-2-yl]-2-methyl-propionsäureethylester
schmilzt bei 134–136°.

In analoger Weise kann man herstellen:

2-[4(5)-Phenyl-5(4)-(3-pyridyl)-imidazol-2-yl]-propionsäureethylesterhemihydrat, Öl, ausgehend von 1-Phenyl-2-(3-pyridyl)-glyoxal und 2-(2-Methylcarbethoxymethyl)-4,4,6-trimethyl-2,3,5,6-tetrahydro-1,3-oxazin.

2-[4(5)-Phenyl-5(4)-(3-pyridyl)-imidazol-2-yl]-essigsäureethylester, gelbes Öl, ausgehend von 1-Phenyl-2-(3-pyridyl)-glyoxal und 2-(Carbethoxymethyl)-4,4,6-trimethyl-2,3,5,6-tetrahydro-1,3-oxazin.

2-[4(5)-Phenyl-5(4)-(3-pyridyl)-imidazol-2-yl]-2-allyl-essigsäureethylester, Smp. 106–108° (aus Ethylacetat-Ether), ausgehend von 1-Phenyl-2-(3-pyridyl)-glyoxal und 2-(2-Allyl-carbethoxymethyl)-4,4,6-trimethyl-2,3,5,6-tetrahydro-1,3-oxazin.

1-[4(5)-Phenyl-5(4)-(3-pyridyl)-imidazol-2-yl]-1-carbethoxy-cyclopentan, Smp. 115–117°, ausgehend von 1-Phenyl-2-(3-pyridyl)-glyoxal und 1-Carbethoxy-1-(4,4,6-trimethyl-2,3,5,6-tetrahydro-1,3-oxazin-2-yl)-cyclopentan.

2-[4(5)-(p-Methoxyphenyl)-5(4)-(3-pyridyl)-imidazol-2-yl]-2-methylpropionsäureethylester, Smp. 125–128° (aus Ether), ausgehend von 1-(p-Methoxyphenyl)-2-(3-pyridyl)-glyoxal und 2-(2,2-Dimethyl-carbethoxymethyl)-4,4,6-trimethyl-2,3,5,6-tetrahydro-1,3-oxazin.

2-[4(5)-(m-Methoxyphenyl)-5(4)-(3-pyridyl)-imidazol-2-yl]-2-methylpropionsäureethylester, Smp. 135–137° (aus Ethylacetat-Ether), ausgehend von 1-(m-Methoxyphenyl)-2-(3-pyridyl)-glyoxal und 2-(2,2-Dimethyl-carbethoxymethyl)-4,4,6-trimethyl-2,3,5,6-tetrahydro-1,3-oxazin.

2-[4(5)-(3,4-Dimethoxyphenyl)-5(4)-(3-pyridyl)-imidazol-2-yl]-2-methylpropionsäureethylester, Smp. 144–146° (aus Ethylacetat-Ether), ausgehend von 1-(3,4-Dimethoxyphenyl)-2-(3-pyridyl)-glyoxal und 2-(2,2-Dimethyl-carbethoxymethyl)-4,4,6-trimethyl-2,3,5,6-tetrahydro-1,3-oxazin.

2-[4(5)-(p-Chlorphenyl)-5(4)-(3-pyridyl)-imidazol-2-yl]-2-methyl-propionsäureethylester, Smp. 161–163° (aus Methylenchlorid/n-Hexan), ausgehend von 1-(p-Chlorphenyl)-2-(3-pyridyl)-glyoxal und 2-(2,2-Dimethyl-carbethoxymethyl)-4,4,6-trimethyl-2,3,5,6-tetrahydro-1,3-oxazin.

2-[4(5)-Phenyl-5(4)-(4-pyridyl)-imidazol-2-yl]-2-methyl-propionsäureethylester, Smp. 210–212° (aus Ethylacetat-Ether), ausgehend von 1-Phenyl-2-(4-pyridyl)-glyoxal und 2-(2,2-Dimethyl-carbethoxymethyl)-4,4,6-trimethyl-2,3,5,6-tetrahydro-1,3-oxazin.

Beispiel 5:

Eine Mischung von 7,14 g α-Brom-(3-pyridyl)-benzylketon-hydrobromid und 19,56 g Methylmalonsäuremonoethylester-Ammoniumsalz in 30 ml wasserfreiem Dimethylformamid wird unter Rühren und Einleiten von Stickstoff während 5 Stunden auf 100° erhitzt. Dann kühlt man ab und engt unter 1466,5 Pa bei einer Badtemperatur von 70° zur Trockene ein. Den Rückstand versetzt man mit 300 ml Ethylacetat und 200 ml Wasser. Die Mischung wird mit konzentrierter wässriger Ammoniaklösung auf pH 8–9 gestellt. Die organische Phase wird abgetrennt, zweimal mit je 50 ml Wasser gewaschen, über Magnesiumsulfat getrocknet und unter 1466,5 Pa zur Trockene eingedampft. Den Rückstand chromatographiert man an 100 g Silikagel. Die Fraktionen 1–8, eluiert mit je 600 ml Chloroform, werden verworfen. Die Fraktionen 9–16, eluiert mit je 600 ml Chloroform-Methanol (99:1), werden vereinigt und unter 1466,5 Pa zur Trockene eingedampft. Der Rückstand, der
2-[4(5)-Phenyl-5(4)-(3-pyridyl)-imidazol-2-yl]-propionsäureethylester,
liegt als gelbes Öl vor.

Analog kann hergestellt werden:

2-[4(5)-Phenyl-5(4)-(1-oxido-3-pyridyl)-imidazol-2-yl]-2-methyl-buttersäureethylester, ausgehend von α-Brom-(1-oxido-3-pyridyl)-benzyl-keton und 2-Ethyl-2-methyl-malonsäuremonoethylester-Ammoniumsalz.

2-[4(5)-Phenyl-5(4)-(3-pyridyl)-imidazol-2-yl]-2-methyl-propionsäureethylester, Smp. 134–136° (aus Ether), ausgehend von α-Brom-(3-pyridyl)-benzyl-keton und Dimethylmalonsäuremonoethylester-Ammoniumsalz.

Der Ausgangsstoff wird wie folgt hergestellt:

Eine Lösung von 42,5 g Benzyl-(3-pyridyl)-keton in 400 ml Ethylenchlorid wird auf 50° erwärmt. Bei

dieser Temperatur wird eine Lösung von 36,2 g Brom in 30 ml Ethylenchlorid zugetropft. Die Suspension wird 15 Stunden bei 50° gerührt, dann abgekühlt und filtriert. Die abfiltrierten Kristalle werden dreimal mit je 30 ml Ethylenchlorid gewaschen und unter 13,3 Pa bei 50° getrocknet. Das α-Brom-benzyl-(3-pyridyl)-keton-hydrobromid schmilzt bei 218–219,5°.

Beispiel 6:

46,0 g N-[4-Methoxy-α-(p-methoxyphenyl)-phenacyl]-malonsäuremonoethylester-amid werden mit 70,1 g Ammoniumacetat in 400 ml Eisessig zwei Stunden unter Rückfluss gekocht. Dann wird die Lösung auf 800 ml konzentriertes Ammoniak und 900 g Eis gegossen und mit Ethylacetat extrahiert. Die organische Phase wird abgetrennt, mit gesättigter Natriumchloridlösung neutral gewaschen, über Magnesiumsulfat getrocknet und unter vermindertem Druck bei 40° eingedampft. Der Rückstand wird in 500 ml einer Ether-Ethylacetatmischung (9:1) gelöst. Die Lösung wird durch eine Schicht Kieselgel filtriert. Das Filtrat wird unter vermindertem Druck bei 40° eingeengt. Den Rückstand kristallisiert man aus Ethylacetat-Ether. Der 2-[4,5-Bis-(p-methoxyphenyl)-imidazol-2-yl]-essigsäureethylester schmilzt bei 131–132°.

In analoger Weise kann man herstellen:

2-[4,5-Bis-(p-methoxyphenyl)-imidazol-2-yl]-2-methyl-propionsäureethylester,
Öl, ausgehend von
N-[4-Methoxy-α-(p-methoxyphenyl)-phenacyl]-dimethyl-malonsäuremonoethylester-amid.

2-[4(5)-Phenyl-5(4)-(3-pyridyl)-imidazol-2-yl]-2-methyl-propionsäureethylester,
Smp. 134–136° (aus Ether), ausgehend von N-[α-(3-Pyridylcarbonyl)-benzyl]-dimethylmalonsäure-monoethylester-amid.

Das Ausgangsmaterial kann wie folgt hergestellt werden:

Eine Suspension von 17,8 g 2-Amino-4′-methoxy-2-(p-methoxyphenyl)-acetophenon-hydrochlorid in 150 ml wasserfreiem Benzol wird unter Rühren mit 18 ml Triethylamin versetzt. Anschliessend werden 9,6 g Malonsäuremonoethylester-chlorid unter Eiskühlung innerhalb 15 Minuten derart zugetropft, dass die Innentemperatur 20° nicht übersteigt. Nach weiteren 10 Minuten werden nochmals 9 ml Triethylamin zugesetzt. Die Suspension wird 16 Stunden bei 20–25° gerührt, anschliessend mit Wasser versetzt und mit Ethylacetat verdünnt. Die organische Phase wird abgetrennt, mit 2 N Natriumcarbonatlösung, gesättigter Natriumchloridlösung und nochmals mit Wasser gewaschen, über Magnesiumsulfat getrocknet und unter vermindertem Druck bei 40° zur Trockene eingedampft. Den Rückstand kristallisiert man aus Ethylacetat-Ether. Das
N-[4-Methoxy-α-(p-methoxyphenyl)-phenacyl]-malonsäuremonoethylester-amid
schmilzt bei 96–97°.

In analoger Weise kann man herstellen:

N-[4-Methoxy-α-(p-methoxyphenyl)-phenacyl]-dimethyl-malonsäuremonoethylester-amid,
ausgehend von
2-Amino-4′-methoxy-2-(p-methoxyphenyl)-acetophenon-hydrochlorid
und Dimethylmalonsäuremonoethylester-chlorid.

N-[α-(3-Pyridyl-carbonyl)-benzyl]-dimethyl-malonsäuremonoethylester-amid,
Öl, ausgehend von
α-Amino-benzyl-(3-pyridyl)-keton und Dimethyl-malonsäuremonoethylester-chlorid.

α-Amino-benzyl-(3-pyridyl)-keton kann auf folgende Weise hergestellt werden:

10,8 g Benzyl-(3-pyridyl)-keton werden zusammen mit 40 ml Pyridin und einer Lösung von 8 g Hydroxylaminhydrochlorid in 15 ml Pyridin 6 Stunden bei 100° gerührt. Das Reaktionsgemisch wird auf Eis/Wasser gegossen und 15 Minuten nachgerührt. Die ausgefallenen Kristalle werden abgenutscht, mit Wasser gewaschen und im Hochvakuum getrocknet. Man erhält das Benzyl-(3-pyridyl)-keton-oxim vom Smp. 122–126°.

Zu einer bei −10° gerührten Lösung von 8,5 g Benzyl-(3-pyridyl)-keton-oxim in 20 ml Pyridin wird innerhalb von 5 Minuten eine Lösung von 7,7 g p-Toluolsulfochlorid in 15 ml Pyridin zugetropft. Das Reaktionsgemisch wird 24 Stunden im Eisschrank aufbewahrt und dann auf Eis/Wasser gegossen. Nach einigem Rühren und Verreiben erstarrt das ausgefallene Öl zu Kristallen. Diese werden abgenutscht, mit Wasser gewaschen und im Hochvakuum getrocknet. Man erhält den Benzyl-(3-pyridyl)-keton-oxim-p-toluolsulfonsäureester, der ohne weitere Reinigung in der nächsten Stufe eingesetzt wird.

11,6 g roher Benzyl-(3-pyridyl)-keton-oxim-p-toluolsulfoester werden in 90 ml absolutem Äthanol suspendiert. Dann werden bei 0° unter Rühren die Lösung von 3,7 g Kalium-tert.-butylat in 30 ml absolutem Äthanol zugetropft. Das Reaktionsgemisch wird 2 Stunden bei 0° gerührt. Die Suspension wird abgenutscht und das Filtrat, welches das gewünschte α-Amino-benzyl-(3-pyridyl)-keton enthält, sofort in der nächsten Stufe weiter umgesetzt.

Beispiel 7:

1,7 g 2-[1-Benzyl-4(5)-phenyl-5(4)-(3-pyridyl)-imidazol-2-yl)-2-methyl-propionsäureethylester
werden in 40 ml Methylenchlorid gelöst und nach Zusatz von 0,6 g Palladium-Kohle bei Raumtemperatur hydriert. Nach beendeter Wasserstoffaufnahme filtriert man vom Katalysator ab und engt das Filtrat unter 1466,5 Pa bei 40° zur Trockne ein. Den Rückstand kristallisiert man aus Ether. Der
2-[4(5)-Phenyl-5(4)-(3-pyridyl)-imidazol-2-yl]-2-methyl-propionsäureethylester
schmilzt bei 134–136°.

In analoger Weise kann man herstellen:

Natriumsalz der
2-[4(5)-Phenyl-5(4)-(3-pyridyl)-imidazol-2-yl]-2-methyl-propionsäure,

als Hydrat, Smp. 273–276°, ausgehend von
2-[1-Benzyl-4(5)-phenyl-5(4)-(3-pyridyl)-imidazol-2-yl)-2-methyl-propionsäure.

Die Ausgangsstoffe können wie folgt hergestellt werden:

Eine Mischung von 3,5 g α-Brom-(3-pyridyl)-benzylketon-hydrobromid und 9,68 g 3-Ethoxypivalinsäure-ammoniumsalz in 25 ml wasserfreiem Dimethylformamid wird unter Rühren und Einleiten von Stickstoff während 6 Stunden auf 100° erhitzt. Die Mischung wird abgekühlt und unter 1466,5 Pa bei einer Badtemperatur von 60° zur Trockene eingedampft. Der Rückstand wird mit 140 ml Ethylacetat und 100 ml Wasser versetzt. Der pH der Mischung wird mit konzentrierter wässriger Ammoniaklösung auf 8–9 gestellt. Die Ethylacetatlösung wird abgetrennt, zweimal mit je 30 ml Wasser gewaschen, über Magnesiumsulfat getrocknet und unter 1466,5 Pa zur Trockene eingedampft. Den Rückstand chromatographiert man an 60 g Silikagel. Die Fraktionen 1–4, eluiert mit je 250 ml Chloroform, werden verworfen. Die Fraktionen 5–12, eluiert mit je 250 ml Chloroform-Methanol (99:2), werden vereinigt und unter 1466,5 Pa zur Trockene eingedampft. Der Rückstand, das
2-[4(5)-Phenyl-5(4)-(3-pyridyl)-imidazol-2-yl]-2-methyl-1-ethoxypropan,
liegt als Öl vor.

Eine Lösung von 1,5 g
2-[4(5)-Phenyl-5(4)-(3-pyridyl)-imidazol-2-yl]-2-methyl-1-ethoxy-propan
in 15 ml wasserfreiem Dimethylformamid wird bei 0° mit 0,25 g Natriumhydrid-Mineralöldispersion versetzt. Die Mischung wird unter Einleiten von Stickstoff während einer Stunde bei Raumtemperatur gerührt und anschliessend tropfenweise mit einer Lösung von 0,65 ml Benzylbromid in 7,0 g wasserfreiem Dimethylformamid versetzt. Man rührt während 30 Minuten bei Raumtemperatur und giesst die Mischung auf 100 ml Eiswasser. Das ausgeschiedene Öl wird dreimal mit je 80 ml Ethylacetat extrahiert. Die vereinigten organischen Phasen werden mit Wasser gewaschen, über Magnesiumsulfat getrocknet und unter 1466,5 Pa bei Raumtemperatur zur Trockene eingedampft. Den Rückstand chromatographiert man an 40 g Silikagel. Die Fraktionen 1–5 eluiert mit je 100 ml Chloroform werden verworfen. Die Fraktionen 6–10, eluiert mit je 100 ml Chloroform-Methanol (99:1), enthalten das
2-[1-Benzyl-4(5)-phenyl-5(4)-(3-pyridyl)-imidazol-2-yl]-2-methyl-1-ethoxy-propan.
Sie werden vereinigt und unter 1466,5 Pa bei Raumtemperatur zur Trockene eingedampft. Der Rückstand liegt als Öl vor.

Eine Lösung von 1,3 g
2-[1-Benzyl-4(5)-phenyl-5(4)-(3-pyridyl)-imidazol-2-yl]-2-methyl-1-ethoxy-propan
in 30 ml Aceton und 10 ml Wasser wird bei Raumtemperatur und unter schnellem Rühren portionenweise mit Kaliumpermanganat versetzt, bis keine Entfärbung mehr eintritt. Die Mischung wird während 10 Stunden bei Raumtemperatur gerührt und filtriert. Man engt das Filtrat unter 1466,5 Pa bei 50° zur Trockene ein. Den Rückstand versetzt man mit 10 ml Eiswasser und extrahiert das ausgeschiedene Öl mit Ethylacetat. Die organische Phase wird mit gesättigter Natriumchloridlösung gewaschen. Nach Trocknen über Natriumsulfat und Filtrieren durch eine Schicht Silikagel engt man das Filtrat unter 1466,5 Pa bei Raumtemperatur zur Trockene ein. Den Rückstand reibt man mit Ether an. Der 2-[1-Benzyl-4(5)-phenyl-5(4)-(3-pyridyl)-imidazol-2-yl)-2-methyl-propionsäureäthylester liegt nach Trocknen unter 13,3 Pa bei Raumtemperatur als fester Schaum vor.

Beispiel 8:

Eine Lösung von
3,32 g 2-[4(5)-Phenyl-5(4)-(3-pyridyl)imidazol-2-yl]-2-methyl-propionsäureethylester
in 20 ml Methanol wird mit 40 ml 0,5 N Natronlauge versetzt. Die Lösung wird vier Stunden bei Raumtemperatur gerührt und unter vermindertem Druck bei 40° eingedampft. Man versetzt den Rückstand mit 50 ml Methylenchlorid und filtriert die gelblichen Kristalle ab. Das Natriumsalz der
2-[4(5)-Phenyl-5(4)-(3-pyridyl)-imidazol-2-yl]-2-methyl-propionsäure
(als Hydrat) schmilzt bei 273–276°.

In analoger Weise kann man herstellen:
2-[4,5-Bis-(p-methoxyphenyl)-imidazol-2-yl]-essigsäure-Natriumsalz-monohydrat,
Smp. 187–190°, ausgehend von
2-[4,5-Bis-(p-methoxyphenyl)-imidazol-2-yl]-essigsäureethylester.

Beispiel 9:

Eine Lösung von
0,9 g 2-[4(5)-Phenyl-5(4)-(1-oxido-3-pyridyl-imidazol-2-yl]-2-methyl-propionsäureethylester
in 10 ml Methanol wird unter Rühren mit 3,0 ml 1 N Natronlauge versetzt. Die Lösung wird 15 Stunden bei Raumtemperatur gerührt und unter 1466,5 Pa bei 40° vom Methanol befreit. Den Rückstand versetzt man mit 20 ml Wasser und extrahiert die gelbe Lösung mit 20 ml Chloroform. Dann wird die wässrige Phase abgetrennt und bei 0° mit 2 N Salzsäure angesäuert. Die klare Lösung wird nochmals mit 10 ml Chloroform extrahiert. Anschliessend wird die wässrige Phase unter 1466,5 Pa bei 40° zur Trockene eingedampft. Den weissen kristallinen Rückstand trocknet man unter 13,3 Pa bei Raumtemperatur während 20 Stunden. Die
2-[4(5)-Phenyl-5(4)-(1-oxido-3-pyridyl)-imidazol-2-yl]-2-methyl-propionsäure
schmilzt bei 178–180°.

Beispiel 10:

Eine Lösung von
5,9 g 2-[4(5)-Phenyl-5(4)-(3-pyridyl)-imidazol-2-yl]-2-methyl-propionsäureethylester
in 120 ml Methylenchlorid wird auf 0–5° abgekühlt und mit 3,5 g m-Chlorperbenzoesäure versetzt. Die Mischung wird 24 Stunden bei Raumtemperatur gerührt. Die gelbe Lösung wird anschliessend

zweimal mit je 20 ml 2 N Kaliumhydrogenkarbonatlösung und mit 30 ml Wasser gewaschen, über Magnesiumsulfat getrocknet und bei 40° unter vermindertem Druck eingeengt. Der Rückstand wird in wenig Methanol gelöst. Nach Zusatz von Wasser kristallisiert der

2-[4(5)-Phenyl-5(4)-(1-oxido-3-pyridyl)-imidazol-2-yl]-2-methyl-propionsäure-ethylester

als Monohydrat. Smp. 82–85°.

In analoger Weise kann man herstellen:

2-[4(5)-Phenyl-5(4)-(1-oxido-3-pyridyl)-imidazol-2-yl]-2-methyl-propionsäuremethylester Monohydrat,
Smp. 96–98° (aus Methanol/Wasser).

2-[4(5)-Phenyl-5(4)-(1-oxido-4-pyridyl)-imidazol-2-yl]-2-methyl-propionsäure-ethylester,
Smp. 164–166° (aus Ethylacetat), ausgehend von
2-[4-(5)-phenyl-5(4)-(4-pyridyl)-imidazol-2-yl]-2-methyl-propionsäureethylester.

2-[4(5)-Phenyl-5(4)-(1-oxido-3-pyridyl)-imidazol-2-yl]-propionsäureethylester-hemihydrat,
Öl, ausgehend von
2-[4(5)-Phenyl-5(4)-(3-pyridyl)-imidazol-2-yl]-propionsäureethylester.

1-[4(5)-Phenyl-5(4)-(1-oxido-3-pyridyl)-imidazol-2-yl]-1-carbethoxy-cyclopentan,
Öl, ausgehend von
1-[4(5)-Phenyl-5(4)-(3-pyridyl)-imidazol-2-yl]-1-carbethoxy-cyclopentan.

2-[4(5)-(p-Chlorphenyl)-5(4)-(1-oxido-3-pyridyl)-imidazol-2-yl]-2-methyl-propionsäureethylester,
Smp. 137–140° (aus Methylenchlorid-Petrolether), ausgehend von
2-[4(5)-(p-Chlorphenyl)-5(4)-(3-pyridyl)-imidazol-2-yl]-2-methyl-propionsäureethylester.

Beispiel 11:

Eine Lösung von

1,3 g 2-[4(5)-(p-Methoxyphenyl)-5(4)-(3-pyridyl)-imidazol-2-yl]-2-methyl-propionsäure-ethylester

in 50 ml Methylenchlorid wird in einer Stickstoffatmosphäre unter Rühren bei −70° mit einer Lösung von 4,7 g Bortribromid in 20 ml Methylenchlorid tropfenweise innerhalb 3 Minuten versetzt. Die Mischung wird 30 Minuten bei −70° gerührt. Dann wird das Kühlbad entfernt und solange gerührt, bis die Innentemperatur 25° erreicht hat. Dann wird die weisse Suspension auf 50 ml eines Eis-Wassergemisches gegossen und gerührt. Die wässrige Phase wird abgetrennt, zweimal mit 20 ml Methylenchlorid extrahiert und mit 2 N Natriumkarbonatlösung auf pH 8 gestellt. Die ausgeschiedenen Kristalle werden zweimal mit je 30 ml Ethylacetat extrahiert. Die vereinigten Ethylacetatphasen werden über Magnesiumsulfat getrocknet und unter vermindertem Druck zur Trockene eingedampft. Den Rückstand kristallisiert man aus Ethylacetat-Ether. Der

2-[4(5)-(p-Hydroxyphenyl)-5(4)-(3-pyridyl)-imidazol-2-yl]-2-methyl-propionsäureethylester schmilzt bei 186–187°.

Beispiel 12:

100 ml mit Salzsäuregas gesättigtes wasserfreies Methanol wird mit

5,0 g 2-[4,5-Bis-(p-methoxyphenyl)-imidazol-2-yl]-propionsäure

versetzt. Die Mischung wird 15 Stunden unter Rückfluss erhitzt, abgekühlt und unter vermindertem Druck zur Trockne eingedampft. Den Rückstand versetzt man mit 10 ml Wasser und stellt mit wässriger konzentrierter Ammoniaklösung alkalisch. Man extrahiert zweimal mit 40 ml Ethylacetat und wäscht die organische Phase bei 5° mit 20 ml 2 N Kaliumbikarbonatlösung und 20 ml Wasser, trocknet sie über Magnesiumsulfat und engt sie unter vermindertem Druck zur Trockene ein. Der

2-[4,5-Bis-(p-methoxyphenyl)-imidazol-2-yl]-propionsäuremethylester

kristallisiert aus Ether-Petrolether.

Beispiel 13:

Eine Lösung von 3,52 g Kaliumsalz der

2-[4,5-Bis-(p-methoxyphenyl)-imidazol-2-yl]-propionsäure

in 30 ml wasserfreiem Dimethylformamid wird bei 80° unter Rühren mit 3,0 g Diethylsulfat versetzt. Die Mischung wird 15 Minuten bei 80° gerührt, abgekühlt und auf Eis-Wasser gegossen. Das ausgeschiedene Öl wird in Ethylacetat gelöst und die organische Phase zweimal mit 2 N Kaliumbikarbonatlösung extrahiert, über Magnesiumsulfat getrocknet und unter vermindertem Druck zur Trockene eingedampft. Der

2-[4,5-Bis-(p-methoxyphenyl)-imidazol-2-yl]-propionsäureethylester

wird aus Ether-Petrolether kristallisiert.

In analoger Weise kann man herstellen:

2-[4(5)-Phenyl-5(4)-(3-pyridyl)-imidazol-2-yl]-2-methylpropionsäuremethylester,
Smp. 158–162° (aus Ether).

Beispiel 14:

Eine Suspension von

3,5 g 2-[4(5)-Phenyl-5(4)-(3-pyridyl)-imidazol-2-yl]-2-methyl-propionsäure-Natriumsalz-monohydrat

in 50 ml wasserfreiem Dimethylformamid wird unter Einleiten von Stickstoff und Rühren bei Raumtemperatur tropfenweise mit 2,5 g Pivaloyloxymethyliodid versetzt. Die Mischung wird 15 Stunden bei Raumtemperatur gerührt und anschliessend unter 1466,5 Pa zur Trockene eingedampft. Den Rückstand verteilt man zwischen 20 ml Wasser und 50 ml Ethylacetat. Die organische Phase wird abgetrennt, über Magnesiumsulfat getrocknet und unter 1466,5 Pa zur Trockene eingeengt. Den Rückstand chromatographiert man an 300 g Silikagel. Die Fraktionen 1–15, eluiert mit je 300 ml Chloroform-Ethylacetat (95:5), werden verworfen. Die Fraktionen 16–26, eluiert mit je 300 ml Chloroform-Ethylacetat (80:20), werden vereinigt und unter 1466,5 Pa zur Trockene eingedampft. Den Rückstand kristallisiert man aus Ether-Petrolether. Der

2-[4(5)-Phenyl-5(4)-(3-pyridyl)-imidazol-2-yl]-2-methyl-propionsäure-pivaloyloxymethylester

schmilzt bei 143–145°.

Beispiel 15:

Eine Lösung von

5,0 g 2-[4,5-Bis-(p-methoxyphenyl)-imidazol-2-yl]-propionsäureamid

und 5,0 g Kaliumhydroxid in 100 ml n-Butanol wird 8 Stunden unter Rückfluss erhitzt. Dann wird abgekühlt und unter 13,3 Pa bei 50° zur Trockene eingedampft. Den Rückstand löst man in 200 ml Wasser. Die Lösung wird filtriert und das Filtrat mit konzentrierter Salzsäure angesäuert. Die ausgeschiedenen Kristalle,

2-[4,5-Bis-(p-methoxyphenyl)-imidazol-2-yl]-propionsäure,

werden abfiltriert.

In analoger Weise kann man herstellen:

2-[4,5-Bis-(p-methoxyphenyl)-imidazol-2-yl]-essigsäure,

ausgehend von

2-[4,5-Bis-(p-methoxyphenyl)-imidazol-2-yl]-essigsäureamid.

Das Ausgangsmaterial kann wie folgt erhalten werden:

Zu einer Lösung von

10 g 2-[1-Benzyl-4,5-bis-(p-methoxyphenyl)-imidazol-2-yl]-propionsäureethylester

in 150 ml flüssigem Ammoniak wird solange portionenweise Natrium zugesetzt, bis die Blaufärbung der Lösung bestehen bleibt. Anschliessend wird noch 45 Minuten weitergerührt und der Natriumamid-Überschuss durch Zugabe von Ammoniumchlorid zersetzt. Man entfernt das Kühlbad und lässt den Ammoniak abdampfen. Den festen Rückstand versetzt man mit 100 ml Eis-Wasser und filtriert das kristalline

2-[4,5-Bis-(p-methoxyphenyl)-imidazol-2-yl]-propionsäureamid

ab.

11,1 g 2-[4,5-Bis-(p-methoxyphenyl)-oxazol-2-yl]-essigsäureethylester

wird mit 97,0 g flüssigem Ammoniak und 64 g Formamid im Autoklav während 5 Stunden auf 200° erhitzt. (Der Druck beträgt $1{,}8 \cdot 10^7$ Pa). Man kühlt ab und giesst das Reaktionsgemisch auf 300 ml Wasser. Das ausgeschiedene Öl wird mit 150 ml Ethylacetat extrahiert. Die organische Phase wird abgetrennt, mit 30 ml gesättigter Kochsalzlösung und Wasser gewaschen, über Magnesiumsulfat getrocknet und unter vermindertem Druck zur Trockene eingedampft. Das

2-(4,5-Bis-(p-methoxyphenyl)-imidazol-2-yl]-essigsäureamid

wird aus Methanol kristallisiert.

Beispiel 16:

In eine Lösung aus

1,0 g 2-[4(5)-Phenyl-5(4)-(3-pyridyl)-imidazol-2-yl]-2-methyl-propionitril

in 15 ml wasserfreiem Ether und 20 ml wasserfreiem Ethanol wird bei 0° Salzsäuregas eingeleitet. Nach 3 Stunden wird die Temperatur auf 20° erhöht und eine weitere Stunde Salzsäuregas eingeleitet. Man lässt die Mischung 15 Stunden stehen und engt sie dann unter vermindertem Druck zur Trockene ein. Den Rückstand versetzt man mit 10 ml Wasser und 20 ml Ether und erwärmt die Mischung während 2½ Stunden bei 40°. Dann kühlt man ab, setzt sowie 2 N Natriumhydroxidlösung zu, bis der pH der Mischung 7,5 beträgt. Man trennt die organische Phase ab, wäscht sie mit Wasser, trocknet sie über Magnesiumsulfat und engt sie unter 1466,5 Pa bei 40° zur Trockene ein. Der Rückstand wird aus Ether kristallisiert. Der

2-[4(5)-Phenyl-5(4)-(3-pyridyl)-imidazol-2-yl]-2-methyl-propionsäureethylester

schmilzt bei 134–136°.

Das Ausgangsmaterial kann wie folgt hergestellt werden:

Eine Mischung von 3,8 g α-Brom-(3-pyridyl)-benzylketon-hydrobromid und 10 g 2-Cyano-2-methyl-propionsäure-Ammoniumsalz in 20 ml wasserfreiem Dimethylformamid wird unter Rühren und Einleiten von Stickstoff 6 Stunden bei 100° erhitzt. Nach dem Abkühlen engt man unter 1466,5 Pa bei einer Temperatur von 70° zur Trockene ein. Der Rückstand wird mit 200 ml Ethylacetat und 150 ml Wasser versetzt. Anschliessend wird das Reaktionsgemisch mit konzentrierter wässriger Ammoniak-Lösung auf pH 8–9 gestellt. Die organische Phase wird abgetrennt, zweimal mit je 40 ml Wasser gewaschen, über Magnesiumsulfat getrocknet und unter 1466,5 Pa bei Raumtemperatur zu Trockene eingedampft. Den Rückstand chromatographiert man an 100 g Silikagel. Die ersten 8 Fraktionen, eluiert mit je 600 ml Chloroform, werden verworfen, und die Fraktionen 9–18, eluiert mit Chloroform-Methanol (99:1), werden vereinigt und unter 1466,5 Pa zur Trockene eingedampft. Man erhält das

2-[4(5)-Phenyl-5(4)-(3-pyridyl)-imidazol-2-yl]-2-methyl-propionitril,

welches ohne weitere Reinigung weiter umgesetzt wird.

Beispiel 17:

Eine Lösung von

2,0 g 2-[4(5)-Phenyl-5(4)-(3-pyridyl)-imidazol-2-yl]-acetaldehyd-dimethylacetal

in 25 ml Dioxan und 10 ml Wasser wird auf 60° erwärmt und mit 0,2 ml Methansulfonsäure versetzt. Die Lösung wird während einer Stunde auf 80° erwärmt, abgekühlt und auf Eis-Wasser gegossen. Man stellt mit konzentrierter wässriger Ammoniaklösung auf pH 8,0 und extrahiert das ausgeschiedene Öl mit 50 ml Ethylacetat. Die organische Phase wird abgetrennt, zweimal mit je 10 ml Wasser gewaschen, über Magnesiumsulfat getrocknet und unter vermindertem Druck zur Trockene eingedampft. Der Rückstand, der

2-[4(5)-Phenyl-5(4)-(3-pyridyl)-imidazol-2-yl]-acetaldehyd

liegt als gelbes Öl vor.

Das Ausgangsmaterial kann wie folgt hergestellt werden:

Eine Mischung von 30,0 g α-Brom-benzyl-(3-pyridyl)-keton-hydrobromid und 50,0 g Ammoniumsalz des Malonaldehydsäure-dimethylacetals in 180 ml wasserfreiem Dimethylformamid werden unter Rühren und Einleiten von Stickstoffgas während vier Stunden auf 100° erhitzt. Dann wird die Mischung abgekühlt und bei 70° unter 1466,5 Pa zur Trockene eingedampft. Den Rückstand versetzt man mit 100 ml Wasser und 400 ml Ethylacetat. Durch Zugabe von konzentrierter wässriger Ammoniaklösung wird der pH auf 8,0 gestellt. Die organische Phase wird abgetrennt, mit 50 ml Wasser gewaschen, über Magnesiumsulfat getrocknet und unter 1466,5 Pa zur Trockene eingedampft. Den Rückstand chromatographiert man an 500 g Silikagel. Die Fraktionen 1–4, eluiert mit je 600 ml Chloroform, werden verworfen. Die Fraktionen 5–16, eluiert mit je 600 ml Chloroform-Methanol (98:2), werden vereinigt und unter vermindertem Druck zur Trockene eingedampft. Der Rückstand, das
N-[α-(3-Pyridyl)-phenacyl]-malonaldehyd-säure-dimethylacetal-amid
liegt als gelbes Öl vor.

Die Fraktionen 19–24, eluiert mit je 600 ml Chloroform-Methanol (97:3), werden vereinigt und unter vermindertem Druck zur Trockene eingedampft. Der Rückstand wird auf Ether-Petrolether kristallisiert. Das
2-[4(5)-Phenyl-5(4)-(3-pyridyl)-imidazol-2-yl]-acetaldehyd-dimethylacetal
schmilzt bei 142–145°.

Ein Gemisch von
10,0 g N-[α-(3-Pyridyl)-phenacyl]-malonaldehyd-säure-dimethylacetal-amid,
30,0 g Ammoniumacetat und 100 ml Eisessig wird zwei Stunden unter Rückfluss gekocht und anschliessend unter kräftigem Rühren in ein Gemisch aus 200 g Eis und 150 ml konzentrierter wässriger Ammoniaklösung gegossen. Der Kristallbrei wird zweimal mit je 150 ml Ethylacetat extrahiert und die organische Phase mit Wasser neutral gewaschen, mit Magnesiumsulfat getrocknet und unter 1466,5 Pa bei 40° zur Trockene eingedampft. Den Rückstand chromatographiert man an 500 g Silikagel. Die Fraktionen 1–4, eluiert mit je 500 ml Chloroform-Methanol (99:1), werden verworfen. Die Fraktionen 5–15, eluiert mit je 500 ml Chloroform-Methanol (99:2), werden vereinigt und unter vermindertem Druck zur Trockene eingedampft. Den Rückstand kristallisiert man aus Ether-Petrolether. Das
2-[4(5)-Phenyl-5(4)-(3-pyridyl)-imidazol-2-yl]-acetaldehyd-dimethylacetal
schmilzt bei 142–145°.

Der Ausgangsstoff wird wie folgt hergestellt: 19,7 g Malonaldehyd-säure-dimethylacetal [V.V. Shikina et al. J. Gen. Chem. U.S.S.R. 25, 723–725 (1955)] werden in 300 ml wasserfreiem Ether gelöst. In die Lösung wird bei 0° während einer Stunde Ammoniakgas eingeleitet. Anschliessend wird die Mischung unter vermindertem Druck zur Trockene eingedampft. Das Ammoniumsalz des Malonaldehyd-säure-dimethylacetals liegt als Öl vor.

Beispiel 18:

Eine Lösung von
5,0 g 2-[4(5)-Phenyl-5(4)-(3-pyridyl)-imidazol-2-yl]-acetaldehyd
in 50 ml Methanol wird unter Rühren mit 5,5 g Iod versetzt. Die Mischung wird bei 50° tropfenweise mit einer 4%-igen methanolischen Kaliumhydroxidlösung versetzt, bis Entfärbung eintritt. Zur Verseifung des entstandenen Esters werden 10 ml 2 N Kaliumhydroxidlösung zugesetzt. Die Lösung wird 10 Minuten auf 50° erwärmt und unter vermindertem Druck zur Trockene eingedampft. Den Rückstand versetzt man mit 15,0 ml 2 N Salzsäure und 50 ml Ether. Die organische Phase wird abgetrennt, mit 10 ml Wasser gewaschen, über Magnesiumsulfat getrocknet und unter vermindertem Druck eingedampft. Die
2-[4(5)-Phenyl-5(4)-(3-pyridyl)-imidazol-2-yl]-essigsäure
liegt als Öl vor.

Beispiel 19:

In eine Lösung von 2 mmol Bortrifluorid-diethyletherat in 10 ml Cyanessigsäureethylester wird unter Einleiten von Stickstoff und unter Rühren 2,2 g 2,3-Bis-(p-methoxyphenyl)-2H-azirin, gelöst in 15 ml Cyanoessigsäureethylester, bei 0° zugetropft. Das Gemisch wird ca. 5 Stunden bei dieser Temperatur gerührt und anschliessend in eine 5-prozentige wässrige Natriumbicarbonat-Lösung gegossen. Das Reaktionsgemisch wird 3 mal mit je 70 ml Methylenchlorid extrahiert. Die Methylenchloridphase wird über wasserfreiem Magnesiumsulfat getrocknet, filtriert und im Vakuum eingedampft. Der Nitrilüberschuss wird anschliessend im Hochvakuum destillativ entfernt. Nach Umkristallisieren aus Ethylacetat-Ether erhält man den
2-[4,5-Bis-(p-methoxyphenyl)-imidazol-2-yl]-essigsäureethylester,
Smp. 131–132°.

Das Ausgangsmaterial kann analog der in J. Amer. Chem. Soc., 89, 2077f (1967) beschriebenen Verfahrensweise hergestellt werden. So geht man beispielsweise zur Herstellung von 2,3-Bis-(p-methoxyphenyl)-2H-azirin von 1-Azido-1-iodo-1,2-bis-(p-methoxyphenyl)-ethan (über das 1-Azido-1,2-bis-(p-methoxyphenyl)-ethen aus.

Beispiel 20:

Eine Mischung von
1,8 g 2-(trans-4,5-(p-Methoxyphenyl)-4,5-dihydroimidazolin-2-yl)-2-methyl-propionsäureamid
und 2,0 g 2,3-Dichlor-5,6-dicyan-1,4-benzochinon in 60 ml wasserfreiem Dioxan wird während 5 Stunden unter Rückfluss erhitzt. Man kühlt ab, filtriert und engt das Filtrat unter vermindertem Druck bei 40° zur Trockene ein. Den Rückstand löst man in 50 ml Ethylacetat. Die Ethylacetatlösung wird mit 20 ml Wasser, zweimal mit je 20 ml 2 N Natriumkarbonatlösung und nochmals mit

20 ml Wasser gewaschen, über Magnesiumsulfat getrocknet, durch eine Schicht Silikagel filtriert und unter vermindertem Druck bei 40° zur Trockene eingedampft. Das
2-[4,5-Bis-(p-methoxyphenyl)-imidazol-2-yl]-2-methyl-propionsäureamid
kristallisiert aus Ethanol-Wasser.

Das Ausgangsmaterial kann wie folgt hergestellt werden:

Eine Lösung von 2,7 g dl-1,2-Bis-(4-methoxyphenyl)-ethylendiamin und 1,6 g Dimethylmalonsäuremonoethylester-monoamid in 40 ml Diphenylether wird während 3 Stunden auf 150° erhitzt. Der gebildete Ethylalkohol wird abdestilliert. Anschliessend kühlt man ab, giesst auf 100 ml Wasser und extrahiert das ausgeschiedene Öl zweimal mit je 100 ml Ether. Die vereinigten organischen Phasen werden mit 50 ml Wasser gewaschen, über Magnesiumsulfat getrocknet und unter 1466,5 Pa bei 30° von Ether und anschliessend unter 13,3 Pa bei 60° vom restlichen Diphenylether befreit. Das
2-[trans-4,5-(p-Methoxyphenyl)-4,5-dihydro-imidazol-2-yl]-2-methyl-propionsäureamid
liegt als Öl vor und wird sofort weiter umgesetzt.

Beispiel 21:

Eine Mischung von 2,7 g 4,4'-Dimethoxystilbendiamin und 1,2 g Dimethylmalonsäurealdehydmonoamid in 50 ml Nitrobenzol wird 40 Minuten unter Rückfluss erhitzt. Die Lösung wird abgekühlt und unter 13,3 Pa bei 60° zur Trockene eingedampft. Der Rückstand, das
2-[4,5-Bis-(p-methoxyphenyl)-imidazol-2-yl]-2-methyl-propionsäureamid
kristallisiert aus Ethanol-Wasser.

Beispiel 22:

Eine Lösung von
3,8 g 2-[4(5)-Phenyl-5(4)-(3-pyridyl)-imidazol-2-yl]-2-methyl-1-ethoxy-propan
in 80 ml Aceton und 25 ml Wasser wird bei Raumtemperatur unter kräftigem Rühren portionenweise mit Kaliumpermanganat versetzt bis keine Entfärbung mehr zu beobachten ist. Anschliessend wird die Mischung 10 Stunden bei Raumtemperatur gerührt und danach filtriert. Man engt das Filtrat unter 1466,5 Pa bei 50° zur Trockene ein. Den Rückstand versetzt man mit 20 ml Eiswasser und extrahiert mit Ethylacetat. Die organische Phase wird mit gesättigter Natriumchlorid-Lösung gewaschen, über Natriumsulfat getrocknet und durch eine Schicht Silikagel filtriert. Dann engt man das Filtrat unter 1466,5 Pa bei Raumtemperatur ein. Der Rückstand wird aus Ether umkristallisiert. Man erhält den
2-[4(5)-Phenyl-5(4)-(3-pyridyl)-imidazol-2-yl]-2-methyl-propionsäureethylester,
Smp. 134–136°.

Beispiel 23:

Eine Salbe, enthaltend 5%
2-[4(5)-Phenyl-5(4)-(1-oxido-3-pyridyl)-imidazol-2-yl]-2-methyl-propionsäure-ethylester,
kann wie folgt hergestellt werden:

Zusammensetzung:

| | |
|---|---|
| Wirkstoff | 5,0% |
| Vaseline | 45,0% |
| Paraffinöl | 19,6% |
| Cetylalkohol | 5,0% |
| Bienenwachs | 5,0% |
| Sorbitan-sesquioleat | 5,0% |
| p-Hydroxybenzoesäureester | 0,2% |
| Wasser, entmineralisiert bis zu | 100,0% |

Die Fettstoffe und Emulgatoren werden zusammengeschmolzen. Das Konservierungsmittel wird in Wasser gelöst, und die Lösung in die Fettschmelze bei erhöhter Temperatur einemulgiert. Nach dem Erkalten wird eine Suspension des Wirkstoffs in einem Teil der Fettschmelze in die Emulsion eingearbeitet.

Beispiel 24:

Eine Crème, enthaltend 10%
2-[4(5)-Phenyl-5(4)-(3-pyridyl)-imidazol-2-yl]-2-methyl-propionsäureethylester,
kann wie folgt hergestellt werden:

Zusammensetzung:

| | |
|---|---|
| Wirkstoff | 10,0% |
| Isopropylpalmitat | 8,0% |
| Cetylpalmitat | 1,5% |
| Siliconöl 100 | 0,5% |
| Sorbitanmonostearat | 3,0% |
| Polysorbat 60 | 3,5% |
| 1,2-Propylenglykol PH | 20,0% |
| Acrylsäurepolymerisat | 0,5% |
| Triethanolamin | 0,7% |
| Wasser, entmineralisiert bis zu | 100,0% |

Das Acrylsäurepolymerisat wird in einem Gemisch aus entmineralisiertem Wasser und 1,2-Propylenglykol suspendiert. Unter Rühren wird hierauf Triethanolamin zugegeben, wodurch ein Schleim erhalten wird. Ein Gemisch aus Isopropylpalmitat, Cetylpalmitat, Siliconöl, Sorbitanmonostearat und Polysorbat wird auf ca. 75° erwärmt und unter Rühren in den gleichfalls auf ca. 75° erwärmten Schleim eingearbeitet. Die auf Raumtemperatur abgekühlten Crème-Grundlage wird hierauf zur Herstellung eines Konzentrates mit dem Wirkstoff verwendet. Das Konzentrat wird mittels eines Durchlaufhomogenisators homogenisiert und dann portionsweise der Grundlage hinzugefügt.

Beispiel 25:

Eine Crème, enthaltend 5%
2-[4(5)-Phenyl-5(4)-(1-oxido-3-pyridyl)-imidazol-2-yl]-2-methyl-propionsäureethylester,
kann wie folgt erhalten werden:

Zusammensetzung:

| | |
|---|---|
| Wirkstoff | 5,0% |
| Cetylpalmitat PH | 2,0% |
| Cetylalkohol PH | 2,0% |
| Triglyceridgemisch gesättigter mittelfettiger Fettsäuren | 5,0% |
| Stearinsäure | 3,0% |
| Glycerinstearat PH | 4,0% |
| Cetomacrogol 1000 | 1,0% |

| | |
|---|---|
| mikrokristalline Cellulose | 0,5% |
| 1,2-Propylenglykol, dest. | 20,0% |
| Wasser, entmineralisiert, bis zu | 100,0% |

Cetylalkohol, Cetylpalmitat, das Triglyceridgemisch, Stearinsäure und Glycerinstearat werden zusammengeschmolzen. Die mikrokristalline Cellulose wird in einen Teil des Wassers dispergiert. Im restlichen Teil des Wassers wird Cetomacrogol gelöst und das Propylenglykol sowie der Schleim dazu gemischt. Die Fettphase wird anschliessend unter Rühren zu Wasserphase gegeben und kaltgerührt. Schliesslich wird der Wirkstoff mit einem Teil der Grundlage angerieben und hierauf die Anreibung in den Rest der Crème eingearbeitet.

Beispiel 26:

Ein transparentes Hydrogel enthaltend
5% 2-[4(5)-Phenyl-5(4)-(1-oxido-3-pyridyl)-imidazol-2-yl]-2-methyl-propionsäure-ethylester
wird wie folgt hergestellt:

Zusammensetzung:

| | |
|---|---|
| Wirkstoff | 5% |
| Propylenglykol | 10–20% |
| Isopropanol | 20% |
| Hydroxypropyl-methylcellulose | 2% |
| Wasser | ad 100% |

Die Hydroxypropyl-methylcellulose wird im Wasser gequollen. Der Wirkstoff wird in einem Gemisch aus Isopropanol und Propylenglykol gelöst. Anschliessend wird die Wirkstofflösung mit einem gequollenen Cellulose-Derivat vermischt und, wenn erwünscht, mit Riechstoffen (0,1%) versetzt.

Beispiel 27:

Ein transparentes Hydrogel, enthaltend
5% 2-[4(5)-(1-oxido-3-pyridyl)-imidazol-2-yl]-2-methyl-propionsäureethylester,
wird wie folgt hergestellt:

Zusammensetzung:

| | |
|---|---|
| Wirkstoff | 5% |
| Propylenglykol | 20% |
| Isopropanol | 20% |
| Acrylsäurepolymerisat | 2% |
| Triethanolamin | 3% |
| Wasser | ad 100% |

Acrylsäurepolymerisat und Wasser werden dispergiert und mit Triethanolamin neutralisiert. Der Wirkstoff wird in einem Gemisch aus Isopropanol und Propylenglykol gelöst. Anschliessend wird die Wirkstofflösung mit dem Gel vermischt, wobei, wenn erwünscht, Riechstoff (0,1%) zugegeben werden kann.

Beispiel 28:

Ein Schaumspray, enthaltend
1% 2-[4(5)-Phenyl-5(4)-(1-oxido-3-pyridyl)-imidazol-2-yl]-2-methyl-propionsäure,
kann folgendermassen hergestellt werden:

Zusammensetzung:

| | |
|---|---|
| Wirkstoff | 1,00% |
| Cetylalkohol PH | 1,70% |
| Paraffinöl, dickflüssig | 1,00% |
| Isopropylmyristat | 2,00% |
| Cetomacrogol 1000 | 2,40% |
| Sorbitanmonostearat | 1,50% |
| 1,2-Propylenglykol PH | 5,00% |
| Methylparaben | 0,18% |
| Propylparaben | 0,02% |
| Chemoderm 314 | 0,10% |
| Wasser, entmineralisiert, bis zu | 100,00% |

Cetylalkohol, Paraffinöl, Isopropylmyristat, Cetomacrogol und Sorbitanstearat werden zusammengeschmolzen. Methyl- und Propylparaben werden in heissem Wasser gelöst. Die Schmelze und die Lösung werden anschliessend vermischt. Der Wirkstoff, in Propylenglykol suspendiert, wird in die Grundlage eingearbeitet. Anschliessend wird Chemoderm zugeführt und mit Wasser auf das Endgewicht ergänzt.

Abfüllung:

20 ml des Gemisches wird in eine Aluminiumblockdose eingefüllt. Die Dose wird mit einem Ventil versehen, und das Treibgas wird unter Druck eingefüllt.

Beispiel 29:

Eine Lösung von 5 g α-Hydroxybenzyl-3-pyridylketon (hergestellt nach J. Chem. Soc. 1956, 2913) und 6 ml Triethylamin in 50 ml wasserfreiem Benzol wird bei 10° unter Rühren während einer Stunde mit einer Lösung von 2 g Malonsäuremonoethylesterchlorid in 10,0 ml wasserfreiem Benzol versetzt. Die Mischung wird zwei Stunden bei 10° gerührt und anschliessend mit 60 ml Wasser versetzt. Man extrahiert zweimal mit je 60 ml Ethylacetat, trennt die organischen Phasen ab, vereinigt sie und wäscht sie mit 30 ml gesättigter wässriger Kochsalzlösung, zweimal mit je 30 ml 1 N Natriumbikarbonatlösung und nochmals mit 30 ml gesättigter Kochsalzlösung.

Die organische Phase wird dann über Magnesiumsulfat getrocknet und unter 1466,5 Pa bei 30° zur Trockene eingedampft. Der Rückstand, der Malonsäuremonoethylester-[1-phenyl-2-oxo-2-(3-pyridyl)]-ethylester liegt als Öl vor.

Eine Mischung von
3,5 g Malonsäuremonoethylester-[1-phenyl-2-oxo-2-(3-pyridyl)]-ethylester,
1,15 g Ammoniumacetat und 100 ml Eisessig wird zwei Stunden unter Rückfluss gekocht und anschliessend unter 1466,5 Pa bei 50° zur Trockene eingedampft. Den Rückstand chromatographiert man an 120 g Silikagel. Die Fraktionen 1–4, eluiert mit je 300 ml Benzol-Ethylacetat-Eisessig (94:5:1), werden verworfen. Die Fraktionen 5–9, eluiert mit dem gleichen Lösungsmittelgemisch, werden vereinigt und unter 1466,5 Pa zur Trockene eingedampft. Der Rückstand enthält das
2-[4-(5)-Phenyl-5(4)-(3-pyridyl)-oxazol-2-yl]-essigsäureethylester
als Öl (amorpher Schaum).

Beispiel 30:

Eine Lösung von 16,6 g
2-[4(5)-Phenyl-5(4)-(3-pyridyl)-oxazol-2-yl]-essigsäureethylester

in 200 ml Methylenchlorid wird auf 0° abgekühlt und unter Rühren mit einer Lösung von 11,3 g m-Chlorperbenzoesäure in 200 ml Methylenchlorid versetzt.

Die Mischung wird eine Stunde bei 0° gerührt und nochmals mit einer Lösung von 11,3 g m-Chlorperbenzoesäure in 200 ml Methylenchlorid versetzt. Die Mischung wird nochmals ½ Stunde bei 0° gerührt und dann zweimal mit je 50 ml 2 N Kaliumbikarbonatlösung und 50 ml Wasser extrahiert. Die Methylenchloridlösung wird abgetrennt, über Magnesiumsulfat getrocknet und unter 1466,5 Pa zur Trockne eingedampft. Den Rückstand chromatographiert man an 500 g Silikagel. Die Fraktionen 1–4, eluiert mit je 800 ml Chloroform-Methanol (99:1), werden verworfen. Die Fraktionen 5–12, eluiert mit je 800 ml Chloroform-Methanol (99:1), werden vereinigt und unter 1466,5 Pa zur Trockene eingedampft. Der Rückstand
2-[4(5)-Phenyl-5(4)-(1-oxido-3-pyridyl)-oxazol-2-yl]-essigsäure-ethylester
ist ein Öl (amorpher Schaum).

Beispiel 31:

Analog Beispiel 29 oder 30 wird hergestellt:
2-[4(5)-Phenyl-5(4)-(3-pyridyl)-oxazol-2-yl]-2-methyl-propionsäure-ethylester,
Öl (amorpher Schaum),
2-[4(5)-Phenyl-5(4)-(3-pyridyl)-oxazol-2-yl]-2-allyl-essigsäureethylester,
1-[4(5)-Phenyl-5(4)-(3-pyridyl)-oxazol-2-yl]-1-carbethoxycyclopentan,
2-[4(5)-(p-Methoxyphenyl)-5(4)-(3-pyridyl)-oxazol-2-yl]-2-methyl-propionsäureethylester,
2-[4(5)-Phenyl-5(4)-(1-oxido-3-pyridyl)-oxazol-2-yl]-2-methyl-propionsäureethylester,
Öl (amorpher Schaum),
2-[4(5)-Phenyl-5(4)-(3-pyridyl)-oxazol-2-yl]-propionsäureethylester,
2-[4(5)-(p-Chlorphenyl)-5(4)-(3-pyridyl)-oxazol-2-yl]-2-methylpropionsäureethylester,
Öl (amorpher Schaum),
2-[4(5)-Phenyl-5(4)-(4-pyridyl)-oxazol-2-yl]-2-methyl-propionsäureethylester,
2-[4(5)-Phenyl-5(4)-(1-oxido-4-pyridyl)-oxazol-2-yl]-2-methyl-propionsäureethylester,
2-[4(5)-(p-Hydroxyphenyl)-5(4)-(3-pyridyl)-oxazol-2-yl]-2-methyl-propionsäureethylester,
Öl (amorpher Schaum),
2-[4(5)-Phenyl-5(4)-(3-pyridyl)-oxazol-2-yl]-2-methyl-propionsäurepivaloyloxymethylester,
Öl (amorpher Schaum).

**Patentansprüche für die Vertragsstaaten: BE CH DE FR IT LI LU NL SE**

1. Neue trisubstituierte Imidazolderivate der allgemeinen Formel

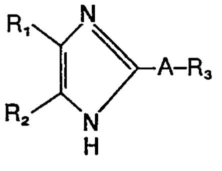

(I),

worin einerseits $R_1$ und $R_2$ unabhängig voneinander Phenyl und/oder durch Halogen, Niederalkyl, Hydroxy, Niederalkoxy und/oder Niederalkanoyl substituiertes Phenyl bedeuten oder worin andererseits einer der Reste $R_1$ und $R_2$ Pyrrolyl, Furyl, Thienyl, Pyridyl, 1-Oxidopyridyl oder Pyrimidyl bedeutet, welche jeweils unsubstituiert oder durch Halogen, Niederalkyl, Hydroxy, Niederalkoxy und/oder Niederalkanoyloxy substituiert sein können, und der andere Phenyl, Pyrrolyl, Furyl, Thienyl, Pyridyl, 1-Oxidopyridyl oder Pyrimidyl bedeutet, welche jeweils unsubstituiert oder durch Halogen, Niederalkyl, Hydroxy, Niederalkoxy und/oder Niederalkanoyloxy substituiert sein können, und jeweils A Niederalkylen, Niederalkyliden, Niederalkenylen, Niederalkenyliden, Cycloalkylen, Cycloalkyliden oder Cycloalkyl-niederalkyliden darstellen, und $R_3$ Carboxy, mit einem Niederalkanol, 3- bis 8-gliedrigem Cycloalkanol, Phenol, einem Hydroxypyridin, einem substituierten Phenol bzw. Hydroxypyridin verestertes Carboxy, Carbamoyl oder durch Hydroxy, Amino, Phenyl oder substituiertes Phenyl mono-substituiertes, durch Niederalkyl mono- oder disubstituiertes oder durch 4- bis 7-gliedriges Alkylen bzw. 3-Aza-, 3-Niederalkylaza-, 3-Oxa- oder 3-Thiaalkylen disubstituiertes Carbamoyl bedeutet, wobei ein Niederalkanol bzw. Cycloalkanol unsubstituiert oder durch Hydroxy, Mercapto, gegebenenfalls substituiertes Phenyl, Niederalkoxy, gegebenenfalls im Phenylteil substituiertes Phenylniederalkoxy, Niederalkylthio, gegebenenfalls im Phenylteil substituiertes Phenylniederalkylthio, Hydroxyniederalkoxy, Niederalkoxyniederalkoxy, gegebenenfalls im Phenylteil substituiertes Phenylniederalkoxyniederalkoxy, Carboxyniederalkoxy, Niederalkoxycarbonyl-niederalkoxy, gegebenenfalls substituiertes Phenyl aufweisendes Niederalkoxycarbonyl-niederalkoxy oder Niederalkanoyloxy und substituiertes Phenyl, Phenol bzw. Hydroxypyridin jeweils durch Niederalkyl, Niederalkoxy, Halogen und/oder Trifluormethyl substituiert sein kann, ihre Isomeren sowie ihre Salze, insbesondere pharmazeutisch verwendbare Salze, mit der Massgabe, dass, wenn A für Methylen bzw. Ethyliden und $R_3$ für Ethoxycarbonyl stehen, nicht beide Reste $R_1$ und $R_2$ zugleich Phenyl bedeuten, und der weiteren Massgabe, dass, wenn A für Ethyliden und $R_3$ für Carboxy bzw. Carbamoyl stehen, nicht beide Reste $R_1$ und $R_2$ zugleich Phenyl, p-Methoxyphenyl oder p-Chlorphenyl bedeuten, wobei unter mit «nieder» bezeichneten Resten solche zu verstehen sind, die bis und mit 7 C-Atome enthalten.

2. Verbindungen gemäss Anspruch 1 der Formel I, worin $R_1$ und $R_2$ unabhängig voneinander Phenyl und/oder durch Halogen, Hydroxy, Niederalkyl, Niederalkoxy und/oder Niederalkanoyloxy substituiertes Phenyl bedeuten, A Niederalkylen mit bis und mit 4 C-Atomen, Niederalkyliden mit bis und mit 7 C-Atomen, Niederalkenylen mit bis und mit 4 C-Atomen, Niederalkenyliden mit bis und mit 7 C-Atomen, 3- bis 8-gliedriges Cycloalkylen, 3- bis 8-gliedriges Cycloalkyliden oder Cycloalkyl-niederalkyliden mit bis und mit 7 C-Atomen

im Alkylidenteil und mit einem 3- bis 8-gliedrigen Cycloalkylteil bedeutet und $R_3$ Carboxy, mit einem Niederalkanol einem 3- bis 8-gliedrigen Cycloalkanol, Phenol oder einem substituierten Phenol verestertes Carboxy, Carbamoyl, N-Mono-, N,N-Diniederalkylcarbamoyl, Pyrrolidino-, Piperidino-, Morpholino-, Piperazino-, 4-Niederalkyl-piperazino-, Thiomorpholino-, Anilino- oder durch Niederalkyl, Niederalkoxy und/oder Halogen substituiertes Anilinocarbonyl darstellt, wobei das Niederalkanol bzw. Cycloalkanol unsubstituiert oder durch Hydroxy, Mercapto, Phenyl, substituiertes Phenyl, Niederalkoxy, Phenylniederalkoxy, im Phenylteil substituiertes Phenylniederalkoxy, Niederalkylthio, Phenylniederalkylthio, im Phenylteil substituiertes Phenylniederalkylthio, Hydroxyniederalkoxy, Niederalkoxyniederalkoxy, Phenylniederalkoxyniederalkoxy, im Phenylteil substituiertes Phenylniederalkoxyniederalkoxy, Carboxyniederalkoxy, Niederalkoxycarbonyl-niederalkoxy, gegebenenfalls substituiertes Phenyl aufweisendes Niederalkoxycarbonyl-niederalkoxy oder Niederalkanoyloxy und substituiertes Phenol bzw. Phenyl jeweils durch Niederalkyl, Niederalkoxy, Halogen und/oder Trifluormethyl substituiert sein kann, ihre Isomeren sowie ihre Salze, insbesondere pharmazeutisch verwendbare Salze, mit der Massgabe, dass, wenn A für Methylen bzw. Ethyliden und $R_3$ für Ethoxycarbonyl stehen, nicht beide Reste $R_1$ und $R_2$ zugleich Phenyl bedeuten, und der weiteren Massgabe, dass, wenn A für Ethyliden und $R_3$ für Carboxy bzw. Carbamoyl stehen, nicht beide Reste $R_1$ und $R_2$ zugleich Phenyl, p-Methoxyphenyl oder p-Chlorphenyl bedeuten.

3. Verbindungen gemäss Anspruch 1 der Formel I, worin einer der Reste $R_1$ und $R_2$ Pyridyl oder 1-Oxido-pyridyl, die unsubstituiert und/oder jeweils durch Halogen, Hydroxy, Niederalkyl, Niederalkoxy und/oder Niederalkanoyloxy substituiert sein können, und der andere Phenyl, Pyridyl oder 1-Oxidopyridyl, die unsubstituiert und/oder jeweils durch Halogen, Hydroxy, Niederalkyl, Niederalkoxy und/oder Niederalkanoyloxy substituiert sein können, bedeutet, A Niederalkylen mit bis und mit 4 C-Atomen, Niederalkyliden mit bis und mit 7 C-Atomen, Niederalkenylen mit bis und mit 4 C-Atomen, Niederalkenyliden mit bis und mit 7 C-Atomen, 3- bis 8-gliedriges Cycloalkylen, 3- bis 8-gliedriges Cycloalkyliden oder Cycloalkylniederalkyliden mit bis und mit 7 C-Atomen im Alkylidenteil und mit einem 3- bis 8-gliedrigen Cycloalkylteil bedeutet und $R_3$ Carboxy, mit einem Niederalkanol, einem 3- bis 8-gliedrigen Cycloalkanol, Phenol oder einem substituierten Phenol verestertes Carboxy, Carbamoyl, N-Mono-, N,N-Diniederalkylcarbamoyl, Pyrrolidino-, Piperidino-, Morpholino-, Piperazino-, 4-Niederalkyl-piperazino-, Thiomorpholino-, Anilino- oder durch Niederalkyl, Niederalkoxy und/oder Halogen substituiertes Anilinocarbonyl darstellt, wobei das Niederalkanol bzw. Cycloalkanol unsubstituiert oder durch Hydroxy, Mercapto, Phenyl, substituiertes Phenyl, Niederalkoxy, Phenylniederalkoxy, im Phenylteil substituiertes Phenylniederalkoxy, Niederalkylthio, Phenylniederalkylthio, im Phenylteil substituiertes Phenylniederalkylthio, Hydroxyniederalkoxy, Niederalkoxyniederalkoxy, Phenylniederalkoxyniederalkoxy, im Phenylteil substituiertes Phenylniederalkoxyniederalkoxy, Carboxyniederalkoxy, Niederalkoxycarbonyl-niederalkoxy, gegebenenfalls substituiertes Phenyl aufweisendes Niederalkoxycarbonyl-niederalkoxy oder Niederalkanoyloxy und substituiertes Phenol bzw. Phenyl jeweils durch Niederalkyl, Niederalkoxy, Halogen und/oder Trifluormethyl substituiert sein kann, ihre Isomeren sowie ihre Salze, insbesondere pharmazeutisch verwendbare Salze.

4. Verbindungen gemäss Anspruch 1 der Formel I, worin $R_1$ und $R_2$ unabhängig voneinander gegebenenfalls durch Halogen mit Atomnummer bis und mit 35, Hydroxy, Niederalkyl mit bis und mit 4 C-Atomen, Niederalkoxy mit bis und mit 4 C-Atomen und/oder Niederalkanoyloxy mit bis und mit 5 C-Atomen substituiertes Phenyl, Pyridyl, und/oder 1-Oxidopyridyl bedeuten, A Niederalkylen mit bis und mit 4 C-Atomen oder Niederalkyliden mit bis und mit 4 C-Atomen bedeutet, $R_3$ gegebenenfalls mit einem Niederalkanol mit bis und mit 4 C-Atomen verestertes Carboxy darstellt, wobei das Niederalkanol gegebenenfalls durch Hydroxy, Mercapto, gegebenenfalls substituiertes Phenyl, Niederalkoxy mit bis und mit 4 C-Atomen, gegebenenfalls im Phenylteil substituiertes Phenylniederalkoxy mit bis und mit 4 C-Atomen im Alkylteil, Niederalkylthio mit bis und mit 4 C-Atomen, Hydroxyniederalkoxy mit bis und mit 4 C-Atomen im Niederalkylteil, Niederalkoxyniederalkoxy jeweils mit bis und mit 4 C-Atomen im Niederalkylteil oder Niederalkanoyloxy mit bis und mit 5 C-Atomen und Phenyl gegebenenfalls durch Niederalkyl mit bis und mit 4 C-Atomen, Niederalkoxy mit bis und mit 4 C-Atomen, Halogen mit Atomnummer bis und mit 35 und/oder Trifluormethyl substituiert sein kann, ihre Isomeren sowie ihre Salze, mit der Massgabe, dass, wenn A für Methylen bzw. Ethyliden und $R_3$ für Ethoxycarbonyl stehen, nicht beide Reste $R_1$ und $R_2$ zugleich Phenyl bedeuten, und der weiteren Massgabe, dass, wenn A für Ethyliden und $R_3$ für Carboxy stehen, nicht beide Reste $R_1$ und $R_2$ zugleich Phenyl, p-Methoxyphenyl oder p-Chlorphenyl bedeuten.

5. Verbindungen gemäss Anspruch 1 der Formel I, worin $R_1$ und $R_2$ unabhängig voneinander Phenyl und/oder durch Halogen mit Atomnummer bis und mit 35, Hydroxy, Niederalkyl mit bis und mit 4 C-Atomen und/oder Niederalkoxy mit bis und mit 4 C-Atomen substituiertes Phenyl bedeuten, A Niederalkylen mit bis und mit 4 C-Atomen, Niederalkyliden mit bis und mit 7 C-Atomen, Niederalkenyliden mit bis und mit 7 C-Atomen oder 3- bis 8-gliedriges Cycloniederalkyliden darstellen und $R_3$ Carboxy, Niederalkoxycarbonyl mit bis und mit 5 C-Atomen, Carbamoyl, N-Mononiederalkylcarbamoyl mit bis und mit 4 C-Atomen im Niederalkyl oder N,N-Diniederalkylcarbamoyl jeweils mit bis und mit 4 C-Atomen im Niederalkyl bedeuten, wobei das Niederalkoxycarbonyl durch Niederalkanoyloxy mit bis und mit 5 C-Atomen substituiert sein kann, ihre Isomeren sowie ihre Salze,

insbesondere pharmazeutisch verwendbare Salze, mit der Massgabe, dass, wenn A für Methylen bzw. Ethyliden und $R_3$ für Ethoxycarbonyl stehen, nicht beide Reste $R_1$ und $R_2$ zugleich Phenyl bedeuten, und der weiteren Massgabe, dass, wenn A für Ethyliden und $R_3$ für Carboxy bzw. Carbamoyl stehen, nicht beide Reste $R_1$ und $R_2$ zugleich Phenyl, p-Methoxyphenyl oder p-Chlorphenyl bedeuten.

6. Verbindungen gemäss Anspruch 1 der Formel I, worin einer der Reste $R_1$ und $R_2$ Phenyl oder durch Halogen mit Atomnummer bis und mit 35, Hydroxy, Niederalkyl mit bis und mit 4 C-Atomen und/oder Niederalkoxy mit bis und mit 4 C-Atomen substituiertes Phenyl bedeutet und der andere Pyridyl oder 1-Oxido-pyridyl bedeutet, die jeweils unsubstituiert oder durch Halogen mit Atomnummer bis und mit 35, Hydroxy und/oder Niederalkoxy mit bis und mit 4 C-Atomen substituiert sein können, A Niederalkylen mit bis und mit 4 C-Atomen, Niederalkyliden mit bis und mit 7 C-Atomen, Niederalkenyliden mit bis und mit 7 C-Atomen, oder 3- bis 8-gliedriges Cycloniederalkyliden darstellen und $R_3$ Carboxy, Niederalkoxycarbonyl mit bis und mit 5 C-Atomen, Carbamoyl, N-Mononiederalkyl-carbamoyl mit bis und mit 4 C-Atomen im Niederalkyl oder N,N-Diniederalkylcarbamoyl jeweils mit bis und mit 4 C-Atomen in Niederalkyl bedeuten, wobei das Niederalkoxycarbonyl durch Niederalkanoyloxy mit bis und mit 5 C-Atomen substituiert sein kann, ihre Isomeren sowie ihre Salze, insbesondere pharmazeutisch verwendbare Salze.

7. Verbindungen gemäss Anspruch 1 der Formel I, worin $R_1$ und $R_2$ unabhängig voneinander Phenyl und/oder durch Niederalkoxy mit bis und mit 4 C-Atomen substituiertes Phenyl bedeuten, A Niederalkylen mit bis und mit 4 C-Atomen oder Niederalkyliden mit bis und mit 4 C-Atomen darstellt und $R_3$ Carboxy oder Niederalkoxycarbonyl mit bis und mit 5 C-Atomen bedeutet, ihre Isomeren sowie ihre Salze, insbesondere pharmazeutisch verwendbare Salze, mit der Massgabe, dass, wenn A für Methylen bzw. Ethyliden und $R_3$ für Ethoxycarbonyl stehen, nicht beide Reste $R_1$ und $R_2$ zugleich Phenyl bedeuten, und der weiteren Massgabe, dass, wenn A für Ethyliden und $R_3$ für Carboxy stehen, nicht beide Reste $R_1$ und $R_2$ zugleich Phenyl oder p-Methoxyphenyl bedeuten.

8. Verbindungen gemäss Anspruch 1 der Formel I, worin einer der Reste $R_1$ und $R_2$ Phenyl oder durch Halogen mit Atomnummer bis und mit 35, Hydroxy oder Niederalkoxy mit bis und mit 4 C-Atomen substituiertes Phenyl bedeutet und der andere Pyridyl oder 1-Oxidopyridyl darstellt, A ein ein quartäres C-Atom aufweisendes Niederalkyliden mit bis und mit 4 C-Atomen, wobei das quartäre C-Atom direkt an den Imidazolring gebunden ist, bedeutet, und $R_3$ Niederalkoxycarbonyl mit bis und mit 5 C-Atomen darstellt, ihre Isomeren sowie ihre Salze, insbesondere pharmazeutisch verwendbare Salze.

9. Verbindungen gemäss Anspruch 1 der Formel I, worin einer der Reste $R_1$ und $R_2$ Phenyl und der andere 1-Oxidopyridyl bedeutet, A 2,2-Propyliden darstellt und $R_3$ Niederalkoxycarbonyl mit bis und mit 5 C-Atomen bedeutet, ihre Isomeren sowie ihre Salze, insbesondere pharmazeutisch verwendbare Salze.

10. 2-[4(5)-Phenyl-5(4)-(3-pyridyl)-imidazol-2-yl]-2-methyl-propionsäureethylester oder ein Salz davon.

11. 2-[4(5)-Phenyl-5(4)-(3-pyridyl)-imidazol-2-yl]-2-allyl-essigsäureethylester oder ein Salz davon.

12. 1-[4(5)-Phenyl-5(4)-(3-pyridyl)-imidazol-2-yl]-1-carbethoxycyclopentan oder ein Salz davon.

13. 2-[4(5)-(p-Methoxyphenyl)-5(4)-(3-pyridyl)-imidazol-2-yl]-2-methyl-propionsäureethylester oder ein Salz davon.

14. 2-[4(5)-Phenyl-5(4)-(1-oxido-3-pyridyl)-imidazol-2-yl]-2-methyl-propionsäureethylester oder ein Salz davon.

15. 2-[4(5)-Phenyl-5(4)-(3-pyridyl)-imidazol-2-yl]-propionsäureethylester oder ein Salz davon.

16. 2-[4(5)-(p-Chlorphenyl)-5(4)-(3-pyridyl)-imidazol-2-yl]-2-methyl-propionsäureethylester oder ein Salz davon.

17. 2-[4(5)-Phenyl-5(4)-(4-pyridyl)-imidazol-2-yl]-2-methyl-propionsäureethylester oder ein Salz davon.

18. 2-[4(5)-Phenyl-5(4)-(1-oxido-4-pyridyl)-imidazol-2-yl]-2-methyl-propionsäureethylester oder ein Salz davon.

19. 2-[4(5)-(p-Hydroxyphenyl)-5(4)-(3-pyridyl)-imidazol-2-yl]-2-methyl-propionsäureethylester oder ein Salz davon.

20. Verbindungen der Formel I gemäss Anspruch 1, mit der Massgabe, dass, wenn A für Ethyliden und $R_3$ für Carboxy stehen, nicht beide Reste $R_1$ und $R_2$ zugleich p-Methoxyphenyl bedeuten, zur Anwendung in einem Verfahren zur therapeutischen Behandlung des tierischen oder menschlichen Körpers.

21. Verbindungen gemäss Anspruch 20 der Formel (I), mit der Massgabe, dass, wenn A für Methylen bzw. Ethyliden und $R_3$ für Ethoxycarbonyl stehen, nicht beide Reste $R_1$ und $R_2$ zugleich Phenyl bedeuten, und der weiteren Massgabe, dass, wenn A für Ethyliden und $R_3$ für Carboxy stehen, nicht beide Reste $R_1$ und $R_2$ zugleich Phenyl, p-Methoxyphenyl oder p-Chlorphenyl bedeuten, zur Anwendung gemäss Anspruch 20.

22. Verbindungen gemäss einem der Ansprüche 1 bis 19 zur Anwendung gemäss Anspruch 20.

23. Verbindungen der Formel I gemäss einem der Ansprüche 1–19 zur Anwendung gemäss Anspruch 20 als topische Hautphlogistatika, Anti-Herpes-Mittel und/oder Sonnenschutzmittel.

24. Verwendung von Verbindungen der Formel I gemäss einem der Ansprüche 1–19 zur Herstellung von Arzneimitteln, wie von topischen Hautphlogistatika, Anti-Herpes-Mitteln und/oder Sonnenschutzmitteln.

25. Pharmazeutische Präparate, enthaltend eine Verbindung gemäss einem der Ansprüche 20–23 in freier Form oder in Form eines pharmazeutisch verwendbaren Salzes neben üblichen pharmazeutischen Hilfs- und Trägerstoffen.

26. Pharmazeutische Präparate gemäss Anspruch 25 enthaltend eine Verbindung gemäss einem der Ansprüche 1–19 in freier Form oder in Form eines pharmazeutisch verwendbaren Salzes neben üblichen pharmazeutischen Hilfs- und Trägerstoffen.

27. Verfahren zur Herstellung von Verbindungen der Formel (I) gemäss Anspruch 1, dadurch gekennzeichnet, dass man aus einer Verbindung der Formel

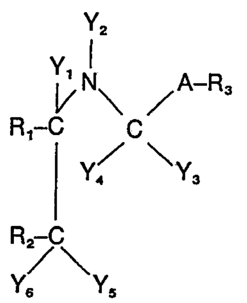

(II),

worin einer der Reste $Y_1$ und $Y_6$ Hydroxy oder Amino und der andere sowie $Y_2$ Wasserstoff darstellt und $Y_3$ gemeinsam mit $Y_4$ und $Y_5$ eine Gruppe der Formel =N– bedeutet oder worin $Y_1$ gemeinsam mit $Y_6$ eine Bindung darstellt, $Y_2$ Wasserstoff ist, $Y_3$ Hydroxy oder Amino bedeutet und $Y_4$ gemeinsam mit $Y_5$ eine Gruppe der Formel –NH– darstellt oder worin $Y_1$ gemeinsam mit $Y_6$ eine Bindung darstellt, $Y_2$ gemeinsam mit $Y_3$ eine zusätzliche Bindung und einer der Reste $Y_4$ und $Y_5$ Amino und der andere Amino, Hydroxy oder reaktionsfähiges verestertes Hydroxy darstellt oder worin $Y_1$ Hydroxy ist, $Y_2$ sowie $Y_3$ Wasserstoff bedeutet, $Y_4$ Hydroxy oder Amino darstellt und $Y_5$ gemeinsam mit $Y_6$ eine Gruppe der Formel =NH oder, sofern $Y_4$ Amino ist, Oxo bedeutet, oder einem Tautomeren und/oder Salz davon, unter Einführung einer zusätzlichen Bindung H–Z, wobei Z Hydroxy oder Amino $Y_1$ bzw. $Y_6$, $Y_3$ oder $Y_4$ bzw. $Y_5$ oder verestertes Hydroxy $Y_4$ bzw. $Y_5$ bedeutet, abspaltet, oder eine Verbindung der Formel

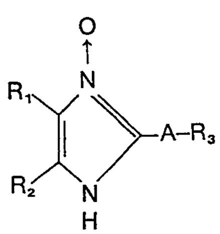

(IV)

oder ein Salz davon zu einer Verbindung der Formel I reduziert oder in einer Verbindung der Formel

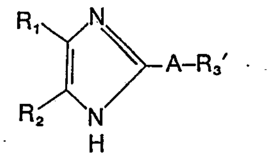

(V),

worin $R_3'$ einen im $R_3$ überführbaren Rest bedeutet, den Rest $R_3'$ in einen Rest $R_3$ überführt oder in einer Verbindung der Formel

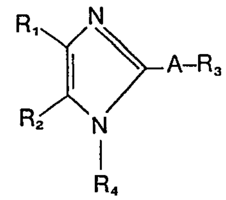

(VI),

worin $R_4$ eine in Wasserstoff überführbare Gruppe darstellt, oder einem Salz davon $R_4$ in Wasserstoff überführt oder Verbindungen der Formel

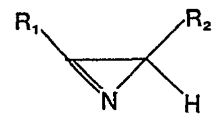

(VIIa)

oder Tautomere davon mit Verbindungen der Formel $R_3$–A–CN (VIIb) umsetzt oder eine Verbindung der Formel

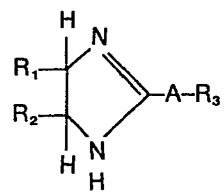

(VIII)

oder ein Isomeres davon zu Verbindungen der Formel I dehydriert und gewünschtenfalls die verfahrensgemäss erhältliche freie Verbindung in ein Salz oder ein verfahrensgemäss erhältliches Salz in die freie Verbindung oder in ein anderes Salz überführt oder eine verfahrensgemäss erhältliche freie Verbindung in eine andere freie Verbindung überführt und/oder, wenn erwünscht, ein erfindungsgemäss erhältliches Gemisch von isomeren Verbindungen der Formel I in die einzelnen Isomeren auftrennt.

28. Verfahren gemäss Anspruch 27, dadurch gekennzeichnet, dass man von irgendeiner Stufe des Verfahrens ausgeht und die fehlenden Schritte durchführt oder einen Ausgangsstoff in Form eines Salzes und/oder Tautomeren verwendet und/oder unter den Reaktionsbedingungen bildet.

29. Verfahren gemäss einem der Ansprüche 27–28, dadurch gekennzeichnet, dass die Ausgangsstoffe der Formel II, ihre Tautomeren und/oder Salze nach an sich bekannten Verfahren zum überwiegenden Teil in situ gebildet und unter den Reaktionsbedingungen ohne Isolierung weiter zu der Verbindung der Formel I umgesetzt werden.

30. Verfahren gemäss Anspruch 29, dadurch gekennzeichnet, dass man ein Diketon der Formel

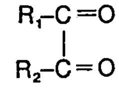

(IIIa)

mit einem Aldehyd der Formel $R_3$–A–C(=O)–H (IIIb) oder einem Salz davon, mit einem Überschuss an Ammoniak unter Erwärmen umsetzt oder ein acyliertes α-Aminoketon der Formel

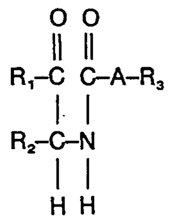

(IIIc)

oder ein Salz davon mit Ammoniak oder eine Verbindung der Formel

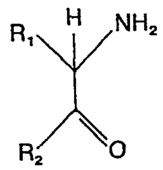

(IIIh)

oder ein Salz davon mit einem Säurederivat der Formel $R_3$–A–COOH (IIIi) umsetzt oder eine Verbindung der Formel

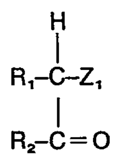

(IIId),

worin $Z_1$ reaktionsfähiges verestertes Hydroxy bedeutet, oder ein Salz davon mit einer Verbindung der Formel

$$R_3\text{–A–}Z_2 \qquad \text{(IIIe)},$$

worin $Z_2$ ein Amidinorest oder Ammoniumcarboxylat bedeutet, oder ein Salz davon gegebenenfalls mit Ammoniak umsetzt oder ein Oxazol der Formel

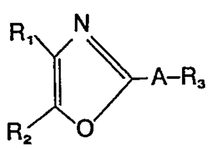

(IIIf)

mit Ammoniak umsetzt oder eine Verbindung der Formel

$$R_1\text{–C(NH}_2\text{)=C(NH}_2\text{)–}R_2 \qquad \text{(IIIk)}$$

oder ein Salz davon mit einer gegebenenfalls funktionell abgewandelten Verbindung der Formel

$$R_3\text{–A–COOH} \qquad \text{(IIIp)}$$

bzw. unter oxidierenden Bedingungen, mit einer Verbindung der Formel

$$R_3\text{–A–C(=O)–H} \qquad \text{(IIIb)}$$

umsetzt.

31. Die nach den Verfahren der Ansprüche 27–30 erhältlichen neuen Verbindungen.

32. Verbindungen der Formel

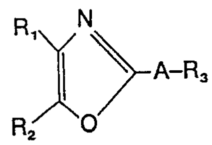

(IIIf),

worin einer der Reste $R_1$ und $R_2$ Phenyl, Pyrrolyl, Pyridyl, 1-Oxidopyridyl oder Pyrimidyl bedeutet, welche jeweils unsubstituiert oder durch Halogen, Niederalkyl, Hydroxy, Niederalkoxy und/oder Niederalkanoyloxy substituiert sein können, und der andere Pyrrolyl, Pyridyl, 1-Oxidopyridyl oder Pyrimidyl bedeutet, welche jeweils unsubstituiert oder durch Halogen, Niederalkyl, Hydroxy, Niederalkoxy und/oder Niederalkanoyloxy substituiert sein können und A Niederalkylen, Niederalkyliden, Niederalkenylen, Niederalkenyliden, Cycloalkylen, Cycloalkyliden oder Cycloalkyl-niederalkyliden darstellen, und $R_3$ Carboxy, mit einem Niederalkanol, 3- bis 8-gliedrigem Cycloalkanol, Phenol, einem Hydroxypyridin, einem substituierten Phenol bzw. Hydroxypyridin verestertes Carboxy, Carbamoyl oder durch Hydroxy, Amino, Phenyl oder substituiertes Phenyl mono-substituiertes, durch Niederalkyl mono- oder disubstituiertes oder durch 4- bis 7-gliedriges Alkylen bzw. 3-Aza-, 3-Niederalkylaza-, 3-Oxa- oder 3-Thiaalkylen disubstituiertes Carbamoyl bedeutet, wobei ein Niederalkanol bzw. Cycloalkanol unsubstituiert oder durch Hydroxy, Mercapto, gegebenenfalls substituiertes Phenyl, Niederalkoxy, gegebenenfalls im Phenylteil substituiertes Phenylniederalkoxy, Niederalkylthio, gegebenenfalls im Phenylteil substituiertes Phenylniederalkylthio, Hydroxyniederalkoxy, Niederalkoxyniederalkoxy, gegebenenfalls im Phenylteil substituiertes Phenylniederalkoxyniederalkoxy, Carboxyniederalkoxy, Niederalkoxycarbonyl-niederalkoxy, gegebenenfalls substituiertes Phenyl aufweisendes Niederalkoxycarbonyl-niederalkoxy oder Niederalkanoyloxy und substituiertes Phenyl, Phenol bzw. Hydroxypyridin jeweils durch Niederalkyl, Niederalkoxy, Halogen und/oder Trifluormethyl substituiert sein kann, ihre Isomeren sowie ihre Salze.

33. Verbindungen der Formel IIIf gemäss Anspruch 32, worin einer der Reste $R_1$ und $R_2$ Pyridyl oder 1-Oxido-pyridyl, die unsubstituiert und/oder jeweils durch Halogen, Hydroxy, Niederalkyl, Niederalkoxy und/oder Niederalkanoyloxy substituiert sein können, und der andere Phenyl, Pyridyl oder 1-Oxidopyridyl, die unsubstituiert und/oder jeweils durch Halogen, Hydroxy, Niederalkyl, Niederalkoxy und/oder Niederalkanoyloxy substituiert sein können, bedeutet, A Niederalkylen mit bis und mit 4 C-Atomen, Niederalkyliden mit bis und mit 7 C-Atomen, Niederalkenylen mit bis und mit 4 C-Atomen, Niederalkenyliden mit bis und mit 7 C-Atomen, 3- bis 8-gliedriges Cycloalkylen, 3- bis 8-gliedriges Cycloalkyliden oder Cycloalkylniederalkyliden mit bis und mit 7 C-Atomen im

Alkylidenteil und mit einem 3- bis 8-gliedrigen Cycloalkylteil bedeutet und $R_3$ Carboxy, mit einem Niederalkanol, einem 3- bis 8-gliedrigen Cycloalkanol, Phenol oder einem substituierten Phenol verestertes Carboxy, Carbamoyl, N-Mono-, N,N-Diniederalkylcarbamoyl, Pyrrolidino-, Piperidino-, Morpholino-, Piperazino-, 4-Niederalkyl-piperazino-, Thiomorpholino-, Anilino- oder durch Niederalkyl, Niederalkoxy und/oder Halogen substituiertes Anilinocarbonyl darstellt, wobei das Niederalkanol bzw. Cycloalkanol unsubstituiert oder durch Hydroxy, Mercapto, Phenyl, substituiertes Phenyl, Niederalkoxy, Phenylniederalkoxy, im Phenylteil substituiertes Phenylniederalkoxy, Niederalkylthio, Phenylniederalkylthio, im Phenylteil substituiertes Phenylniederalkylthio, Hydroxyniederalkoxy, Niederalkoxyniederalkoxy, Phenylniederalkoxyniederalkoxy, im Phenylteil substituiertes Phenylniederalkoxyniederalkoxy, Carboxyniederalkoxy, Niederalkoxycarbonyl-niederalkoxy, gegebenenfalls substituiertes Phenyl aufweisendes Niederalkoxycarbonyl-niederalkoxy oder Niederalkanoyloxy und substituiertes Phenol bzw. Phenyl jeweils durch Niederalkyl, Niederalkoxy, Halogen und/oder Trifluormethyl substituiert sein kann, ihre Isomeren sowie ihre Salze.

34. Verbindungen der Formel IIIf gemäss Anspruch 32, worin einer der Reste $R_1$ und $R_2$ Phenyl oder durch Halogen mit Atomnummer bis und mit 35, Hydroxy, Niederalkyl mit bis und mit 4 C-Atomen und/oder Niederalkoxy mit bis und mit 4 C-Atomen substituiertes Phenyl bedeutet und der andere Pyridyl oder 1-Oxido-pyridyl bedeutet, die jeweils unsubstituiert oder durch Halogen mit Atomnummer bis und mit 35, Hydroxy und/oder Niederalkoxy mit bis und mit 4 C-Atomen substituiert sein können, A Niederalkylen mit bis und mit 4 C-Atomen, Niederalkyliden mit bis und mit 7 C-Atomen, Niederalkenyliden mit bis und mit 7 C-Atomen, oder 3- bis 8-gliedriges Cycloniederalkyliden darstellen und $R_3$ Carboxy, Niederalkoxycarbonyl mit bis und mit 5 C-Atomen, Carbamoyl, N-Mononiederalkyl-carbamoyl mit bis und mit 4 C-Atomen im Niederalkyl oder N,N-Diniederalkylcarbamoyl jeweils mit bis und mit 4 C-Atomen in Niederalkyl bedeuten, wobei das Niederalkoxycarbonyl durch Niederalkanoyloxy mit bis und mit 5 C-Atomen substituiert sein kann, ihre Isomeren sowie ihre Salze.

35. Verbindungen der Formel IIIf gemäss Anspruch 32, worin einer der Reste $R_1$ und $R_2$ Phenyl oder durch Halogen mit Atomnummer bis und mit 35, Hydroxy oder Niederalkoxy mit bis und mit 4 C-Atomen substituiertes Phenyl bedeutet und der andere Pyridyl oder 1-Oxidopyridyl darstellt, A ein ein quartäres C-Atom aufweisendes Niederalkyliden mit bis und mit 4 C-Atomen, wobei das quartäre C-Atom direkt an den Imidazolring gebunden ist, bedeutet, und $R_3$ Niederalkoxycarbonyl mit bis und mit 5 C-Atomen darstellt, ihre Isomeren sowie ihre Salze.

36. 2-[4-Phenyl-5-(3-pyridyl)-oxazol-2-yl]-essigsäure-ethylester
oder ein Salz davon.

37. 2-[4-Phenyl-5-(1-oxido-3-pyridyl)-oxazol-2-yl]-2-methyl-propionsäureethylester
oder ein Salz davon.

38. Verbindungen der Formel IIIf gemäss einem der Ansprüche 32 bis 37 zur Anwendung in einem Verfahren zur therapeutischen Behandlung des tierischen oder menschlichen Körpers.

39. Verbindungen der Formel IIIf gemäss Anspruch 38 zur Anwendung als topische Hautphlogistatika.

40. Verwendung von Verbindungen der Formel IIIf gemäss einem der Ansprüche 32–37 zur Herstellung von Arzneimitteln.

41. Verfahren zur Herstellung von Verbindungen der Formel IIIf gemäss Anspruch 32, dadurch gekennzeichnet, dass man Verbindungen der Formel

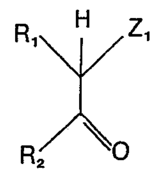

(IIId),

worin $Z_1$ gegebenenfalls reaktionsfähig verestertes Hydroxy bedeutet, mit Carbonsäuren der Formel $R_3$–A–COOH (IIIp), deren Salze oder deren funktionelle Derivate und mit Ammoniak umsetzt oder Verbindungen der Formel

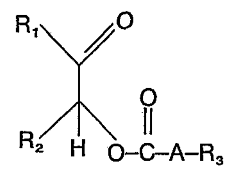

(IIIq),

welche durch Umsetzung von Verbindungen der Formel

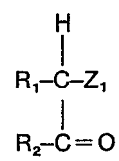

(IIId)

mit gegebenenfalls funktionellen Derivaten von Verbindungen der Formel

$$R_3\text{–A–COOH} \quad \text{(IIIp)}$$

erhältlich sind, mit Ammoniak umsetzt, gewünschtenfalls in einer so erhaltenen Verbindung der Formel IIIf nicht N-oxidierte Heteroarylreste $R_1$ und $R_2$ mit Hilfe eines Oxidationsmittels N-oxidiert, und gewünschtenfalls die verfahrensgemäss erhältliche freie Verbindung in ein Salz oder ein verfahrensgemäss erhältliches Salz in die freie Verbindung oder in ein anderes Salz überführt oder eine verfahrensgemäss erhältliche freie Verbindung in eine andere freie Verbindung überführt und/oder, wenn erwünscht, ein erfindungsgemäss erhältliches Gemisch von isomeren Verbindungen der Formel I in die einzelnen Isomeren auftrennt.

42. Verfahren gemäss Anspruch 41, dadurch gekennzeichnet, dass man von irgendeiner Stufe des Verfahrens ausgeht und die fehlenden Schritte durchführt oder einen Ausgangsstoff in Form eines Salzes und/oder Tautomeren verwendet und/oder unter den Reaktionsbedingungen bildet.

43. Die nach dem Verfahren der Ansprüche 41 und 42 erhältlichen neuen Verbindungen.

**Patentansprüche für den Vertragsstaat: AT**

1. Verfahren zur Herstellung von Verbindungen der Formel I

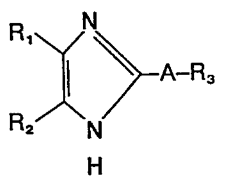

(I) ,

worin einerseits $R_1$ und $R_2$ unabhängig voneinander Phenyl und/oder durch Halogen, Niederalkyl, Hydroxy, Niederalkoxy und/oder Niederalkanoyl substituiertes Phenyl bedeuten oder worin andererseits einer der Reste $R_1$ und $R_2$ Pyrrolyl, Furyl, Thienyl, Pyridyl, 1-Oxidopyridyl oder Pyrimidyl bedeutet, welche jeweils unsubstituiert oder durch Halogen, Niederalkyl, Hydroxy, Niederalkoxy und/oder Niederalkanoyloxy substituiert sein können, und der andere Phenyl, Pyrrolyl, Furyl, Thienyl, Pyridyl, 1-Oxidopyridyl oder Pyrimidyl bedeutet, welche jeweils unsubstituiert oder durch Halogen, Niederalkyl, Hydroxy, Niederalkoxy und/oder Niederalkanoyloxy substituiert sein können, und jeweils A Niederalkylen, Niederalkyliden, Niederalkenylen, Niederalkenyliden, Cycloalkylen, Cycloalkyliden oder Cycloalkyl-niederalkyliden darstellen, und $R_3$ Carboxy, mit einem Niederalkanol, 3- bis 8-gliedrigem Cycloalkanol, Phenol, einem Hydroxypyridin, einem substituierten Phenol bzw. Hydroxypyridin verestertes Carboxy, Carbamoyl oder durch Hydroxy, Amino, Phenyl oder substituiertes Phenyl mono-substituiertes, durch Niederalkyl mono- oder disubstituiertes oder durch 4- bis 7-gliedriges Alkylen bzw. 3-Aza-, 3-Niederalkylaza-, 3-Oxa- oder 3-Thiaalkylen disubstituiertes Carbamoyl bedeutet, wobei ein Niederalkanol bzw. Cycloalkanol unsubstituiert oder durch Hydroxy, Mercapto, gegebenenfalls substituiertes Phenyl, Niederalkoxy, gegebenenfalls im Phenylteil substituiertes Phenylniederalkoxy, Niederalkylthio, gegebenenfalls im Phenylteil substituiertes Phenylniederalkylthio, Hydroxyniederalkoxy, Niederalkoxyniederalkoxy, gegebenenfalls im Phenylteil substituiertes Phenylniederalkoxyniederalkoxy, Carboxyniederalkoxy, Niederalkoxycarbonyl-niederalkoxy, gegebenenfalls substituiertes Phenyl aufweisendes Niederalkoxycarbonyl-niederalkoxy oder Niederalkanoyloxy und substituiertes Phenyl, Phenol bzw. Hydroxypyridin jeweils durch Niederalkyl, Niederalkoxy, Halogen und/oder Trifluormethyl substituiert sein kann, ihre Isomeren sowie ihre Salze, insbesondere pharmazeutisch verwendbare Salze, mit der Massgabe, dass, wenn A für Methylen bzw. Ethyliden und $R_3$ für Ethoxycarbonyl stehen, nicht beide Reste $R_1$ und $R_2$ zugleich Phenyl bedeuten, und der weiteren Massgabe, dass, wenn A für Ethyliden und $R_3$ für Carboxy bzw. Carbamoyl stehen, nicht beide Reste $R_1$ und $R_2$ zugleich Phenyl, p-Methoxyphenyl oder p-Chlorphenyl bedeuten, wobei unter mit «nieder» bezeichneten Resten solche zu verstehen sind, die bis und mit 7 C-Atome enthalten, dadurch gekennzeichnet, dass man aus einer Verbindung der Formel

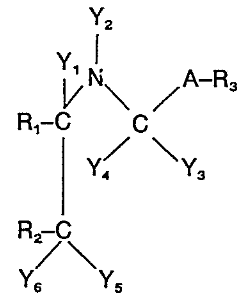

(II),

worin einer der Reste $Y_1$ und $Y_6$ Hydroxy oder Amino und der andere sowie $Y_2$ Wasserstoff darstellt und $Y_3$ gemeinsam mit $Y_4$ und $Y_5$ eine Gruppe der Formel =N– bedeutet oder worin $Y_1$ gemeinsam mit $Y_6$ eine Bindung darstellt, $Y_2$ Wasserstoff ist, $Y_3$ Hydroxy oder Amino bedeutet und $Y_4$ gemeinsam mit $Y_5$ eine Gruppe der Formel –NH– darstellt oder worin $Y_1$ gemeinsam mit $Y_6$ eine Bindung darstellt, $Y_2$ gemeinsam mit $Y_3$ eine zusätzliche Bindung und einer der Reste $Y_4$ und $Y_5$ Amino und der andere Amino, Hydroxy oder reaktionsfähiges verestertes Hydroxy, darstellt oder worin $Y_1$ Hydroxy ist, $Y_2$ sowie $Y_3$ Wasserstoff bedeutet, $Y_4$ Hydroxy oder Amino darstellt und $Y_5$ gemeinsam mit $Y_6$ eine Gruppe der Formel =NH oder, sofern $Y_4$ Amino ist, Oxo bedeutet, oder einem Tautomeren und/oder Salz davon, unter Einführung einer gegebenenfalls zusätzlichen Bindung H–Z abspaltet, wobei Z Hydroxy oder Amino $Y_1$ bzw. $Y_6$, $Y_3$ oder $Y_4$ bzw. $Y_5$ oder verestertes Hydroxy $Y_4$ bzw. $Y_5$ bedeutet, oder eine Verbindung der Formel

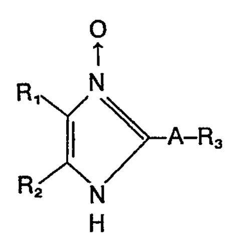

(IV)

oder ein Salz davon zu einer Verbindung der Formel I reduziert oder in einer Verbindung der Formel

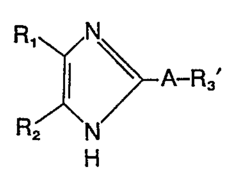

(V),

worin $R_3'$ einen im $R_3$ überführbaren Rest bedeutet, den Rest $R_3'$ in einen Rest $R_3$ überführt oder in einer Verbindung der Formel

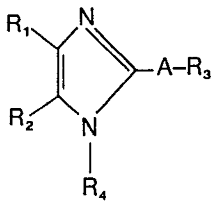
(VI),

worin $R_4$ eine in Wasserstoff überführbare Gruppe darstellt, oder einem Salz davon $R_4$ Wasserstoff überführt oder indem man Verbindungen der Formel

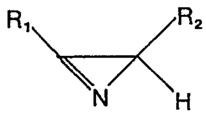
(VIIa)

oder Tautomere davon mit Verbindungen der Formel $R_3$–A–CN (VIIb) umsetzt oder eine Verbindung der Formel

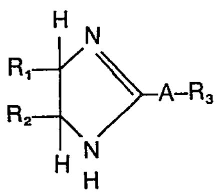
(VIII)

oder ein Isomeres davon zu Verbindungen der Formel I dehydriert und gewünschtenfalls die verfahrensgemäss erhältliche freie Verbindung in ein Salz oder ein verfahrensgemäss erhältliches Salz in die freie Verbindung oder in ein anderes Salz überführt oder eine verfahrensgemäss erhältliche freie Verbindung in eine andere freie Verbindung überführt und/oder, wenn erwünscht, ein erfindungsgemäss erhältliches Gemisch von isomeren Verbindungen der Formel I in die einzelnen Isomeren auftrennt.

2. Verfahren gemäss Anspruch 1, dadurch gekennzeichnet, dass man ein Diketon der Formel

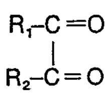
(IIIa)

mit einem Aldehyd der Formel $R_3$–A–C(=O)–H (IIIb) oder einem Salz davon, mit einem Überschuss an Ammoniak unter Erwärmen umsetzt oder ein acyliertes α-Aminoketon der Formel

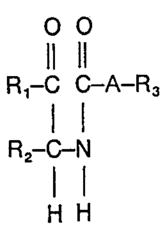
(IIIc)

oder ein Salz davon mit Ammoniak oder eine Verbindung der Formel

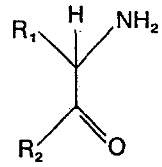
(IIIh)

oder ein Salz davon mit einem Säurederivat der Formel $R_3$–A–COOH (IIIi) umsetzt oder eine Verbindung der Formel

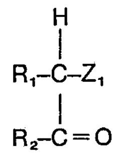
(IIId),

worin $Z_1$ reaktionsfähiges verestertes Hydroxy bedeutet, oder ein Salz davon mit einer Verbindung der Formel

$R_3$–A–$Z_2$ (IIIe),

worin $Z_2$ ein Amidinorest oder Ammoniumcarboxylat bedeutet, oder ein Salz davon gegebenenfalls mit Ammoniak umsetzt oder ein Oxazol der Formel

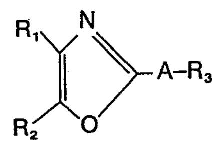
(IIIf)

mit Ammoniak umsetzt oder eine Verbindung der Formel $R_1$–C($NH_2$)=C($NH_2$)–$R_2$ (IIIk) oder ein Salz davon mit einer gegebenenfalls funktionell abgewandelten Verbindung der Formel

$R_3$–A–COOH (IIIp)

bzw., unter oxidierenden Bedingungen, mit einer Verbindung der Formel

$R_3$–A–C(=O)–H (IIIb)

umsetzt.

3. Verfahren gemäss einem der Ansprüche 1–2, dadurch gekennzeichnet, dass die Ausgangsstoffe der Formel II, ihre Tautomeren und/oder Salze nach an sich bekannten Verfahren zum überwiegenden Teil in situ gebildet und unter den Reaktionsbedingungen ohne Isolierung weiter zu der Verbindung der Formel I umgesetzt werden.

4. Verfahren gemäss einem der Ansprüche 1–3, dadurch gekennzeichnet, dass man von irgendeiner Stufe des Verfahrens ausgeht und die fehlenden Schritte durchführt oder einen Ausgangsstoff in Form eines Salzes und/oder Tautomeren verwendet und/oder unter den Reaktionsbedingungen bildet.

5. Verfahren gemäss Anspruch 1 zur Herstellung von Verbindungen der Formel I, worin

einerseits $R_1$ und $R_2$ unabhängig voneinander Phenyl und/oder durch Halogen, Niederalkyl, Hydroxy, Niederalkoxy und/oder Niederalkanoyl

substituiertes Phenyl bedeuten oder worin andererseits einer der Reste $R_1$ und $R_2$ Pyrrolyl, Furyl, Thienyl, Pyridyl, 1-Oxidopyridyl oder Pyrimidyl bedeutet, welche jeweils unsubstituiert oder durch Halogen, Niederalkyl, Hydroxy, Niederalkoxy und/oder Niederalkanoyloxy substituiert sein können, und der andere Phenyl, Pyrrolyl, Furyl, Thienyl, Pyridyl, 1-Oxidopyridyl oder Pyrimidyl bedeutet, welche jeweils unsubstituiert oder durch Halogen, Niederalkyl, Hydroxy, Niederalkoxy und/oder Niederalkanoyloxy substituiert sein können, und jeweils A Niederalkylen, Niederalkyliden, Niederalkenylen, Niederalkenyliden, Cycloalkylen, Cycloalkyliden oder Cycloalkyl-niederalkyliden darstellen, und $R_3$ Carboxy, mit einem Niederalkanol, 3- bis 8-gliedrigem Cycloalkanol, Phenol, einem Hydroxypyridin, einem substituierten Phenol bzw. Hydroxypyridin verestertes Carboxy, bedeutet, wobei ein Niederalkanol bzw. Cycloalkanol unsubstituiert oder

durch Hydroxy, Mercapto, gegebenenfalls substituiertes Phenyl, Niederalkoxy, gegebenenfalls im Phenylteil substituiertes Phenylniederalkoxy, Niederalkylthio, gegebenenfalls im Phenylteil substituiertes Phenylniederalkylthio, Hydroxyniederalkoxy, Niederalkoxyniederalkoxy, gegebenenfalls im Phenylteil substituiertes Phenylniederalkoxyniederalkoxy, oder Niederalkanoyloxy und substituiertes Phenyl, Phenol bzw. Hydroxypyridin jeweils durch Niederalkyl, Niederalkoxy, Halogen und/oder Trifluormethyl substituiert sein kann, ihre Isomeren sowie ihre Salze, insbesondere pharmazeutisch verwendbare Salze, mit der Massgabe, dass, wenn A für Methylen bzw. Ethyliden und $R_3$ für Ethoxycarbonyl stehen, nicht beide Reste $R_1$ und $R_2$ zugleich Phenyl bedeuten, und der weiteren Massgabe, dass, wenn A für Ethyliden und $R_3$ für Carboxy bzw. Carbamoyl stehen, nicht beide Reste $R_1$ und $R_2$ zugleich Phenyl, p-Methoxyphenyl oder p-Chlorphenyl bedeuten, dadurch gekennzeichnet, dass man

a) aus einer Verbindung der Formel

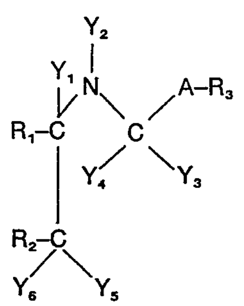

(II),

worin einer der Reste $Y_1$ und $Y_6$ Hydroxy oder Amino und der andere sowie $Y_2$ Wasserstoff darstellt und $Y_3$ gemeinsam mit $Y_4$ und $Y_5$ eine Gruppe der Formel $=N-$ bedeutet oder worin $Y_1$ gemeinsam mit $Y_6$ eine Bindung darstellt, $Y_2$ Wasserstoff ist, $Y_3$ Hydroxy oder Amino bedeutet und $Y_4$ gemeinsam mit $Y_5$ eine Gruppe der Formel $-NH-$ darstellt oder $Y_2$ gemeinsam mit $Y_3$ eine zusätzliche Bindung und einer der Reste $Y_4$ und $Y_5$ Amino und der andere Amino, Hydroxy, Halogen oder Sulfonyloxy darstellt oder worin $Y_1$ Hydroxy ist, $Y_2$ sowie $Y_3$ Wasserstoff bedeutet, $Y_4$ Hydroxy oder Amino darstellt und $Y_5$ gemeinsam mit $Y_6$ eine Gruppe der Formel $=NH-$ oder, sofern $Y_4$ Amino ist, Oxo bedeutet, oder einem Tautomeren und/oder Salz davon, unter Einführung einer gegebenenfalls zusätzlichen Bindung H–Z abspaltet, wobei Z Hydroxy oder Amino $Y_1$ bzw. $Y_6$, $Y_3$ oder $Y_4$ bzw. $Y_5$ oder Halogen bzw. Sulfonyloxy $Y_4$ bzw. $Y_5$ bedeutet,

b) eine Verbindung der Formel

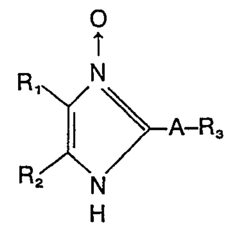

(IV)

oder ein Salz davon zu einer Verbindung der Formel I reduziert,

c) in einer Verbindung der Formel

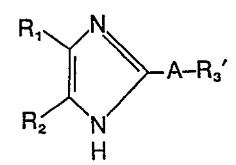

(V),

worin $R_3'$ einen im $R_3$ überführbaren Rest bedeutet, den Rest $R_3'$ in einen Rest $R_3$ überführt oder

d) in einer Verbindung der Formel

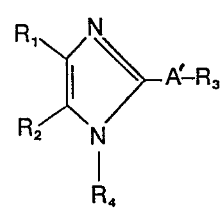

(VI),

worin A' Niederalkyliden, Niederalkenyliden, Cycloalkyliden oder Cycloalkyl-niederalkyliden bedeutet und $R_4$ eine in Wasserstoff überführbare Gruppe darstellt, oder einem Salz davon $R_4$ in Wasserstoff überführt und gewünschtenfalls die verfahrensgemäss erhältliche freie Verbindung in ein Salz oder ein verfahrensgemäss erhältliches Salz in die freie Verbindung oder in ein anderes Salz überführt.

6. Verfahren gemäss Anspruch 5, dadurch gekennzeichnet, dass man von irgendeiner Stufe des Verfahrens ausgeht und die fehlenden Schritte durchführt oder einen Ausgangstoff in Form eines Salzes, und/oder Tautomeren verwendet oder unter Reaktionsbedingungen bildet.

7. Verfahren gemäss einem der Ansprüche 1–4, zur Herstellung von Verbindungen der Formel I, worin $R_1$ und $R_2$ unabhängig voneinander Phenyl und/oder durch Halogen, Hydroxy, Niederalkyl, Niederalkoxy und/oder Niederalkanoyloxy substituiertes Phenyl bedeuten, A Niederalkylen mit bis und mit 4 C-Atomen, Niederalkyliden mit bis und mit 7 C-Atomen, Niederalkenylen mit bis und mit

4 C-Atomen, Niederalkenyliden mit bis und mit 7 C-Atomen, 3- bis 8-gliedriges Cycloalkylen, 3- bis 8-gliedriges Cycloalkyliden oder Cycloalkylniederalkyliden mit bis und mit 7 C-Atomen im Alkylidenteil und mit einem 3- bis 8-gliedrigen Cycloalkylteil bedeutet und $R_3$ Carboxy, mit einem Niederalkanol einem 3- bis 8-gliedrigen Cycloalkanol, Phenol oder einem substituierten Phenol verestertes Carboxy, Carbamoyl, N-Mono-, N,N-Diniederalkylcarbamoyl, Pyrrolidino-, Piperidino-, Morpholino-, Piperazino-, 4-Niederalkyl-piperazino-, Thiomorpholino-, Anilino- oder durch Niederalkyl, Niederalkoxy und/oder Halogen substituiertes Anilinocarbonyl darstellt, wobei das Niederalkanol bzw. Cycloalkanol unsubstituiert oder durch Hydroxy, Mercapto, Phenyl, substituiertes Phenyl, Niederalkoxy, Phenylniederalkoxy, im Phenylteil substituiertes Phenylniederalkoxy, Niederalkylthio, Phenylniederalkylthio, im Phenylteil substituiertes Phenylniederalkylthio, Hydroxyniederalkoxy, Niederalkoxyniederalkoxy, Phenylniederalkoxyniederalkoxy, im Phenylteil substituiertes Phenylniederalkoxyniederalkoxy, Carboxyniederalkoxy, Niederalkoxycarbonyl-niederalkoxy, gegebenenfalls substituiertes Phenyl aufweisendes Niederalkoxycarbonyl-niederalkoxy oder Niederalkanoyloxy und substituiertes Phenol bzw. Phenyl jeweils durch Niederalkyl, Niederalkoxy, Halogen und/oder Trifluormethyl substituiert sein kann, ihrer Isomeren sowie ihrer Salze, insbesondere pharmazeutisch verwendbare Salze, mit der Massgabe, dass, wenn A für Methylen bzw. Ethyliden und $R_3$ für Ethoxycarbonyl stehen, nicht beide Reste $R_1$ und $R_2$ zugleich Phenyl bedeuten, und der weiteren Massgabe, dass, wenn A für Ethyliden und $R_3$ für Carboxy bzw. Carbamoyl stehen, nicht beide Reste $R_1$ und $R_2$ zugleich Phenyl, p-Methoxyphenyl oder p-Chlorphenyl bedeuten, dadurch gekennzeichnet, dass man von Verbindungen der Formeln II bzw. IIIa und IIIb, IIIc, IIIh und IIIi, IIId und IIIe, IIIk und IIIp bzw. IIIb sowie IIIf oder V oder VI oder VIIa und VIIb oder VIII ausgeht, worin die Variablen $Y_1$–$Y_6$, $R_3'$ und $R_4$ die in Anspruch 1 und die Variablen $Z_1$ und $Z_2$ die in Anspruch 2 angegebenen Bedeutungen haben.

8. Verfahren gemäss einem der Ansprüche 1–4, zur Herstellung von Verbindungen der Formel I, worin einer der Reste $R_1$ und $R_2$ Pyridyl oder 1-Oxido-pyridyl, die unsubstituiert und/oder jeweils durch Halogen, Hydroxy, Niederalkyl, Niederalkoxy und/oder Niederalkanoyloxy substituiert sein können, und der andere Phenyl, Pyridyl oder 1-Oxidopyridyl, die unsubstituiert und/oder jeweils durch Halogen, Hydroxy, Niederalkyl, Niederalkoxy und/oder Niederalkanoyloxy substituiert sein können, bedeutet, A Niederalkylen mit bis und mit 4 C-Atomen, Niederalkyliden mit bis und mit 7 C-Atomen, Niederalkenylen mit bis und mit 4 C-Atomen, Niederalkenyliden mit bis und mit 7 C-Atomen, 3- bis 8-gliedriges Cycloalkylen, 3- bis 8-gliedriges Cycloalkyliden oder Cycloalkylniederalkyliden mit bis und mit 7 C-Atomen im Alkylidenteil und mit einem 3- bis 8-gliedrigen Cycloalkylteil bedeutet und $R_3$ Carboxy, mit einem Niederalkanol, einem 3- bis 8-gliedrigen Cycloalkanol, Phenol oder einem substituierten Phenol verestertes Carboxy, Carbamoyl, N-Mono-, N,N-Diniederalkylcarbamoyl, Pyrrolidino-, Piperidino-, Morpholino-, Piperazino-, 4-Niederalkyl-piperazino-, Thiomorpholino-, Anilino- oder durch Niederalkyl, Niederalkoxy und/oder Halogen substituiertes Anilinocarbonyl darstellt, wobei das Niederalkanol bzw. Cycloalkanol unsubstituiert oder durch Hydroxy, Mercapto, Phenyl, substituiertes Phenyl, Niederalkoxy, Phenylniederalkoxy, im Phenylteil substituiertes Phenylniederalkoxy, Niederalkylthio, Phenylniederalkylthio, im Phenylteil substituiertes Phenylniederalkylthio, Hydroxyniederalkoxy, Niederalkoxyniederalkoxy, Phenylniederalkoxyniederalkoxy, im Phenylteil substituiertes Phenylniederalkoxyniederalkoxy, Carboxyniederalkoxy, Niederalkoxycarbonyl-niederalkoxy, gegebenenfalls substituiertes Phenyl aufweisendes Niederalkoxycarbonyl-niederalkoxy oder Niederalkanoyloxy und substituiertes Phenol bzw. Phenyl jeweils durch Niederalkyl, Niederalkoxy, Halogen und/oder Trifluormethyl substituiert sein kann, ihre Isomeren sowie ihre Salze, insbesondere pharmazeutisch verwendbare Salze, dadurch gekennzeichnet, dass man Verbindungen der Formeln II bzw. IIIa und IIIb, IIIc, IIIh und IIIi, IIId und IIIe, IIIk und IIIp bzw. IIIb sowie IIIf oder V oder VI oder VIIa und VIIb oder VIII ausgeht, worin die Variablen $Y_1$–$Y_6$, $R_3'$ und $R_4$ die in Anspruch 1 und die Variablen $Z_1$ und $Z_2$ die in Anspruch 2 angegebenen Bedeutungen haben.

9. Verfahren gemäss einem der Ansprüche 5 und 6, zur Herstellung von Verbindungen der Formel I, worin $R_1$ und $R_2$ unabhängig voneinander gegebenenfalls durch Halogen mit Atomnummer bis und mit 35, Hydroxy, Niederalkyl mit bis und mit 4 C-Atomen, Niederalkoxy mit bis und mit 4 C-Atomen und/oder Niederalkanoyloxy mit bis und mit 5 C-Atomen substituiertes Phenyl, Pyridyl, und/oder 1-Oxidopyridyl, bedeuten, A Niederalkylen mit bis und mit 4 C-Atomen, oder Niederalkyliden mit bis und mit 4 C-Atomen bedeutet, $R_3$ gegebenenfalls mit einem Niederalkanol mit bis und mit 4 C-Atomen verestertes Carboxy darstellt, wobei das Niederalkanol gegebenenfalls durch Hydroxy, Mercapto, gegebenenfalls substituiertes Phenyl, Niederalkoxy mit bis und mit 4 C-Atomen, gegebenenfalls im Phenylteil substituiertes Phenylniederalkoxy mit bis und mit 4 C-Atomen im Alkylteil, Niederalkylthio mit bis und mit 4 C-Atomen, Hydroxyniederalkoxy mit bis und mit 4 C-Atomen im Niederalkylteil, Niederalkoxyniederalkoxy jeweils mit bis und mit 4 C-Atomen im Niederalkylteil oder Niederalkanoyloxy mit bis und mit 5 C-Atomen und Phenyl gegebenenfalls durch Niederalkyl mit bis und mit 4 C-Atomen, Niederalkoxy mit bis und mit 4 C-Atomen, Halogen mit Atomnummer bis und mit 35 und/oder Trifluormethyl substituiert sein kann, ihrer Isomeren sowie ihrer Salze, mit der Massgabe, das, wenn A für Methylen oder Ethyliden und $R_3$ für Ethoxycarbonyl stehen, mindestens einer der Reste $R_1$ und $R_2$ von Phenyl verschieden ist, und der weiteren Massgabe, dass, wenn A für Ethyliden und $R_3$ für Carboxy stehen, mindestens einer der Reste $R_1$

und $R_2$ von Phenyl, p-Methoxyphenyl und p-Chlorphenyl verschieden ist, dadurch gekennzeichnet, dass man von Verbindungen der Formeln II, IV, V oder VI ausgeht, worin die Variablen $Y_1$–$Y_6$, $R_3'$ und $R_4$ die in Anspruch 5 angegebenen Bedeutungen haben.

10. Verfahren gemäss einem der Ansprüche 1–4 zur Herstellung von Verbindungen der Formel I, worin $R_1$ und $R_2$ unabhängig voneinander Phenyl und/oder durch Halogen mit Atomnummer bis und mit 35, Hydroxy, Niederalkyl mit bis und mit 4 C-Atomen und/oder Niederalkoxy mit bis und mit 4 C-Atomen substituiertes Phenyl bedeuten, A Niederalkylen mit bis und mit 4 C-Atomen, Niederalkyliden mit bis und mit 7 C-Atomen, Niederalkenyliden mit bis und mit 7 C-Atomen oder 3- bis 8-gliedriges Cycloniederalkyliden darstellen und $R_3$ Carboxy, Niederalkoxycarbonyl mit bis und mit 5 C-Atomen, Carbamoyl, N-Mononiederalkylcarbamoyl mit bis und mit 4 C-Atomen im Niederalkyl oder N,N-Diniederalkylcarbamoyl jeweils mit bis und mit 4 C-Atomen im Niederalkyl bedeuten, wobei das Niederalkoxycarbonyl durch Niederalkanoyloxy mit bis und mit 5 C-Atomen substituiert sein kann, ihrer Isomeren sowie ihrer Salze, insbesondere pharmazeutisch verwendbare Salze, mit der Massgabe, dass, wenn A für Methylen oder Ethyliden und $R_3$ für Ethoxycarbonyl stehen, mindestens einer der Reste $R_1$ und $R_2$ von Phenyl verschieden ist, und der weiteren Massgabe, 228dass, wenn A für Ethyliden und $R_3$ für Carboxy stehen, mindestens einer der Reste $R_1$ und $R_2$ von Phenyl, p-Methoxyphenyl und p-Chlorphenyl verschieden ist, dadurch gekennzeichnet, dass man von Verbindungen der Formeln II bzw. IIIa und IIIb, IIIc, IIIh und IIIi, IIId und IIIe, IIIk und IIIp bzw. IIIb sowie IIIf oder V oder VI oder VIIa und VIIb oder VIII ausgeht, worin die Variablen $Y_1$–$Y_6$, $R_3'$ und $R_4$ die im Anspruch 1 und die Variablen $Z_1$ und $Z_2$ die in Anspruch 2 angegebenen Bedeutungen haben.

11. Verfahren gemäss einem der Ansprüche 1–4, zur Herstellung von Verbindungen der Formel I, worin einer der Reste $R_1$ und $R_2$ Phenyl oder durch Halogen mit Atomnummer bis und mit 35, Hydroxy, Niederalkyl mit bis und mit 4 C-Atomen und/oder Niederalkoxy mit bis und mit 4 C-Atomen substituiertes Phenyl bedeutet und der andere Pyridyl oder 1-Oxido-pyridyl bedeutet, die jeweils unsubstituiert oder durch Halogen mit Atomnummer bis und mit 35, Hydroxy und/oder Niederalkoxy mit bis und mit 4 C-Atomen substituiert sein können, A Niederalkylen mit bis und mit 4 C-Atomen, Niederalkyliden mit bis und mit 7 C-Atomen, Niederalkenyliden mit bis und mit 7 C-Atomen, oder 3- bis 8-gliedriges Cycloniederalkyliden darstellen und $R_3$ Carboxy, Niederalkoxycarbonyl mit bis und mit 5 C-Atomen, Carbamoyl, N-Mononiederalkyl-carbamoyl mit bis und mit 4 C-Atomen im Niederalkyl, oder N,N-Diniederalkyl-carbamoyl jeweils mit bis und mit 4 C-Atomen in Niederalkyl bedeuten, wobei das Niederalkoxycarbonyl durch Niederalkanoyloxy mit bis und mit 5 C-Atomen substituiert sein kann, ihrer Isomeren sowie ihrer Salze, insbesondere pharmazeutisch verwendbare Salze, dadurch gekennzeichnet, dass man von Verbindungen der Formeln II bzw. IIIa und IIIb, IIIc, IIIh und IIIi, IIId und IIIe, IIIk und IIIp bzw. IIIb sowie IIIf oder V oder VI oder VIIa und VIIb oder VIII ausgeht, worin die Variablen $Y_1$–$Y_6$, $R_3'$ und $R_4$ die in Anspruch 1 und die Variablen $Z_1$ und $Z_2$ die in Anspruch 2 angegebenen Bedeutungen haben.

12. Verfahren gemäss einem der Ansprüche 1–4, zur Herstellung von Verbindungen der Formel I, worin $R_1$ und $R_2$ unabhängig voneinander Phenyl und/oder durch Niederalkoxy mit bis und mit 4 C-Atomen substituiertes Phenyl bedeuten, A Niederalkylen mit bis und mit 4 C-Atomen oder Niederalkyliden mit bis und mit 4 C-Atomen darstellt und $R_3$ Carboxy oder Niederalkoxycarbonyl mit bis und mit 5 C-Atomen bedeutet, ihrer Isomeren sowie ihrer Salze, insbesondere pharmazeutisch verwendbare Salze, mit der Massgabe, dass, wenn A für Methylen oder Ethyliden und $R_3$ für Ethoxycarbonyl steht, mindestens einer der Reste $R_1$ und $R_2$ von Phenyl verschieden ist, und der weiteren Massgabe, dass, wenn A für Ethyliden und $R_3$ für Carboxy stehen, mindestens einer der Reste $R_1$ und $R_2$ von Phenyl und p-Methoxyphenyl verschieden ist, dadurch gekennzeichnet, dass man von Verbindungen der Formeln II bzw. IIIa und IIIb, IIIc, IIIh und IIIi, IIId und IIIe, IIIk und IIIp bzw. IIIb sowie IIIf oder V oder VI oder VIIa und VIIb oder VIII ausgeht, worin die Variablen $Y_1$–$Y_6$, $R_3'$ und $R_4$ die in Anspruch 1 und die Variablen $Z_1$ und $Z_2$ die in Anspruch 2 angegebenen Bedeutungen haben.

13. Verfahren gemäss einem der Ansprüche 1–4, zur Herstellung von Verbindungen der Formel I, worin einer der Reste $R_1$ und $R_2$ Phenyl oder durch Halogen mit Atomnummer bis und mit 35, Hydroxy oder Niederalkoxy mit bis und mit 4 C-Atomen substituiertes Phenyl bedeutet und der andere Pyridyl oder 1-Oxidopyridyl darstellt, A ein ein quartäres C-Atom aufweisendes Niederalkyliden mit bis und mit 4 C-Atomen, wobei das quartäre C-Atom direkt an den Imidazolring gebunden ist, bedeutet, und $R_3$ Niederalkoxycarbonyl mit bis und mit 5 C-Atomen darstellt, ihrer Isomeren sowie ihrer Salze, insbesondere pharmazeutisch verwendbare Salze, dadurch gekennzeichnet, dass man von Verbindungen der Formeln II bzw. IIIa und IIIb, IIIc, IIIh und IIIi, IIId und IIIe, IIIk und IIIp bzw. IIIb sowie IIIf oder V oder VI oder VIIa und VIIb oder VIII ausgeht, worin die Variablen $Y_1$–$Y_6$, $R_3'$ und $R_4$ die in Anspruch 1 und die Variablen $Z_1$ und $Z_2$ die in Anspruch 2 angegebenen Bedeutungen haben.

14. Verfahren gemäss einem der Ansprüche 1–4, zur Herstellung von Verbindungen der Formel I, worin einer der Reste $R_1$ und $R_2$ Phenyl und der andere 1-Oxidopyridyl bedeutet, A 2,2-Propyliden darstellt und $R_3$ Niederalkoxycarbonyl mit bis und mit 5 C-Atomen bedeutet, ihrer Isomeren sowie ihrer Salze, insbesondere pharmazeutisch verwendbare Salze, dadurch gekennzeichnet, dass man von Verbindungen der Formeln II bzw. IIIa und IIIb, IIIc, IIIh und IIIi, IIId und IIIe, IIIk und IIIp bzw. IIIb sowie IIIf oder V oder VI oder VIIa und VIIb oder VIII ausgeht, worin die Variablen $Y_1$–$Y_6$, $R_3'$

und $R_4$ die in Anspruch 1 und die Variablen $Z_1$ und $Z_2$ die in Anspruch 2 angegebenen Bedeutungen haben.

15. Verfahren gemäss einem der Ansprüche 5 und 6 zur Herstellung von
2-[4(5)-Phenyl-5(4)-(3-pyridyl)-imidazol-2-yl]-2-methyl-propionsäureethylester
oder eines Salzes davon, dadurch gekennzeichnet, dass man von Verbindungen der Formeln II, IV, V oder VI ausgeht, worin die Variablen $Y_1$–$Y_6$, $R_3'$ und $R_4$ die im Anspruch 5 angegebenen Bedeutungen haben.

16. Verfahren gemäss einem der Ansprüche 1–4 zur Herstellung von
2-[4(5)-Phenyl-5(4)-(3-pyridyl)-imidazol-2-yl]-2-methyl-propionsäureethylester
oder eines Salzes davon, dadurch gekennzeichnet, dass man von Verbindungen der Formeln II bzw. IIIa und IIIb, IIIc, IIIh und IIIi, IIId und IIIe, IIIk und IIIp bzw. IIIb sowie IIIf oder V oder VI oder VIIa und VIIb oder VIII ausgeht, worin die Variablen $Y_1$–$Y_6$, $R_3'$ und $R_4$ die in Anspruch 1 und die Variablen $Z_1$ und $Z_2$ die in Anspruch 2 angegebenen Bedeutungen haben.

17. Verfahren gemäss einem der Ansprüche 5 und 6 zur Herstellung von
2-[4(5)-Phenyl-5(4)-(3-pyridyl)-imidazol-2-yl]-2-allyl-essigsäureethylester
oder eines Salzes davon, dadurch gekennzeichnet, dass man von Verbindungen der Formeln, II, IV, V oder VI ausgeht, worin die Variablen $Y_1$–$Y_6$, $R_3'$ und $R_4$ die im Anspruch 5 angegebenen Bedeutungen haben.

18. Verfahren gemäss einem der Ansprüche 1–4 zur Herstellung von
2-[4(5)-Phenyl-5(4)-(3-pyridyl)-imidazol-2-yl]-2-allyl-essigsäureethylester,
dadurch gekennzeichnet, dass man von Verbindungen der Formeln II bzw. IIIa und IIIb, IIIc, IIIh und IIIi, IIId und IIIe, IIIk und IIIp bzw. IIIb sowie IIIf oder V oder VI oder VIIa und VIIb oder VIII ausgeht, worin die Variablen $Y_1$–$Y_6$, $R_3'$ und $R_4$ die in Anspruch 1 und die Variablen $Z_1$ und $Z_2$ die in Anspruch 2 angegebenen Bedeutungen haben.

19. Verfahren gemäss einem der Ansprüche 5 und 6 zur Herstellung von
1-[4(5)-Phenyl-5(4)-(3-pyridyl)-imidazol-2-yl]-1-carbethoxycyclopentan,
oder eines Salzes davon, dadurch gekennzeichnet, dass man von Verbindungen der Formeln II, IV, V oder VI ausgeht, worin die Variablen $Y_1$–$Y_6$, $R_3'$ und $R_4$ die in Anspruch 5 angegebenen Bedeutungen haben.

20. Verfahren gemäss einem der Ansprüche 1–4 zur Herstellung von
1-[4(5)-Phenyl-5(4)-(3-pyridyl)-imidazol-2-yl]-1-carbethoxycyclopentan
oder eines Salzes davon, dadurch gekennzeichnet, dass man von Verbindungen der Formeln II bzw. IIIa und IIIb, IIIc, IIIh und IIIi, IIId und IIIe, IIIk und IIIp bzw. IIIb sowie IIIf oder V oder VI oder VIIa und VIIb oder VIII ausgeht, worin die Variablen $Y_1$–$Y_6$, $R_3'$ und $R_4$ die in Anspruch 1 und die Variablen $Z_1$ und $Z_2$ die in Anspruch 2 angegebenen Bedeutungen haben.

21. Verfahren gemäss einem der Ansprüche 5 und 6 zur Herstellung von
2-[4(5)-(p-Methoxyphenyl)-5(4)-(3-pyridyl)-imidazol-2-yl]-2-methyl-propionsäureethylester
oder eines Salzes davon, dadurch gekennzeichnet, dass man von Verbindungen der Formeln II, IV, V oder VI ausgeht, worin die Variablen $Y_1$–$Y_6$, $R_3'$ und $R_4$ die in Anspruch 5 angegebenen Bedeutungen haben.

22. Verfahren gemäss einem der Ansprüche 1–4 zur Herstellung von
2-[4(5)-(p-Methoxyphenyl)-5(4)-(3-pyridyl)-imidazol-2-yl]-2-methyl-propionsäureethylester
oder eines Salzes davon, dadurch gekennzeichnet, dass man von Verbindungen der Formeln II bzw. IIIa und IIIb, IIIc, IIIh und IIIi, IIId und IIIe, IIIk und IIIp bzw. IIIb sowie IIIf oder V oder VI oder VIIa und VIIb oder VIII ausgeht, worin die Variablen $Y_1$–$Y_6$, $R_3'$ und $R_4$ die in Anspruch 1 und die Variablen $Z_1$ und $Z_2$ die in Anspruch 2 angegebenen Bedeutungen haben.

23. Verfahren gemäss einem der Ansprüche 5 und 6 zur Herstellung von
2-[4(5)-Phenyl-5(4)-(1-oxido-3-pyridyl)-imidazol-2-yl]-2-methyl-propionsäureethylester
oder eines Salzes davon, dadurch gekennzeichnet, dass man von Verbindungen der Formeln II, IV, V oder VI ausgeht, worin die Variablen $Y_1$–$Y_6$, $R_3'$ und $R_4$ die in Anspruch 5 angegebenen Bedeutungen haben.

24. Verfahren gemäss einem der Ansprüche 1–4 zur Herstellung von
2-[4(5)-Phenyl-5(4)-(1-oxido-3-pyridyl)-imidazol-2-yl]-2-methyl-propionsäureethylester
oder eines Salzes davon, dadurch gekennzeichnet, dass man von Verbindungen der Formeln II bzw. IIIa und IIIb, IIIc, IIIh und IIIi, IIId und IIIe, IIIk und IIIp bzw. IIIb sowie IIIf oder V oder VI oder VIIa und VIIb oder VIII ausgeht, worin die Variablen $Y_1$–$Y_6$, $R_3'$ und $R_4$ die in Anspruch 1 und die Variablen $Z_1$ und $Z_2$ die in Anspruch 2 angegebenen Bedeutungen haben.

25. Verfahren gemäss einem der Ansprüche 5 und 6 zur Herstellung von
2-[4(5)-Phenyl-5(4)-(3-pyridyl)-imidazol-2-yl]-propionsäureethylester
oder eines Salzes davon, dadurch gekennzeichnet, dass man von Verbindungen der Formeln II, IV, V oder VI ausgeht, worin die Variablen $Y_1$–$Y_6$, $R_3'$ und $R_4$ die in Anspruch 5 angegebenen Bedeutungen haben.

26. Verfahren gemäss einem der Ansprüche 1–4 zur Herstellung von
2-[4(5)-Phenyl-5(4)-(3-pyridyl)-imidazol-2-yl]-propionsäureethylester
oder eines Salzes davon, dadurch gekennzeichnet, dass man von Verbindungen der Formeln II bzw. IIIa und IIIb, IIIc, IIIh und IIIi, IIId und IIIe, IIIk und IIIp bzw. IIIb sowie IIIf oder V oder VI oder VIIa und VIIb oder VIII ausgeht, worin die Variablen $Y_1$–$Y_6$, $R_3'$ und $R_4$ die in Anspruch 1 und die Variablen $Z_1$ und $Z_2$ die in Anspruch 2 angegebenen Bedeutungen haben.

27. Verfahren gemäss einem der Ansprüche 1–4 zur Herstellung von
2-[4(5)-(p-Chlorphenyl)-5(4)-(3-pyridyl)-imidazol-2-yl]-2-methyl-propionsäureethylester
oder eines Salzes davon, dadurch gekennzeichnet, dass man von Verbindungen der Formeln II bzw. IIIa und IIIb, IIIc, IIIh und IIIi, IIId und IIIe, IIIk und IIIp bzw. IIIb sowie IIIf oder V oder VI oder VIIa und VIIb oder VIII ausgeht, worin die Variablen $Y_1$–$Y_6$, $R_3'$ und $R_4$ die in Anspruch 1 und die Variablen $Z_1$ und $Z_2$ die in Anspruch 2 angegebenen Bedeutungen haben.

28. Verfahren gemäss einem der Ansprüche 1–4 zur Herstellung von
2-[4(5)-Phenyl-5(4)-(4-pyridyl)-imidazol-2-yl]-2-methyl-propionsäureethylester
oder eines Salzes davon, dadurch gekennzeichnet, dass man von Verbindungen der Formeln II bzw. IIIa und IIIb, IIIc, IIIh und IIIi, IIId und IIIe, IIIk und IIIp bzw. IIIb sowie IIIf oder V oder VI oder VIIa und VIIb oder VIII ausgeht, worin die Variablen $Y_1$–$Y_6$, $R_3'$ und $R_4$ die in Anspruch 1 und die Variablen $Z_1$ und $Z_2$ die in Anspruch 2 angegebenen Bedeutungen haben.

29. Verfahren gemäss einem der Ansprüche 1–4 zur Herstellung von
2-[4(5)-Phenyl-5(4)-(1-oxido-4-pyridyl)-imidazol-2-yl]-2-methyl-propionsäureethylester
oder eines Salzes davon, dadurch gekennzeichnet, dass man von Verbindungen der Formeln II bzw. IIIa und IIIb, IIIc, IIIh und IIIi, IIId und IIIe, IIIk und IIIp bzw. IIIb sowie IIIf oder V oder VI oder VIIa und VIIb oder VIII ausgeht, worin die Variablen $Y_1$–$Y_6$, $R_3'$ und $R_4$ die in Anspruch 1 und die Variablen $Z_1$ und $Z_2$ die in Anspruch 2 angegebenen Bedeutungen haben.

30. Verfahren gemäss einem der Ansprüche 1–4 zur Herstellung von
2-[4(5)-(p-Hydroxyphenyl)-5(4)-(3-pyridyl)-imidazol-2-yl]-2-methyl-propionsäureethylester
oder eines Salzes davon, dadurch gekennzeichnet, dass man von Verbindungen der Formeln II bzw. IIIa und IIIb, IIIc, IIIh und IIIi, IIId und IIIe, IIIk und IIIp bzw. IIIb sowie IIIf oder V oder VI oder VIIa und VIIb oder VIII ausgeht, worin die Variablen $Y_1$–$Y_6$, $R_3'$ und $R_4$ die in Anspruch 1 und die Variablen $Z_1$ und $Z_2$ die in Anspruch 2 angegebenen Bedeutungen haben.

31. Verfahren zur Herstellung pharmazeutischer Präparate, dadurch gekennzeichnet, dass man eine gemäss einem der Ansprüche 5, 6, 9, 15, 17, 19, 21, 23 oder 25 erhältliche Verbindung, in freier Form oder in Form eines pharmazeutisch verwendbaren Salzes, mit üblichen pharmazeutischen Hilfs- und Trägerstoffen vermischt.

32. Verfahren zur Herstellung pharmazeutischer Präparate, dadurch gekennzeichnet, dass man eine gemäss einem der Ansprüche 1–4, 7, 8, 10–14, 15, 18, 20, 22, 24 oder 26–30 erhältliche Verbindung, in freier Form oder in Form eines pharmazeutisch verwendbaren Salzes, mit üblichen pharmazeutischen Hilfs- und Trägerstoffen vermischt.

33. Verfahren zur Herstellung von Verbindungen der Formel

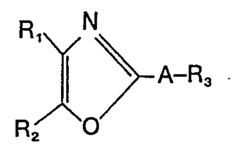 (IIIf)

worin einer der Reste $R_1$ und $R_2$ Phenyl, Pyrrolyl, Pyridyl, 1-Oxidopyridyl oder Pyrimidyl bedeutet, welche jeweils unsubstituiert oder durch Halogen, Niederalkyl, Hydroxy, Niederalkoxy und/oder Niederalkanoyloxy substituiert sein können, und der andere Pyrrolyl, Pyridyl, 1-Oxidopyridyl oder Pyrimidyl bedeutet, welche jeweils unsubstituiert oder durch Halogen, Niederalkyl, Hydroxy, Niederalkoxy und/oder Niederalkanoyloxy substituiert sein können und A Niederalkylen, Niederalkyliden, Niederalkenylen, Niederalkenyliden, Cycloalkylen, Cycloalkyliden oder Cycloalkyl-niederalkyliden darstellen, und $R_3$ Carboxy, mit einem Niederalkanol, 3- bis 8-gliedrigem Cycloalkanol, Phenol, einem Hydroxypyridin, einem substituierten Phenol bzw. Hydroxypyridin verestertes Carboxy, Carbamoyl oder durch Hydroxy, Amino, Phenyl oder substituiertes Phenyl mono-substituiertes, durch Niederalkyl mono- oder disubstituiertes oder durch 4- bis 7-gliedriges Alkylen bzw. 3-Aza-, 3-Niederalkylaza-, 3-Oxa- oder 3-Thiaalkylen disubstituiertes Carbamoyl bedeutet, wobei ein Niederalkanol bzw. Cycloalkanol unsubstituiert oder durch Hydroxy, Mercapto, gegebenenfalls substituiertes Phenyl, Niederalkoxy, gegebenenfalls im Phenylteil substituiertes Phenylniederalkoxy, Niederalkylthio, gegebenenfalls im Phenylteil substituiertes Phenylniederalkylthio, Hydroxyniederalkoxy, Niederalkoxyniederalkoxy, gegebenenfalls im Phenylteil substituiertes Phenylniederalkoxyniederalkoxy, Carboxyniederalkoxy, Niederalkoxycarbonyl-niederalkoxy, gegebenenfalls substituiertes Phenyl aufweisendes Niederalkoxycarbonyl-niederalkoxy oder Niederalkanoyloxy und substituiertes Phenyl, Phenol bzw. Hydroxypyridin jeweils durch Niederalkyl, Niederalkoxy, Halogen und/oder Trifluormethyl substituiert sein kann, ihre Isomeren sowie ihre Salze, dadurch gekennzeichnet, dass man Verbindungen der Formel

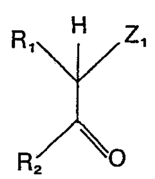 (IIId),

worin $Z_1$ gegebenenfalls reaktionsfähig verestertes Hydroxy bedeutet, mit Carbonsäuren der Formel $R_3$–A–COOH (IIIp), deren Salze oder deren funktionelle Derivate und mit Ammoniak umsetzt oder Verbindungen der Formel

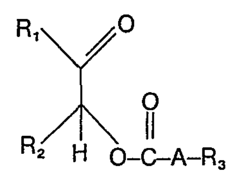 (IIIq),

welche durch Umsetzung von Verbindungen der Formel

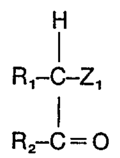

(IIId)

mit gegebenenfalls funktionellen Derivaten von Verbindungen der Formel

$R_3$–A–COOH (IIIp)

erhältlich sind, mit Ammoniak umsetzt, gewünschtenfalls in einer so erhaltenen Verbindung der Formel IIIf nicht N-oxidierte Heteroarylreste $R_1$ und $R_2$ mit Hilfe eines Oxidationsmittels N-oxidiert, und gewünschtenfalls die verfahrensgemäss erhältliche freie Verbindung in ein Salz oder ein verfahrensgemäss erhältliches Salz in die freie Verbindung oder in ein anderes Salz überführt oder eine verfahrensgemäss erhältliche freie Verbindung in eine andere freie Verbindung überführt und/oder, wenn erwünscht, ein erfindungsgemäss erhältliches Gemisch von isomeren Verbindungen der Formel I in die einzelnen Isomeren auftrennt.

34. Verfahren nach Anspruch 33, dadurch gekennzeichnet, dass man von irgendeiner Stufe des Verfahrens ausgeht und die fehlenden Schritte durchführt oder einen Ausgangsstoff in Form eines Salzes und/oder Tautomeren verwendet und/oder unter den Reaktionsbedingungen bildet.

35. Verfahren nach Anspruch 33 und 34 zur Herstellung von Verbindungen der Formel IIIf, worin einer der Reste $R_1$ und $R_2$ Pyridyl oder 1-Oxidopyridyl, die unsubstituiert und/oder jeweils durch Halogen, Hydroxy, Niederalkyl, Niederalkoxy und/oder Niederalkanoyloxy substituiert sein können, und der andere Phenyl, Pyridyl oder 1-Oxidopyridyl, die unsubstituiert und/oder jeweils durch Halogen, Hydroxy, Niederalkyl, Niederalkoxy und/oder Niederalkanoyloxy substituiert sein können, bedeutet, A Niederalkylen mit bis und mit 4 C-Atomen, Niederalkyliden mit bis und mit 7 C-Atomen, Niederalkenylen mit bis und mit 4 C-Atomen, Niederalkenyliden mit bis und mit 7 C-Atomen, 3- bis 8-gliedriges Cycloalkylen, 3- bis 8-gliedriges Cycloalkyliden oder Cycloalkylniederalkyliden mit bis und mit 7 C-Atomen im Alkylidenteil und mit einem 3- bis 8-gliedrigen Cycloalkylteil bedeutet und $R_3$ Carboxy, mit einem Niederalkanol, einem 3- bis 8-gliedrigen Cycloalkanol, Phenol oder einem substituierten Phenol verestertes Carboxy, Carbamoyl, N-Mono-, N,N-Diniederalkylcarbamoyl, Pyrrolidino-, Piperidino-, Morpholino-, Piperazino-, 4-Niederalkyl-piperazino-, Thiomorpholino-, Anilino- oder durch Niederalkyl, Niederalkoxy und/oder Halogen substituiertes Anilinocarbonyl darstellt, wobei das Niederalkanol bzw. Cycloalkanol unsubstituiert oder durch Hydroxy, Mercapto, Phenyl, substituiertes Phenyl, Niederalkoxy, Phenylniederalkoxy, im Phenylteil substituiertes Phenylniederalkoxy, Niederalkylthio, Phenylniederalkylthio, im Phenylteil substituiertes Phenylniederalkylthio, Hydroxyniederalkoxy, Niederalkoxyniederalkoxy, Phenylniederalkoxyniederalkoxy, im Phenylteil substituiertes Phenylniederalkoxyniederalkoxy, Carboxyniederalkoxy, Niederalkoxycarbonyl-niederalkoxy, gegebenenfalls substituiertes Phenyl aufweisendes Niederalkoxycarbonyl-niederalkoxy oder Niederalkanoyloxy und substituiertes Phenol bzw. Phenyl jeweils durch Niederalkyl, Niederalkoxy, Halogen und/oder Trifluormethyl substituiert sein kann, ihre Isomeren sowie ihre Salze.

36. Verfahren nach Anspruch 33 und 34 zur Herstellung von Verbindungen der Formel IIIf, worin einer der Reste $R_1$ und $R_2$ Phenyl oder durch Halogen mit Atomnummer bis und mit 35, Hydroxy, Niederalkyl mit bis und mit 4 C-Atomen und/oder Niederalkoxy mit bis und mit 4 C-Atomen substituiertes Phenyl bedeutet und der andere Pyridyl oder 1-Oxido-pyridyl bedeutet, die jeweils unsubstituiert oder durch Halogen mit Atomnummer bis und mit 35, Hydroxy und/oder Niederalkoxy mit bis und mit 4 C-Atomen substituiert sein können, A Niederalkylen mit bis und mit 4 C-Atomen, Niederalkyliden mit bis und mit 7 C-Atomen, Niederalkenyliden mit bis und mit 7 C-Atomen, oder 3- bis 8-gliedriges Cycloniederalkyliden darstellen und $R_3$ Carboxy, Niederalkoxycarbonyl mit bis und mit 5 C-Atomen, Carbamoyl, N-Mononiederalkylcarbamoyl mit bis und mit 4 C-Atomen im Niederalkyl oder N,N-Diniederalkyl-carbamoyl jeweils mit bis und mit 4 C-Atomen in Niederalkyl bedeuten, wobei das Niederalkoxycarbonyl durch Niederalkanoyloxy mit bis und mit 5 C-Atomen substituiert sein kann, ihre Isomeren sowie ihre Salze.

37. Verfahren nach Anspruch 33 und 34 zur Herstellung von Verbindungen der Formel IIIf, worin einer der Reste $R_1$ und $R_2$ Phenyl oder durch Halogen mit Atomnummer bis und mit 35, Hydroxy oder Niederalkoxy mit bis und mit 4 C-Atomen substituiertes Phenyl bedeutet und der andere Pyridyl oder 1-Oxidopyridyl darstellt, A ein ein quartäres C-Atom aufweisendes Niederalkyliden mit bis und mit 4 C-Atomen, wobei das quartäre C-Atom direkt an den Imidazolring gebunden ist, bedeutet, und $R_3$ Niederalkoxycarbonyl mit bis und mit 5 C-Atomen darstellt, ihre Isomeren sowie ihre Salze.

38. Verfahren nach Anspruch 33 und 34 zur Herstellung von

2-[4-Phenyl-5-(3-pyridyl)-oxazol-2-yl]-essigsäureethylester

oder ein Salz davon.

39. Verfahren nach Ansprüchen 33 und 34 zur Herstellung von

2-[4-Phenyl-5-(1-oxido-3-pyridyl)-oxazol-2-yl]-2-methyl-propionsäureethylester

oder ein Salz davon.

**Claims for the Contracting States: BE CH DE FR IT LI LU NL SE**

1. Novel trisubstituted imidazole derivatives of the general formula

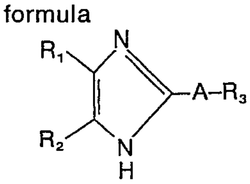

(I) ,

in which, on the one hand, each of $R_1$ and $R_2$, independently of the other, represents phenyl and/or phenyl substituted by halogen, lower alkyl, hydroxy, lower alkoxy and/or by lower alkanoyl, or wherein, on the other hand, one of the radicals $R_1$ and $R_2$ represent pyrrolyl, furyl, thienyl, pyridyl, 1-oxidopyridyl or pyrimidyl, each of which can be unsubstituted or substituted by halogen, lower alkyl, hydroxy, lower alkoxy and/or lower alkanoyloxy, and the other represents phenyl, pyrrolyl, furyl, thienyl, pyridyl, 1-oxidopyridyl or pyrimidyl, each of which can be unsubstituted or substituted by halogen, lower alkyl, hydroxy, lower alkoxy and/or lower alkanoyloxy, and in each case A represents lower alkylene, lower alkylidene, lower alkenylene, lower alkenylidene, cycloalkylene, cycloalkylidene or cycloalkyl-lower alkylidene, and $R_3$ represents carboxy, carboxy esterified by a lower alkanol, by 3- to 8-membered cycloalkanol, by phenol, by a hydroxypyridine or, by a substituted phenol or hydroxypyridine, carbamoyl or carbamoyl mono-substituted by hydroxy, amino, phenyl or substituted phenyl, carbamoyl mono- or di-substituted by lower alkyl, or carbamoyl di-substituted by 4- to 7-membered alkylene or 3-aza-, 3-lower alkylaza-, 3-oxa- or 3-thia-alkylene, wherein a lower alkanol or cycloalkanol can be unsubstituted or substituted by hydroxy, mercapto, optionally substituted phenyl, lower alkoxy, phenyl-lower alkoxy optionally substituted in the phenyl moiety, lower alkylthio, phenyl-lower alkylthio optionally substituted in the phenyl moiety, hydroxy-lower alkoxy, lower alkoxy-lower alkoxy, phenyl-lower alkoxy-lower alkoxy optionally substituted in the phenyl moiety, carboxy-lower alkoxy, lower alkoxycarbonyl-lower alkoxy, or lower alkoxycarbonyl-lower alkoxy or lower alkanoyloxy containing optionally substituted phenyl and wherein substituted phenyl, phenol and hydroxypyridine can each be substituted by lower alkyl, lower alkoxy, halogen und/or trifluoromethyl, their isomers and their salts, especially pharmaceutically acceptable salts, with the proviso that when A represents methylene or ethylidene and $R_3$ represents ethoxycarbonyl at least one of the radicals $R_1$ and $R_2$ is other than phenyl, and the further proviso that if A represents ethylidene and $R_3$ represents carboxy or carbamoyl at least one of the radicals $R_1$ and $R_2$ is other than phenyl, p-methoxyphenyl or p-chlorophenyl, wherein there are to be understood by radicals referred to a "lower" those having up to and including 7 carbon atoms.

2. Compounds according to claim 1 of the formula I in which each of $R_1$ and $R_2$ represents, independently of the other, phenyl und/or phenyl substituted by halogen, hydroxy, lower alkyl, lower alkoxy and/or lower alkanoyloxy, A represents lower alkylene having up to and including 4 carbon atoms, lower alkylidene having up to and including 7 carbon atoms, lower alkenylene having up to and including 4 carbon atoms, lower alkenylidene having up to and including 7 carbon atoms, 3- to 8-membered cycloalkylene, 3- to 8-membered cycloalkylidene, or cycloalkyl-lower alkylidene having up to and including 7 carbon atoms in the alkylidene moiety and having a 3- to 8-membered cycloalkyl moiety, and $R_3$ represents carboxy, carboxy esterified by a lower alkanol, a 3- to 8-membered cycloalkanol, phenol or a substituted phenol, or represents carbamoyl, N-mono- or N,N-di-lower alkylcarbamoyl, pyrrolidino-, piperidino-, morpholino-, piperazino-, 4-lower alkylpiperazino-, thiomorpholino- or anilino-carboxyl, or anilinocarbonyl substituted by lower alkyl, lower alkoxy and/or halogen, wherein the lower alkanol or cycloalkanol can be unsubstituted or substituted by hydroxy, mercapto, phenyl, substituted phenyl, lower alkoxy, phenyl-lower alkoxy, phenyl-lower alkoxy substituted in the phenyl moiety, lower alkylthio, phenyl-lower alkylthio, phenyl-lower alkylthio substituted in the phenyl moiety, hydroxy-lower alkoxy, lower alkoxy-lower alkoxy, phenyl-lower alkoxy-lower alkoxy, phenyl-lower alkoxy-lower alkoxy substituted in the phenyl moiety, carboxy-lower alkoxy, lower alkoxycarbonyl-lower alkoxy, or lower alkoxycarbonyl-lower alkoxy or lower alkanoyloxy containing optionally substituted phenyl, and wherein substituted phenol or phenyl can each be substituted by lower alkyl, lower alkoxy, halogen and/or trifluoromethyl, their isomers and their salts, especially pharmaceutically acceptable salts, with the proviso that, if A represents methylene or ethylidene and $R_3$ represents ethoxycarbonyl, at least one of the radicals $R_1$ and $R_2$ is different from phenyl, and the further proviso that, if A represents ethylidene and $R_3$ represents carboxy or carbamoyl, at least one of the radicals $R_1$ and $R_2$ is different from phenyl, p-methoxyphenyl and p-chlorophenyl.

3. Compounds according to claim 1 of the formula I in which one of the radicals $R_1$ and $R_2$ represents pyridyl or 1-oxidopyridyl, each of which can be unsubstituted and/or substituted by halogen, hydroxy, lower alkyl, lower alkoxy and/or lower alkanoyloxy, and the other represents phenyl, pyridyl or 1-oxidopyridyl each of which can be unsubstituted and/or substituted by halogen, hydroxy, lower alkyl, lower alkoxy and/or lower alkanoyloxy, A represents lower alkylene having up to and including 4 carbon atoms, lower alkylidene having up to and including 7 carbon atoms, lower alkenylene having up to and including 4 carbon atoms, lower alkenylidene having up to and including 7 carbon atoms, 3- to 8-membered cycloalkylene, 3- to 8-membered cycloalkylidene, or cycloalkyl-lower alkylidene having up to and including 7 carbon atoms in the alkylidene moiety and having a 3- to 8-membered cycloalkyl moiety, and $R_3$ represents carboxy, carboxy esterified by a lower alkanol, by a 3- to 8-membered cycloalkanol, by phenol or by a substituted phenol, or represents carbamoyl, N-mono- or N,N-di-lower alkylcarbamoyl, pyrrolidino-, piperidino-, morpholino-, piperazino-, 4-lower alkylpiperazino-, thiomorpholino- or anilinocarbonyl, or anilinocarbonyl substituted by lower alkyl, lower alkoxy and/or halogen, wherein the lower alkanol or cycloalkanol can be unsubstituted or substituted by hydroxy, mercapto, phenyl, substituted phenyl, lower alkoxy, phenyl-lower alkoxy, phenyl-lower alkoxy substituted in the phenyl moiety, lower alkylthio, phenyl-lower alkylthio, phenyl-lower alkylthio substituted in the phenyl moiety, hydroxy-lower alkoxy, lower alkoxy-lower alkoxy, phenyl-lower alkoxy-lower alkoxy, phenyl-lower alkoxy-lower alkoxy substituted in the phenyl moiety, carboxy-lower alkoxy, lower alkoxycarbonyl-lower alkoxy, or lower alkoxycarbonyl-lower alkoxy or lower alkanoyloxy containing optionally substituted phenyl, and wherein substituted phenol or phenyl can each be substituted by lower alkyl, lower alkoxy, halogen and/or trifluoromethyl, their isomers and their salts, especially pharmaceutically acceptable salts.

4. Compounds according to claim 1 of the formula I in which each of $R_1$ and $R_2$ represents, independently of the other, phenyl, pyridyl, and/or 1-oxidopyridyl each optionally substituted by halogen having an atomic number of up to and including 35, by hydroxy, by lower alkyl having up to and including 4 carbon atoms, by lower alkoxy having up to and including 4 carbon atoms, and/or by lower alkanoyloxy having up to and including 5 carbon atoms, A represents lower alkylene having up to and including 4 carbon atoms, or lower alkylidene having up to and including 4 carbon atoms, $R_3$ represents carboxy optionally esterified by a lower alkanol having up to and including 4 carbon atoms, wherein the lower alkanol can optionally be substituted by hydroxy, mercapto, optionally substituted phenyl, lower alkoxy having up to and including 4 carbon atoms, phenyl-lower alkoxy having up to and including 4 carbon atoms in the alkyl moiety and optionally substituted in the phenyl moiety, lower alkylthio having up to and including 4 carbon atoms, hydroxy-lower alkoxy having up to and including 4 carbon atoms in the lower alkyl moiety, lower alkoxy-lower alkoxy having up to and including 4 carbon atoms in each lower alkyl moiety, or lower alkanoyloxy having up to and including 5 carbon atoms, and wherein phenyl can optionally be substituted by lower alkyl having up to and including 4 carbon atoms, lower alkoxy having up to and including 4 carbon atoms, halogen having an atomic number of up to and including 35, and/or by trifluoromethyl, their isomers and their salts, with the proviso that, if A represents methylene or ethylidene and $R_3$ represents ethoxycarbonyl, at least one of the radicals $R_1$ and $R_2$ is different from phenyl, and the further proviso that, if A represents ethylidene and $R_3$ represents carboxy, at least one of the radicals $R_1$ and $R_2$ is different from phenyl, p-methoxyphenyl and p-chlorophenyl.

5. Compounds according to claim 1 of the formula I in which each of $R_1$ and $R_2$ represents, independently of the other, phenyl, and/or phenyl substituted by halogen having an atomic number of up to and including 35, by hydroxy, by lower alkyl having up to and including 4 carbon atoms, and/or by lower alkoxy having up to and including 4 carbon atoms, A represents lower alkylene having up to and including 4 carbon atoms, lower alkylidene having up to and including 7 carbon atoms, lower alkenylidene having up to and including 7 carbon atoms, or 3- to 8-menbered cyclo-lower alkylidene, and $R_3$ represents carboxy, lower alkoxycarbonyl having up to and including 5 carbon atoms, carbamoyl, N-mono-lower alkylcarbamoyl having up to and including 4 carbon atoms in the lower alkyl, or N,N-di-lower alkylcarbamoyl having up to and including 4 carbon atoms in each lower alkyl, wherein the lower alkoxycarbonyl can be substituted by lower alkanoyloxy having up to and including 5 carbon atoms, their isomers and their salts, especially pharmaceutically acceptable salts, with the proviso that, if A represents methylene or ethylidene and $R_3$ represents ethoxycarbonyl, at least one of the radicals $R_1$ and $R_2$ is different from phenyl, and the further proviso that, if A represents ethylidene and $R_3$ represents carboxy, at least one of the radicals $R_1$ and $R_2$ is different from phenyl, p-methoxyphenyl and p-chlorophenyl.

6. Compounds according to claim 1 of the formula I in which one of the radicals $R_1$ and $R_2$ represents phenyl or phenyl substituted by halogen having an atomic number of up to and including 35, by hydroxy, by lower alkyl having up to and including 4 carbon atoms, and/or by lower alkoxy having up to and including 4 carbon atoms, and the other represents pyridyl or 1-oxidopyridyl each of which can be unsubstituted or substituted by halogen having an atomic number of up to and including 35, by hydroxy, and/or by lower alkoxy having up to and including 4 carbon atoms, A represents lower alkylene having up to and including 4 carbon atoms, lower alkylidene having up to and including 7 carbon atoms, lower alkenylidene having up to and including 7 carbon atoms, or 3- to 8-membered cyclo-lower alkylidene, and $R_3$ represents carboxy, lower alkoxycarbonyl having up to and including 5 carbon atoms, carbamoyl, N-mono-lower alkylcarbamoyl having up to and including 4 carbon atoms in the lower alkyl, or N,N-di-lower alkylcarbamoyl having up to and including 4 carbon atoms in each lower alkyl, wherein the lower alkoxycarbonyl can be substituted by lower alkanoyloxy having up to and including 5 carbon atoms, their isomers and their salts, especially pharmaceutically acceptable salts.

7. Compounds according to claim 1 of the formula I in which each of $R_1$ and $R_2$ represents, independently of the other, phenyl and/or phenyl substituted by lower alkoxy having up to and including 4 carbon atoms, A represents lower alkylene having up to and including 4 carbon atoms or lower alkylidene having up to and including 4 carbon atoms, and $R_3$ represents carboxy or lower alkoxycarbonyl having up to and including 5 carbon atoms, their isomers and their salts, especially pharmaceutically acceptable salts, with the proviso that, of A represents methylene or ethylidene and $R_3$ represents ethoxycarbonyl, at least one of the radicals $R_1$ and $R_2$ is different from phenyl, and the further proviso that, if A repre-

sents ethylidene and $R_3$ represents carboxy, at least one of the radicals $R_1$ and $R_2$ is different from phenyl and p-methoxy-phenyl.

8. Compounds according to claim 1 of the formula I in which one of the radicals $R_1$ and $R_2$ represents phenyl or phenyl substituted by halogen having an atomic number of up to and including 35, by hydroxy or by lower alkoxy having up to and including 4 carbon atoms, and the other represents pyridyl or 1-oxidopyridyl, A represents lower alkylidene having up to and including 4 carbon atoms and containing a quaternary carbon atom, wherein the quaternary carbon atom is bonded directly to the imidazole ring, and $R_3$ represents lower alkoxycarbonyl having up to and including 5 carbon atoms, their isomers and their salts, especially pharmaceutically acceptable salts.

9. Compounds according to claim 1 of the formula I in which one of the radicals $R_1$ and $R_2$ represents phenyl and the other represents 1-oxidopyridyl, A represents 2,2-propylidene and $R_3$ represents lower alkoxycarbonyl having up to and including 5 carbon atoms, their isomers and their salts, especially pharmaceutically acceptable salts.

10. 2-[4(5)-phenyl-5(4)-(3-pyridyl)-imidazol-2-yl]-2-methyl ethyl propionate
or a salt thereof.

11. 2-[4(5)-phenyl-5(4)-(3-pyridyl)-imidazol-2-yl]-2-allyl ethyl acetate
or a salt thereof.

12. 1-[4(5)-phenyl-5(4)-(3-pyridyl)-imidazol-2-yl]-1-ethoxycarbonylcyclopentane
or a salt thereof.

13. 2-[4-(5)-(p-methoxyphenyl)-5(4)-(3-pyridyl)-imidazol-2-yl]-2-methyl ethyl propionate
or a salt thereof.

14. 2-[4(5)-phenyl-5(4)-(1-oxido-3-pyridyl)-imidazol-2-yl]-2-methyl ethyl propionate
or a salt thereof.

15. 2-(4(5)-phenyl-5(4)-(3-pyridyl)-imidazol-2-yl]-ethyl propionate
or a salt thereof.

16. 2-[4(5)-(p-chlorophenyl)-5(4)-(3-pyridyl)-imidazol-2-yl]-2-methyl ethyl propionate
or a salt thereof.

17. 2-(4(5)-phenyl-5(4)-(4-pyridyl)-imidazol-2-yl]-2-methyl ethyl propionate
or a salt thereof.

18. 2-[4(5)-phenyl-5(4)-(1-oxido-4-pyridyl)-imidazol-2-yl]-2-methyl ethyl propionate
or a salt thereof.

19. 2-[4(5)-(p-hydroxyphenyl)-5(4)-(3-pyridyl)-imidazol-2-yl]-2-methyl ethyl propionate
or a salt thereof.

20. Compounds of the formula I according to claim 1 with the proviso that if A represents ethylidene and $R_3$ represents carboxy, at least one of the radicals $R_1$ and $R_2$ is different from p-methoxyphenyl, for use in a method for the therapeutic treatment of the animal or human body.

21. Compounds according to claim 20 of the formula (I) with the proviso that, if A represents methylene or ethylidene and $R^3$ represents ethoxycarbonyl, at least one of the radicals $R_1$ and $R_2$ ist different from phenyl, and the further proviso that, if A represents ethylidene and $R_3$ represents carboxy, at least one of the radicals $R_1$ and $R_2$ ist different from phenyl, p-methoxyphenyl or p-chlorophenyl, for use in accordance with claim 20.

22. Compounds according to any one of claims 1 to 19 for use in accordance with claim 20.

23. Compounds of the formula I according to any one of claims 1–19 for use in accordance with claim 20 as topical skin-phlogistatics, anti-herpes agents and/or sun-screening agents.

24. Use of compounds of the formula I according to any one of claims 1–19 for the manufacture of medicaments such as topical skin-phlogistatics, anti-herpes agents and/or sun-screening agents.

25. Pharmaceutical preparations containing a compound according to any one of claims 20–23 in the free form or in the form of a pharmaceutically acceptable salt together with customary pharmaceutical adjuncts and carriers.

26. Pharmaceutical preparations according to claim 25 containing a compound according to any one of claims 1 to 10 in free form or in the form of a pharmaceutically acceptable salt together with customary pharmaceutical adjuncts and carriers.

27. Process for the manufacture of compounds of the formula (I) according to claim 1, characterised in that, while introducing an additional bond, H–Z, Z representing hydroxy or amino $Y_1$ and $Y_6$ respectively, $Y_3$ or $Y_4$ and $Y_5$ respectively, or esterified hydroxy $Y_4$ and $Y_5$ respectively, is removed from a compound of the formula

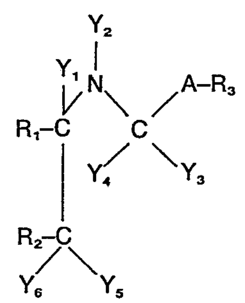

(II)

in which one of the radicals $Y_1$ and $Y_6$ represents hydroxy or amino, and the other radical and $Y_2$ each represents hydrogen, and $Y_3$ together with $Y_4$ and $Y_5$ represents a group of the formula =N–, or in which $Y_1$ together with $Y_6$ represents a bond, $Y_2$ is hydrogen, $Y_3$ represents hydroxy or amino, and $Y_4$ together with $Y_5$ represents a group of the formula –NH–, or in which $Y_1$ together with $Y_6$ represents a bond, $Y_2$ together with $Y_3$ represents an additional bond and one of the radicals $Y_4$ and $Y_5$ represents amino and the other represents amino, hydroxy or reactive esterified hydroxy, or in which $Y_1$ is hydroxy, $Y_2$ and $Y_3$ each represents hydrogen, $Y_4$ represents hydroxy or amino and $Y_5$ together with $Y_6$ represents a group of the formula

=NH or, if $Y_4$ is amino, represents oxo, or from a tautomer and/or a salt thereof, or a compound of the formula

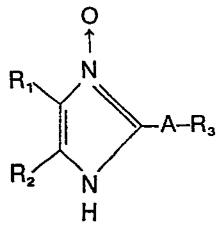

(IV)

or a salt thereof is reduced to a compound of the formula I, or, in a compound of the formula

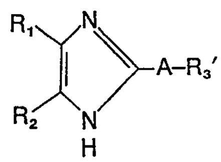

(V)

in which $R_3'$ represents a radical that can be converted into $R_3$, the radical $R_3'$ is converted into a radical $R_3$, or, in a compound of the formula

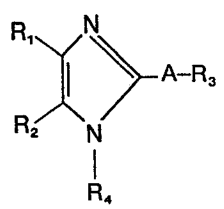

(VI)

in which $R_4$ represents a group that can be converted into hydrogen, or in a salt thereof, $R_4$ is converted into hydrogen, or compounds of the formula

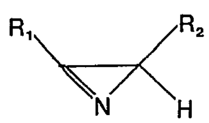

(VIIa)

or tautomers thereof are reacted with compounds of the formula $R_3$–A–CN (VIIb), or a compound of the formula

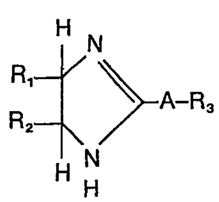

(VIII)

or an isomer thereof is dehydrogenated to form compounds of the formula I, and if desired, the free compound obtainable in accordance with the process is converted into a salt or a salt obtainable in accordance with the process is converted into the free compound or into a different salt or a free compound obtainable in accordance with the process is converted into a different free compound and/or, if desired, a mixture of isomeric compounds of the formula I obtainable in accordance with the invention is separated into the individual isomers.

28. Process according to claim 27, characterised in that a start is made at any stage of the process and the remaining steps are carried out or a starting material is used in the form of a salt and/or tautomer and/or is formed under the reaction conditions.

29. Process according to claim 27 or 28, characterised in that the starting materials of the formula II, their tautomers and/or salts are for the most part formed in situ according to processes known per se and further reacted under the reaction conditions to form the compound of the formula I without being isolated.

30. Process according to claim 29, characterised in that a diketone of the formula

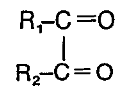

(IIIa)

is reacted, while heating, with an aldehyde of the formula $R_3$–A–C(=O)–H (IIIb) or a salt thereof, and an excess of ammonia, or an acylated α-aminoketone of the formula

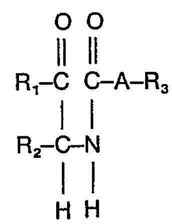

(IIIc)

or a salt thereof is reacted with ammonia, or a compound of the formula

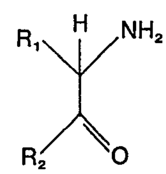

(IIIh)

or a salt thereof is reacted with an acid derivative of the formula $R_3$–A–COOH (IIIi), or a compound of the formula

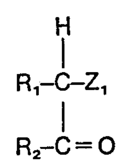

(IIId) ,

in which $Z_1$ represents reactive esterified hydroxy, or a salt thereof, is reacted with a compound of the formula

$R_3$–A–$Z_2$ (IIIe)

in which $Z_2$ represents an amidino radical or ammonium carboxylate, or a salt thereof, optionally with ammonia, or an oxazole of the formula

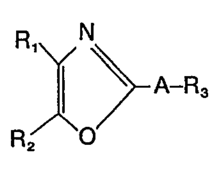

(IIIf)

is reacted with ammonia, or a compound of the formula

$$R_1–C(NH_2) = C(NH_2)–R_2 \quad (IIIk)$$

or a salt thereof is reacted with an optionally functionally modified compound of the formula

$$R_3–A–COOH \quad (IIIp)$$

or, under oxidising conditions, with a compound of the formula

$$R_3–A–C(=O)–H \quad (IIIb).$$

31. The compounds of the formula I obtainable in accordance with the processes of claims 27 to 39.

32. Compounds of the formula

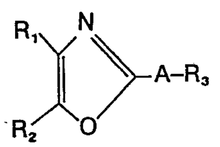

(IIIf),

in which one of the radicals $R_1$ and $R_2$ represents phenyl, pyrrolyl, pyridyl, 1-oxidopyridyl or pyrimidyl each of which can be unsubstituted or substituted by halogen, lower alkyl, hydroxy, lower alkoxy and/or lower alkanoyloxy, and the other represents pyrrolyl, pyridyl, 1-oxidopyridyl or pyrimidyl each of which can be unsubstituted or substituted by halogen, lower alkyl, hydroxy, lower alkoxy and/or lower alkanoyloxy, A represents lower alkylene, lower alkylidene, lower alkenylene, lower alkenylidene, cycloalkylene, cycloalkylidene or cycloalkyl-lower alkylidene, and $R_3$ represents carboxy, carboxy esterified by a lower alkanol, by a 3- to 8-membered cycloalkanol, by phenol, by a hydroxypyridine, or by a substituted phenol or substituted hydroxypyridine, or represents carbamoyl or carbamoyl monosubstituted by hydroxy, amino, phenyl or by substituted phenyl, carbamoyl mono- or disubstituted by lower alkyl, or carbamoyl di-substituted by 4- to 7-membered alkylene or 3-aza-, 3-lower alkylaza-, 3-oxa- or 3-thia-alkylene, wherein a lower alkanol or cycloalkanol can be unsubstituted or substituted by hydroxy, mercapto, optionally substituted phenyl, lower alkoxy, phenyl-lower alkoxy optionally substituted in the phenyl moiety, lower alkylthio, phenyl-lower alkylthio optionally substituted in the phenyl moiety, hydroxy-lower alkoxy, lower alkoxy-lower alkoxy, phenyl-lower alkoxy-lower alkoxy optionally substituted in the phenyl moiety, carboxy-lower alkoxy, lower alkoxycarbonyl-lower alkoxy, or lower alkoxycarbonyl-lower alkoxy or lower alkanoyloxy containing optionally substituted phenyl, and wherein substituted phenyl, phenol or hydroxypyridine can each be substituted by lower alkyl, lower alkoxy, halogen and/or trifluoromethyl, their isomers and their salts.

33. Compounds of the formula IIIf according to claim 32, in which one of the radicals $R_1$ and $R_2$ represents pyridyl or 1-oxidopyridyl each of which can be unsubstituted and/or substituted by halogen, hydroxy, lower alkyl, lower alkoxy and/or lower alkanoyloxy, and the other represents phenyl, pyridyl or 1-oxidopyridyl each of which can be unsubstituted and/or substituted by halogen, hydroxy, lower alkyl, lower alkoxy and/or lower alkanoyloxy, A represents lower alkylene having up to and including 4 carbon atoms, lower alkylidene having up to and including 7 carbon atoms, lower alkenylene having up to and including 4 carbon atoms, lower alkenylidene having up to and including 7 carbon atoms, 3- to 8-membered cycloalkylene, 3- to 8-membered cycloalkylidene, or cycloalkyl-lower alkylidene having up to and including 7 carbon atoms in the alkylidene moiety and having a 3- to 8-membered cycloalkyl moiety, and $R_3$ represents carboxy, carboxy esterified by a lower alkanol, by a 3- to 8-membered cycloalkanol, by phenol or by a substituted phenol, or represents carbamoyl, N-mono- or N,N-di-lower alkylcarbamoyl, pyrrolidino-, piperidino-, morpholino-, piperazino-, 4-lower alkylpiperazino-, thiomorpholino- or anilino-carbonyl, or anilinocarbonyl substituted by lower alkyl, lower alkoxy and/or halogen, wherein the lower alkanol or cycloalkanol can be unsubstituted or substituted by hydroxy, mercapto, phenyl, substituted phenyl, lower alkoxy, phenyl-lower alkoxy, phenyl-lower alkoxy substituted in the phenyl moiety, lower alkylthio, phenyl-lower alkylthio, phenyl-lower alkylthio substituted in the phenyl moiety, hydroxy-lower alkoxy, lower alkoxy-lower alkoxy, phenyl-lower alkoxy-lower alkoxy, phenyl-lower alkoxy-lower alkoxy substituted in the phenyl moiety, carboxy-lower alkoxy, lower alkoxycarbonyl-lower alkoxy, or lower alkoxycarbonyl-lower alkoxy or lower alkanoyloxy containing optionally substituted phenyl, and wherein substituted phenol or phenyl can each be substituted by lower alkyl, lower alkoxy, halogen and/or trifluoromethyl, their isomers and their salts.

34. Compounds of the formula IIIf according to claim 32, in which one of the radicals $R_1$ and $R_2$ represents phenyl or phenyl substituted by halogen having an atomic number of up to and including 35, by hydroxy, by lower alkyl having up to and including 4 carbon atoms, and/or by lower alkoxy having up to and including 4 carbon atoms, and the other represents pyridyl or 1-oxidopyridyl each of which can be unsubstituted or substituted by halogen having an atomic number of up to and including 35, by hydroxy, and/or by lower alkoxy having up to and including 4 carbon atoms, A represents lower alkylene having up to and including 4 carbon atoms, lower alkylidene having up to and including 7 carbon atoms, lower alkenylidene having up to and including 7 carbon atoms, or 3- to 8-membered cyclo-lower alkylidene, and $R_3$ represents carboxy, lower alkoxycarbonyl having up to and including 5 carbon atoms, carbamoyl, N-mono-lower alkylcarbamoyl having up to and including 4 carbon atoms in the lower alkyl, or N,N-di-lower alkylcarbamoyl having up to and including 4 carbon atoms in each lower alkyl,

wherein the lower alkoxycarbonyl can be substituted by lower alkanoyloxy having up to and including 5 carbon atoms, their isomers and their salts.

35. Compounds of the formula IIIf according to claim 32, in which one of the radicals $R_1$ and $R_2$ represents phenyl or phenyl substituted by halogen having an atomic number of up to and including 35, by hydroxy or by lower alkoxy having up to and including 4 carbon atoms, and the other represents pyridyl or 1-oxidopyridyl, A represents lower alkylidene having up to and including 4 carbon atoms and containing a quaternary carbon atom, wherein the quaternary carbon atom is bonded directly to the imidazole ring, and $R_3$ represents lower alkoxycarbonyl having up to and including 5 carbon atoms, their isomers and their salts.

36. 2-[4-phenyl-5-(3-pyridyl)-oxazol-2-yl]-ethyl acetate
or a salt thereof.

37. 2-[4-phenyl-5-(1-oxido-3-pyridyl)-oxazol-2-yl]-2-methyl ethyl propionate
or a salt thereof.

38. Compounds of the formula IIIf according to any one of claims 32 to 37 for use in a method for the therapeutic treatment of the animal or human body.

39. Compounds of the formula IIIf according to claim 38 for use as topical skin-phlogistatics.

40. Use of compounds of the formula IIIf according to any one of claims 32 to 37 for the manufacture of medicaments.

41. Process for the manufacture of compounds of the formula IIIf according to claim 33, characterised in that compounds of the formula

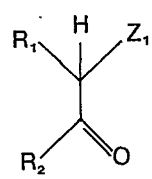

(IIId)

in which $Z_1$ represents optionally reactively esterified hydroxy, are reacted with carboxylic acids of the formula $R_3$–A–COOH (IIIp), the salts or functional derivatives thereof and with ammonia, or compounds of the formula

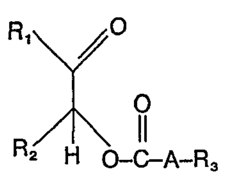

(IIIq)

which are obtainable by reacting compounds of the formula

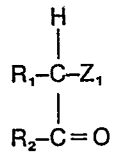

(IIId)

with optionally functional derivatives of compounds of the formula

$R_3$–A–COOH (IIIp)

are reacted with ammonia, if desired non N-oxidised heteroaryl radicals $R_1$ and $R_2$ in a resulting compound of the formula IIIf are N-oxidised by means of an oxidising agent and, if desired, the free compound obtainable in accordance with the process is converted into a salt, or a salt obtainable in accordance with the process is converted into the free compound or into a different salt, or a free compound obtainable in accordance with the process is converted into a different free compound and/or, if desired, a mixture of isomeric compounds of the formula I obtainable in accordance with the invention is separated into the indivicual isomers.

42. Process according to claim 41, characterised in that a start is made at any stage of the process and the remaining steps are carried out or a starting material is used in the form of a salt and/or a tautomer and/or is formed under the reaction conditions.

43. The compounds of the formula IIIf obtainable in accordance with the process of claims 41 and 42.

**Claims for the Contracting State: AT**

1. Process for the manufacture of compounds of the formula (I)

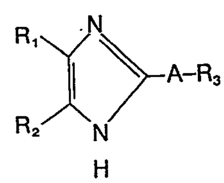

(I) ,

in which, on the one hand, each of $R_1$ and $R_2$, independently of the other, represents phenyl and/or phenyl substituted by halogen, lower alkyl, hydroxy, lower alkoxy and/or by lower alkanoyl, or wherein, on the other hand, one of the radicals $R_1$ and $R_2$ represent pyrrolyl, furyl, thienyl, pyridyl, 1-oxidopyridyl or pyrimidyl, each of which can be unsubstituted or substituted by halogen, lower alkyl, hydroxy, lower alkoxy and/or lower alkanoyloxy, and the other represents phenyl, pyrrolyl, furyl, thienyl, pyridyl, 1-oxidopyridyl or pyrimidyl, each of which can be unsubstituted or substituted by halogen, lower alkyl, hydroxy, lower alkoxy and/or lower alkanoyloxy, and in each case A represents lower alkylene, lower alkylidene, lower alkenylene, lower alkenylidene, cycloalkylene, cycloalkylidene or cycloalkyl-lower alklyidene, and $R_3$ represents carboxy, carboxy esterified by a lower alkanol, by 3- to 8-membered cycloalkanol, by phenol, by a hydroxypyridine or by a substituted phenol or hydroxypyridine, carbamoyl or carbamoyl mono-substituted by hydroxy, amino, phenyl or substituted phenyl, carbamoyl mono- or di-substituted by lower alkyl, or carbamoyl di-substituted by 4- to 7-membered alkylene or 3-aza-, 3-lower alkylaza-, 3-oxa- or 3-thia-alkylene, wherein a lower alkanol or cycloalkanol can be unsubstituted or substituted by hydroxy, mercapto, optionally substituted phenyl, lower alkoxy, phenyl-lower alkoxy optionally substituted in the

phenyl moiety, lower alkylthio, phenyl-lower alkylthio optionally substituted in the phenyl moiety, hydroxy-lower alkoxy, lower alkoxy-lower alkoxy, phenyl-lower alkoxy-lower alkoxy optionally substituted in the phenyl moiety, carboxy-lower alkoxy, lower alkoxycarbonyl-lower alkoxy, or lower alkoxycarbonyl-lower alkoxy or lower alkanoyloxy containing optionally substituted phenyl and wherein substituted phenyl, phenol and hydroxypyridine can each be substituted by lower alkyl, lower alkoxy, halogen and/or trifluoromethyl, their isomers and their salts, especially pharmaceutically acceptable salts, with the proviso that when A represents methylene or ethylidene and $R_3$ represents ethoxycarbonyl at least one of the radicals $R_1$ and $R_2$ is other than phenyl, and the further proviso that if A represents ethylidene and $R_3$ represents carboxy or carbamoyl at least one of the radicals $R_1$ and $R_2$ is other than phenyl, p-methoxyphenyl or p-chlorophenyl, wherein there are to be understood by radicals referred to as "lower" those having up to and including 7 carbon atoms, characterised in that while introducing an optional additional bond, H–Z, Z representing hydroxy or amino $Y_1$ and $Y_6$ respectively, $Y_3$ or $Y_4$ and $Y_5$ respectively, or esterified hydroxy $Y_4$ and $Y_5$ respectively, is removed from a compound of the formula

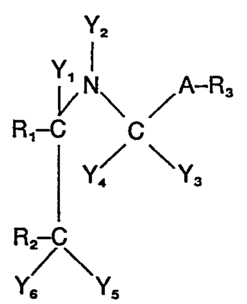

(II)

in which one of the radicals $Y_1$ and $Y_6$ represents hydroxy or amino, and the other radical and $Y_2$ each represents hydrogen, and $Y_3$ together with $Y_4$ and $Y_5$ represents a group of the formula =N–, or in which $Y_1$ together with $Y_6$ represents a bond, $Y_2$ is hydrogen, $Y_3$ represents hydroxy or amino, and $Y_4$ together with $Y_5$ represents a group of the formula –NH–, or in which $Y_1$ together with $Y_6$ represents a bond, $Y_2$ together with $Y_3$ represents an additional bond and one of the radicals $Y_4$ and $Y_5$ represents amino and the other represents amino, hydroxy or reactive esterified hydroxy, or in which $Y_1$ is hydroxy, $Y_2$ and $Y_3$ each represents hydrogen, $Y_4$ represents hydroxy or amino and $Y_5$ together with $Y_6$ represents a group of the formula =NH– or, if $Y_4$ is amino, represents oxo, or from a tautomer and/or a salt thereof, or a compound of the formula

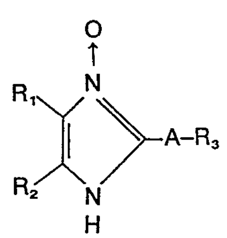

(IV)

or a salt thereof is reduced to a compound of the formula I, or, in a compound of the formula

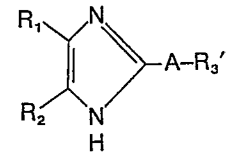

(V)

in which $R_3'$ represents a radical that can be converted into $R_3'$, the radical $R_3'$ is converted into a radical $R_3$, or, in a compound of the formula

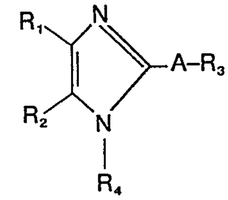

(VI)

in which $R_4$ represents a group that can be converted into hydrogen, or in a salt thereof, $R_4$ is converted into hydrogen, or compounds of the formula

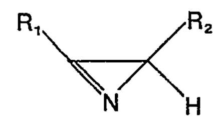

(VIIa)

or tautomers thereof are reacted with compounds of the formula $R_3$–A–CN (VIIb), or a compound of the formula

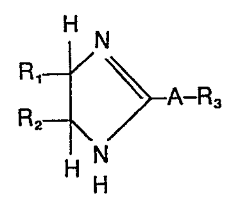

(VIII)

or an isomer thereof is dehydrogenated to form compounds of the formula I, and, if desired, the free compound obtainable in accordance with the process is converted into a salt or a salt obtainable in accordance with the process is converted into the free compound or into a different salt or a free compound obtainable in accordance with the process is converted into a different free compound and/or, if desired, a mixture of isomeric compounds of the formula I obtainable in accordance with the invention is separated into the individual isomers.

2. Process according to claim 1, characterised in that a diketone of the formula

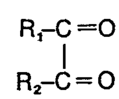

(IIIa)

is reacted, while heating, with an aldehyde of the formula $R_3$–A–C(=O)–H (IIIb) or a salt thereof, and an excess of ammonia, or an acylated W-aminoketone of the formula

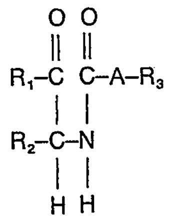

(IIIc)

or a salt thereof is reacted with ammonia, or a compound of the formula

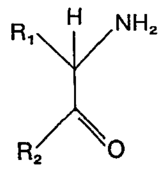

(IIIh)

or a salt thereof is reacted with an acid derivative of the formula $R_3$–A–COOH (IIIi), or a compound of the formula

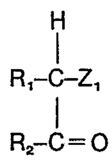

(IIId),

in which $Z_1$ represents reactive esterified hydroxy, or a salt thereof, is reacted with a compound of the formula

$$R_3\text{–A–}Z_2 \qquad \text{(IIIe)}$$

in which $Z_2$ represents an amidino radical or ammonium carboxylate, or a salt thereof, optionally with ammonia, or an oxazole of the formula

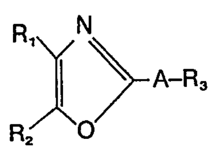

(IIIf)

is reacted with ammonia, or a compound of the formula

$$R_1\text{–C}(NH_2)=C(NH_2)\text{–}R_2 \qquad \text{(IIIk)}$$

or a salt thereof is reacted with an optionally functionally modified compound of the formula

$$R_3\text{–A–COOH} \qquad \text{(IIIp)}$$

or, under oxidising conditions, with a compound of the formula

$$R_3\text{–A–C}(=O)\text{–H} \qquad \text{(IIIb).}$$

3. Process according to claim 1 or 2, characterised in that the starting materials of the formula II, their tautomers and/or salts are for the most part formed in situ according to processes known per se and are further reacted under the reaction conditions, without being isolated, to form the compound of the formula I.

4. Process according to any one of claims 1 to 3, characterised in that a start is made at any stage of the process and the remaining steps are carried out or a starting material is used in the form of a salt and/or a tautomer and/or is formed under the reaction conditions.

5. Process according to claim 1 for the manufacture of compounds of the formula I in which on the one hand, each of $R_1$ and $R_2$, independently of the other, represents phenyl and/or phenyl substituted by halogen, lower alkyl, hydroxy, lower alkoxy and/or by lower alkanoyl, or wherein, on the other hand, one of the radicals $R_1$ and $R_2$ represent pyrrolyl, furyl, thienyl, pyridyl, 1-oxidopyridyl or pyrimidyl, each of which can be unsubstituted or substituted by halogen, lower alkyl, hydroxy, lower alkoxy and/or lower alkanoyloxy, and the other represents phenyl, pyrrolyl, furyl, thienyl, pyridyl, 1-oxidopyridyl or pyrimidyl, each of which can be unsubstituted or substituted by halogen, lower alkyl, hydroxy, lower alkoxy and/or lower alkanoyloxy, and in each case A represents lower alkylene, lower alkylidene, lower alkenylene, lower alkenylidene, cycloalkylene, cycloalkylidene or cycloalkyl-lower alkylidene, and $R_3$ represents carboxy, carboxy esterified by a lower alkanol, by 3- to 8-membered cycloalkanol, by phenol, by a hydroxypyridine or by a substituted phenol or hydroxypyridine, wherein a lower alkanol or cycloalkanol can be unsubstituted or substituted by hydroxy, mercapto, optionally substituted phenyl, lower alkoxy, phenyl-lower alkoxy optionally substituted in the phenyl moiety, lower alkylthio, phenyl-lower alkylthio optionally substituted in the phenyl moiety, hydroxy-lower alkoxy, lower alkoxy-lower alkoxy, phenyl-lower alkoxy-lower alkoxy optionally substituted in the phenyl moiety, or lower alkanoyl wherein substituted phenyl, phenol and hydroxypyridine can each be substituted by lower alkyl, lower alkoxy, halogen and/or trifluoromethyl, their isomers and their salts, especially pharmaceutically acceptable salts, with the proviso that when A represents methylene or ethylidene and $R_3$ represents ethoxycarbonyl at least one of the radicals $R_1$ and $R_2$ is other than phenyl, and the further proviso that if a represents ethylidene and $R_3$ represents carboxy or carbamoyl at least one of the radicals $R_1$ and $R_2$ is other than phenyl, p-methoxyphenyl or p-chlorophenyl, characterised in that, while introducing an optional additional bond, H–Z, Z representing hydroxy or amino, $Y_1$ and $Y_6$ respectively, $Y_3$ or $Y_4$ and $Y_5$ respectively, or halogen or sulphonyloxy $Y_4$ and $Y_5$ respectively, is removed from a compound of the formula

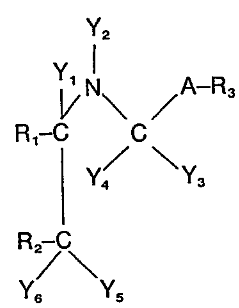

(II)

in which one of the radicals $Y_1$ and $Y_6$ represents hydroxy or amino, and the other radical and $Y_2$ each represents hydrogen, and $Y_3$ together with $Y_4$ and $Y_5$ represents a group of the formula =N–, or in which $Y_1$ together with $Y_6$ represents a bond, $Y_2$ is hydrogen, $Y_3$ represents hydroxy or amino, and $Y_4$ together with $Y_5$ represents a group of the formula –NH–, $Y_2$ together with $Y_3$ represents an additional bond and one of the radicals $Y_4$ and $Y_5$ represents amino and the other represents amino, hydroxy, halogen or sulphonyloxy, or in which $Y_1$ is hydroxy, $Y_2$ and $Y_3$ each represents hydrogen, $Y_4$ represents hydroxy or amino and $Y_5$ together with $Y_6$ represents a group of the formula =NH– or, if $Y_4$ is amino, represents oxo, or from a tautomer and/or a salt thereof,

b) a compound of the formula

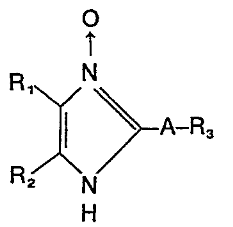

(IV)

or a salt thereof is reduced to a compound of the formula I,

c) in a compound of the formula

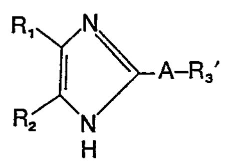

(V)

in which $R_3'$ represents a radical that can be converted into $R_3$, the radical $R_3'$ is converted into a radical $R_3$ or

d) in a compound of the formula

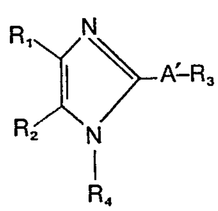

(VI)

in which A′ represents lower alkylidene, lower alkenylidene, cycloalkylidene or cycloalkyl-lower alkylidene, and $R_4$ represents a group that can be converted into hydrogen, or in a salt thereof, $R_4$ is converted into hydrogen and, if desired, the free compound obtainable in accordance with the process is converted into a salt or a salt obtainable in accordance with the process is converted into the free compound or into a different salt.

6. Process according to claim 5, characterised in that a start is made at any stage of the process and the remaining steps are carried out or a starting material is used in the form of a salt and/or a tautomer, or is formed under the reaction conditions.

7. Process according to any one of claims 1 to 4 for the manufacture of compounds of the formula I in which each of $R_1$ and $R_2$ represents, independently of the other, phenyl and/or phenyl substituted by halogen, hydroxy, lower alkyl, lower alkoxy and/or lower alkanoyloxy, A represents lower alkylene having up to and including 4 carbon atoms, lower alkylidene having up to and including 7 carbon atoms, lower alkenylene having up to and including 4 carbon atoms, lower alkenylidene having up to and including 7 carbon atoms, 3- to 8-membered cycloalkylene, 3- to 8-membered cycloalkylidene, or cycloalkyl-lower alkylidene having up to and including 7 carbon atoms in the alkylidene moiety and having a 3- to 8-membered cycloalkyl moiety, and $R_3$ represents carboxy, carboxy esterified by a lower alkanol, a 3- to 8-membered cycloalkanol, phenol or a substituted phenol, or represents carbamoyl, N-mono- or N,N-di-lower alkylcarbamoyl, pyrrolidino-, piperidino-, morpholino-, piperazino-, 4-lower alkylpiperazino-, thiomorpholino- or anilino-carbonyl, or anilinocarbonyl substituted by lower alkyl, lower alkoxy and/or halogen, wherein the lower alkanol or cycloalkanol can be unsubstituted or substituted by hydroxy, mercapto, phenyl, substituted phenyl, lower alkoxy, phenyl-lower alkoxy, phenyl-lower alkoxy substituted in the phenyl moiety, lower alkylthio, phenyl-lower alkylthio, phenyl-lower alkylthio substituted in the phenyl moiety, hydroxy-lower alkoxy, lower alkoxy-lower alkoxy, phenyl-lower alkoxy-lower alkoxy, phenyl-lower alkoxy-lower alkoxy substituted in the phenyl moiety, carboxy-lower alkoxy, lower alkoxycarbonyl-lower alkoxy, or lower alkoxycarbonyl-lower alkoxy or lower alkanoyloxy containing optionally substituted phenyl, and wherein substituted phenol or phenyl can each be substituted by lower alkyl, lower alkoxy, halogen and/or trifluoromethyl, their isomers and their salts, especially pharmaceutically acceptable salts, with the proviso that, if A represents methylene or ethylidene and $R_3$ represents ethoxycarbonyl, at least one of the radicals $R_1$ and $R_2$ is different from phenyl, and the further proviso that, if A represents ethylidene and $R_3$ represents carboxy or carbamoyl, at least one of the radicals $R_1$ and $R_2$ is different from phenyl, p-methoxyphenyl and p-chlorophenyl, characterised in that compounds of the formulae II or IIIa and IIIb, IIIc, IIIh and IIIi, IIId and IIIe, IIIk and IIIp or IIIb as well as IIIf or V or VI or VIIa and VIIb or VIII are used as starting materials, in which the variables $Y_1$ to $Y_6$, $R_3'$ and $R_4$ have the meanings given in claim 1 and the variables $Z_1$ and $Z_2$ have the meanings given in claim 2.

8. Process according to any one of claims 1 to 4 for the manufacture of compounds of the formula I in which one of the radical $R_1$ and $R_2$ represents pyridyl or 1-oxidopyridyl, each of which can be unsubstituted and/or substituted by halogen, hydroxy, lower alkyl, lower alkoxy and/or lower alkanoyloxy, and the other represents phenyl, pyridyl or 1-oxidopyridyl each of which can be unsubstituted and/or substituted by halogen, hydroxy, lower alkyl, lower alkoxy and/or lower alkanoyloxy, A represents lower alkylene having up to and

including 4 carbon atoms, lower alkylidene having up to and including 7 carbon atoms, lower alkenylene having up to and including 4 carbon atoms, lower alkenylidene having up to and including 7 carbon atoms, 3- to 8-membered cycloalkylene, 3- to 8-membered cycloalkylidene, or cycloalkyl-lower alkylidene having up to and including 7 carbon atoms in the alkylidene moiety and having a 3- to 8-membered cycloalkyl moiety, and $R_3$ represents carboxy, carboxy esterified by a lower alkanol, by a 3- to 8-membered cycloalkanol, by phenol or by a substituted phenol, or represents carbamoyl, N-mono- or N,N-di-lower alkylcarbamoyl, pyrrolidino-, piperidino-, morpholino-, piperazino-, 4-lower alkylpiperazino-, thiomorpholino- or anilino-carbonyl, or anilinocarbonyl substituted by lower alkyl, lower alkoxy and/or halogen, wherein the lower alkanol or cycloalkanol can be unsubstituted or substituted by hydroxy, mercapto, phenyl, substituted phenyl, lower alkoxy, phenyl-lower alkoxy, phenyl-lower alkoxy substituted in the phenyl moiety, lower alkylthio, phenyl-lower alkylthio, phenyl-lower alkylthio substituted in the phenyl moiety, hydroxy-lower alkoxy, lower alkoxy-lower alkoxy, phenyl-lower alkoxy-lower alkoxy, phenyl-lower alkoxy-lower alkoxy substituted in the phenyl moiety, carboxy-lower alkoxy, lower alkoxycarbonyl-lower alkoxy, or lower alkoxycarbonyl-lower alkoxy or lower alkanoyloxy containing optionally substituted phenyl, and wherein substituted phenol or phenyl can each be substituted by lower alkyl, lower alkoxy, halogen and/or trifluoromethyl, their isomers and their salts, especially pharmaceutically acceptable salts, characterised in that compounds of the formulae II or IIIa and IIIb, IIIc, IIIh and IIIi, IIId and IIIe, IIIk and IIIp or IIIb as well as IIIf or V or VI or VIIa and VIIb or VIII are used as starting materials, in which the variables $Y_1$ to $Y_6$, $R_3'$ and $R_4$ have the meanings given in claim 1 and the variables $Z_1$ and $Z_2$ have the meanings given in claim 2.

9. Process according to claim 5 or 6 for the manufacture of compounds of the formula I in which each of $R_1$ and $R_2$ represents, independently of the other, phenyl, pyridyl, and/or 1-oxidopyridyl each optionally substituted by halogen having an atomic number of up to and including 35, by hydroxy, by lower alkyl having up to and including 4 carbon atoms, by lower alkoxy having up to and including 4 carbon atoms, and/or by lower alkanoyloxy having up to and including 5 carbon atoms, A represents lower alkylene having up to and including 4 carbon atoms, or lower alkylidene having up to and including 4 carbon atoms, $R_3$ represents carboxy optionally esterified by a lower alkanol having up to and including 4 carbon atoms, wherein the lower alkanol can optionally be substituted by hydroxy, mercapto, optionally substituted phenyl, lower alkoxy having up to and including 4 carbon atoms, phenyl-lower alkoxy having up to and including 4 carbon atoms in the alkyl moiety and optionally substituted in the phenyl moiety, lower alkylthio having up to and including 4 carbon atoms, hydroxy-lower alkoxy having up to and including 4 carbon atoms in the lower alkyl moiety, lower alkoxy-lower alkoxy having up to and including 4 carbon atoms in each lower alkyl moiety, or lower alkanoyloxy having up to and including 5 carbon atoms, and wherein phenyl can optionally be substituted by lower alkyl having up to and including 4 carbon atoms, lower alkoxy having up to and including 4 carbon atoms, halogen having an atomic number of up to and including 35, and/or by trifluoromethyl, their isomers and their salts, with the proviso that, if A represents methylene or ethylidene and $R_3$ represents ethoxycarbonyl, at least one of the radicals $R_1$ and $R_2$ is different from phenyl, and the further proviso that, if A represents ethylidene and $R_3$ represents carboxy, at least one of the radicals $R_1$ and $R_2$ is different from phenyl, p-methoxyphenyl and p-chlorophenyl, characterised in that compounds of the formulae II, IV, V or VI are used as starting materials in which the variables $Y_1$ to $Y_6$, $R_3'$ and $R_4$ have the meanings given in claim 5.

10. Process according to any one of claims 1 to 4 for the manufacture of compounds of the formula I in which each of $R_1$ and $R_2$ represents, independently of the other, phenyl, and/or phenyl substituted by halogen having an atomic number of up to and including 35, by hydroxy, by lower alkyl having up to and including 4 carbon atoms, and/or by lower alkoxy having up to and including 4 carbon atoms, A represents lower alkylene having up to and including 4 carbon atoms, lower alkylidene having up to and including 7 carbon atoms, lower alkenylidene having up to and including 7 carbon atoms, or 3- to 8-membered cyclo-lower alkylidene, and $R_3$ represents carboxy, lower alkoxycarbonyl having up to and including 5 carbon atoms, carbamoyl, N-mono-lower alkylcarbamoyl having up to and including 4 carbon atoms in the lower alkyl, or N,N-di-lower alkylcarbamoyl having up to and including 4 carbon atoms in each lower alkyl, wherein the lower alkoxycarbonyl can be substituted by lower alkanoyloxy having up to and including 5 carbon atoms, their isomers and their salts, especially pharmaceutically acceptable salts, with the proviso that, if A represents methylene or ethylidene and $R_3$ represents ethoxycarbonyl, at least one of the radicals $R_1$ and $R_2$ is different from phenyl, and the further proviso that, if A represents ethylidene and $R_3$ represents carboxy, at least one of the radicals $R_1$ and $R_2$ is different from phenyl, p-methoxyphenyl and p-chlorophenyl, characterised in that compounds of the formulae II or IIIa and IIIb, IIIc, IIIh and IIIi, IIId and IIIe, IIIk and IIIp or IIIb as well as IIIf or V or VI or VIIa and VIIb or VIII are used as starting materials, in which the variables $Y_1$ to $Y_6$, $R_3'$ and $R_4$ have the meanings given in claim 1 and the variables $Z_1$ and $Z_2$ have the meanings given in claim 2.

11. Process according to any one of claims 1 to 4 for the manufacture of compounds of the formula I in which one of the radicals $R_1$ and $R_2$ represents phenyl or phenyl substituted by halogen having an atomic number of up to and including 35, by hydroxy, by lower alkyl having up to and including

4 carbon atoms, and/or by lower alkoxy having up to and including 4 carbon atoms, and the other represents pyridyl or 1-oxidopyridyl each of which can be unsubstituted or substituted by halogen having an atomic number of up to and including 35, by hydroxy, and/or by lower alkoxy having up to and including 4 carbon atoms, A represents lower alkylene having up to and including 4 carbon atoms, lower alkylidene having up to and including 7 carbon atoms, lower alkenylidene having up to and including 7 carbon atoms, or 3- to 8-membered cyclo-lower alkylidene, and $R_3$ represents carboxy, lower alkoxycarbonyl having up to and including 5 carbon atoms, carbamoyl, N-mono-lower alkylcarbamoyl having up to and including 4 carbon atoms in the lower alkyl, or N,N-di-lower alkylcarbamoyl having up to and in cluding 4 carbon atoms in each lower alkyl, wherein the lower alkoxycarbonyl can be substituted by lower alkanoyloxy having up to and including 5 carbon atoms, their isomers and their salts, especially pharmaceutically acceptable salts, characterised in that compounds of the formulae II or IIIa and IIIb, IIIc, IIIh and IIIi, IIId and IIIe, IIIk and IIIp or IIIb as well as IIIf or V or VI or VIIa and VIIb or VIII are used as starting materials, in which the variables $Y_1$ to $Y_6$, $R_3'$ and $R_4$ have the meanings given in claim 1 and the variables $Z_1$ and $Z_2$ have the meanings given in claim 2.

12. Process according to any one of claims 1 to 4 for the manufacture of compounds of the formula I in which each of $R_1$ and $R_2$ represents, independently of the other, phenyl and/or phenyl substituted by lower alkoxy having up to and including 4 carbon atoms, A represents lower alkylene having up to and including 4 carbon atoms or lower alkylidene having up to and including 4 carbon atoms, and $R_3$ represents carboxy or lower alkoxycarbonyl having up to and including 5 carbon atoms, their isomers and their salts, especially pharmaceutically acceptable salts, with the proviso that, if A represents methylene or ethylidene and $R_3$ represents ethoxycarbonyl, at least one of the radicals $R_1$ and $R_2$ is different from phenyl, and the further proviso that, if A represents ethylidene and $R_3$ represents carboxy, at least one of the radicals $R_1$ and $R_2$ is different from phenyl and p-methoxyphenyl, characterised in that compounds of the formulae II or IIIa and IIIb, IIIc, IIIh and IIIi, IIId and IIIe, IIIk and IIIp or IIIb as well as IIIf or V or VI or VIIa and VIIb or VIII are used as starting materials, in which the variables $Y_1$ to $Y_6$, $R_3'$ and $R_4$ have the meanings given in claim 1 and the variables $Z_1$ and $Z_2$ have the meanings given in claim 2.

13. Process according to any one of claims 1 to 4 for the manufacture of compounds of the formula I in which one of the radicals $R_1$ and $R_2$ represents phenyl or phenyl substituted by halogen having an atomic number of up to and including 35, by hydroxy or by lower alkoxy having up to and including 4 carbon atoms, and the other represents pyridyl or 1-oxidopyridyl, A represents lower alkylidene having up to and including 4 carbon atoms and containing a quaternary carbon atom, wherein the quaternary carbon atom is bonded directly to the imidazole ring, and $R_3$ represents lower alkoxycarbonyl having up to and including 5 carbon atoms, their isomers and their salts, especially pharmaceutically acceptable salts, characterised in that compounds of the formulae II or IIIa and IIIb, IIIc, IIIh and IIIi, IIId and IIIe, IIIk and IIIp or IIIb as well as IIIf or V or VI or VIIa and VIIb or VIII are used as starting materials, in which the variables $Y_1$ to $Y_6$, $R_3'$ and $R_4$ have the meanings given in claim 1 and the variables $Z_1$ and $Z_2$ have the meanings given in claim 2.

14. Process according to any one of claims 1 to 4 for the manufacture of compounds of the formula I in which one of the radicals $R_1$ and $R_2$ represents phenyl and the other represents 1-oxidopyridyl, A represents 2,2-propylidene and $R_3$ represents lower alkoxycarbonyl having up to and including 5 carbon atoms, their isomers and their salts, especially pharmaceutically acceptable salts, characterised in that compounds of the formulae II or IIIa and IIIb, IIIc, IIIh and IIIi, IIId and IIIe, IIIk and IIIp or IIIb as well as IIIf or V or VI or VIIa and VIIb or VIII are used as starting materials, in which the variables $Y_1$ to $Y_6$, $R_3'$ and $R_4$ have the meanings given in claim 1 and the variables $Z_1$ and $Z_2$ have the meanings given in claim 2.

15. Process according to claim 5 or 6 for the manufacture of
2-[4(5)-phenyl-5(4)-(3-pyridyl)-imidazol-2-yl]-2-methyl ethyl propionate
or a salt thereof, characterised in that compounds of the formulae II, IV, V or VI are used as starting materials in which the variables $Y_1$ to $Y_6$, $R_3'$ and $R_4$ have the meanings given in claim 5.

16. Process according to any one of claims 1 to 4 for the manufacture of
2-[4(5)-phenyl-5(4)-(3-pyridyl)-imidazol-2-yl]-2-methyl ether propionate
or a salt thereof, characterised in that compounds of the formulae II or IIIa and IIIb, IIIc, IIIh and IIIi, IIId and IIIe, IIIk and IIIp or IIIb as well as IIIf or V or VI or VIIa and VIIb or VIII are used as starting materials, in which the variables $Y_1$ to $Y_6$, $R_3'$ and $R_4$ have the meanings given in claim 1 and the variables $Z_1$ and $Z_2$ have the meanings given in claim 2.

17. Process according to claim 5 or 6 for the manufacture of
2-[4(5)-phenyl-5(4)-(3-pyridyl)-imidazol-2-yl]-2-allyl ethyl acetate
or a salt thereof, characterised in that compounds of the formulae II, IV, V or VI are used as starting materials in which the variables $Y_1$ to $Y_6$, $R_3'$ and $R_4$ have the meanings given in claim 5.

18. Process according to any one of claims 1 to 4 for the manufacture of
2-[4(5)-phenyl-5(4)-(3-pyridyl)-imidazol-2-yl]-2-allyl ethyl acetate,
characterised in that compounds of the formulae II or IIIa and IIIb, IIIc, IIIh and IIIi, IIId and IIIe, IIIk and IIIp or IIIb as well as IIIf or V or VI or VIIa and VIIb or VIII are used as starting materials, in which

the variables $Y_1$ to $Y_6$, $R_3'$ and $R_4$ have the meanings given in claim 1 and the variables $Z_1$ and $Z_2$ have the meanings given in claim 2.

19. Process according to claim 5 or 6 for the manufacture of
1-[4(5)-phenyl-5(4)-(3-pyridyl)-imidazol-2-yl]-1-ethoxycarbonylcyclopentane
or a salt thereof, characterised in that compounds of the formulae II, IV, V or VI are used as starting materials in which the variables $Y_1$ to $Y_6$, $R_3'$ and $R_4$ have the meanings given in claim 5.

20. Process according to any one of claims 1 to 4 for the manufacture of
1-[4(5)-phenyl-5(4)-(3-pyridyl)-imidazol-2-yl]-1-ethoxycarbonylcyclopentane
or a salt thereof, characterised in that compounds of the formulae II or IIIa and IIIb, IIIc, IIIh and IIIi, IIId and IIIe, IIIk and IIIp or IIIb as well as IIIf or V or VI or VIIa and VIIb or VIII are used as starting materials, in which the variables $Y_1$ to $Y_6$, $R_3'$ and $R_4$ have the meanings given in claim 1 and the variables $Z_1$ and $Z_2$ have the meanings given in claim 2.

21. Process according to claim 5 or 6 for the manufacture of
2-[4(5)-(p-methoxyphenyl)-5(4)-(3-pyridyl)-imidazol-2-yl]-2-methyl ethyl propionate
or a salt thereof, characterised in that compounds of the formulae II, IV, V or VI are used as starting materials in which the variables $Y_1$ to $Y_6$, $R_3'$ and $R_4$ have the meanings given in claim 5.

22. Process according to any one of claims 1 to 4 for the manufacture of
2-[4(5)-(p-methoxyphenyl)-5(4)-(3-pyridyl)-imidazol-2-yl]-2-methyl ethyl propionate
or a salt thereof, characterised in that compounds of the formulae II or IIIa and IIIb, IIIc, IIIh and IIIi, IIId and IIIe, IIIk and IIIp or IIIb as well as IIIf or V or VI or VIIa and VIIb or VIII are used as starting materials, in which the variables $Y_1$ to $Y_6$, $R_3'$ and $R_4$ have the meanings given in claim 1 and the variables $Z_1$ and $Z_2$ have the meanings given in claim 2.

23. Process according to claim 5 or 6 for the manufacture of
2-[4(5)-phenyl-5(4)-(1-oxido-3-pyridyl)-imidazol-2-yl]-2-methyl ethyl propionate
or a salt thereof, characterised in that compounds of the formulae II, IV, V or VI are used as starting materials in which the variables $Y_1$ to $Y_6$, $R_3'$ and $R_4$ have the meanings given in claim 5.

24. Process according to any one of claims 1 to 4 for the manufacture of
2-[4(5)-phenyl-5(4)-(1-oxido-3-pyridyl)-imidazol-2-yl]-2-methyl ethyl propionate
or a salt thereof, characterised in that compounds of the formulae II or IIIa and IIIb, IIIc, IIIh and IIIi, IIId and IIIe, IIIk and IIIp or IIIb as well as IIIf or V or VI or VIIa and VIIb or VIII are used as starting materials, in which the variables $Y_1$ to $Y_6$, $R_3'$ and $R_4$ have the meanings given in claim 1 and the variables $Z_1$ and $Z_2$ have the meanings given in claim 2.

25. Process according to claim 5 or 6 for the manufacture of
2-[4(5)-phenyl-5(4)-(3-pyridyl)-imidazol-2-yl]-ethyl propionate
or a salt thereof, characterised in that compounds of the formulae II, IV, V or VI are used as starting materials in which the variables $Y_1$ to $Y_6$, $R_3'$ and $R_4$ have the meanings given in claim 5.

26. Process according to any one of claims 1 to 4 for the manufacture of
2-[4(5)-phenyl-5(4)-(3-pyridyl)-imidazol-2-yl]-ethyl propionate
or a salt thereof, characterised in that compounds of the formulae II or IIIa and IIIb, IIIc, IIIh and IIIi, IIId and IIIe, IIIk and IIIp or IIIb as well as IIIf or V or VI or VIIa and VIIb or VIII are used as starting materials, in which the variables $Y_1$ to $Y_6$, $R_3'$ and $R_4$ have the meanings given in claim 1 and the variables $Z_1$ and $Z_2$ have the meanings given in claim 2.

27. Process according to any one of claims 1 to 4 for the manufacture of
2-[4(5)-(p-chlorophenyl)-5(4)-(3-pyridyl)-imidazol-2-yl]-2-methyl ethyl propionate
or a salt thereof, characterised in that compounds of the formulae II or IIIa and IIIb, IIIc, IIIh and IIIi, IIId and IIIe, IIIk and IIIp or IIIb as well as IIIf or V or VI or VIIa and VIIb or VIII are used as starting materials, in which the variables $Y_1$ to $Y_6$, $R_3'$ and $R_4$ have the meanings given in claim 1 and the variables $Z_1$ and $Z_2$ have the meanings given in claim 2.

28. Process according to any one of claims 1 to 4 for the manufacture of
2-[4(5)-phenyl-5(4)-(4-pyridyl)-imidazol-2-yl]-2-methyl ethyl propionate
or a salt thereof, characterised in that compounds of the formulae II or IIIa and IIIb, IIIc, IIIh and IIIi, IIId and IIIe, IIIk and IIIp or IIIb as well as IIIf or V or VI or VIIa and VIIb or VIII are used as starting materials, in which the variables $Y_1$ to $Y_6$, $R_3'$ and $R_4$ have the meanings given in claim 1 and the variables $Z_1$ and $Z_2$ have the meanings given in claim 2.

29. Process according to any one of claims 1 to 4 for the manufacture of
2-[4(5)-phenyl-5(4)-(1-oxido-4-pyridyl)-imidazol-2-yl]-2-methyl ethyl propionate
or a salt thereof, characterised in that compounds of the formulae II or IIIa and IIIb, IIIc, IIIh and IIIi, IIId and IIIe, IIIk and IIIp or IIIb as well as IIIf or V or VI or VIIa and VIIb or VIII are used as starting materials, in which the variables $Y_1$ to $Y_6$, $R_3'$ and $R_4$ have the meanings given in claim 1 and the variables $Z_1$ and $Z_2$ have the meanings given in claim 2.

30. Process according to any one of claims 1 to 4 for the manufacture of
2-[4(5)-(p-hydroxyphenyl)-5(4)-(3-pyridyl)-imidazol-2-yl]-2-methyl ethyl propionate
or a salt thereof, characterised in that compounds of the formulae II or IIIa and IIIb, IIIc, IIIh and IIIi, IIId and IIIe, IIIk and IIIp or IIIb as well as IIIf or V or VI or VIIa and VIIb or VIII are used as starting materials, in which the variables $Y_1$ to $Y_6$, $F_3'$ and

$R_4$ have the meanings given in claim 1 and the variables $Z_1$ and $Z_2$ have the meanings given in claim 2.

31. Process for the manufacture of pharmaceutical preparations, characterised in that a compound obtainable in accordance with any one of claims 5, 6, 9, 15, 17, 19, 21, 23 or 25 in free form or in the form of a pharmaceutically acceptable salt, is mixed with customary pharmaceutical adjuncts and carriers.

32. Process for the manufacture of pharmaceutical preparations, characterised in that a compound obtainable in accordance with any one of claims 1 to 4, 7, 8, 10 to 14, 15, 18, 20, 22, 24 or 26 to 30 in free form or in the form of a pharmaceutically acceptable salt, is mixed with customary pharmaceutical adjuncts and carriers.

33. Process for the manufacture of compounds of the formula

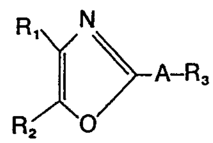

(IIIf),

in which one of the radicals $R_1$ and $R_2$ represents phenyl, pyrrolyl, pyridyl, 1-oxidopyridyl or pyrimidyl each of which can be unsubstituted or substituted by halogen, lower alkyl, hydroxy, lower alkoxy and/or lower alkanoyloxy, and the other represents pyrrolyl, pyridyl, 1-oxidopyridyl or pyrimidyl each of which can be unsubstituted or substituted by halogen, lower alkyl, hydroxy, lower alkoxy and/or lower alkanoyloxy and A represents lower alkylene, lower alkylidene, lower alkenylene, lower alkenylidene, cycloalkylene, cycloalkylidene or cycloalkyl-lower alkylidene, and $R_3$ represents carboxy, carboxy esterified by a lower alkanol, by a 3- to 8-membered cycloalkanol, by phenol, by a hydroxypyridine, or by a substituted phenol or substituted hydroxypyridine, or represents carbamoyl or carbamoyl mono-substituted by hydroxy, amino, phenyl or by substituted phenyl, carbamoyl mono- or di-substituted by lower alkyl, or carbamoyl di-substituted by 4- to 7-membered alkylene or 3-aza-, 3-lower alkylaza-, 3-oxa- or 3-thia-alkylene, wherein a lower alkanol or cycloalkanol can be unsubstituted or substituted by hydroxy, mercapto, optionally substituted phenyl, lower alkoxy, phenyl-lower alkoxy optionally substituted in the phenyl moiety, lower alkylthio, phenyl-lower alkylthio optionally substituted in the phenyl moiety, hydroxy-lower alkoxy, lower alkoxy-lower alkoxy, phenyl-lower alkoxy-lower alkoxy optionally substituted in the phenyl moiety, carboxy-lower alkoxy, lower alkoxycarbonyl-lower alkoxy, or lower alkoxycarbonyl-lower alkoxy or lower alkanoyloxy containing optionally substituted phenyl, and wherein substituted phenyl, phenol or hydroxypyridine can each be substituted by lower alkyl, lower alkoxy, halogen and/or trifluoromethyl, their isomers and their salts, characterised in that compounds of the formula

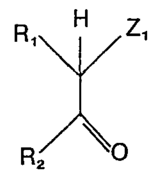

(IIId)

in which $Z_1$ represents optionally reactively esterified hydroxy, are reacted with carboxylic acids of the formula $R_3$–A–COOH (IIIp), the salts or functional derivatives thereof and with ammonia, or compounds of the formula

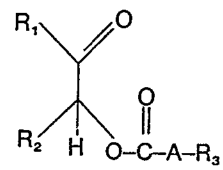

(IIIq)

which are obtainable by reacting compounds of the formula

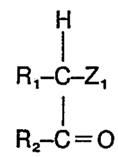

(IIId)

with optionally functional derivatives of compounds of the formula

$$R_3\text{–A–COOH} \qquad \text{(IIIp)}$$

are reacted with ammonia, if desired non N-oxidised heteroaryl radicals $R_1$ and $R_2$ in a resulting compound of the formula IIIf are N-oxidised by means of an oxidising agent and, if desired, the free compound obtainable in accordance with the process is converted into a salt, or a salt obtainable in accordance with the process is converted into the free compound or into a different salt, or a free compound obtainable in accordance with the process is converted into a different free compound and/or, if desired, a mixture of isomeric compounds of the formula I obtainable in accordance with the invention is separated into the individual isomers.

34. Process according to claim 33, characterised in that a start is made at any stage of the process and the remaining steps are carried out or a starting material is used in the form of a salt and/or a tautomer and/or is formed under the reaction conditions.

35. Process according to claims 33 and 34 for the manufacture of compounds of the formula IIIf in which one of the radicals $R_1$ and $R_2$ represents pyridyl or 1-oxidopyridyl each of which can be unsubstituted and/or substituted by halogen, hydroxy, lower alkyl, lower alkoxy and/or lower alkanoyloxy, and the other represents phenyl or 1-oxidopyridyl each of which can be unsubstituted and/or substituted by halogen, hydroxy, lower alkyl, lower alkoxy and/or lower alkanoyloxy, A represents lower alkylene having up to and including 4 carbon atoms, lower alkylidene having up to and including 7 carbon atoms, lower alkenylene having up to and including 4 carbon atoms, lower alkenylidene having up to and including 7 carbon atoms, 3- to 8-membered cycloalkylene, 3- to 8-membered cycloalkylidene, or cycloalkyl-lower alkylidene having up to and including 7 carbon atoms in the alkylidene moiety and having a 3- to 8-membered cycloalkyl moiety, and $R_3$ represents carboxy, carboxy esterified by a lower alkanol, by a 3- to 8-membered cycloalkanol, by phenol or by a substituted phenol, or represents carbamoyl, N-mono- or N,N-di-lower alkylcarbamoyl, pyrrolidino-, piperidino-, morpholino-, piperazino-, 4-lower alkylpiperazino-, thiomorpholino- or anilinocarbonyl, or anilinocarbonyl substituted by lower alkyl, lower alkoxy and/or halogen, wherein the lower alkanol or cycloalkanol can be unsubstituted or substituted by hydroxy, mercapto, phenyl, substituted phenyl, lower alkoxy, phenyl-lower alkoxy, phenyl-lower alkoxy substituted in the phenyl moiety, lower alkylthio, phenyl-lower alkylthio, phenyl-lower alkylthio substituted in the phenyl moiety, hydroxy-lower alkoxy, lower alkoxy-lower alkoxy, phenyl-lower alkoxy-lower alkoxy, phenyl-lower alkoxy-lower alkoxy substituted in the phenyl moiety, carboxy-lower alkoxy, lower alkoxycarbonyl-lower alkoxy, or lower alkoxycarbonyl-lower alkoxy or lower alkanoyloxy containing optionally substituted phenyl, and wherein substituted phenol or phenyl can each be substituted by lower alkyl, lower alkoxy, halogen and/or trifluoromethyl, their isomers and their salts.

36. Process according to claim 33 and 34 for the manufacture of compounds of the formula IIIf in which one of the radicals $R_1$ and $R_2$ represents phenyl or phenyl substituted by halogen having an atomic number of up to and including 35, by hydroxy, by lower alkyl having up to and including 4 carbon atoms, and/or by lower alkoxy having up to and including 4 carbon atoms, and the other represents pyridyl and/or 1-oxidopyridyl each of which can be unsubstituted or substituted by halogen having an atomic number of up to and including 35, by hydroxy, or by lower alkoxy having up to and including 4 carbon atoms, A represents lower alkylene having up to and including 4 carbon atoms, lower alkylidene having up to and including 7 carbon atoms, lower alkenylidene having up to and including 7 carbon atoms, or 3- to 8-membered cyclo-lower alkylidene, and $R_3$ represents carboxy, lower alkoxycarbonyl having up to and including 5 carbon atoms, carbamoyl, N-mono-lower alkylcarbamoyl having up to and including 4 carbon atoms in the lower alkyl, or N,N-di-lower alkylcarbamoyl having up to and including 4 carbon atoms in each lower alkyl, wherein the lower alkoxycarbonyl can be substituted by lower alkanoyloxy having up to and including 5 carbon atoms, their isomers and their salts.

37. Process according to claim 33 or 34 for the manufacture of compounds of the formula IIIf in which one of the radicals $R_1$ and $R_2$ represents phenyl or phenyl substituted by halogen having an atomic number of up to and including 35, by hydroxy or by lower alkoxy having up to and including 4 carbon atoms, and the other represents pyridyl or 1-oxidopyridyl, A represents lower alkylidene having up to and including 4 carbon atoms and containing a quaternary carbon atom, wherein the quaternary carbon atom is bonded directly to the imidazole ring, and $R_3$ represents lower alkoxycarbonyl having up to and including 5 carbon atoms, their isomers and their salts.

38. Process according to claims 33 and 34 for the manufacture of 2-[4-phenyl-5-(3-pyridyl)-oxazol-2-yl]-ethyl acetate or a salt thereof.

39. Process according to claims 33 and 34 for the manufacture of 2-[4-phenyl-5-(1-oxido-3-pyridyl)-oxazol-2-yl]-2-methyl ethyl propionate or a salt thereof.

**Revendications pour les Etats contractants: BE CH DE FR IT LI LU NL SE**

1. Nouveaux dérivés d'imidazole trisubstitués de formule générale

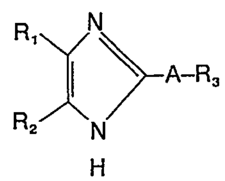

(I) ,

où d'une part R1 et R2 représentent indépendamment l'un de l'autre un phényle et/ou un phényle substitué par halogène, alcoyle inférieur, hydroxy, alcoxy inférieur et/ou alcanoyle inférieur, ou bien où d'autre part l'un des radicaux R1 et R2 représentent pyrrolyle, furyle, thiényle, pyridyle, 1-oxydopyridyle ou pyrimidyle, qui peuvent être chacun non substitués ou substitués par halogène, alcoyle inférieur, hydroxy, alcoxy inférieur et/ou alcanoyloxy inférieur, et l'autre représente phényle, pyrrolyle, furyle, thiényle, pyridyle, 1-oxydopyridyle ou pyrimidyle, qui peuvent chacun être non substitués ou substitués par halogène, alcoyle inférieur, hydroxy, alcoxy inférieur et/ou alcanoyloxy inférieur, et où à chaque fois A représente alcoylène inférieur, alcoylidène inférieur, alcénylène inférieur, alcénylidène inférieur, cycloalcoylène, cycloalcoylidène ou cycloalcoyl-alcoylidène inférieur, et R3 représente un carboxy, un carboxy estérifié par un alcanol inférieur, un cycloalcanol à 3 à 8 chaînons, un phénol, une hydroxypyridine, un phénol substitué ou selon les cas une hydroxypyridine, un carbamoyle ou un carbamoyle monosubstitué par hydroxy, amino, phényle ou phényle substitué, mono- ou disubstitué par alcoyle inférieur ou disubstitué par alcoylène à 4 à 7 chaînons ou selon les cas 3-aza-, 3-alcoyle inférieur-aza, 3-oxa- ou 3-thiaalcoylène, où un alcanol inférieur ou selon les cas un cycloalcanol peut être non substitué ou substitué par hydroxy, mercapto, phényle éventuellement substitué, alcoxy inférieur, phénylalcoxy inférieur éventuellement substitué dans la fraction phényle, alcoyle inférieur-thio, phénylalcoyle inférieur-thio

éventuellement substitué dans la fraction phényle, hydroxyalcoxy inférieur, alcoxy inférieur-alcoxy inférieur, phénylalcoxy inférieur-alcoxy inférieur éventuellement substitué dans la fraction phényle, carboxy-alcoxy inférieur, alcoxy inférieur-carbonyl-alcoxy inférieur, alcoxy inférieur-carbonyl-alcoxy inférieur présentant un phényle éventuellement substitué, ou alcanoyloxy inférieur et phényle substitué, phénol ou selon les cas hydroxypyridine à chaque fois substituée par alcoyle inférieur, alcoxy inférieur, halogène et/ou trifluorométhyle, leurs isomères ainsi que leurs sels, en particulier leurs sels pharmaceutiquement acceptables, avec la précision que lorsque A représente un méthylène ou selon les cas un éthylidène et R3 représente un éthoxycarbonyle, les deux radicaux R1 et R2 ne représentent pas simultanément un phényle, et la précision supplémentaire que, lorsque A représente un éthylidène et R3 un carboxy ou selon les cas un carbamoyle, les deux radicaux R1 et R2 ne représentent pas simultanément un phényle, un p-méthoxyphényle ou un p-chlorophényle, où les radicaux décrits comme «inférieurs» doivent être compris comme ceux qui contiennent jusqu'à 7 atomes de carbone compris.

2. Composés selon la revendication 1 de formule I, où R1 et R2 représentent indépendamment l'un de l'autre phényle et/ou phényle substitué par halogène, hydroxy, alcoyle inférieur, alcoxy inférieur et/ou alcanoyloxy inférieur, A représente un alcoylène inférieur ayant jusqu'à 4 atomes de carbone compris, un alcoylidène inférieur ayant jusqu'à 7 atomes de carbone compris, un cycloalcoylène à 3 à 8 chaînons, un cycloalcoylidène à 3 à 8 chaînons ou un cycloalcoyl-alcoylidène inférieur ayant jusqu'à 7 atomes de carbone compris dans la fraction alcoylidène, et avec une fraction cycloalcoyle à 3 à 8 chaînons, et R3 représente un carboxy, un carboxy estérifié avec un alcanol inférieur, un cycloalcanol à 3 à 8 chaînons, un phénol ou un phénol substitué, un carbamoyle, un N-mono-, N,N-dialcoyle inférieur-carbamoyle, un pyrrolidino-, pipéridino-, morpholino-, pipérazino-, 4-alcoyle inférieur-pipérazino-, thiomorpholino-, anilino- ou anilino-carbonyle substitué par alcoyle inférieur, alcoxy inférieur et/ou halogène, où l'alcanol inférieur ou selon les cas cycloalcanol est non substitué ou peut être substitué par hydroxy, mercapto, phényle, phényle substitué, alcoxy inférieur, phénylalcoxy inférieur, phénylalcoxy inférieur substitué dans la fraction phényle, alcoyle inférieur-thio, phénylalcoyle inférieur-thio, phénylalcoyle inférieur-thio substitué dans la fraction phényle, hydroxyalcoxy inférieur, alcoxy inférieur-alcoxy inférieur, phénylalcoxy inférieur-alcoxy inférieur, phénylalcoxy inférieur-alcoxy inférieur substitué dans la fraction phényle, carboxyalcoxy inférieur, alcoxy inférieur-carbonyl-alcoxy inférieur, alcoxy inférieur-carbonyl-alcoxy inférieur présentant éventuellement un phényle substitué, ou alcanoyloxy inférieur et phénol substitué ou selon les cas phényle à chaque fois substitué par alcoyle inférieur, alcoxy inférieur, halogène et/ou trifluorométhyle, leurs isomères ainsi que leurs sels, en particulier les sels pharmaceutiquement acceptables, avec la précision que, lorsque A représente méthylène ou selon les cas éthylidène et R3 représente éthoxycarbonyle, les deux radicaux R1 et R2 ne représentent pas simultanément un phényle, et la précision supplémentaire que, lorsque A représente un éthylidène et R3 un carboxy ou selon les cas un carbamoyle, les deux radicaux R1 et R2 ne représentent pas simultanément un phényle, un p-méthoxyphényle ou un p-chlorophényle.

3. Composés selon la revendication 1 de formule I, où l'un des radicaux R1 et R2 représente un pyridyle ou un 1-oxydo-pyridyle, qui peuvent être non substitués et/ou à chaque fois substitués par halogène, hydroxy, alcoyle inférieur, alcoxy inférieur et/ou alcanoyloxy inférieur, et l'autre représente un phényle, un pyridyle ou un 1-oxydopyridyle, qui peuvent être non substitués et/ou substitués à chaque fois halogène, hydroxy, alcoyle inférieur, alcoxy inférieur et/ou alcanoyloxy inférieur, A représente un alcoylène inférieur ayant jusqu'à 4 atomes de carbone compris, un alcoylidène inférieur ayant jusqu'à 7 atomes de carbone compris, un alcénylène inférieur ayant jusqu'à 4 atomes de carbone compris, un alcénylidène inférieur ayant jusqu'à 7 atomes de carbone compris, un cycloalcoylène à 3 à 8 chaînons, un cycloalcoylidène à 3 à 8 chaînons ou un cycloalcoyl-alcoylidène inférieur ayant jusqu'à 7 atomes de carbone compris dans la fraction alcoylidène et avec une fraction cycloalcoyle à 3 à 8 chaînons et R3 représente un carboxy, un carboxy estérifié avec un alcanol inférieur, un cycloalcanol à 3 à 8 chaînons, un phénol ou un phénol substitué; un carbamoyle, un N-mono-, N,N-dialcoyle inférieur-carbamoyle, un pyrrolidino-, pipéridino-, morpholino-, pipérazino-, 4-alcoyle inférieur-pipérazino-, thiomorpholino-, anilino- ou anilino-carbonyle substitué par alcoyle inférieur, alcoxy inférieur et/ou halogène, où l'alcanol inférieur ou selon les cas cycloalcanol est non substitué ou peut être substitué par hydroxy, mercapto, phényle, phényle substitué, alcoxy inférieur, phénylalcoxy inférieur, phénylalcoxy inférieur substitué dans la fraction phényle, alcoyle inférieur-thio, phénylalcoyle inférieur-thio, phénylalcoyle inférieur-thio substitué dans la fraction phényle, hydroxyalcoxy inférieur, alcoxy inférieur-alcoxy inférieur, phénylalcoxy inférieur-alcoxy inférieur, phénylalcoxy inférieur-alcoxy inférieur substitué dans la fraction phényle, carboxyalcoxy inférieur, alcoxy inférieur-carbonyl-alcoxy inférieur, alcoxy inférieur-carbonyl-alcoxy inférieur présentant éventuellement un phényle substitué, ou alcanoyloxy inférieur et phénol substitué ou selon les cas phényle à chaque fois substitué par alcoyle inférieur, alcoxy inférieur, halogène et/ou trifluorométhyle, leurs isomères ainsi que leurs sels, en particulier les sels pharmaceutiquement acceptables.

4. Composés selon la revendication 1 de formule I, où R1 et R2 représentent indépendamment l'un de l'autre un phényle éventuellement substitué par un halogène dont le numéro atomique va jusqu'à 35 compris, un alcoyle inférieur ayant jusqu'à 4 atomes de carbone compris, un alcoxy

inférieur ayant jusqu'à 4 atomes de carbone compris et/ou un alcanoyloxy inférieur ayant jusqu'à 5 atomes de carbone compris, un pyridyle et/ou un 1-oxydopyridyle, A représente un alcoylène inférieur ayant jusqu'à 4 atomes de carbone compris, R3 représente un carboxy éventuellement estérifié par un alcanol inférieur ayant jusqu'à 4 atomes de carbone compris, où l'alcanol inférieur peut être éventuellement substitué par un hydroxy, un mercapto, un phényle éventuellement substitué, un alcoxy inférieur ayant jusqu'à 4 atomes de carbone compris, un phénylalcoxy inférieur éventuellement substitué dans le noyau phényle et ayant jusqu'à 4 atomes de carbone compris dans la fraction alcoyle, un alcoyle inférieur-thio ayant jusqu'à 4 atomes de carbone compris, un hydroxyalcoxy inférieur ayant jusqu'à 4 atomes de carbone compris dans la fraction alcoyle inférieur, un alcoxy inférieur-alcoxy inférieur ayant à chaque fois jusqu'à 4 atomes de carbone compris dans la fraction alcoyle inférieur ou un alcanoyloxy inférieur ayant jusqu'à 5 atomes de carbone compris et un phényle éventuellement substitué par un alcoyle inférieur ayant jusqu'à 4 atomes de carbone compris, un alcoxy inférieur ayant jusqu'à 4 atomes de carbone compris, un halogène dont le numéro atomique va jusqu'à 35 compris et/ou un trifluorométhyle, leurs isomères ainsi que leurs sels, avec la précision que lorsque A représente un méthylène ou selon les cas un éthylidène et R3 représente un éthoxycarbonyle, les deux radicaux R1 et R2 ne représentant pas ensemble un phényle, et la précision supplémentaire que lorsque A représente un éthylidène et R3 un carboxy, les deux radicaux R1 et R2 ne représentent pas simultanément un phényle, un p-méthoxyphényle ou un p-chlorophényle.

5. Composés selon la revendication 1 de la formule I, où R1 et R2 représentent indépendamment l'un de l'autre un phényle et/ou un phényle substitué par un halogène dont le numéro atomique va jusqu'à 35 compris, un hydroxy, un alcoyle inférieur ayant jusqu'à 4 atomes de carbone compris et/ou un alcoxy inférieur ayant jusqu'à 4 atomes de carbone compris, A représente un alcoylène inférieur ayant jusqu'à 4 atomes de carbone compris, un alcoylidène inférieur ayant jusqu'à 7 atomes de carbone compris, un alcénylidène inférieur ayant jusqu'à 7 atomes de carbone compris ou un cycloalcoylidène inférieur à 3 à 8 chaînons et R3 représente un carboxy, un alcoxy inférieur-carbonyle ayant jusqu'à 5 atomes de carbone compris, un carbamoyle, un N-monoalcoyle inférieur-carbamoyle ayant jusqu'à 4 atomes de carbone compris dans la partie alcoyle inférieur ou N,N-dialcoyle inférieur-carbamoyle ayant à chaque fois jusqu'à 4 atomes de carbone compris dans la partie alcoyle inférieur, où l'alcoxy inférieur-carbonyle peut être substitué par un alcanoyloxy inférieur ayant jusqu'à 5 atomes de carbone compris, leurs isomères ainsi que leurs sels, en particulier leurs sels pharmaceutiquement acceptables, avec la précision que lorsque A représente un méthylène ou selon les cas un éthylidène et R3 représente un éthoxycarbonyle, les deux radicaux R1 et R2 ne représentent pas simultanément un phényle, et la précision ultérieure que lorsque A représente un éthylidène et R3 un carboxy ou selon les cas un carbamoyle, les deux radicaux R1 et R2 ne représentent pas tous deux simultanément un phényle, un p-méthoxyphényle ou un p-chlorophényle.

6. Composés selon la revendication 1 de formule I, où l'un des radicaux R1 et R2 représente un phényle ou un phényle substitué par un halogène dont le numéro atomique va jusqu'à 35 compris, un hydroxy, un alcoyle inférieur ayant jusqu'à 4 atomes de carbone compris et/ou un alcoxy inférieur ayant jusqu'à 4 atomes de carbone compris, et l'autre représente un pyridyle ou un 1-oxydo-pyridyle, qui à chaque fois peuvent être non substitués ou substitués par un halogène dont le numéro atomique va jusqu'à 35 compris, un hydroxy et/ou un alcoxy inférieur ayant jusqu'à 4 atomes de carbone compris, A représente un alcoylène inférieur ayant jusqu'à 4 atomes de carbone compris, un alcoylidène inférieur ayant jusqu'à 7 atomes de carbone compris, ou un cycloalcoylidène inférieur à 3 à 7 chaînons et R3 représente un carboxy, un alcoxy inférieur-carbonyle ayant jusqu'à 5 atomes de carbone compris, un carbamoyle, un N-monoalcoyle inférieur-carbamoyle ayant jusqu'à 4 atomes de carbone compris dans la partie alcoyle inférieur ou un N,N-dialcoyle inférieur-carbamoyle ayant à chaque fois jusqu'à 4 atomes de carbone compris dans la partie alcoyle inférieur, où l'alcoxy inférieur-carbonyle peut être substitué par un alcanoyloxy inférieur ayant jusqu'à 5 atomes de carbone compris, leurs isomères ainsi que leurs sels, en particulier les sels pharmaceutiquement acceptables.

7. Composés selon la revendication 1 de formule I, où R1 et R2 représentent indépendamment l'un de l'autre un phényle et/ou un phényle substitué par un alcoxy inférieur ayant jusqu'à 4 atomes de carbone compris, A représente un alcoylène inférieur ayant jusqu'à 4 atomes de carbone compris ou un alcoylidène inférieur ayant jusqu'à 4 atomes de carbone compris et R3 représente un carboxy ou un alcoxy inférieur-carbonyle ayant jusqu'à 5 atomes de carbone compris, leurs isomères ainsi que leurs sels, en particulier les sels pharmaceutiquement acceptables, avec la précision que lorsque A représente un méthylène ou selon les cas un éthylidène et R3 un éthoxycarbonyle, les deux radicaux R1 et R2 ne représentent pas simultanément un phényle, et la précision supplémentaire que lorsque A représente un éthylidène et R3 un carboxy, les deux radicaux R1 et R2 ne représentent pas simultanément un phényle ou un p-méthoxyphényle.

8. Composés selon la revendication 1 de formule I, où l'un des radicaux R1 et R2 représente un phényle ou un phényle substitué par un halogène de numéro atomique allant jusqu'à 35 compris, un hydroxy ou un alcoxy inférieur ayant jusqu'à 4 atomes de carbone compris et l'autre représente un pyridyle ou un 1-oxydopyridyle, A représente un alcoylidène inférieur présentant un atome de carbone quaternaire et ayant jusqu'à 4

atomes de carbone compris, où l'atome de carbone quaternaire est lié directement au noyau imidazole, et R3 représente un alcoxy inférieur-carbonyle ayant jusqu'à 5 atomes de carbone compris, leurs isomères ainsi que leurs sels, en particulier les sels pharmaceutiquement acceptables.

9. Composés selon la revendication 1 de formule I, où l'un des radicaux R1 et R2 représente un phényle et l'autre un 1-oxydopyridyle, A représente un 2,2-propylidène et R3 représente un alcoxy inférieur-carbonyle ayant jusqu'à 5 atomes de carbone compris, leurs isomères ainsi que leurs sels, en particulier leurs sels pharmaceutiquement acceptables.

10. Ester éthylique de l'acide
2-[4(5)-phényl-5(4)-(3-pyridyl)-imidazol-2-yl]-2-méthyl-propionique
ou un de ses sels.

11. Ester éthylique de l'acide
2-[4(5)-phényl-5(4)-(3-pyridyl-imidazol-2-yl]-2-allyl-acétique
ou un de ses sels.

12. 1-[4(5)-phényl-5(4)-(3-pyridyl)-imidazol-2-yl]-1-carbéthoxy-cyclopentane
ou un de ses sels.

13. Ester éthylique de l'acide
2-[4(5)-(p-méthoxyphényl)-5(4)-(3-pyridyl)-imidazol-2-yl]-2-méthyl-propionique
ou un de ses sels.

14. Ester éthylique de l'acide
2-[4(5)-phényl-5(4)-(1-oxydo-3-pyridyl)-imidazol-2-yl]-2-méthyl-propionique
ou un de ses sels.

15. Ester éthylique de l'acide
2-[4(5)-phényl-5(4)-(3-pyridyl)-imidazol-2-yl]-propionique
ou un de ses sels.

16. Ester éthylique de l'acide
2-[4(5)-(p-chlorophényl)-5(4)-(3-pyridyl)-imidazol-2-yl]-2-méthyl-propionique
ou un de ses sels.

17. Ester éthylique de l'acide
2-[4(5)-phényl-5(4)-(4-pyridyl)-imidazol-2-yl]-2-méthyl-propionique
ou un de ses sels.

18. Ester éthylique de l'acide
2-[4(5)-phényl-5(4)-(1-oxydo-4-pyridyl)-imidazol-2-yl]-2-méthyl-propionique
ou un de ses sels.

19. Ester éthylique de l'acide
2-[4(5)-(p-hydroxyphényl)-5(4)-(3-pyridyl)-imidazol-2-yl]-2-méthyl-propionique
ou un de ses sels.

20. Composés de formule I selon la revendication 1, avec la précision que lorsque A représente un éthylidène et R3 un carboxy, les deux radicaux R1 et R2 ne représentent pas simultanément un p-méthoxyphényle, aux fins d'application dans un procédé de traitement thérapeutique du corps animal ou humain.

21. Composés selon la revendication 20 de formule (I), avec la précision que lorsque A représente un méthylène ou selon les cas un éthylidène et R3 représente un éthoxycarbonyle, les deux radicaux R1 et R2 ne représentent pas simultanément un phényle, et la précision supplémentaire que lorsque A représente un éthylidène et R3 un carboxy, les deux radicaux R1 et R2 ne représentent pas simultanément un phényle, un p-méthoxyphényle ou un p-chlorophényle, aux fins d'application selon la revendication 20.

22. Composés selon l'une des revendications 1 à 19 aux fins d'application selon la revendication 20.

23. Composés de formule I selon l'une des revendications 1–19 aux fins d'application selon la revendication 20 comme stabilisateurs des inflammations locales de la peau, agents anti-herpès et/ou agents de protection contre les rayons solaires.

24. Application de composés de formule I selon l'une des revendications 1–19 aux fins de préparation de médicaments, comme de stabilisateurs des inflammations locales de la peau, d'agents anti-herpès et/ou d'agents de protection contre les rayons solaires.

25. Préparations pharmaceutiques contenant un composé selon l'une des revendications 20–23 sous forme libre ou sous la forme d'un sel pharmaceutiquement acceptable outre les additifs et supports pharmaceutiques habituels.

26. Préparations pharmaceutiques selon la revendication 25 contenant un composé selon l'une des revendications 1–19 sous forme libre ou sous la forme d'un sel pharmaceutiquement acceptable outre les additifs et supports pharmaceutiques habituels.

27. Procédé de préparation de composés de formule (I) selon la revendication 1, caractérisé en ce qu'à partir d'un composé de formule

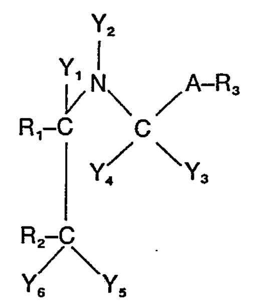

(II),

où l'un des radicaux Y1 et Y6 représente un hydroxy ou un amino et l'autre ainsi que Y2 représente un hydrogène et Y3 représente avec Y4 et Y5 un groupe de formule =N– ou bien où Y1 avec Y6 représente une liaison, Y2 est un hydrogène, Y3 est un hydroxy ou un amino et Y4 représente avec Y5 un groupe de formule –NH– ou bien où Y1 représente avec Y6 une liaison, Y2 représente avec Y3 une liaison supplémentaire et l'un des radicaux Y4 et Y5 représente un amino et l'autre un amino, un hydroxy ou un hydroxy estérifié réactif ou bien où Y1 est un hydroxy, Y2 ainsi qu'Y3 représente un hydrogène, Y4 représente un hydroxy ou un amino et Y5 représente avec Y6 un groupe de formule =NH ou, dans la mesure où Y4 est un amino, représente un oxo, ou un de ses tautomères et/ou sels, avec introduction d'une

liaison supplémentaire on clive H–Z, où Z représente hydroxy ou amino Y1 ou selon les cas Y6, Y3 ou Y4 ou selon les cas Y5 ou hydroxy estérifié Y4 ou selon les cas Y5, ou en ce qu'on réduit un composé de formule

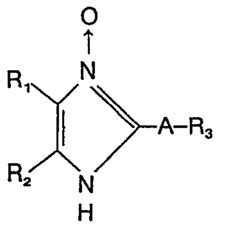
(IV)

ou un de ses sels en un composé de formule I, ou bien en ce que dans un composé de formule

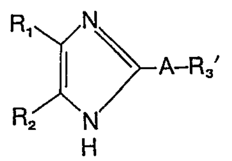
(V),

où R3′ représente un radical transformable en R3, on transforme le radical R3′ en un radical R3 ou en ce que dans un composé de formule

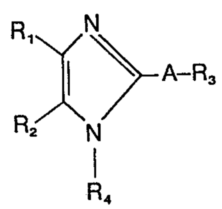
(VI),

où R4 représente un groupe transformable en hydrogène, ou un de ses sels, on transforme R4 en hydrogène, ou en ce qu'on fait réagir des composés de formule

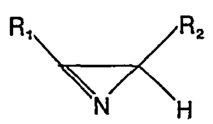
(VIIa)

ou leurs tautomères avec des composés de formule R3–A–CN (VIIb) ou en ce qu'on déshydrogène un composé de formule

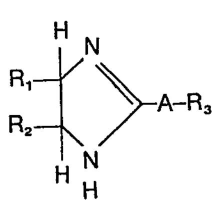
(VIII)

ou un de ses isomères en composés de formule I et si on le désire en ce qu'on transforme un composé libre obtenu selon le procédé en un sel ou un sel obtenu selon le procédé en le composé libre ou en un autre sel ou en ce qu'on transforme un composé libre obtenu selon le procédé en un autre composé libre et/ou, si on le désire, en ce qu'on sépare un mélange de composés isomères de formule I obtenu selon l'invention en les isomères isolés.

28. Procédé selon la revendication 27, caractérisé en ce qu'on part d'une étape quelconque du procédé et en ce qu'on effectue les étapes manquantes ou bien en ce qu'on utilise un produit de départ sous la forme d'un sel et/ou de tautomères et/ou en ce qu'on le forme dans les conditions de la réaction.

29. Procédé selon l'une des revendications 27–28, caractérisé en ce qu'on forme pour la plus grande partie in situ les produits de départ de formule II, leurs tautomères et/ou sels selon des procédés connus et en ce qu'on les fait réagir plus avant dans les conditions de la réaction sans les isoler pour donner le composé de formule I.

30. Procédé selon la revendication 29, caractérisé en ce qu'on fait réagir une dicétone de formule

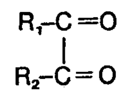
(IIIa)

avec un aldéhyde de formule R3–A–C(=O)–H (IIIb) ou un de ses sels, avec un excès d'ammoniac en chauffant, ou en ce qu'on fait réagir une α-aminocétone acylée de formule

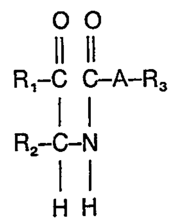
(IIIc)

ou un de ses sels avec de l'ammoniac, ou en ce qu'on fait réagir un composé de formule

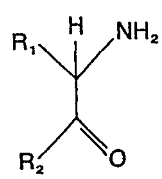
(IIIh)

ou un de ses sels avec un dérivé d'acide de formule R3–A–COOH (IIIi), ou en ce qu'on fait réagir un composé de formule

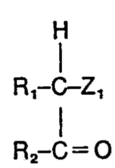
(IIId),

où Z1 représente un hydroxy estérifié réactif, ou un de ses sels, avec un composé de formule

$R_3$–A–$Z_2$ (IIIe),

où Z représente un radical amidino ou le carboxylate d'ammonium, ou un de ses sels, éventuellement avec de l'ammoniac, ou en ce qu'on fait réagir un oxazole de formule

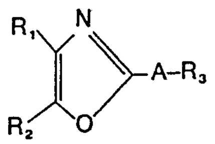

(IIIf)

avec de l'ammoniac, ou en ce qu'on fait réagir un composé de formule

$$R_1-C(NH_2)=C(NH_2)-R_2 \quad (IIIk)$$

ou un de ses sels avec un composé éventuellement fonctionnellement modifié de formule

$$R_3-A-COOH \quad (IIIp)$$

ou selon les cas, dans des conditions oxydantes, avec un composé de formule

$$R_3-A-C(=O)-H \quad (IIIb)$$

31. Les composés de formule I que l'on peut obtenir selon les procédés des revendications 27–30.

32. Composés de formule

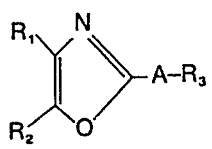

(IIIf),

où l'un des radicaux R1 et R2 représente phényle, pyrrolyle, pyridyle, 1-oxydopyridyle ou pyrimidyle, qui peuvent être chacun non substitué ou substitué par halogène, alcoyle inférieur, hydroxy, alcoxy inférieur et/ou alcanoyloxy inférieur, et l'autre représente pyrrolyle, pyridyle, 1-oxydopyridyle ou pyrimidyle, qui peuvent être chacun non substitués ou substitués par halogène, alcoyle inférieur, hydroxy, alcoxy inférieur et/ou alcanoyloxy inférieur et A représente alcoylène inférieur, alcoylidène inférieur, alcénylène inférieur, alcénylidène inférieur, cycloalcoylène, cycloalcoylidène ou cycloalcoyl-alcoylidène inférieur, et R3 représente carboxy, carboxy estérifié par un alcanol inférieur, un cycloalcanol à 3 à 8 chaînons, un phénol, une hydroxypyridine, un phénol substitué ou selon les cas une hydroxypyridine, un carbamoyle ou un carbamoyle monosubstitué par hydroxy, amino, phényle ou phényle substitué, mono- ou disubstitué par un alcoyle inférieur ou disubstitué par un alcoylène à 4 à 7 chaînons ou selon les cas un 3-aza-, 3-alcoyle inférieur-aza-, 3-oxa- ou 3-thiaalcoylène, où un alcanol inférieur ou selon les cas un cycloalcanol peut être non substitué ou substitué par hydroxy, mercapto, phényle éventuellement substitué, alcoxy inférieur, phénylalcoxy inférieur éventuellement substitué dans la partie phényle, alcoyle inférieur-thio, phénylalcoyle inférieur-thio éventuellement substitué dans la partie phényle, hydroxyalcoxy inférieur, alcoxy inférieur-alcoxy inférieur, phénylalcoxy inférieur-alcoxy inférieur éventuellement substitué dans la partie phényle, carboxyalcoxy inférieur, alcoxy inférieur-carbonyl-alcoxy inférieur, alcoxy inférieur-carbonyl-alcoxy inférieur présentant éventuellement un phényle substitué ou alcanoyloxy inférieur et phényle substitué, phénol ou selon les cas hydroxypyridine à chaque fois substitué par alcoyle inférieur, alcoxy inférieur, halogène et/ou trifluorométhyle, leurs isomères ainsi que leurs sels.

33. Composés de formule IIIf selon la revendication 32, où l'un des radicaux R1 et R2 représente un pyridyle ou un 1-oxydo-pyridyle, qui peuvent être non substitués et/ou chacun substitué par halogène, hydroxy, alcoyle inférieur, alcoxy inférieur et/ou alcanoyloxy inférieur, et l'autre représente un phényle, un pyridyle ou un 1-oxydopyridyle, qui peuvent être non substitués et/ou chacun substitué par halogène, hydroxy, alcoyle inférieur, alcoxy inférieur et/ou alcanoyloxy inférieur, A représente un alcoylène inférieur ayant jusqu'à 4 atomes de carbone compris, un alcoylidène inférieur ayant jusqu'à 7 atomes de carbone compris, un alcénylène inférieur ayant jusqu'à 4 atomes de carbone compris, un alcénylidène inférieur ayant jusqu'à 7 atomes de carbone compris, un cycloalcoylène à 3 à 8 chaînons, un cycloalcoylidène à 3 à 8 chaînons ou un cycloalcoyl-alcoylidène inférieur ayant jusqu'à 7 atomes de carbone compris dans la partie alcoylidène et avec une partie cycloalcoyle à 3 à 8 chaînons et R3 représente un carboxy, un carboxy estérifié par un alcanol inférieur, un cycloalcanol à 3 à 8 chaînons, un phénol ou un phénol substitué, un carbamoyle, un pyrrolidino-, pipéridino-, morpholino-, pipérazino-, 4-alcoyle inférieur-pipérazino-, thiomorpholino-, anilino- ou anilino-carbonyle substitué par alcoyle inférieur, alcoxy inférieur et/ou halogène, où l'alcanol inférieur ou selon les cas cycloalcanol est non substitué ou peut être substitué par hydroxy, mercapto, phényle, phényle substitué, alcoxy inférieur, phénylalcoxy inférieur, phénylalcoxy inférieur substitué dans la fraction phényle, alcoyle inférieur-thio, phénylalcoyle inférieur-thio, phénylalcoyle inférieur-thio substitué dans la fraction phényle, hydroxyalcoxy inférieur, alcoxy inférieur-alcoxy inférieur, phénylalcoxy inférieur-alcoxy inférieur, phénylalcoxy inférieur-alcoxy inférieur substitué dans la fraction phényle, carboxyalcoxy inférieur, alcoxy inférieur-carbonyl-alcoxy inférieur, alcoxy inférieur-carbonyl-alcoxy inférieur présentant éventuellement un phényle substitué, ou alcanoyloxy inférieur et phénol substitué ou selon les cas phényle à chaque fois substitué par alcoyle inférieur, alcoxy inférieur, halogène et/ou trifluorométhyle, leurs isomères ainsi que leurs sels.

34. Composés de formule IIIf selon la revendication 32, où l'un des radicaux R1 et R2 représente un phényle ou un phényle substitué par un halogène dont le numéro atomique va jusqu'à 35 compris, un hydroxy, un alcoyle inférieur ayant jusqu'à 4 atomes de carbone compris et/ou un alcoxy inférieur ayant jusqu'à 4 atomes de carbone compris, et l'autre représente un pyridyle ou un 1-oxydo-pyridyle, qui à chaque fois peuvent être non substitués ou substitués par un halogène dont le

numéro atomique va jusqu'à 35 compris, un hydroxy et/ou un alcoxy inférieur ayant jusqu'à 4 atomes de carbone compris, A représente un alcoylène inférieur ayant jusqu'à 4 atomes de carbone compris, un alcoylidène inférieur ayant jusqu'à 7 atomes de carbone compris, ou un cycloalcoylidène inférieur à 3 à 7 chaînons et R3 représente un carboxy, un alcoxy inférieur-carbonyle ayant jusqu'à 5 atomes de carbone compris, un carbamoyle, un N-monoalcoyle inférieur-carbamoyle ayant jusqu'à 4 atomes de carbone compris dans la partie alcoyle inférieur ou un N,N-dialcoyle inférieur-carbamoyle ayant à chaque fois jusqu'à 4 atomes de carbone compris dans la partie alcoyle inférieur, où l'alcoxy inférieur-carbonyle peut être substitué par un alcanoyloxy inférieur ayant jusqu'à 5 atomes de carbone compris, leurs isomères ainsi que leurs sels.

35. Composés de formule IIIf selon la revendication 32, où l'un des radicaux R1 et R2 représente un phényle ou un phényle substitué par un halogène de numéro atomique allant jusqu'à 35 compris, un hydroxy ou un alcoxy inférieur ayant jusqu'à 4 atomes de carbone compris et l'autre représente un pyridyle ou un 1-oxydopyridyle, A représente un alcoylidène inférieur présentant un atome de carbone quaternaire et ayant jusqu'à 4 atomes de carbone compris, où l'atome de carbone quaternaire C est lié directement au noyau imidazole, et R3 représente un alcoxy inférieur-carbonyle ayant jusqu'à 5 atomes de carbone compris, leurs isomères ainsi que leurs sels.

36. Ester éthylique de l'acide
2-[4-phényl-5-(3-pyridyl)-oxazol-2-yl]-
acétique
ou un de ses sels.

37. Ester éthylique de l'acide
2-[4-phényl-5-(1-oxydo-3-pyridyl)-oxazol-
2-yl]-2-méthylpropionique
ou un de ses sels.

38. Composés de formule IIIf selon l'une des revendications 32 à 37 aux fins d'application dans un procédé de traitement thérapeutique du corps animal ou humain.

39. Composés de formule IIIf selon la revendication 38 aux fins d'application comme stabilisateurs des inflammations locales de la peau.

40. Application de composés de formule IIIf selon l'une des revendications 32–37 à la préparation de médicaments.

41. Procédé de préparation de composés de formule IIIf selon la revendication 33, caractérisé en ce qu'on fait réagir des composés de formule

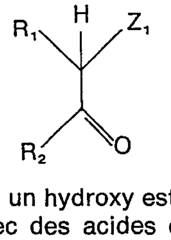

(IIId),

où Z1 représente un hydroxy estérifié éventuellement réactif, avec des acides carboxyliques de formule R3–A–COOH (IIIp), leurs sels ou leurs dérivés fonctionnels et avec de l'ammoniac ou en ce qu'on fait réagir des composés de formule

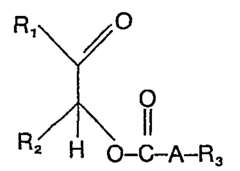

(IIIq),

que l'on peut obtenir par réaction de composés de formule

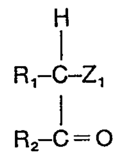

(IIId)

avec des dérivés éventuellement fonctionnels de composés de formule

$R_3–A–COOH$ (IIIp)

avec de l'ammoniac, si on le désire en ce qu'on N-oxyde dans un composé de formule IIIf ainsi obtenu les radicaux hétéroaryle non N-oxydés R1 et R2 à l'aide d'un oxydant, et si on le désire en ce qu'on transforme le composé libre que l'on peut obtenir selon le procédé en un sel ou un sel que l'on peut obtenir selon le procédé en le composé libre ou en un autre sel ou un composé libre que l'on peut obtenir selon le procédé en un autre composé libre et/ou, si on le désire, en ce qu'on sépare un mélange d'isomères de composés isomères de formule I que l'on peut obtenir selon l'invention en les isomères isolés.

42. Procédé selon la revendication 41, caractérisé en ce qu'on part d'une étape quelconque du procédé et en ce qu'on effectue les étapes manquantes ou en ce qu'on utilise un produit de départ sous la forme d'un sel et/ou de tautomères et/ou en ce qu'on le forme dans les conditions de la réaction.

43. Les composés de formule IIIf que l'on peut obtenir selon le procédé des revendications 41 et 42.

**Revendications pour l'Etat contractant: AT**

1. Procédé de préparation de composés de formule I

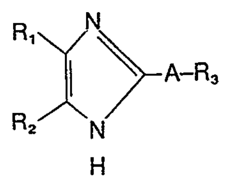

(I) ,

où d'une part R1 et R2 représentent indépendamment l'un de l'autre un phényle et/ou un phényle substitué par halogène, alcoyle inférieur, hydroxy, alcoxy inférieur et/ou alcanoyle inférieur, ou bien où d'autre part l'un des radicaux R1 et R2 représentent pyrrolyle, furyle, thiényle, pyridyle, 1-oxydopyridyle ou pyrimidyle, qui peuvent être chacun non substitués ou substitués par halogène, alcoyle inférieur, hydroxy, alcoxy inférieur et/ou alcanoyloxy inférieur, et l'autre représente phényle, pyrrolyle, furyle, thiényle, pyridyle, 1-oxydopyridyle ou pyrimidyle, qui peuvent chacun être non substitués ou substitués par halogène, alcoyle inférieur, hydroxy, alcoxy inférieur et/ou alcanoyloxy inférieur, et où à chaque fois A représente alcoylène inférieur, alcoylidène inférieur, alcénylène inférieur, alcénylidène inférieur, cycloalcoylène, cycloalcoylidène ou cycloalcoylalcoylidène inférieur, et R3 représente un carboxy, un carboxy estérifié par un alcanol inférieur, un cycloalcanol à 3 à 8 chaînons, un phénol, une hydroxypyridine, un phénol substitué ou selon les cas une hydroxypyridine, un carbamoyle ou un carbamoyle monosubstitué par hydroxy, amino, phényle ou phényle substitué, mono- ou disubstitué par alcoyle inférieur ou disubstitué par alcoylène à 4 à 7 chaînons ou selon les cas 3-aza-, 3-alcoyle inférieur-aza, 3-oxa- ou 3-thiaalcoylène, où un alcanol inférieur ou selon les cas un cycloalcanol peut être non substitué ou substitué par hydroxy, mercapto, phényle éventuellement substitué, alcoxy inférieur, phénylalcoxy inférieur éventuellement substitué dans la fraction phényle, alcoyle inférieur-thio, phénylalcoyle inférieur-thio éventuellement substitué dans la fraction phényle, hydroxyalcoxy inférieur, alcoxy inférieur-alcoxy inférieur, phénylalcoxy inférieur-alcoxy inférieur éventuellement substitué dans la fraction phényle, carboxy-alcoxy inférieur, alcoxy inférieur-carbonyl-alcoxy inférieur, alcoxy inférieur-carbonyl-alcoxy inférieur présentant un phényle éventuellement substitué, ou alcanoyloxy inférieur et phényle substitué, phénol ou selon les cas hydroxypyridine à chaque fois substituée par alcoyle inférieur, alcoxy inférieur, halogène et/ou trifluorométhyle, leurs isomères ainsi que leurs sels, en particulier leurs sels pharmaceutiquement acceptables, avec la précision que lorsque A représente un méthylène ou selon les cas un éthylidène et R3 représente un éthoxycarbonyle, les deux radicaux R1 et R2 ne représentent pas simultanément un phényle, et la précision supplémentaire que, lorsque A représente un éthylidène et R3 un carboxy ou selon les cas un carbamoyle, les deux radicaux R1 et R2 ne représentent pas simultanément un phényle, un p-méthoxyphényle ou un p-chlorophényle, où les radicaux décrits comme «inférieurs» doivent être compris comme ceux qui contiennent jusqu'à 7 atomes de carbone compris, caractérisé en ce qu'à partir d'un composé de formule

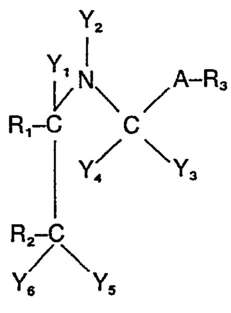

(II),

où l'un des radicaux Y1 et Y6 représente un hydroxy ou un amino et l'autre ainsi que Y2 représente un hydrogène et Y3 représente avec Y4 et Y5 un groupe de formule =N– ou bien où Y1 avec Y6 représente une liaison, Y2 est un hydrogène, Y3 est un hydroxy ou un amino et Y4 représente avec Y5 un groupe de formule –NH– ou bien où Y1 représente avec Y6 une liaison, Y2 représente avec Y3 une liaison supplémentaire et l'un des radicaux Y4 et Y5 représente un amino et l'autre un amino, un hydroxy ou un hydroxy estérifié réactif ou bien où Y1 est un hydroxy, Y2 ainsi qu'Y3 représente un hydrogène, Y4 représente un hydroxy ou un amino et Y5 représente avec Y6 un groupe de formule =NH ou, dans la mesure où Y4 est un amino, représente un oxo, ou un de ses tautomères et/ou sels, avec introduction d'une liaison supplémentaire on clive H–Z, où Z représente hydroxy ou amino Y1 ou selon les cas Y6, Y3 ou Y4 ou selon les cas Y5 ou hydroxy estérifié Y4 ou selon les cas Y5, ou en ce qu'on réduit un composé de formule

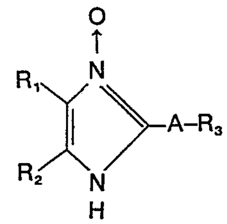

(IV)

ou un de ses sels en un composé de formule I, ou bien en ce que dans un composé de formule

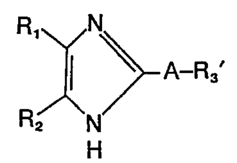

(V),

où R3′ représente un radical transformable en R3, on transforme le radical R3′ en un radical R3 ou en ce que dans un composé de formule

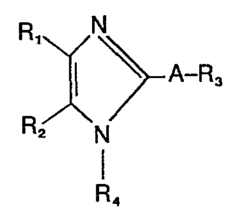

(VI),

où R4 représente un groupe transformable en hydrogène, ou un de ses sels, on transforme R4 en hydrogène, ou en ce qu'on fait réagir des composés de formule

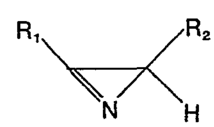

(VIIa)

ou leurs tautomères avec des composés de formule R3–A–CN (VIIb) ou en ce qu'on déshydrogène un composé de formule

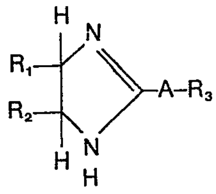

(VIII)

ou un de ses isomères en composés de formule I et si on le désire en ce qu'on transforme un composé libre obtenu selon le procédé en un sel ou un sel obtenu selon le procédé en le composé libre ou en un autre sel ou en ce qu'on transforme un composé libre obtenu selon le procédé en un autre composé libre et/ou, si on le désire, en ce qu'on sépare un mélange de composés isomères de formule I obtenu selon l'invention en les isomères isolés.

2. Procédé selon la revendication 1, caractérisé en ce qu'on fait réagir une dicétone de formule

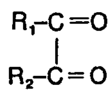

(IIIa)

avec un aldéhyde de formule R3–A–C(=O)–H (IIIb) ou un de ses sels, avec un excès d'ammoniac en chauffant, ou en ce qu'on fait réagir une α-aminocétone acylée de formule

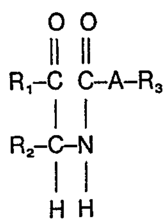

(IIIc)

ou un de ses sels avec de l'ammoniac, ou en ce qu'on fait réagir un composé de formule

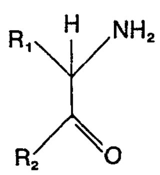

(IIIh)

ou un de ses sels avec un dérivé d'acide de formule R3–A–COOH (IIIi), ou en ce qu'on fait réagir un composé de formule

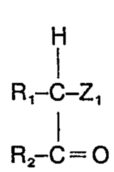

(IIId) ,

où Z1 représente un hydroxy estérifié réactif, ou un de ses sels, avec un composé de formule

$$R_3\text{–A–}Z_2 \qquad \text{(IIIe)},$$

où Z représente un radical amidino ou le carboxylate d'ammonium, ou un de ses sels, éventuellement avec de l'ammoniac, ou en ce qu'on fait réagir un oxazole de formule

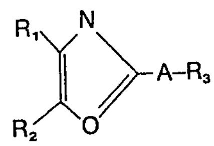

(IIIf)

avec de l'ammoniac, ou en ce qu'on fait réagir un composé de formule

$$R_1\text{–C}(NH_2) = \text{C}(NH_2)\text{–}R_2 \qquad \text{(IIIk)}$$

ou un de ses sels avec un composé éventuellement fonctionnellement modifié de formule

$$R_3\text{–A–COOH} \qquad \text{(IIIp)}$$

ou selon les cas, dans des conditions oxydantes, avec un composé de formule

$$R_3\text{–A–C}(=O)\text{–H} \qquad \text{(IIIb)}$$

3. Procédé selon l'une des revendications 1–2, caractérisé en ce qu'on forme pour la plus grande partie in situ les produits de départ de formule II, leurs tautomères et/ou sels selon des procédés connus et en ce qu'on les fait réagir plus avant dans les conditions de la réaction sans les isoler pour donner le composé de formule I.

4. Procédé selon l'une des revendications 1–3, caractérisé en ce qu'on part d'une étape quelconque du procédé et en ce qu'on effectue les étapes manquantes ou bien en ce qu'on utilise un produit de départ sous la forme d'un sel et/ou de tautomères et/ou en ce qu'on le forme dans les conditions de la réaction.

5. Procédé selon la revendication 1 de préparation de composés de formule I

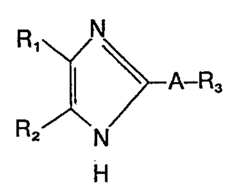

(I) ,

où d'une part R1 et R2 représentent indépendamment l'un de l'autre un phényle et/ou un phényle substitué par halogène, alcoyle inférieur, hydroxy, alcoxy inférieur et/ou alcanoyle inférieur, ou bien où d'autre part l'un des radicaux R1 et R2 représente pyrrolyle, furyle, thiényle, pyridyle, 1-oxydopyridyle ou pyrimidyle, qui peuvent être chacun non substitués ou substitués par halogène, alcoyle inférieur, hydroxy, alcoxy inférieur et/ou alcanoyloxy inférieur, et l'autre représente phényle, pyrrolyle, furyle, thiényle, pyridyle, 1-oxydopyridyle ou pyrimidyle, qui peuvent chacun être non substitués ou substitués par halogène, alcoyle inférieur, hydroxy, alcoxy inférieur et/ou alcanoyloxy inférieur, et où à chaque fois A représente alcoylène inférieur, alcoylidène inférieur, alcénylène inférieur, alcénylidène inférieur, cycloalcoylène, cycloalcoylidène ou cycloalcoyl-alcoylidène inférieur, et R3 représente un carboxy, un carboxy estérifié par un alcanol inférieur, un cycloalcanol à 3 à 8 chaînons, un phénol, un hydroxypyridine, un phénol substitué ou selon les cas une hydroxypyridine, un carbamoyle ou un carbamoyle monosubstitué par hydroxy, amino, phényle ou phényle substitué, mono- ou disubstitué par alcoyle inférieur ou disubstitué par alcoylène à 4 à 7 chaînons ou selon les cas 3-aza-, 3-alcoyle inférieur-aza, 3-oxa- ou 3-thiaalcoylène, où un alcanol inférieur ou selon les cas un cycloalcanol peut être non substitué ou substitué par hydroxy, mercapto, phényle éventuellement substitué, alcoxy inférieur, phénylalcoxy inférieur éventuellement substitué dans la fraction phényle, alcoyle inférieur-thio, phénylalcoyle inférieur-thio éventuellement substitué dans la fraction phényle, hydroxyalcoxy inférieur, alcoxy inférieur-alcoxy inférieur, phénylalcoxy inférieur-alcoxy inférieur éventuellement substitué dans la fraction phényle, carboxy-alcoxy inférieur, alcoxy inférieur-carbonyl-alcoxy inférieur, alcoxy inférieur-carbonyl-alcoxy inférieur présentant un phényle éventuellement substitué, ou alcanoyloxy inférieur et phényle substitué, phénol ou selon les cas hydroxypyridine à chaque fois substituée par alcoyle inférieur, alcoxy inférieur, halogène et/ou trifluorométhyle, leurs isomères ainsi que leurs sels, en particulier leurs sels pharmaceutiquement acceptables, avec la précision que lorsque A représente un méthylène ou selon les cas un éthylidène et R3 représente un éthoxycarbonyle, les deux radicaux R1 et R2 ne représentent pas simultanément un phényle, et la précision supplémentaire que, lorsque A représente un éthylidène et R3 un carboxy ou selon les cas un carbamoyle, les deux radicaux R1 et R2 ne représentent pas simultanément un phényle, un p-méthoxyphényle ou un p-chlorophényle, caractérisé en ce que

a) à partir d'un composé de formule

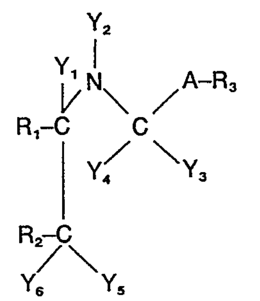

(II),

où l'un des radicaux Y1 et Y6 représente un hydroxy ou un amino et l'autre ainsi que Y2 représente un hydrogène et Y3 représente avec Y4 et Y5 un groupe de formule =N– ou bien où Y1 avec Y6 représente une liaison, Y2 est un hydrogène, Y3 est un hydroxy ou un amino et Y4 représente avec Y5 un groupe de formule –NH– ou bien où Y1 représente avec Y6 une liaison, Y2 représente avec Y3 une liaison supplémentaire et l'un des radicaux Y4 et Y5 représente un amino et l'autre un amino, un hydroxy ou un hydroxy estérifié réactif ou bien où Y1 est un hydroxy, Y2 ainsi qu'Y3 représente un hydrogène, Y4 représente un hydroxy ou un amino et Y5 représente avec Y6 un groupe de formule =NH ou, dans la mesure où Y4 est un amino, représente un oxo, ou un de ses tautomères et/ou sels, avec introduction d'une liaison supplémentaire on clive H–Z, où Z représente hydroxy ou amino Y1 ou selon les cas Y6, Y3 ou Y4 ou selon les cas Y5 ou un halogène ou selon les cas un sulfonyloxy Y4 ou selon les cas Y5,

b) on réduit un composé de formule

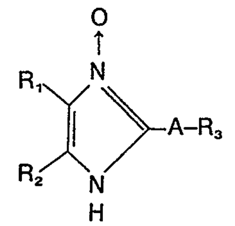

(IV)

ou un de ses sels en un composé de formule I,

c) dans un composé de formule

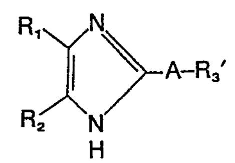

(V),

où R3′ représente un radical transformable en R3, on transforme le radical R3′ en un radical R3 ou

d) dans un composé de formule

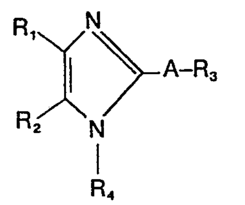

(VI),

où A′ représente un alcoylidène inférieur, un alcénylidène inférieur, un cycloalcoylidène ou un cycloalcoyl-alcoylidène inférieur et R4 représente un groupe transformable en hydrogène, ou un de ses sels, on transforme R4 en hydrogène et si on le désire on transforme un composé libre que l'on peut obtenir selon le procédé en un sel ou un sel que l'on peut obtenir selon le procédé en le composé libre ou en un autre sel.

6. Procédé selon la revendication 5, caractérisé en ce qu'on part d'une étape quelconque du procédé et en ce qu'on effectue les étapes manquantes et/ou en ce qu'on utilise des tautomères et/ou en ce qu'on les forme dans les conditions de la réaction.

7. Procédé selon l'une des revendications 1–4 de préparation de composés de formule I, où R1 et R2 représentent indépendamment l'un de l'autre phényle et/ou phényle substitué par halogène, hydroxy, alcoyle inférieur, alcoxy inférieur et/ou alcanoyloxy inférieur, A représente un alcoylène inférieur ayant jusqu'à 4 atomes de carbone compris, un alcoylidène inférieur ayant jusqu'à 7 atomes de carbone compris, un cycloalcoylène à 3 à 8 chaînons, un cycloalcoylidène à 3 à 8 chaînons ou un cycloalcoyl-alcoylidène inférieur ayant jusqu'à 7 atomes de carbone compris dans la fraction alcoylidène, et avec une fraction cycloalcoyle à 3 à 8 chaînons, et R3 représente un carboxy, un carboxy estérifié avec un alcanol inférieur, un cycloalcanol à 3 à 8 chaînons, un phénol ou un phénol substitué, un carbamoyle, un N-mono-, N,N-dialcoyle inférieur-carbamoyle, un pyrrolidino-, pipéridino-, morpholino-, pipérazino-, 4-alcoyle inférieur-pipérazino-, thiomorpholino-, anilino- ou anilino-carbonyle substitué par alcoyle inférieur, alcoxy inférieur et/ou halogène, où l'alcanol inférieur ou selon les cas cycloalcanol est non substitué ou peut être substitué par hydroxy, mercapto, phényle, phényle substitué, alcoxy inférieur, phénylalcoxy inférieur, phénylalcoxy inférieur substitué dans la fraction phényle, alcoyle inférieur-thio, phénylalcoyle inférieur-thio, phénylalcoyle inférieur-thio substitué dans la fraction phényle, hydroxyalcoxy inférieur, alcoxy inférieur-alcoxy inférieur, phénylalcoxy inférieur-alcoxy inférieur, phénylalcoxy inférieur-alcoxy inférieur substitué dans la fraction phényle, carboxyalcoxy inférieur, alcoxy inférieur-carbonyl-alcoxy inférieur, alcoxy inférieur-carbonyl-alcoxy inférieur présentant éventuellement un phényle substitué, ou alcanoyloxy inférieur et phénol substitué ou selon les cas phényle à chaque fois substitué par alcoyle inférieur, alcoxy inférieur, halogène et/ou trifluorométhyle, leurs isomères ainsi que leurs sels, en particulier les sels pharmaceutiquement acceptables, avec la précision que, lorsque A représente méthylène ou selon les cas éthylidène et R3 représente éthoxycarbonyle, les deux radicaux R1 et R2 ne représentent pas simultanément un phényle, et la précision supplémentaire que, lorsque A représente un éthylidène et R3 un carboxy ou selon les cas un carbamoyle, les deux radicaux R1 et R2 ne représentent pas simultanément un phényle, un p-méthoxyphényle ou un p-chlorophényle, caractérisé en ce qu'on part de composés de formules II ou selon les cas IIIa et IIIb, IIIc, IIIh et IIIi, IIId et IIIe, IIIk et IIIp ou selon les cas IIIb ainsi que IIIf ou V ou VI ou VIIa et VIIb ou VIII, où les variables Y1–Y6, R3′ et R4 ont les significations données dans la revendication 1 et les variables Z1 et Z2 celles données dans la revendication 2.

8. Procédé selon l'une des revendications 1–4 de préparation de composés de formule I, où l'un des radicaux R1 et R2 représente un pyridyle ou un 1-oxydo-pyridyle, qui peuvent être non substitués et/ou à chaque fois substitués par halogène, hydroxy, alcoyle inférieur, alcoxy inférieur et/ou alcanoyloxy inférieur, et l'autre représente un phényle, un pyridyle ou un 1-oxydopyridyle, qui peuvent être non substitués et/ou substitués à chaque fois halogène, hydroxy, alcoyle inférieur, alcoxy inférieur et/ou alcanoyloxy inférieur, A représente un alcoylène inférieur ayant jusqu'à 4 atomes de carbone compris, un alcoylidène inférieur ayant jusqu'à 7 atomes de carbone compris, un alcénylène inférieur ayant jusqu'à 4 atomes de carbone compris, un alcénylidène inférieur ayant jusqu'à 7 atomes de carbone compris, un cycloalcoylène à 3 à 8 chaînons, un cycloalcoylidène à 3 à 8 chaînons ou un cycloalcoyl-alcoylidène inférieur ayant jusqu'à 7 atomes de carbone compris dans la fraction alcoylidène et avec une fraction cycloalcoyle à 3 à 8 chaînons et R3 représente un carboxy, un carboxy estérifié avec un alcanol inférieur, un cycloalcanol à 3 à 8 chaînons, un phénol ou un phénol substitué; un carbamoyle, un N-mono-, N,N-dialcoyle inférieur-carbamoyle, un pyrrolidino-, pipéridino-, morpholino-, pipérazino-, 4-alcoyle inférieur-pipérazino-, thiomorpholino-, anilino- ou anilino-carbonyle substitué par alcoyle inférieur, alcoxy inférieur et/ou halogène, où l'alcanol inférieur ou selon les cas cycloalcanol est non substitué ou peut être substitué par hydroxy, mercapto, phényle, phényle substitué, alcoxy inférieur, phénylalcoxy inférieur, phénylalcoxy inférieur substitué dans la fraction phényle, alcoyle inférieur-thio, phénylalcoyle inférieur-thio, phénylalcoyle inférieur-thio substitué dans la fraction phényle, hydroxyalcoxy inférieur, alcoxy inférieur-alcoxy inférieur, phénylalcoxy inférieur-alcoxy inférieur, phénylalcoxy inférieur-alcoxy inférieur substitué dans la fraction phényle, carboxyalcoxy inférieur, alcoxy inférieur-carbonyl-alcoxy inférieur, alcoxy inférieur-carbonyl-alcoxy inférieur présentant éventuellement un phényle substitué, ou alcanoyloxy inférieur et phénol substitué ou selon les cas phényle à chaque fois substitué par alcoyle inférieur, alcoxy inférieur, halogène et/ou trifluorométhyle, leurs isomères ainsi que leurs sels, en particulier les sels pharmaceutiquement acceptables, caractérisé en ce qu'on part de composés de formules II ou selon les cas IIIa et IIIb, IIIc, IIIh et IIIi, IIId et IIIe, IIIk et IIIp ou selon les cas IIIb ainsi que IIIf ou V ou VI ou VIIa et VIIb ou VIII, où les variables Y1–Y6, R3′ et R4 sont les significations données dans la revendication 1 et les variables Z1 et Z2 celles données dans la revendication 2.

9. Procédé selon l'une des revendications 5 et 6, de préparation de composés de formule I où R1 et R2 représentent indépendamment l'un de l'autre un phényle éventuellement substitué par un halogène dont le numéro atomique va jusqu'à 35 compris, un alcoyle inférieur ayant jusqu'à 4 atomes de carbone compris, un alcoxy inférieur ayant jusqu'à 4 atomes de carbone compris et/ou un alcanoyloxy inférieur ayant jusqu'à 5 atomes de carbone compris, un pyridyle et/ou un 1-oxydopyridyle, A représente un alcoylène inférieur ayant jusqu'à 4 atomes de carbone compris, R3 représente un carboxy éventuellement estérifié par un alcanol inférieur ayant jusqu'à 4 atomes de carbone compris, où l'alcanol inférieur peut être éventuellement substitué par un hydroxy, un mercapto, un phényle éventuellement substitué, un alcoxy inférieur ayant jusqu'à 4 atomes de carbone compris, un phénylalcoxy inférieur éventuellement substitué dans le noyau phényle et ayant jusqu'à 4 atomes de carbone compris dans la fraction alcoyle, un alcoyle inférieur-thio ayant jusqu'à 4 atomes de carbone compris, un hydroxyalcoxy inférieur ayant jusqu'à 4 atomes de carbone compris dans la fraction alcoyle inférieur, un alcoxy inférieur-alcoxy inférieur ayant à chaque fois jusqu'à 4 atomes de carbone compris dans la fraction alcoyle inférieur ou un alcanoyloxy inférieur ayant jusqu'à 5 atomes de carbone compris et un phényle éventuellement substitué par un alcoyle inférieur ayant jusqu'à 4 atomes de carbone compris, un alcoxy inférieur ayant jusqu'à 4 atomes de carbone compris, un halogène dont le numéro atomique va jusqu'à 35 compris et/ou un trifluorométhyle, leurs isomères ainsi que leurs sels, avec la précision que lorsque A représente un méthylène ou selon les cas un éthylidène et R3 représente un éthoxycarbonyle, les deux radicaux R1 et R2 ne représentent pas ensemble un phényle, et la précision supplémentaire que lorsque A représente un éthylidène et R3 un carboxy, au moins l'un des radicaux R1 et R2 est différent de phényle, p-méthoxyphényle et p-chlorophényle, caractérisé en ce qu'on part de composés de formules II, IV, V ou VI où les variables Y1–Y6, R3' et R4 ont les significations données dans la revendication 5.

10. Procédé selon l'une des revendications 1–4 de préparation de composés de formule I, où R1 et R2 représentent

indépendamment l'un de l'autre un phényle et/ou un phényle substitué par un halogène dont le numéro atomique va jusqu'à 35 compris, un hydroxy, un alcoyle inférieur ayant jusqu'à 4 atomes de carbone compris et/ou un alcoxy inférieur ayant jusqu'à 4 atomes de carbone compris, A représente un alcoylène inférieur ayant jusqu'à 4 atomes de carbone compris, un alcoylidène inférieur ayant jusqu'à 7 atomes de carbone compris, un alcénylidène inférieur ayant jusqu'à 7 atomes de carbone compris ou un cycloalcoylidène inférieur à 3 à 8 chaînons et R3 représente un carboxy, un alcoxy inférieur-carbonyle ayant jusqu'à 5 atomes et carbone compris, un carbamoyle, un N-monoalcoyle inférieur-carbamoyle ayant jusqu'à 4 atomes de carbone compris dans la partie alcoyle inférieur ou N,N-dialcoyle inférieur-carbamoyle ayant à chaque fois jusqu'à 4 atomes de carbone compris dans la partie alcoyle inférieur, où l'alcoxy inférieur-carbonyle peut être substitué par un alcanoyloxy inférieur ayant jusqu'à 5 atomes de carbone compris, leurs isomères ainsi que leurs sels, en particulier leurs sels pharmaceutiquement acceptables, avec la précision que lorsque A représente un méthylène ou selon les cas un éthylidène et R3 représente un éthoxycarbonyle, les deux radicaux R1 et R2 ne représentent pas simultanément un phényle, et la précision ultérieure que lorsque A représente un éthylidène et R3 un carboxy,

au moins l'un des radicaux R1 et R2 est différent de phényle, p-méthoxyphényle et p-chlorophényle, caractérisé en ce qu'on part de composés de formules II ou selon les cas IIIa et IIIb, IIIc, IIIh et IIIi, IIId et IIIe, IIIk et IIIp ou selon les cas IIIb ainsi que IIIf ou V ou VI ou VIIa et VIIb ou VIII, où les variables Y1–Y6, R3' et R4 ont les significations données dans la revendication 1 et les variables Z1 et Z2 celles données dans la revendication 2.

11. Procédé selon l'une des revendications 1–4, de préparation de composés de formule I, où l'un des radicaux R1 et R2 représente un phényle ou un phényle substitué par un halogène dont le numéro atomique va jusqu'à 35 compris, un hydroxy, un alcoyle inférieur ayant jusqu'à 4 atomes de carbone compris et/ou un alcoxy inférieur ayant jusqu'à 4 atomes de carbone compris, et l'autre représente un pyridyle ou un 1-oxydo-pyridyle, qui à chaque fois peuvent être non substitués ou substitués par un halogène dont le numéro atomique va jusqu'à 35 compris, un hydroxy et/ou un alcoxy inférieur ayant jusqu'à 4 atomes de carbone compris, A représente un alcoylène inférieur ayant jusqu'à 4 atomes de carbone compris, un alcoylidène inférieur ayant jusqu'à 7 atomes de carbone compris, ou un cycloalcoylidène inférieur à 3 à 7 chaînons et R3 représente un carboxy, un alcoxy inférieur-carbonyle ayant jusqu'à 5 atomes de carbone compris, un carbamoyle, un N-monoalcoyle inférieur-carbamoyle ayant jusqu'à 4 atomes de carbone compris dans la partie alcoyle inférieur ou un N,N-dialcoyle inférieur-carbamoyle ayant à chaque fois jusqu'à 4 atomes de carbone compris dans la partie alcoyle inférieur, où l'alcoxy inférieur-carbonyle peut être substitué par un alcanoyloxy inférieur ayant jusqu'à 5 atomes de carbone compris, leurs isomères ainsi que leurs sels, en particulier les sels pharmaceutiquement acceptables, caractérisé en ce qu'on part de composés de formules II ou selon les cas IIIa et IIIb, IIIc, IIIh et IIIi, IIId et IIIe, IIIk et IIIp ou selon les cas IIIb ainsi que IIIf ou V ou VI ou VIIa et VIIb ou VIII, où les variables Y1–Y6, R3' et R4 ont les significations données dans la revendication 1 et les variables Z1 et Z2 ont les significations données dans la revendication 2.

12. Procédé selon l'une des revendications 1–4 de préparation de composés de formule I, où R1 et R2 représentent indépendamment l'un de l'autre un phényle et/ou un phényle substitué par un alcoxy inférieur ayant jusqu'à 4 atomes de carbone compris, A représente un alcoylène inférieur ayant jusqu'à 4 atomes de carbone compris ou un alcoylidène inférieur ayant jusqu'à 4 atomes de carbone compris et R3 représente un carboxy ou un alcoxy inférieur-carbonyle ayant jusqu'à 5 atomes de carbone compris, leurs isomères ainsi que leurs sels, en particulier les sels pharmaceutiquement acceptables, avec la précision que lorsque A représente un méthylène ou selon les cas un éthylidène et R3 un éthoxycarbonyle, les deux radicaux R1 et R2 ne représentent pas simultanément un phényle, et la précision supplémentaire que lorsque A représente un éthylidène et R3 un carboxy, les deux radicaux R1 et R2 ne représentent pas simultanément un phényle ou un p-méthoxyphényle, caractérisé en ce qu'on part de composés de formules II ou selon les cas IIIa et IIIb, IIIc, IIIh et IIIi, IIId et IIIe, IIIk et IIIp ou selon les cas IIIb ainsi que IIIf ou V ou VI ou VIIa et VIIb ou VIII, où les variables Y1–Y6, R3' et R4 ont les

significations données dans la revendication 1 et les variables Z1 et Z2 celles données dans la revendication 2.

13. Procédé selon l'une des revendications 1–4, de préparation de composés de formule I, où l'un des radicaux R1 et R2 représente un phényle ou un phényle substitué par un halogène de numéro atomique allant jusqu'à 35 compris, un hydroxy ou un alcoxy inférieur ayant jusqu'à 4 atomes de carbone compris et l'autre représente un pyridyle ou un 1-oxydopyridyle, A représente un alcoylidène inférieur présentant un atome de carbone quaternaire et ayant jusqu'à 4 atomes de carbone compris, où l'atome de carbone quaternaire est lié directement au noyau imidazole, et R3 représente un alcoxy inférieur-carbonyle ayant jusqu'à 5 atomes de carbone compris, leurs isomères ainsi que leurs sels, en particulier les sels pharmaceutiquement acceptables, caractérisé en ce qu'on part de composés de formules II ou selon les cas IIIa et IIIb, IIIc, IIIh et IIIi, IIId et IIIe, IIIk et IIIp ou selon les cas IIIb ainsi que IIIf ou V ou VI ou VIIa et VIIb ou VIII, où les variables Y1–Y6, R3′ et R4 ont les significations données dans la revendication 1 et les variables Z1 et Z2 celles données dans la revendication 2.

14. Procédé selon l'une des revendications 1–4, de préparation de composés de formule I, où l'un des radicaux R1 et R2 représente un phényle et l'autre un 1-oxydopyridyle, A représente un 2,2-propylidène et R3 représente un alcoxy inférieur-carbonyle ayant jusqu'à 5 atomes de carbone compris, leurs isomères ainsi que leurs sels, en particulier leurs sels pharmaceutiquement acceptables, caractérisé en ce qu'on part de composés de formules II ou selon les cas IIIa et IIIb, IIIc, IIIh et IIIi, IIId et IIIe, IIIk et IIIp ou selon les cas IIIb ainsi que IIIf ou V ou VI ou VIIa et VIIb ou VIII, où les variables Y1–Y6, R3′ et R4 ont les significations données dans la revendication 1 et les variables Z1 et Z2 ont les significations données dans la revendication 2.

15. Procédé selon l'une des revendications 5 et 6 de préparation d'ester éthylique de l'acide
2-[4(5)-phényl-5(4)-(3-pyridyl)-imidazol-2-yl]-
2-méthyl-propionique
ou d'un de ses sels, caractérisé en ce qu'on part de composés de formules II, IV, V ou VI où les variables Y1–Y6, R3′ et R4 ont les significations données dans la revendication 5.

16. Procédé selon l'une des revendications 1–4 de préparation d'ester éthylique de l'acide
2-[4(5)-phényl-5(4)-(3-pyridyl)-imidazol-2-yl]-
2-méthyl-propionique
ou d'un de ses sels, caractérisé en ce qu'on part de composés de formules II ou selon les cas IIIa et IIIb, IIIc, IIIh et IIIi, IIId et IIIe, IIIk et IIIp ou selon les cas IIIb ainsi que IIIf ou V ou VI ou VIIa et VIIb ou VIII, où les variables Y1–Y6, R3′ et R4 ont les significations données dans la revendication 1 et les variables Z1 et Z2 celles données dans la revendication 2.

17. Procédé selon l'une des revendications 5 et 6 de préparation d'ester éthylique de l'acide
2-[4(5)-phényl-5(4)-(3-pyridyl)-imidazol-2-
yl]-2-allyl-acétique
ou d'un de ses sels, caractérisé en ce qu'on part de composés de formules II, IV, V ou VI où les variables Y1–Y6, R3′ et R4 ont les significations données dans la revendication 5.

18. Procédé selon l'une des revendications 1–4 de préparation d'ester éthylique de l'acide
2-[4(5)-phényl-5(4)-(3-pyridyl)-imidazol-2-
yl]-2-allyl-acétique,
caractérisé en ce qu'on part de composés de formules II ou selon les cas IIIa et IIIb, IIIc, IIIh et IIIi, IIId et IIIe, IIIk et IIIp ou selon les cas IIIb ainsi que IIIf ou V ou VI ou VIIa et VIIb ou VIII, où les variables Z1 et Z2 ont les significations données dans la revendication 2.

19. Procédé selon l'une des revendications 5 et 6 de préparation de
1-[4(5)-phényl-5(4)-(3-pyridyl)-imidazol-2-yl]-
1-carbéthoxycyclopentane,
ou d'un de ses sels, caractérisé en ce qu'on part de composés de formules II, IV, V ou VI où les variables Y1–Y6, R3′ et R4 ont les significations données dans la revendication 5.

20. Procédé selon l'une des revendications 1–4 de préparation de
1-[4(5)-phényl-5(4)-(3-pyridyl)-imidazol-2-yl]-
1-carbéthoxycyclopentane
ou d'un de ses sels, caractérisé en ce qu'on part de composés de formules II ou selon les cas IIIa et IIIb, IIIc, IIIh et IIIi, IIId et IIIe, IIIk et IIIp ou selon les cas IIIb ainsi que IIIf ou V ou VI ou VIIa et VIIb ou VIII, où les variables Y1–Y6, R3′ et R4 ont les significations données dans la revendication 1 et les variables Z1 et Z2 celles données dans la revendication 2.

21. Procédé selon l'une des revendications 5 et 6 de préparation d'ester éthylique de l'acide
2-[4(5)-(p-méthoxyphényl)-5(4)-(3-pyridyl)-
imidazol-2-yl]-2-méthylpropionique
ou d'un de ses sels, caractérisé en ce qu'on part de composés de formules II, IV, V ou VI où les variables Y1–Y6, R3′ et R4 ont les significations données dans la revendication 5.

22. Procédé selon l'une des revendications 1–4 de préparation d'ester éthylique de l'acide
2-[4(5)-(p-méthoxyphényl)-5(4)-(3-pyridyl)-
imidazol-2-yl]-2-méthyl-propionique
ou d'un de ses sels, caractérisé en ce qu'on part de composés de formules II ou selon les cas IIIa et IIIb, IIIc, IIIh et IIIi, IIId et IIIe, IIIk et IIIp ou selon les cas IIIb ainsi que IIIf ou V ou VI ou VIIa et VIIb ou VIII, où les variables Y1–Y6, R3′ et R4 ont les significations données dans la revendication 1 et les variables Z1 et Z2 celles données dans la revendication 2.

23. Procédé selon l'une des revendications 5 et 6 de préparation d'ester éthylique de l'acide
2-[4(5)-phényl-5(4)-(1-oxydo-3-pyridyl)-
imidazol-2-yl]-2-méthyl-propionique
ou d'un de ses sels, caractérisé en ce qu'on part de composés de formules II, IV, V ou VI où les variables Y1—Y6, R3′ et R4 ont les significations données dans la revendication 5.

24. Procédé selon l'une des revendications 1–4 de préparation d'ester éthylique de l'acide
2-[4(5)-phényl-5(4)-(1-oxydo-3-pyridyl)-imidazol-2-yl]-2-méthyl-propionique
ou d'un de ses sels, caractérisé en ce qu'on part de composés de formules II ou selon les cas IIIa et IIIb, IIIc, IIIh et IIIi, IIId et IIIe, IIIk et IIIp ou selon les cas IIIb ainsi que IIIf ou V ou VI ou VIIa et VIIb ou VIII, où les variables Y1—Y6, R3′ et R4 ont les significations données dans la revendication 1 et les variables Z1 et Z2 celles données dans la revendication 2.

25. Procédé selon l'une des revendications 5 et 6 de préparation d'ester éthylique de l'acide
2-[4(5)-phényl-5(4)-(3-pyridyl)-imidazol-2-yl]-propionique
ou d'un de ses sels, caractérisé en ce qu'on part de composés de formules II, IV, V ou VI où les variables Y1–Y6, R3′ et R4 ont les significations données dans la revendication 5.

26. Procédé selon l'une des revendications 1–4 de préparation d'ester éthylique de l'acide
2-[4(5)-phényl-5(4)-(3-pyridyl)-imidazol-2-yl]-propionique
ou d'un de ses sels, caractérisé en ce qu'on part de composés de formules II ou selon les cas IIIa et IIIb, IIIc, IIIh et IIIi, IIId et IIIe, IIIk et IIIp ou selon les cas IIIb ainsi que IIIf ou V ou VI ou VIIa et VIIb ou VIII, où les variables Y1–Y6, R3′ et R4 ont les significations données dans la revendication 1 et les variables Z1 et Z2 celles données dans la revendication 2.

27. Procédé selon l'une des revendications 1–4 de préparation d'ester éthylique de l'acide
2-[4(5)-p-chlorophényl)-5(4)-(3-pyridyl)-imidazol-2-yl]-2-méthyl-propionique
ou d'un de ses sels, caractérisé en ce qu'on part de composés de formules II ou selon les cas IIIa et IIIb, IIIc, IIIh et IIIi, IIId et IIIe, IIIk et IIIp ou selon les cas IIIb, ainsi que IIIf ou V ou VI ou VIIa et VIIb ou VIII, où les variables Y1–Y6, R3′ et R4 ont les significations données dans la revendication 1 et les variables Z1 et Z2 celles données dans la revendication 2.

28. Procédé selon l'une des revendications 1–4 de préparation d'ester éthylique de l'acide
2-[4(5)-phényl-5(4)-(4-pyridyl)-imidazol-2-yl]-2-méthyl-propionique
ou d'un de ses sels, caractérisé en ce qu'on part de composés de formules II ou selon les cas IIIa et IIIb, IIIc, IIIh et IIIi, IIId et IIIe, IIIk et IIIp ou selon les cas IIIb ainsi que IIIf ou V ou VI ou VIIa et VIIb ou VIII, où les variables Y1–Y6, R3′ et R4 ont les significations données dans la revendication 1 et les variables Z1 et Z2 celles données dans la revendication 2.

29. Procédé selon l'une des revendications 5 et 6 de préparation d'ester éthylique de l'acide
2-[4(5)-phényl-5(4)-(1-oxydo-4-pyridyl)-imidazol-2-yl]-2-méthyl-propionique
ou d'un de ses sels, caractérisé en ce qu'on part de composés de formules II, IV, V ou VI où les variables Y1–Y6, R3′ et R4 ont les significations données dans la revendication 5.

30. Procédé selon l'une des revendications 1–4 de préparation d'ester éthylique de l'acide
2-[4(5)-(p-hydroxyphényl)-5(4)-(3-pyridyl)-imidazol-2-yl]-2-méthyl-propionique
ou d'un de ses sels, caractérisé en ce qu'on part de composés de formules II ou selon les cas IIIa et IIIb, IIIc, IIIh et IIIi, IIId et IIIe, IIIk et IIIp ou selon les cas IIIb ainsi que IIIf ou V ou VI ou VIIa et VIIb ou VIII, où les variables Y1–Y6, R3′ et R4 ont les significations données dans la revendication 1 et les variables Z1 et Z2 celles données dans la revendication 2.

31. Procédé de préparation de préparations pharmaceutiques, caractérisé en ce qu'on mélange un composé obtenu selon l'une des revendications 5, 6, 9, 15, 17, 19, 21, 23 ou 25, sous forme libre ou sous la forme d'un sel pharmaceutiquement acceptable, avec des additifs et supports habituels, pharmaceutiques habituels.

32. Procédé de préparation de préparations pharmaceutiques, caractérisé en ce qu'on mélange un composé obtenu selon l'une des revendications 1–4, 7, 8, 10–14, 15, 18, 20, 22, 24 ou 26–30, sous forme libre ou sous la forme d'un sel pharmaceutiquement acceptable, avec des additifs et supports pharmaceutiques habituels.

33. Procédé de préparation de composés de formule

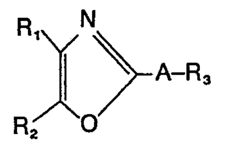 (IIIf) ,

où l'un des radicaux R1 et R2 représente phényle, pyrrolyle, pyridyle, 1-oxydopyridyle ou pyrimidyle, qui peuvent être chacun non substitué ou substitué par halogène, alcoyle inférieur, hydroxy, alcoxy inférieur et/ou alcanoyloxy inférieur, et l'autre représente pyrrolyle, pyridyle, 1-oxydopyridyle ou pyrimidyle, qui peuvent être chacun non substitués ou substitués par halogène, alcoyle inférieur, hydroxy, alcoxy inférieur et/ou alcanoyloxy inférieur et A représente alcoylène inférieur, alcoylidène inférieur, alcénylène inférieur, alcénylidène inférieur, cycloalcoylène, cycloalcoylidène ou cycloalcoyl-alcoylidène inférieur, et R3 représente carboxy, carboxy estérifié par un alcanol inférieur, un cycloalcanol à 3 à 8 chaînons, un phénol, une hydroxypyridine, un phénol substitué ou selon les cas une hydroxypyridine, un carbamoyle ou un carbamoyle monosubstitué par hydroxy, amino, phényle ou phényle substitué, mono- ou disubstitué par un alcoyle inférieur ou disubstitué par un alcoylène à 4 à 7 chaînons ou selon les cas un 3-aza-, 3-alcoyle inférieur-aza-, 3-oxa- ou 3-thiaalcoylène, où un alcanol inférieur ou selon les cas un cycloalcanol peut être non substitué ou substitué par hydroxy, mercapto, phényle éventuellement substitué, alcoxy inférieur, phénylalcoxy inférieur éventuellement substitué dans la partie phényle, alcoyle inférieur-thio, phénylalcoyle inférieur-thio éventuellement substitué dans la partie phényle, hydroxyalcoxy inférieur, alcoxy inférieur-alcoxy inférieur, phénylalcoxy inférieur-alcoxy inférieur éventuellement substitué dans la partie phényle, carboxyalcoxy inférieur, alcoxy inférieur-carbonyl-alcoxy inférieur, alcoxy inférieur-carbonyl-alcoxy inférieur présentant éventuellement un phényle substitué ou alcanoyloxy inférieur et phényle substitué, phénol ou selon les cas hydroxypyridine à chaque fois substitué par alcoyle inférieur, alcoxy inférieur, halogène et/ou trifluorométhyle, leurs isomères ainsi que leurs sels, caractérisé en ce qu'on fait réagir des composés de formule

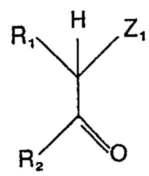

(IIId),

où Z1 représente un hydroxy estérifié éventuellement réactif, avec des acides carboxyliques de formule R3–A–COOH (IIIp), leurs sels ou leurs dérivés fonctionnels et avec de l'ammoniac ou en ce qu'on fait réagir des composés de formule

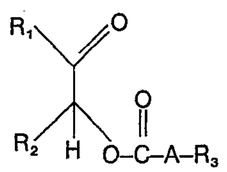

(IIIq),

que l'on peut obtenir par réaction de composés de formule

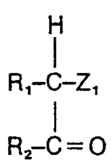

(IIId)

avec des dérivés éventuellement fonctionnels de composés de formule

$R_3$–A–COOH (IIIp)

avec de l'ammoniac, si on le désire en ce qu'on N-oxyde dans un composé de formule IIIf ainsi obtenu les radicaux hétéroaryle non N-oxydés R1 et R2 à l'aide d'un oxydant, et si on le désire en ce qu'on transforme le composé libre que l'on peut obtenir selon le procédé en un sel ou un sel que l'on peut obtenir selon le procédé en le composé libre ou en un autre sel ou un composé libre que l'on peut obtenir selon le procédé en un autre composé libre et/ou, si on le désire, en ce qu'on sépare un mélange d'isomères de de composés isomères de formule I que l'on peut obtenir selon l'invention en les isomères isolés.

34. Procédé selon la revendication 33, caractérisé en ce qu'on part d'une étape quelconque du procédé et en ce qu'on effectue les étapes manquantes ou en ce qu'on utilise un produit de départ sous la forme d'un sel et/ou de tautomères et/ou en ce qu'on le forme dans les conditions de la réaction.

35. Procédé selon les revendications 33 et 34 de préparation de composés de formule IIIf, où l'un des radicaux R1 et R2 représente un pyridyle ou un 1-oxydo-pyridyle, qui peuvent être non substitués et/ou chacun substitué par halogène, hydroxy, alcoyle inférieur, alcoxy inférieur et/ou alcanoyloxy inférieur, et l'autre représente un phényle, un pyridyle ou un 1-oxydopyridyle, qui peuvent être non substitués et/ou chacun substitué par halogène, hydroxy, alcoyle inférieur, alcoxy inférieur et/ou alcanoyloxy inférieur, A représente un alcoylène inférieur ayant jusqu'à 4 atomes de carbone compris, un alcoylidène inférieur ayant jusqu'à 7 atomes de carbone compris, un alcénylène inférieur ayant jusqu'à 4 atomes de carbone compris, un alcénylidène inférieur ayant jusqu'à 7 atomes de carbone compris, un cycloalcoylène à 3 à 8 chaînons, un cycloalcoylidène à 3 à 8 chaînons ou un cycloalcoyl-alcoylidène inférieur ayant jusqu'à 7 atomes de carbone compris dans la partie alcoylidène et avec une partie cycloalcoyle à 3 à 8 chaînons et R3 représente un carboxy, un carboxy estérifié par un alcanol inférieur, un cycloalcanol à 3 à 8 chaînons, un phénol ou un phénol substitué, un carbamoyle, un pyrrolidino-, pipéridino-, morpholino-, pipérazino-, 4-alcoyle inférieur-pipérazino-, thio-morpholino-, anilino- ou anilino-carbonyle substitué par alcoyle inférieur, alcoxy inférieur et/ou halogène, où l'alcanol inférieur ou selon les cas cycloalcanol est non substitué ou peut être substitué par hydroxy, mercapto, phényle, phényle substitué, alcoxy inférieur, phénylalcoxy inférieur, phénylalcoxy inférieur substitué dans la fraction phényle, alcoyle inférieur-thio, phénylalcoyle inférieur-thio, phénylalcoyle inférieur-thio substitué dans la fraction phényle, hydroxyalcoxy inférieur, alcoxy inférieur-alcoxy inférieur, phénylalcoxy inférieur-alcoxy inférieur, phénylalcoxy inférieur-alcoxy inférieur substitué dans la fraction phényle, carboxyalcoxy inférieur, alcoxy inférieur-carbonyl-alcoxy inférieur, alcoxy inférieur-carbonyl-alcoxy inférieur présentant éventuellement un phényle substitué, ou alcanoyloxy inférieur et phénol substitué ou selon les cas phényle à chaque fois substitué par alcoyle inférieur, alcoxy inférieur, halogène et/ou trifluorométhyle, leurs isomères ainsi que leurs sels.

36. Procédé selon les revendications 33 et 34 de préparation de composés de formule IIIf, où l'un des radicaux R1 et R2 représente un phényle ou un phényle substitué par un halogène dont le numéro atomique va jusqu'à 35 compris, un hydroxy, un alcoyle inférieur ayant jusqu'à 4 atomes de carbone compris et/ou un alcoxy inférieur ayant jusqu'à 4 atomes de carbone compris, et l'autre représente un pyridyle ou un 1-oxydo-pyridyle, qui à chaque fois peuvent être non substitués ou substitués par un halogène dont le numéro atomique va jusqu'à 35 compris, un hydroxy et/ou un alcoxy inférieur ayant jusqu'à 4 atomes de carbone compris, A représente un alcoylène inférieur ayant jusqu'à 4 atomes de carbone compris, un alcoylidène inférieur ayant jusqu'à 7 atomes de carbone compris, ou un cycloalcoylidène inférieur à 3 à 7

chaînons et R3 représente un carboxy, un alcoxy inférieur-carbonyle ayant jusqu'à 5 atomes de carbone compris, un carbamoyle, un N-monoalcoyle inférieur-carbamoyle ayant jusqu'à 4 atomes de carbone compris dans la partie alcoyle inférieur ou un N,N-dialcoyle inférieur-carbamoyle ayant à chaque fois jusqu'à 4 atomes de carbone compris dans la partie alcoyle inférieur, où l'alcoxy inférieur-carbonyle peut être substitué par un alcanoyloxy inférieur ayant jusqu'à 5 atomes de carbone compris, leurs isomères ainsi que leurs sels.

37. Procédé selon les revendications 33 et 34 de préparation de composés de formule IIIf, où l'un des radicaux R1 et R2 représente un phényle ou un phényle substitué par un halogène de numéro atomique allant jusqu'à 35 compris, un hydroxy ou un alcoxy inférieur ayant jusqu'à 4 atomes de carbone compris et l'autre représente un pyridyle ou un 1-oxydopyridyle, A représente un alcoylidène inférieur présentant un atome de carbone quaternaire et ayant jusqu'à 4 atomes de carbone compris, où l'atome de carbone quaternaire C est lié directement au noyau imidazole, et R3 représente un alcoxy inférieur-carbonyle ayant jusqu'à 5 atomes de carbone compris, leurs isomères ainsi que leurs sels.

38. Procédé selon les revendications 33 et 34 de préparation de l'ester éthylique de l'acide
2-[4-phényl-5-(3-pyridyl)-oxazol-2-yl]-acétique
ou d'un de ses sels.

39. Procédé selon les revendications 33 et 34 de préparation de l'ester éthylique de l'acide
2-[4-phényl-5-(1-oxydo-3-pyridyl)-oxazol-2-yl]-2-méthyl-propionique
ou d'un de ses sels.